# EUROPÄISCHE PATENTANMELDUNG

(11) **EP 2 070 909 A1**
(43) Veröffentlichungstag der Anmeldung: **17.06.2009**
(21) Anmeldenummer: 07076093.9
(22) Anmeldetag: 15.12.2007
(51) Int. Cl.: C07C 255/58, C07D 211/34, C07D 265/02, C07D 309/14, C07D 335/02, A61K 31/167, A61K 31/536, A61K 31/351, A61K 31/435, A61K 31/382, A61P 5/24, A61P 15/08, A61P 35/00

(54) **Nichtsteroidale Progesteronrezeptor-Modulatoren**

(71) Anmelder: Bayer Schering Pharma AG, 13353 Berlin (DE)
(72) Erfinder: Kirkland, Thomas, Atascadero, CA 93422 (US); Schwede, Wolfgang, 16548 Glienicke (DE); Möller, Carsten, 10115 Berlin (DE); Bäurle, Stefan, 10245 Berlin (DE); Wyrwa, Ralf, 07751 Rothenstein (DE); Rotgeri, Andrea, 13503 Berlin (DE)

(57) **Zusammenfassung**

Die vorliegende Erfindung betrifft nichtsteroidale Progesteronrezeptor-Modulatoren der allgemeinen Formel I, 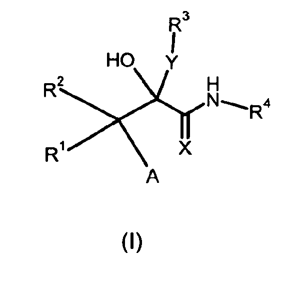 die Verwendung der Progesteronrezeptor-Modulatoren zur Herstellung von Arzneimitteln sowie pharmazeutische Zusammensetzungen, die diese Verbindungen enthalten.

Die erfindungsgemäßen Verbindungen sind geeignet zur Therapie und Prophylaxe von gynäkologischen Erkrankungen wie Endometriose, Leiomyomen des Uterus, dysfunktionelle Blutungen und Dysmenorrhoe sowie für die Therapie und Prophylaxe von hormonabhängigen Tumoren und zur Verwendung für die weibliche Fertilitätskontrolle sowie für die Hormonersatztherapie.

## Beschreibung

Die vorliegende Erfindung betrifft nichtsteroidale Progesteronrezeptor-Modulatoren, ein Verfahren zu deren Herstellung, die Verwendung der Progesteronrezeptor-Modulatoren zur Herstellung von Arzneimitteln sowie pharmazeutische Zusammensetzungen, die diese Verbindungen enthalten.

Das Steroidhormon Progesteron reguliert in entscheidender Weise das Fortpflanzungsgeschehen im weiblichen Organismus. Progesteron wird während des Zyklus und in der Gravidität in großen Mengen vom Ovar bzw. der Plazenta sezerniert. Im Zusammenwirken mit Estrogenen bewirkt Progesteron zyklische Veränderungen der Uterusschleimhaut (Endometrium) im Menstruationszyklus. Unter dem Einfluss erhöhter Progesteronspiegel nach der Ovulation wird die Uterusschleimhaut in einen Zustand überführt, der die Einnistung eines Embryos (Blastozyste) zulässt. In der Gravidität kontrolliert Progesteron die Ruhigstellung des Myometriums und erhält die Funktion des dezidualen Gewebes.

Es ist weiterhin bekannt, dass Progesteron durch die Unterdrückung der estrogenvermittelten Mitose im Uterusgewebe die endometriale Proliferation hemmt (K. Chwalisz, R.M. Brenner, U. Fuhrmann, H. Hess-Stumpp, W. Elger, Steroids 65, 2000, 741-751).

Eine bedeutende Rolle des Progesterons und der Progesteronrezeptoren ist auch in pathophysiologischen Prozessen bekannt. Progesteronrezeptoren sind in Endometrioseherden, aber auch in Tumoren des Uterus, der Mamma und des ZNS nachgewiesen. Weiterhin ist bekannt, dass Leiomyome des Uterus Progesteronabhängig wachsen.

Die Wirkungen von Progesteron in den Geweben der Genitalorgane und in anderen Geweben erfolgen durch Interaktionen mit Progesteronrezeptoren, die für die zellulären Effekte verantwortlich sind.

Progesteronrezeptor-Modulatoren sind entweder reine Agonisten oder hemmen die Wirkung von Progesteron teilweise oder vollständig. Dementsprechend werden Substanzen als reine Agonisten, Partialagonisten (**S**elektive **P**rogesteron**r**ezeptor**m**odulatoren = SPRM) und reine Antagonisten definiert.

Entsprechend der Fähigkeit von Progesteronrezeptor-Modulatoren, ihre Wirkung über den Progesteronrezeptor zu entfalten, besitzen diese Verbindungen ein beträchtliches Potential als Therapeutika für gynäkologische und onkologische Indikationen sowie für die Geburtshilfe und die Fertilitätskontrolle.

Reine Progesteronrezeptor-Antagonisten hemmen die Wirkung von Progesteron am Progesteronrezeptor komplett. Sie haben antiovulatorische Eigenschaften sowie die Fähigkeit, Estrogeneffekte im Endometrium bis zur völligen Atrophie zu hemmen. Sie sind daher besonders geeignet, in den Reproduktionsprozess der Frau einzugreifen, z. B. postovulatorisch, um die Nidation einer befruchteten Eizelle zu verhindern, in der Gravidität, um die Reaktionsbereitschaft des Uterus für Prostaglandine oder Oxytocin zu erhöhen oder um eine Eröffnung und Erweichung ("Reifung") der Cervix zu erreichen sowie um eine hohe Wehenbereitschaft des Myometriums auszulösen.
In Endometrioseherden bzw. in Tumorgeweben, welche mit Progesteronrezeptoren ausgestattet sind, erwartet man nach Applikation von reinen Progesteronrezeptor-Antagonisten eine günstige Beeinflussung des Krankheitsgeschehens. Besondere Vorteile für die Beeinflussung von Krankheitszuständen wie Endometriose oder Leiomyome des Uterus könnten dann gegeben sein, wenn durch die Progesteronrezeptor-Antagonisten zusätzlich eine Hemmung der Ovulation erreicht werden kann. Mit der Hemmung der Ovulation entfällt auch ein Teil der ovariellen Hormonproduktion und damit der auf diesen Anteil entfallenden Stimulationseffekt auf das pathologisch veränderte Gewebe.

Dem ersten beschriebenen Progesteronrezeptor-Antagonisten, RU 486 (auch Mifepristone) folgte die Synthese und Charakterisierung einer großen Zahl Analoga mit variierender Stärke der Progesteronrezeptor-antagonistischen Aktivität. Während RU 486 neben der Progesteronrezeptor-antagonistischen Wirkung auch eine antiglukokortikoide Wirkung zeigt, zeichnen sich später synthetisierte Verbindungen vor allem durch eine selektivere Wirkung als Progesteronrezeptor-Antagonisten aus.

Aus der Literatur sind neben steroidalen Verbindungen wie Onapriston oder Lilopriston, die sich gegenüber RU 486 durch eine bessere Wirkungsdissoziation von Progesteronrezeptor-antagonistischer zu antiglukokortikoider Wirkung auszeichnen, auch verschiedene nicht-steroidale Strukturen bekannt, deren antagonistische Wirkung am Progesteronrezeptor untersucht wird [siehe z.B. S. A. Leonhardt und D. P. Edwards, Exp. Biol. Med. 227: 969-980 (2002) und R. Winneker, A. Fensome, J. E. Wrobel, Z. Zhang, P. Zhang, Seminars in Reproductive Medicine, Volume 23: 46-57 (2005)]. Bisher bekannte nicht-steroidale Verbindungen besitzen jedoch nur mäßige antagonistische Aktivität verglichen mit der Aktivität bekannter steroidaler Strukturen. Die wirksamsten nicht-steroidalen Verbindungen werden mit *in vitro* Aktivitäten von 10 % der Aktivität von RU 486 beschrieben.

Die antiglukokortikoide Aktivität ist nachteilig für eine therapeutische Anwendung, bei der die Hemmung der Progesteronrezeptoren im Vordergrund der Therapie steht. Eine antiglukokortikoide Wirksamkeit verursacht unerwünschte Nebenwirkungen bei den therapeutisch erforderlichen Dosierungen. Dies kann die Applikation einer therapeutisch sinnvollen Dosis verhindern oder zum Abbruch der Behandlung führen.
Die teilweise oder vollständige Reduktion der antiglukokortikoiden Eigenschaften ist deshalb eine wichtige Voraussetzung für die Therapie mit Progesteronrezeptor-Antagonisten, insbesondere für diejenigen Indikationen, die eine über Wochen oder Monate andauernde Behandlung erfordern.

Im Gegensatz zu den reinen Antagonisten zeigen Progesteronrezeptor-Partialagonisten (SPRMs) eine residuelle agonistische Eigenschaft, welche unterschiedlich stark ausgeprägt sein kann. Dies führt dazu, dass diese Substanzen in bestimmten Organsystemen agonistische Wirkungen am Progesteronrezeptor zeigen (D. DeManno, W. Elger, R. Garg, R. Lee, b. Schneider, H. Hess-Stumpp, G. Schuber, K. Chwalisz, Steroids 68, 2003, 1019-1032). Eine solche organspezifische und dissoziierte Wirkung kann für die beschriebenen Indikationen von therapeutischem Nutzen sein.

Aufgabe vorliegender Erfindung ist es daher, weitere nichtsteroidale Progesteronrezeptor-Modulatoren zur Verfügung zu stellen. Diese Verbindungen sollen eine reduzierte antiglukokortikoide Wirkung besitzen und daher geeignet sein für die Therapie und Prophylaxe von gynäkologischen Erkrankungen wie Endometriose, Leiomyomen des Uterus, dysfunktionelle Blutungen und der Dysmenorrhoe. Außerdem sollen die erfindungsgemäßen Verbindungen geeignet sein für die Therapie und Prophylaxe von hormonabhängigen Tumoren, beispielsweise von Mamma-, Endometriums-, Ovar- sowie Prostatakarzinomen. Die Verbindungen sollen weiterhin geeignet sein für die Verwendung in der weiblichen Fertilitätskontrolle als auch für die weibliche Hormonersatztherapie.

Die Aufgabe wird gemäß vorliegender Erfindung durch die Bereitstellung nichtsteroidaler Verbindungen der allgemeinen Formel I gelöst 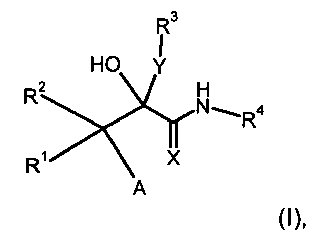 worin
- A: ein Wasserstoff oder ein gegebenenfalls ein- oder mehrfach, gleich oder verschieden mit Z substituierter C₁-C₈-Alkyl-, C₂-C₈-Alkenyl-, C₂-C₈-Alkinylrest ist oder aber direkt für Z steht, wobei Z folgende Bedeutung besitzt: Cyano, Halogen, Hydroxy, Nitro, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d}, -C=NOR^{b}, -NR^{c}R^{d}, -PO₃(R^{b})_{2,} -NR^{e}COR^{b}, -NR^{e}CSR^{b}, -NR^{e}S(O)R^{b}, -NR^{e}S(O)₂R^{b}, -NR^{e}CONR^{c}R^{d}, -NR^{e}COOR^{b}, -NR^{e}C(NH)NR^{c}R^{d}, -NR^{e}CSNR^{c}R^{d}, -NR^{e}S(O)NR^{c}R^{d}, -NR^{e}S(O)₂NR^{c}R^{d}, -S(O)R^{b}, -S(O)NR^{c}R^{d}, -S(O)₂R^{b}, -SO₂OR^{b}, -CSNR^{c}R^{d}, -CR^{b}(OH)-R^{b} mit
- R^{b}: ein Wasserstoff, ein C₁-C₆-Alkyl-, Hydroxy-C₁-C₃-alkyl-, C₁-C₃₋Alkoxy-C₁-C₃-alkyl-, C₂-C₈-Alkenyl-, C₂-C₈-Alkinyl-, C₃-C₁₀-Cycloalkyl-, C₆-C₁₂-Aryl- oder ein teilweise oder vollständig fluorierter C₁-C₃-Fluoralkylrest und
- R^{c} und R^{d}: unabhängig voneinander ein Wasserstoff, ein gegebenenfalls mit W substituiertes C₁-C₆-Alkyl-, C₂-C₈Alkenyl-, C₂-C₈-Alkinyl-, C₃-C₁₀-Cycloalkyl-, C₆-C₁₂-Aryl-, ein 5- bis 12-gliedriger Heteroarylrest, eine Gruppe C(O)R^{b} mit der weiter oben angegebenen Bedeutung für R^{b} oder eine Hydroxygruppe bedeuten oder gemeinsam unter Einschluss des Stickstoffs einen 3- bis 7-gliedrigen
- R^{f}: Ring bilden, der gegebenenfalls um O, S oder NR^{f} erweitert ist, wobei Wasserstoff, C₁-C₃-Alkyl, C₁-C₃-Alkanoyl, C₁-C₃-
- W: Alkylsulfonyl oder C₁-C₃-Alkoxycarbonyl ist, und -NR^{g}R^{h} mit
- R^{g}: Wasserstoff oder C₁-C₃-Alkyl und
- R^{h}: Wasserstoff oder C₁-C₃-Alkyl oder
- R^{g} und R^{h}: gemeinsam unter Einschluss des Stickstoffs einen 3- bis 7-gliedrigen Ring bilden, der gegebenenfalls um O, S oder NR^{f} erweitert ist, wobei
- R^{f}: Wasserstoff, C₁-C₃-Alkyl, C₁-C₃-Alkanoyl, C₁-C₃-Alkylsulfonyl oder C₁-C₃-Alkoxycarbonyl ist,
- und wobei, wenn:
- R^{c}: eine Hydroxygruppe ist, R^{d} nur ein Wasserstoff, ein gegebenenfalls mit W substituiertes C₁-C₆-Alkyl, C₂-C₈-Alkenyl, C₂-C₈-Alkinyl, C₃-C₁₀-Cycloalkyl oder C₆-C₁₂-Aryl sein kann und umgekehrt sowie weiterhin
- R^{e}: ein Wasserstoff, ein gegebenenfalls mit W substituiertes C₁-C₆-Alkyl, C₂-C₈-Alkenyl, C₂-C₈-Alkinyl, C₃-C₁₀-Cyclo-alkyl oder C₆-C₁₂-Aryl bedeutet
- oder:
- A: ein gegebenenfalls ein- oder mehrfach, gleich oder verschieden mit M substituierter C₃-C₁₀-Cycloalkyl- oder 3-12-gliedriger Heterocycloalkylrest bedeutet und
- M: ein C₁-C₆-Alkyl oder eine Gruppe -COR^{b}, CO₂R^{b},O-R^{b}, oder -NR^{c}R^{d} ist, wobei R^{b}, R^{c} und R^{d} die weiter oben angegebenen Bedeutungen haben und
- R¹ und R²: unabhängig voneinander eine unverzweigte oder verzweigte, gegebenenfalls mit Z substituierte C₁-C₅-Alkylgruppe oder gemeinsam mit dem C-Atom der Kette einen gegebenenfalls mit Z substituierten carbocyclischen oder heterocyclischen Ring mit insgesamt 3-7 Gliedern bildend, wobei, wenn
- A: ein Wasserstoff ist und R¹ ein Methylrest ist, R² nicht ein Methylrest oder ein Ethylrest sein können,
- A: ein Wasserstoff ist R¹ und R² nicht gemeinsam einen Ring mit 3-4 Gliedern sein können
- A: ein Methylrest ist R¹ und R² nicht gleichzeitig ein Methylrest sein oder gemeinsam mit dem C-Atom der Kette einen Cyclopropylring bilden können,
- R³: ein Wasserstoff oder ein gegebenenfalls ein- oder mehrfach, gleich oder verschieden mit K substituierter C₁-C₈-Alkyl-, C₂-C₈-Alkenyl-, C₂-C₈-Alkinyl-, C₃-C₁₀-Cycloalkyl-, 3-12-gliedriger Heterocycloalkylrest oder ein gegebenenfalls ein- oder mehrfach, gleich oder verschieden mit L substituierter Aryl- oder 3-12-gliedriger Heteroarylrest bedeutet,
- und:
- K: ein Cyano, Halogen, Hydroxy, Nitro, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d}, -C=NOR^{b} -NR^{c}R^{d} oder ein gegebenenfalls ein- oder mehrfach, gleich oder verschieden mit M substituierter C₃-C₁₀-Cycloalkyl-, 3-12-gliedriger Heterocycloalkylrest oder ein gegebenenfalls ein- oder mehrfach mit L substituierter Aryl- oder 3-12-gliedriger Heteroarylrest ist, mit der für M unter A angegebenen Bedeutung
- und:
- L: C₁-C₈-Alkyl, C₂-C₈-Alkenyl, C₂-C₈-Alkinyl, ein teilweise oder vollständig fluoriertes C₁-C₆-Fluoralkyl, ein teilweise oder vollständig fluoriertes C₁-C₆-Fluoralkoxy, C₁-C₆-Alkoxy-C₁-C₆-alkyl, C₁-C₆-Alkoxy-C₁-C₆-alkoxy, ein mono- oder bicyclischer (CH₂)ₚ-C₃-C₁₀-Cycloalkyl, ein mono- oder bicyclischer 3-12-gliedriger (CH₂)ₚ-Heterocycloalkylrest, (CH₂)ₚCN, (CH₂)ₚHal, (CH₂)ₚNO₂, ein mono- oder bicyclischer gegebenenfalls mit V substituierter (CH₂)ₚ-C₆-C₁₂-Arylrest, ein mono- oder bicyclischer gegebenenfalls mit V substituierter 3-12-gliedriger (CH₂)ₚ-Heteroarylrest, oder -(CH₂)ₚPO₃(R^{b})₂, -(CH₂)ₚNR^{c}R^{d}, -(CH₂)ₚNR^{e}COR^{b}, -(CH₂)ₚNR^{e}CSR^{b}, -(CH₂)ₚNR^{e}S(O)R^{b}, -(CH₂)ₚNR^{e}S(O)₂R^{b}, -(CH₂)ₚNR^{e}CONR^{c}R^{d}, -(CH₂)ₚNR^{e}COOR^{b}, -(CH₂)ₚNR^{e}C(NH)NR^{c}R^{d}, -(CH₂)ₚNR^{e}CSNR^{c}R^{d}, -(CH₂)ₚNR^{e}S(O)NR^{c}R^{d}, -(CH₂)ₚNR^{e}S(O)₂NR^{c}R^{d}, -(CH₂)ₚCOR^{b}, -(CH₂)ₚCSR^{b}, -(CH₂)ₚ S(O)R^{b}, -(CH₂)ₚS(O)(NH)R^{b}, -(CH₂)ₚS(O)₂R^{b}, -(CH₂)ₚS(O)₂NR^{c}R^{d}, -(CH₂)ₚSO₂OR^{b}, -(CH₂)ₚCO₂R^{b}, -(CH₂)ₚCONR^{c}R^{d}, -(CH₂)ₚCSNR^{c}R^{d}, -(CH₂)ₚOR^{b}, -(CH₂)ₚSR^{b}, -(CH₂)ₚCR^{b}(OH)-R^{b}, -(CH₂)ₚ-C=NOR^{b}, -O-(CH₂)ₙ-O-, -O-(CH₂)ₙ-CH₂-, -O-CH=CH- oder -(CH₂)ₙ₊₂- ist und die end-ständigen Sauerstoffatome und/oder Kohlenstoffatome mit direkt benachbarten Ringkohlenstoffatomen verknüpft sind und wobei
- n: 1 oder 2 und
- p: eine Zahl 0, 1, 2, 3, 4, 5 oder 6 ist, sowie
- V: ein Cyano, Halogen, Nitro, -(CH₂)ₚOR^{b}, -(CH₂)ₚS(O)₂R^{b}, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d},-C=NOR^{b} -NR^{c}R^{d}, ein teilweise oder vollständig fluoriertes C₁-C₆-Fluoralkyl oder ein teilweise oder vollständig fluoriertes C₁-C₆-Fluoralkoxy,
- X: ein Sauerstoff- oder zwei Wasserstoffatome
- Y: für (CH₂)ₘ, -C=C- oder -CH=CH- mit m = 0 oder 1 steht, und
- R⁴: ein gegebenenfalls gleich oder verschieden mit 1 bis 3 der unter L genannten Reste substituierter aromatischer oder heteroaromatischer 3 bis 12-gliedriger Mono- oder Bicyclus, oder für eine der folgenden unter B oder C genannten Gruppen steht:

B: 6-Ring/6-Ring-Systeme:

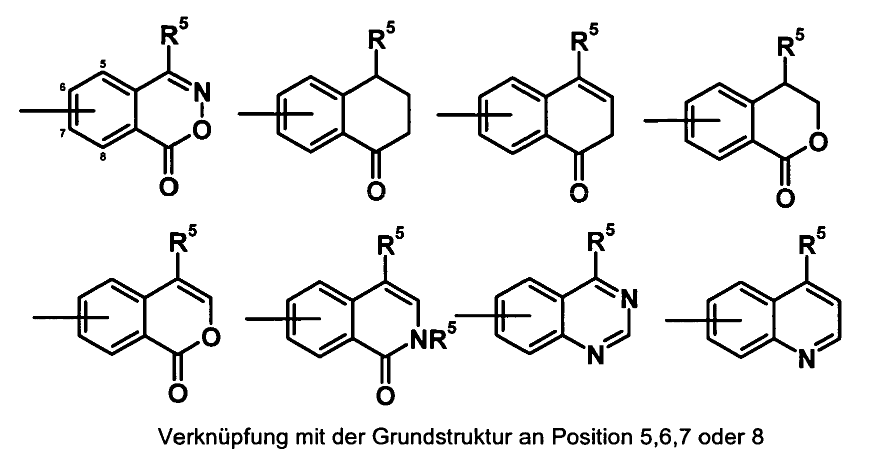

C: 6-Ring/5-Ring-Systeme: 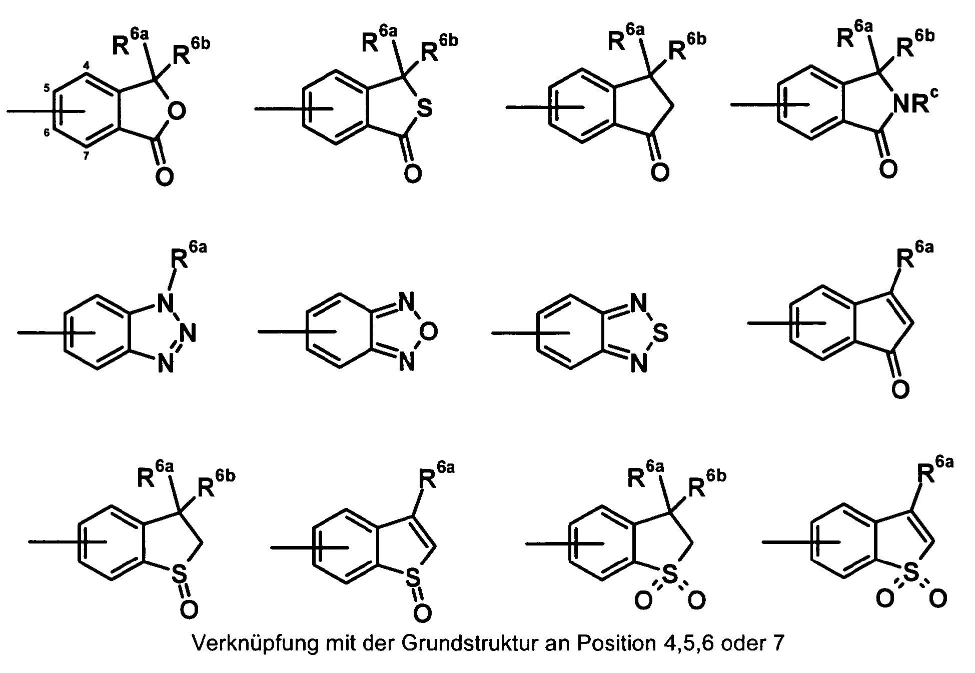 wobei
- R⁵: Wasserstoff oder C₁-C₄ Alkyl, oder ein teilweise oder vollständig fluoriertes C₁-C₄ Fluoralkyl,
R^{6a} und R^{6b} unabhängig voneinander Wasserstoff, C₁-C₄ Alkyl oder ein teilweise oder vollständig fluoriertes C₁-C₄-Fluoralkyl sind oder gemeinsam mit dem Ringkohlenstoffatom einen 3-bis 6-gliedrigen Ring bilden,
sowie ihre pharmazeutisch annehmbaren Salze.

Die erfindungsgemäßen Verbindungen der allgemeinen Formel I können durch das Vorhandensein von Asymmetriezentren als unterschiedliche Stereoisomere vorliegen. Sowohl die Racemate als auch die getrennt vorliegenden Stereoisomere gehören zum Gegenstand der vorliegenden Erfindung.

Weiterhin umfasst die vorliegende Erfindung die neuen Verbindungen als pharma-zeutische Wirkstoffe, ihre therapeutische Anwendung und pharmazeutische Darreichungsformen, die die neuen Substanzen enthalten.

Die erfindungsgemäßen Verbindungen der allgemeinen Formel (I) oder deren pharma-zeutisch annehmbare Salze können für die Herstellung eines Arzneimittels, insbesondere zur Behandlung und Prophylaxe von gynäkologischen Erkrankungen wie Endometriose, Leiomyomen des Uterus, dysfunktionelle Blutungen und der Dysmenorrhoe verwendet werden. Weiterhin können die erfindungsgemäßen Verbindungen für die Behandlung und Prophylaxe von hormonabhängigen Tumoren wie beispielsweise für Mamma-, Prostata- und Endometriumskarzinom verwendet werden.

Die erfindungsgemäßen Verbindungen der allgemeinen Formel (I) oder deren pharmazeutisch annehmbare Salze sind ferner geeignet zur Verwendung für die weibliche Fertilitätskontrolle oder für die weibliche Hormonersatztherapie.

Die erfindungsgemäßen nichtsteroidalen Verbindungen der allgemeinen Formel I wirken stark antagonistisch oder partialagonistisch bei hoher Wirkstärke am Progesteronrezeptor. Sie weisen eine starke Wirkungsdissoziation hinsichtlich ihrer Bindungsstärke am Progesteron- und am Glukokortikoidrezeptor auf. Während bekannte Progesteronrezeptor-Antagonisten wie Mifepristone (RU 486) neben der erwünschten hohen Bindungsaffinität zum Progesteronrezeptor gleichfalls eine hohe

Affinität zum Glukokortikoidrezeptor zeigen, zeichnen sich die erfindungsgemäßen Verbindungen durch eine sehr geringe Glukokortikoidrezeptorbindung bei gleichzeitig vorhandener hoher Progesteronrezeptoraffinität aus.

Die als Gruppen definierten Substituenten der erfindungsgemäßen Verbindungen der allgemeinen Formel I können jeweils die nachfolgenden Bedeutungen haben:
Unter C₁-C₄-, C₁-C₅-, C₁-C₆- bzw. C₁-C₈-Alkylgruppe werden unverzweigte oder gegebenenfalls verzweigte Alkylreste verstanden. Dabei handelt es sich beispielsweise um eine Methyl-, Ethyl-, n-Propyl-, iso-Propyl-, n-, iso-, tert.-Butyl-, eine n-Pentyl-, 2,2-Dimethylpropyl- 3-Methylbutyl-, Hexyl-, Heptyl oder Octylgruppe.
   Im Sinne von R¹, R² und R³, sind dabei die Methyl-, Ethyl-, n-Propyl- oder n-Butylgruppe sowie eine n-Pentylgruppe bevorzugt.
   Erfindungsgemäß wird für R⁵ Methyl oder Ethyl sowie für R^{6a} und R^{6b} ein Wasserstoff bevorzugt.
Unter Alkenyl werden unverzweigte oder gegebenenfalls verzweigte Alkenylreste verstanden. Im Sinne der Erfindung werden unter C₂-C₈-Alkenylgruppe beispielsweise folgende verstanden: Vinyl, Allyl, 3-Buten-1-yl- oder 2,3-Dimethyl-2-propenyl. Ist der Aromat in R³ mit einem C₂-C₈-Alkenylrest substituiert, handelt es sich vorzugsweise um eine Vinylgruppe.
Unter Alkinyl werden unverzweigte oder gegebenenfalls verzweigte Alkinylreste verstanden. Für einen C₂-C₈-Alkinylrest soll beispielsweise eine Ethinyl-, Propinyl-, Butinyl-, Pentinyl-, Hexinyl- sowie Octinylgruppe, vorzugsweise jedoch eine Ethinyloder Propinylgruppe stehen.
Für C₁-C₆-Alkoxyl-C₁-C₆-alkoxygruppe kann beispielsweise Methoxymethoxy, Ethoxymethoxy oder 2-Methoxyethoxy stehen.
Bei einem Rest OR^{b} handelt es sich im Sinne der Erfindung um eine Hydroxy-, Methoxy-, Ethoxy-, n-Propoxy-, iso-Propoxy-, n-, iso-, tert.-Butoxy - oder n-Pentoxy-, 2,2-Dimethylpropoxy- oder 3-Methylbutoxygruppe. Hydroxy, Methoxy und Ethoxy sind bevorzugt.
Für eine teilweise oder vollständig fluorierte C₁-C₄-Fluoralkylgruppe kommen vor allem die Trifluormethyl- oder die Pentafluorethylgruppe in Betracht.
Für ein Halogenatom kann ein Fluor-, Chlor-, Brom- oder lodatom stehen. Bevorzugt ist hier Fluor, Chlor oder Brom.
Unter 3- bis 12-gliedrige Cycloalkyl und Heterocycloalkylgruppen werden sowohl monocyclische wie bicyclische verstanden.
Für monocyclische C₃-C₁₀-Cycloalkyl im Sinne von R^{c} und R^{e} seien beispielsweise Cyclopropan, Cyclobutan, Cyclopentan und Cyclohexan genannt. Cyclopropyl, Cyclopentyl und Cyclohexyl sind bevorzugt.
Für monocyclische 3-10-gliedrige heterocyclische Reste im Sinne von A, Z, K, R³ oder R⁴ stehen, beispielsweise Morpholin, Tetrahydrofuran, Piperidin, Pyrrolidin, Oxiran, Oxetan, Aziridin, Dioxolan, Dioxan, Thiophen, Furan, Pyran, Pyrrol, Imidazol, Pyrazol, Pyridin, Pyrazin, Pyrimidin, Pyridazin, Piperazin, Thiazol, Oxazol, Furazan, Pyrrolin, Thiazolin, Triazol, Tetrazol, wobei alle beliebigen, chemisch möglichen Isomeren bezüglich der Positionen der Heteroatome verwendet werden.
Als Beispiele für bicyclische 3-10-gliedrige Heterocyclen seien Chinolin, Chinazolin und Naphthyridin genannt.
   Für R⁴ sind erfindungsgemäß die unter B und C genannten bicyclischen Ringsysteme bevorzugt.
Im Sinne von R¹ und R², die gemeinsam mit dem C-Atom der Kette einen carbocyclischen Ring mit insgesamt 3-7 Gliedern bilden können, werden Carbocyclen mit 3 bis 7 Kohlenstoffatomen, bevorzugt 3 bis 6 Kohlenstoffatomen verstanden. Besonders bevorzugt sind Cyclopropyl, Cyclopentyl und Cyclohexyl.
   Heterocyclen im Sinne von R¹ und R², die gemeinsam mit dem C-Atom der Kette gebildet werden können, können cyclische Ringverbindungen mit mindestens einem Heteroatom, bevorzugt Sauerstoff, Stickstoff und Schwefel sein. Besonders bevorzugt sind Tetrahydropyranyl, Piperidinyl, Tetrahydrothiopyranyl.
Die Zahl p für den (CH₂)ₚ-Rest kann eine Zahl 0, 1, 2, 3, 4, 5 oder 6, bevorzugt 0, 1 oder 2 sein. Unter "Rest" werden erfindungsgemäß sämtliche funktionellen Gruppen, die in Verbindung mit (CH₂)ₚ unter L aufgeführt werden, verstanden.
Im Falle, dass die Verbindungen der allgemeinen Formel I als Salze vorliegen, kann dies beispielsweise in der Form des Hydrochlorids, Sulfats, Nitrats, Tartrats, Citrats, Fumarats, Succinats oder Benzoats sein.
Wenn die erfindungsgemäßen Verbindungen als racemische Gemische vorliegen, können sie nach dem Fachmann geläufigen Methoden der Racemattrennung in die reinen, optisch aktiven Formen aufgetrennt werden. Beispielsweise lassen sich die racemischen Gemische durch Chromatographie an einem selbst optisch aktiven Trägermaterial (CHIRALPAK AD^{®}) in die reinen Isomere trennen. Es ist auch möglich, die freie Hydroxygruppe in einer racemischen Verbindung der allgemeinen Formel I mit einer optisch aktiven Säure zu verestern und die erhaltenen diastereoisomeren Ester durch fraktionierte Kristallisation oder chromatographisch zu trennen und die getrennten Ester jeweils zu den optisch reinen Isomeren zu verseifen. Als optisch aktive Säure kann beispielsweise Mandelsäure, Camphersulfonsäure oder Weinsäure verwendet werden.
Bevorzugt gemäß vorliegender Erfindung sind Verbindungen der allgemeinen Formel (I), in denen A ein Wasserstoffatom ist.
Weiterhin bevorzugt sind Verbindungen, in denen:
   Y für einen Rest -C≡C- steht und
      - R¹ und R²: gemeinsam mit dem C-Atom der Kette einen carbocyclischen oder heterocyclischen 3-6-gliedrigen Ring bilden und
      - R³: ein gegebenenfalls mit K substituiertes C₁-C₈-Alkyl, ein gegebenenfalls ein- oder mehrfach, gleich oder verschieden mit L substituiertes Aryl oder ein 3- bis 12-gliedriges Heteroaryl bedeuten oder Y für (CH₂)ₘ steht und R³ ein gegebenenfalls ein- oder mehrfach, gleich oder verschieden mit L substituiertes Aryl oder ein 3- bis 12-gliedriges Heteroaryl ist und
      - R⁴: ein gegebenenfalls 1- oder 2-fach gleich oder verschieden mit L substituiertes mono- oder bicyclisches Aryl oder eine der unter R⁴ genannten Gruppen B mit Verknüpfung an Position 6 oder C mit Verknüpfung an Position 5 ist.
   Im Fall, dass Y (CH₂)ₘ bedeutet, steht m bevorzugt für 1.
   Unabhängig von m ist für den Fall, dass Y (CH₂)ₘ bedeutet, R⁴ ein mit bis zu 2 der unter L genannten Reste substituierter Phenylring.
   Für L sind besonders bevorzugt ein Cyanorest, ein Chlor und/ oder ein Trifluormethylrest.

Im Fall, dass Y (CH₂)ₘ bedeutet, kann R⁴ alternativ zum substituierten Phenylring auch folgende Bedeutung besitzen: 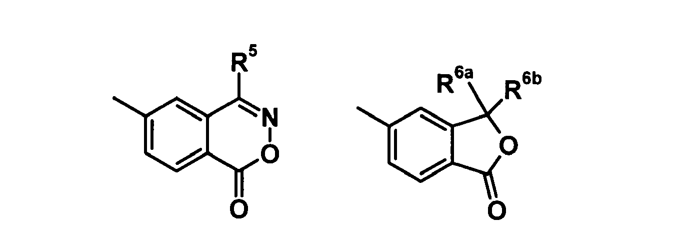

Hierfür sind folgende Substituenten bevorzugt:
- R⁵: ein Methyl oder Ethyl,
- R⁶: ein Wasserstoff.
Bevorzugt sind weiterhin Verbindungen der allgemeinen Formel (I), in denen
- A: ein Wasserstoff ist,
- p: 0, 1 oder 2,
- L: ein C₁-C₈-Alkyl, C₂-C₈-Alkenyl, C₂-C₈-Alkinyl, ein teilweise oder vollständig fluoriertes C₁-C₆-Fluoralkyl, -(CH₂)ₚCN, (CH₂)ₚHal, (CH₂)ₚNO₂, (CH₂)ₚ-C₆-C₁₂-Aryl, -(CH₂)ₚ-Heteroaryl, -(CH₂)ₚNR^{c}R^{d}, -(CH₂)pNR^{e}COR^{b}, -(CH₂)ₚNR^{e}S(O)₂R^{b}, -(CH₂)ₚNR^{e}CONR^{c}R^{d}, -(CH₂)ₚNR^{e}S(O)NR^{c}R^{d}, -(CH₂)ₚNR^{e}S(O)₂NR^{c}R^{d}, -(CH₂)ₚCOR^{b}, -(CH₂)ₚS(O)R^{b}, -(CH₂)ₚS(O)₂R^{b}, -(CH₂)ₚS(O)₂NR^{c}R^{d}, -(CH₂)ₚCO₂R^{b}, -(CH₂)ₚCONR^{c}R^{d}, -(CH₂)ₚOR^{b}, -(CH₂)ₚCR^{b}(OH)-R^{b} und
- Z: ein Cyano, Halogen, Hydroxy, Nitro, -C(O)R^{b}, CO₂R^{b}, -O-R^{b},-SO₂NR^{c}R^{d}, -C(O)-NR^{c}R^{d}, -NR^{c}R^{d}, -NR^{e}COR^{b}, -NR^{e}S(O)R^{b},-NR^{e}S(O)₂R^{b}, -NR^{e}CONR^{c}R^{d}, -S(O)R^{b}, -S(O)NR^{c}R^{d}, -S(O)₂R^{b}, -CR^{b}(OH)-R^{b}, oder ein gegebenenfalls ein- oder mehrfach, gleich oder verschieden mit M substituiertes C₃-C₁₀-Cycloalkyl oder Heterocycloalkyl.
Weiterhin bevorzugt sind Verbindungen der allgemeinen Formel (I), in denen
- A: ein Wasserstoff ist und
- R¹ und R²: gemeinsam mit dem C-Atom der Kette einen Cyclopropyl-, Cyclopentyl- oder Cyclohexylring bilden.

Besonders bevorzugt sind hierbei wiederum ein Tetrahydropyranyl-, ein Piperidinyl-oder ein Tetrahydrothiopyranylring.

Die nachstehend genannten Verbindungen sowie deren Verwendung sind erfindungsgemäß bevorzugt:

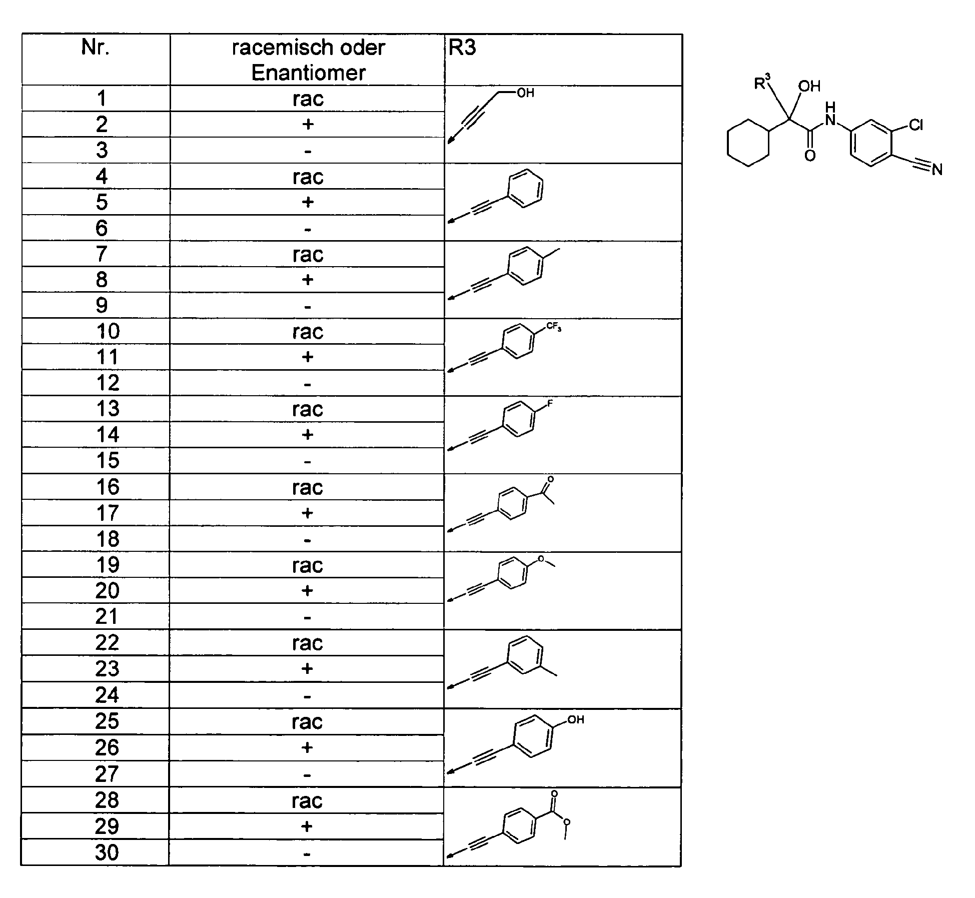

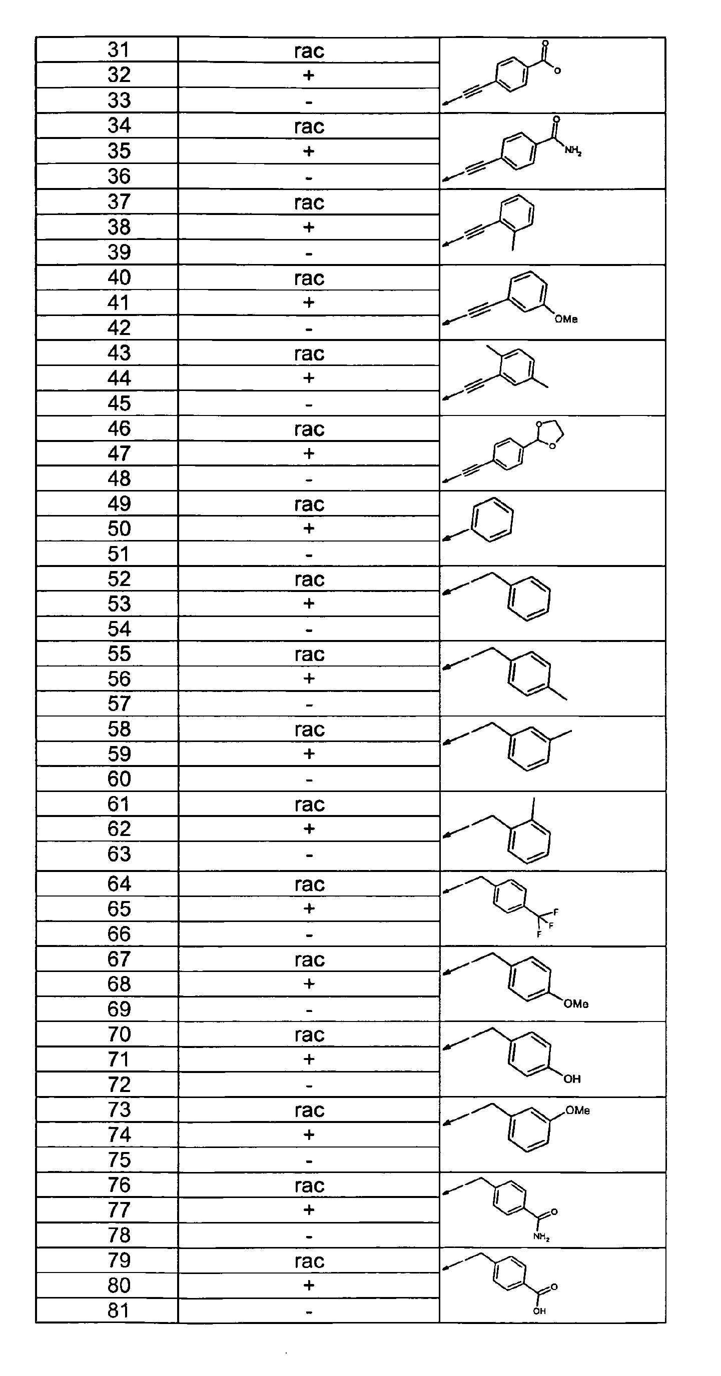

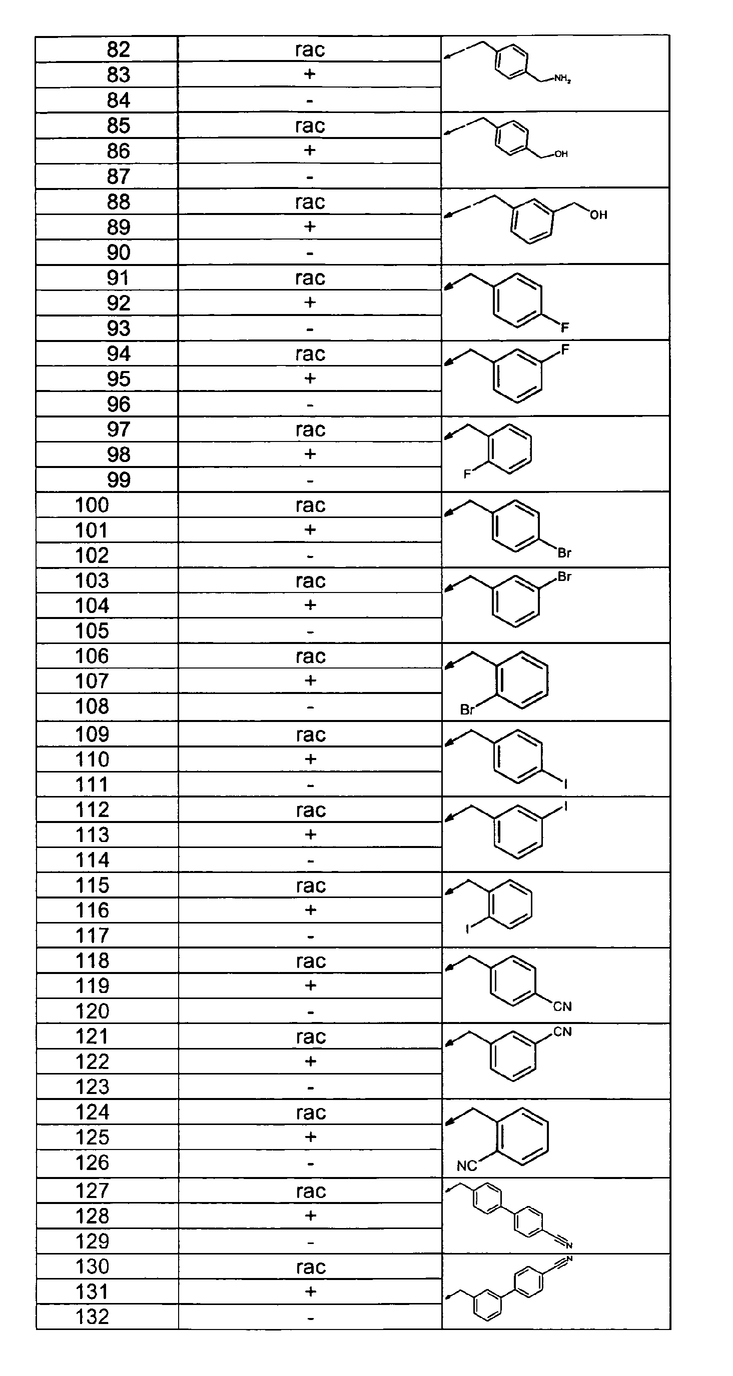

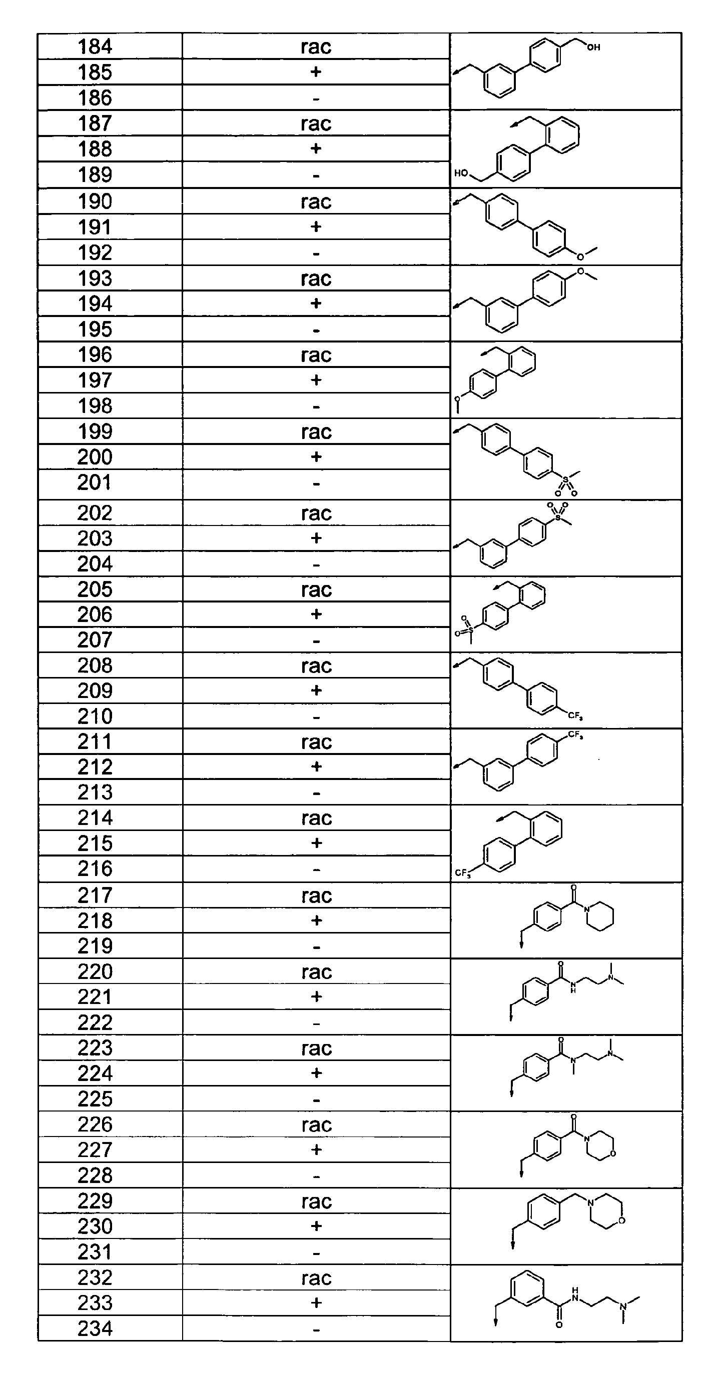

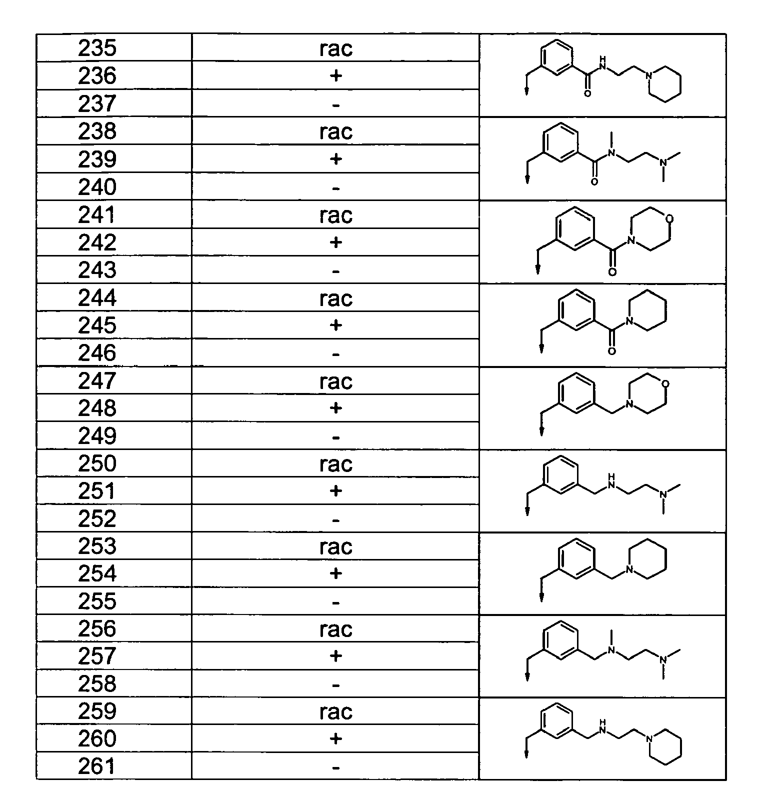

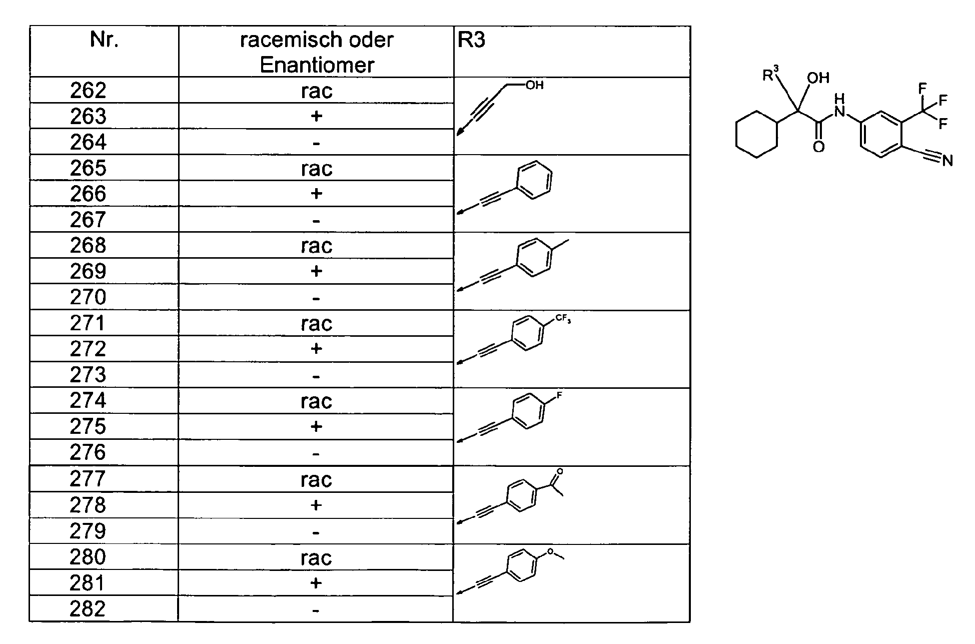

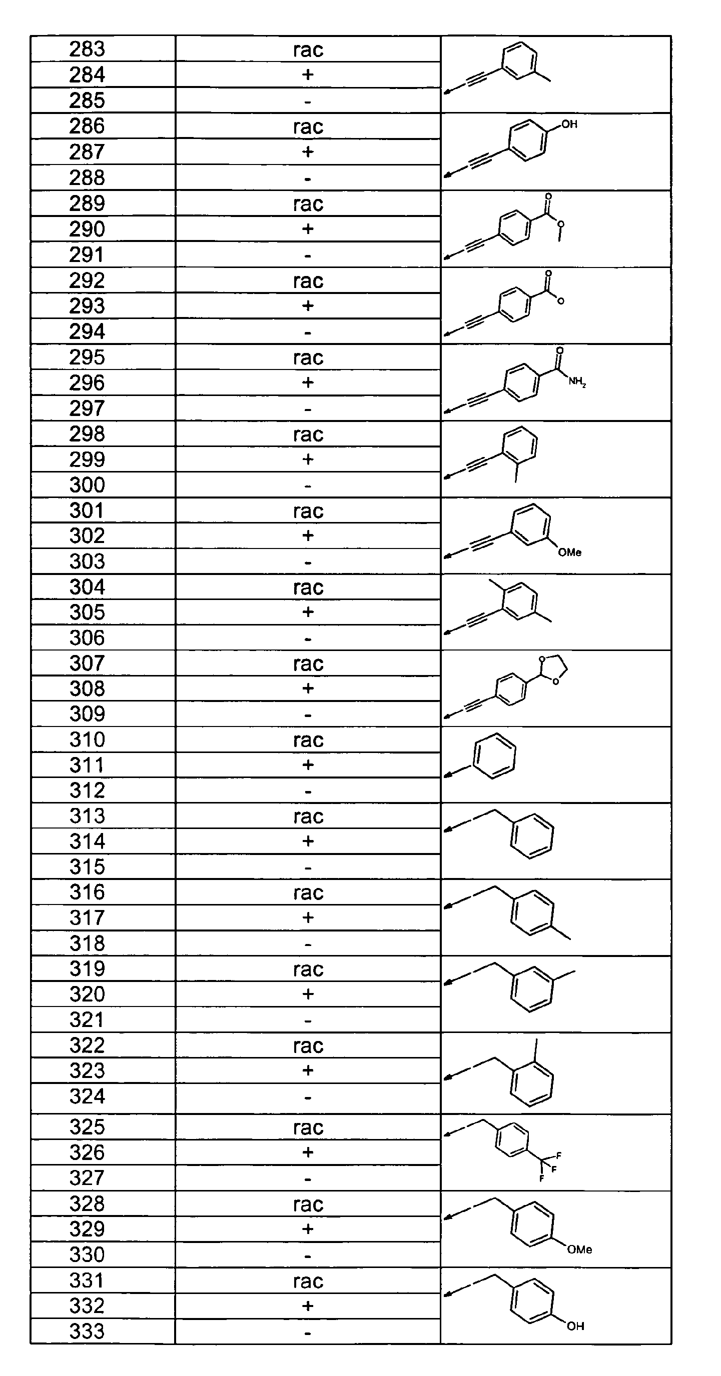

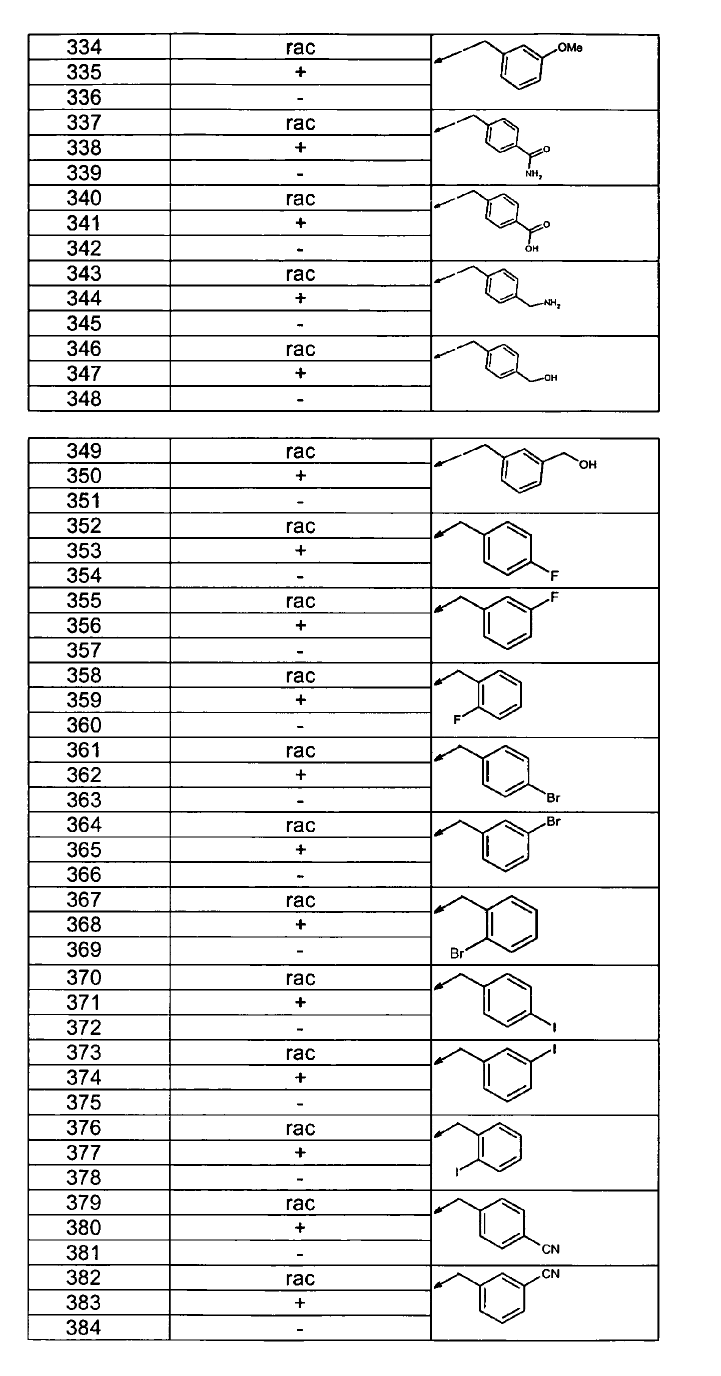

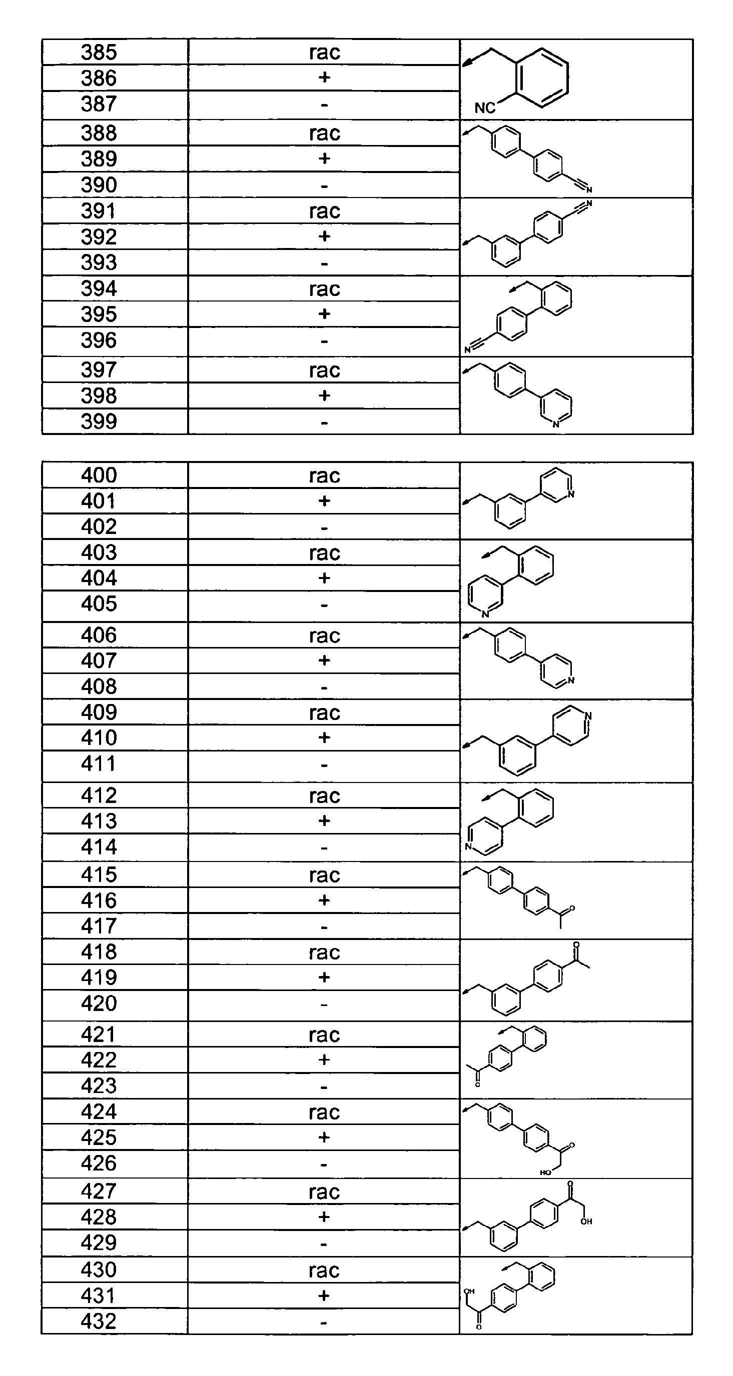

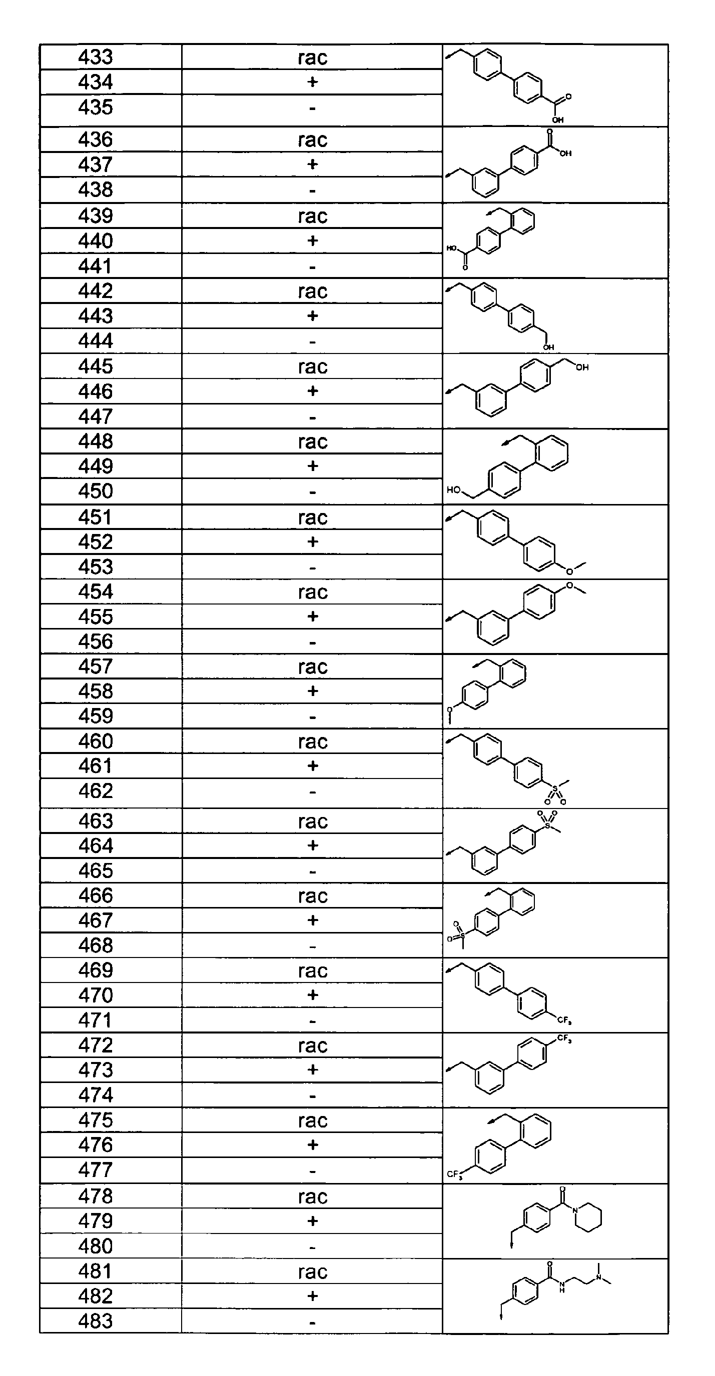

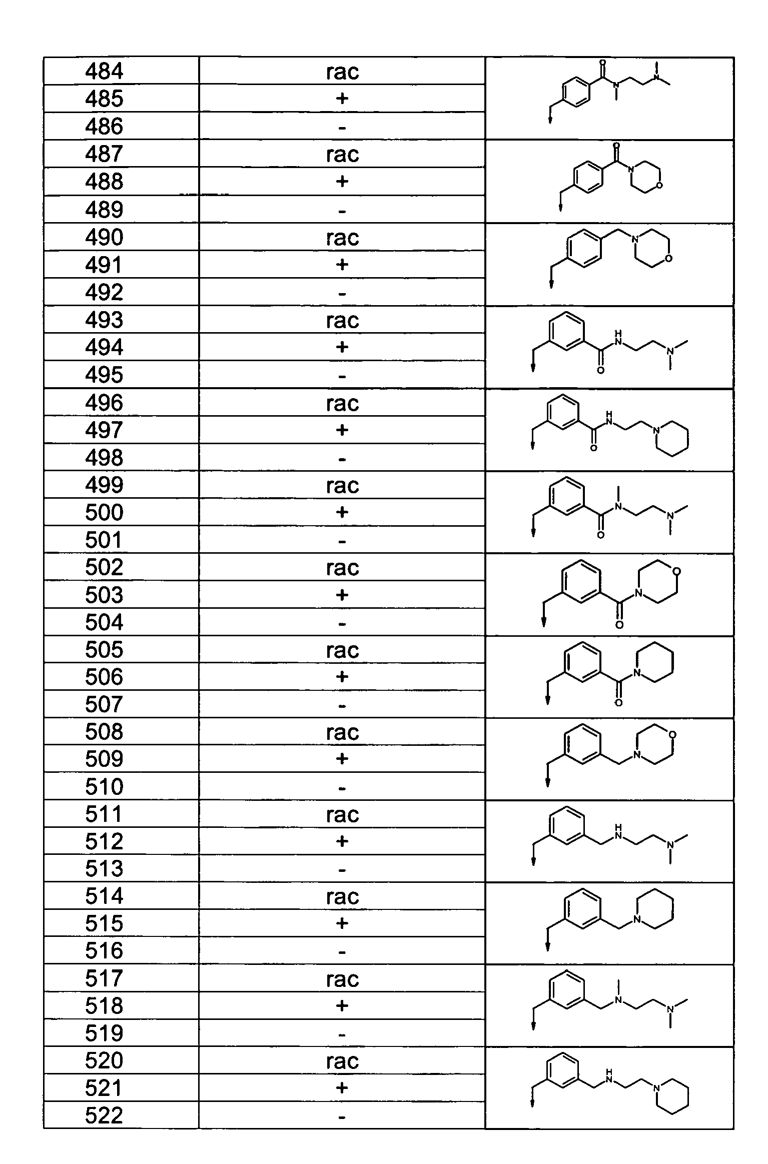

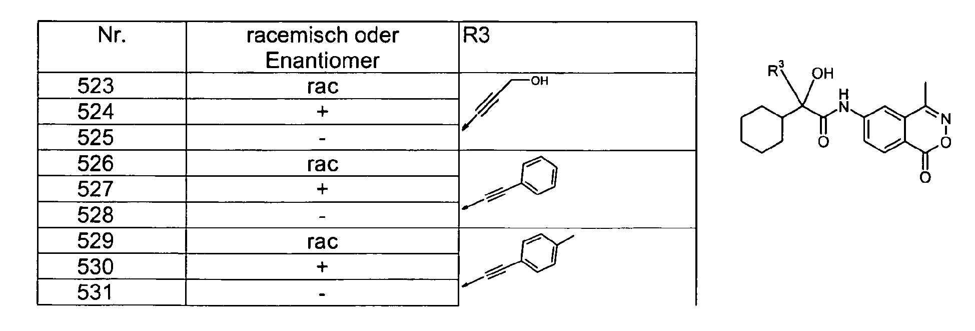

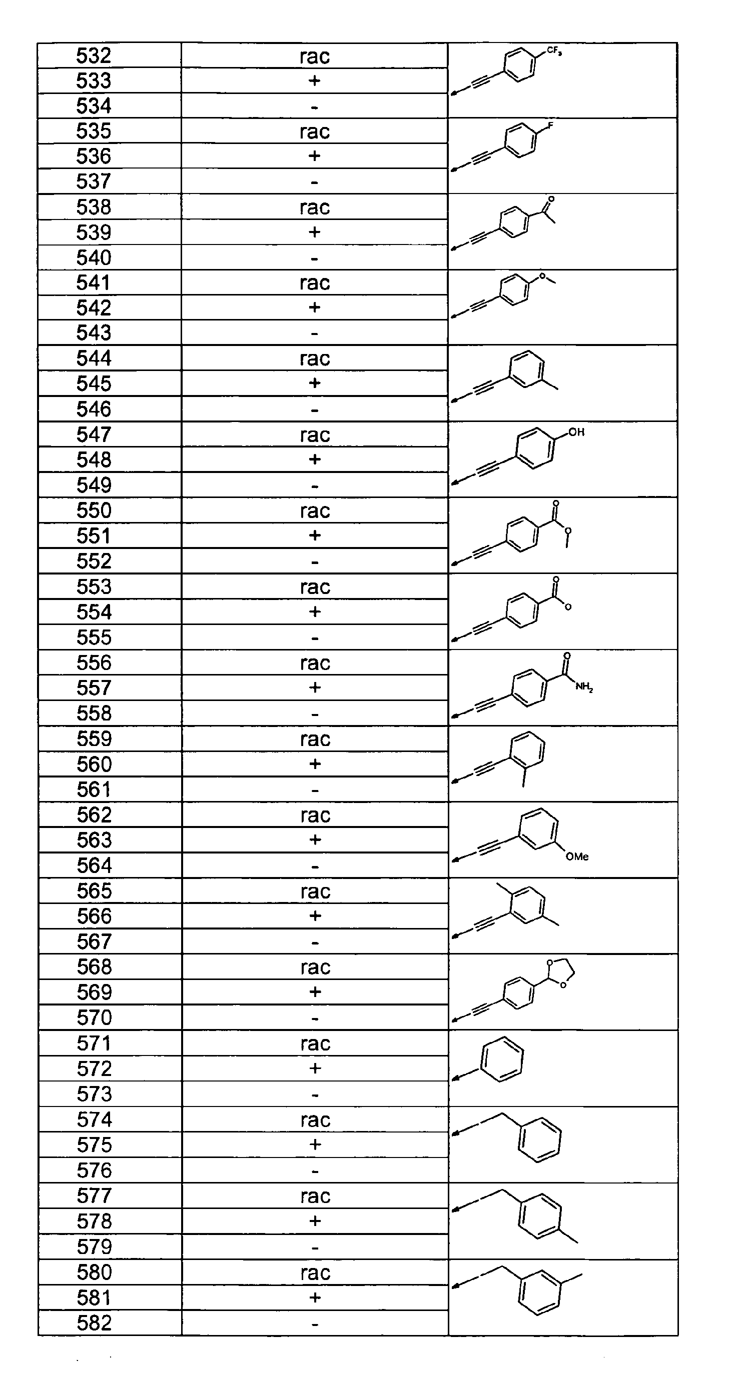

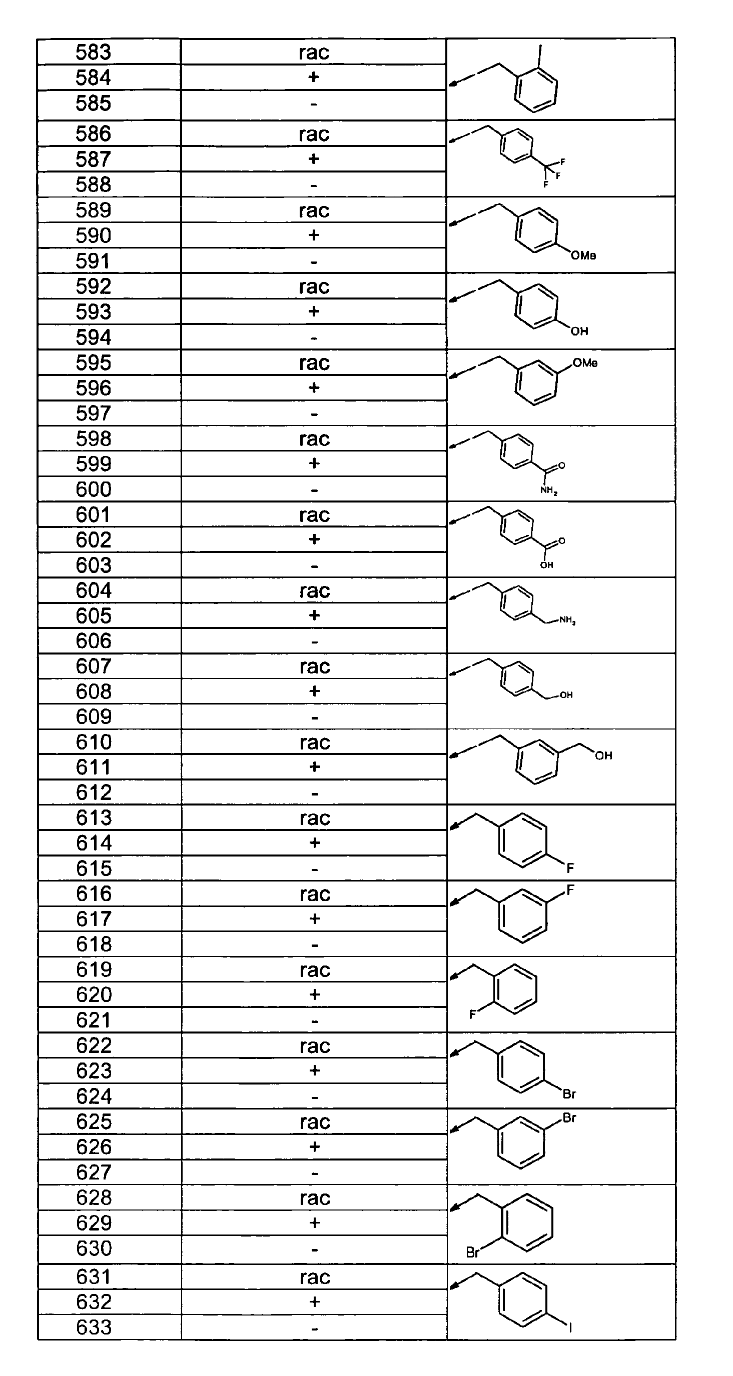

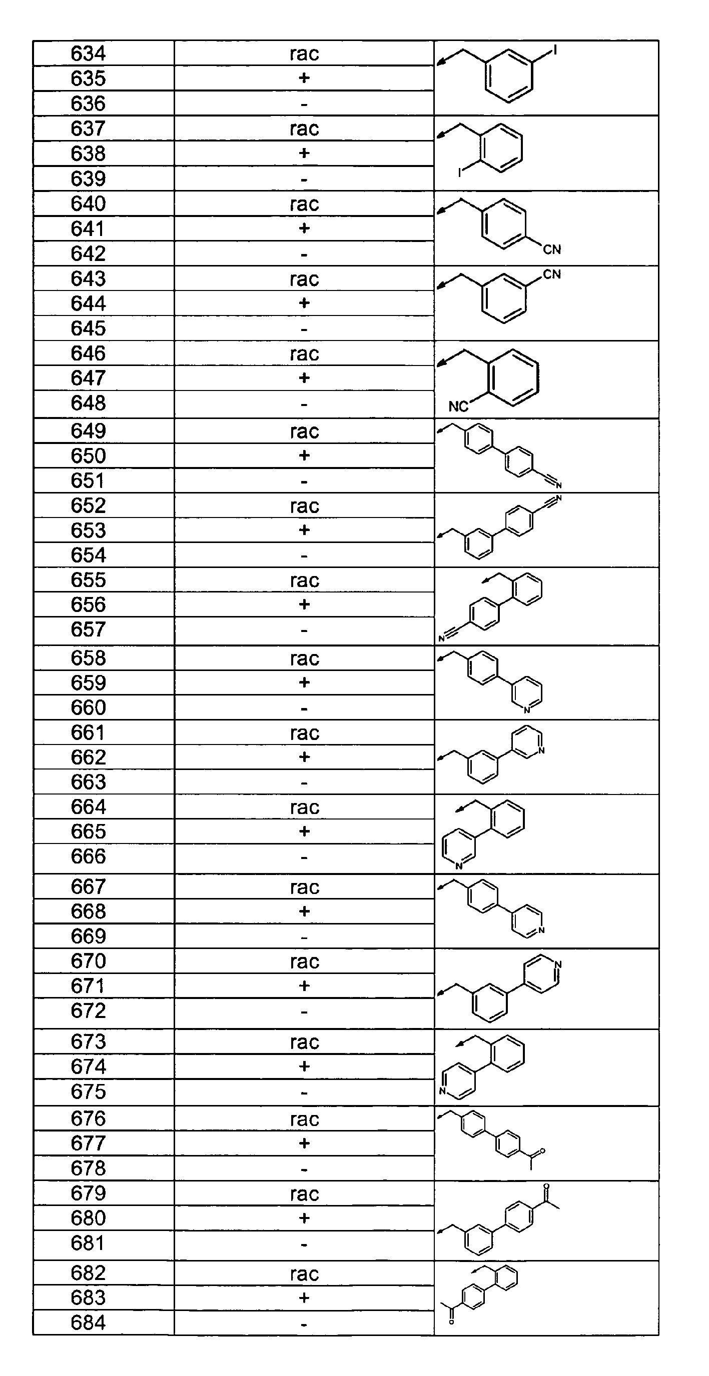

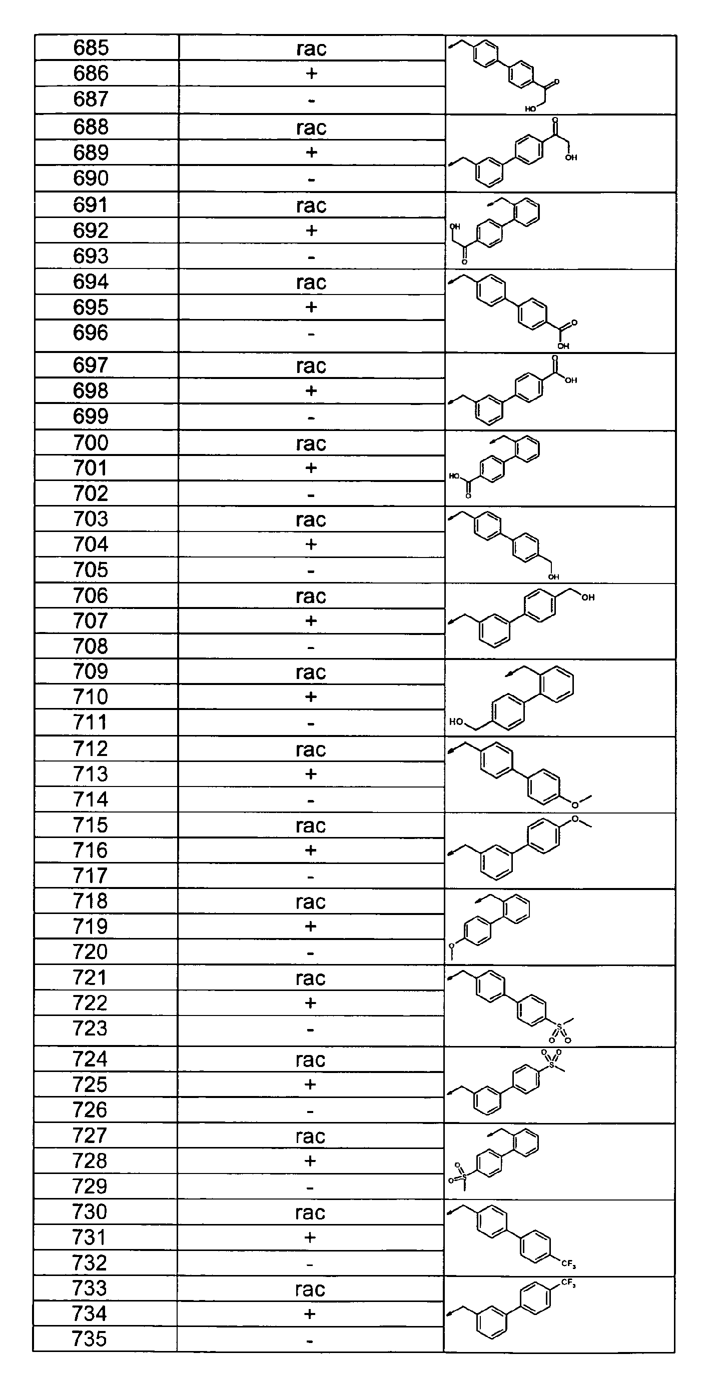

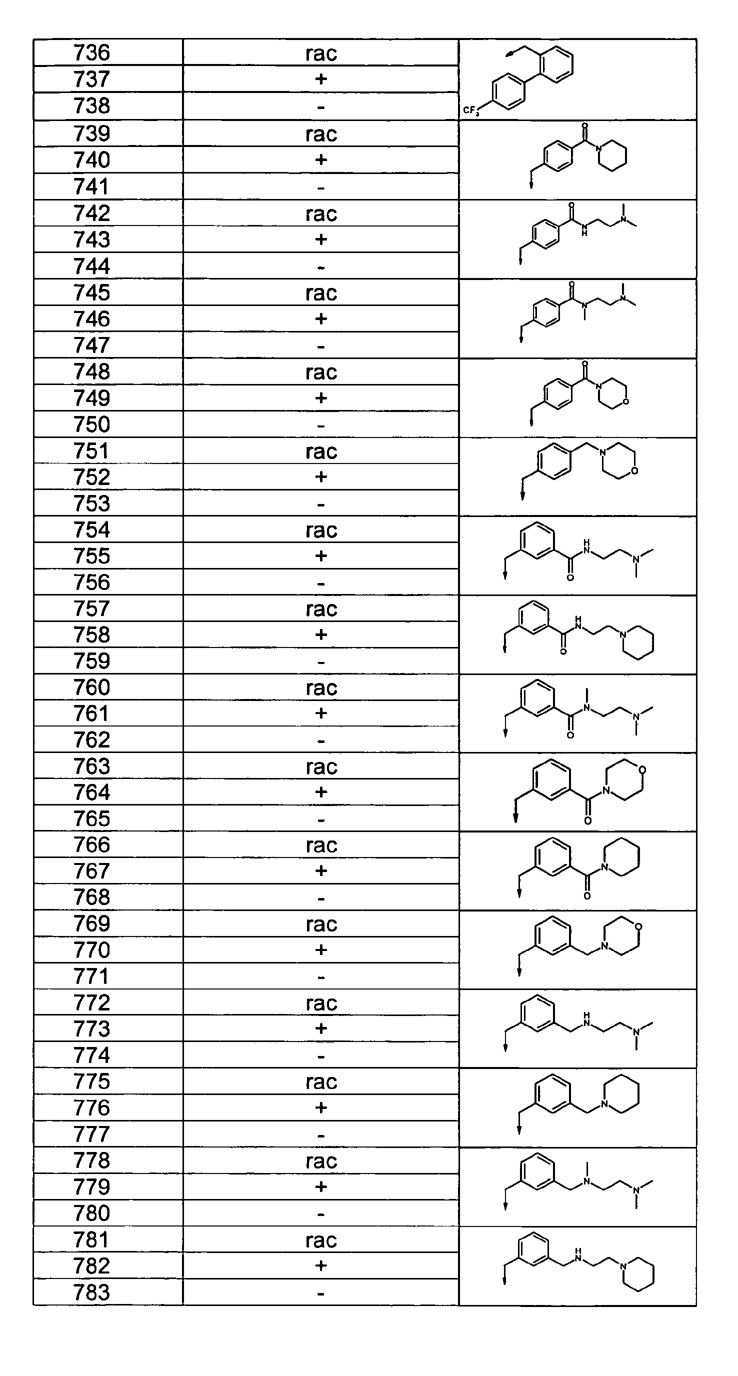

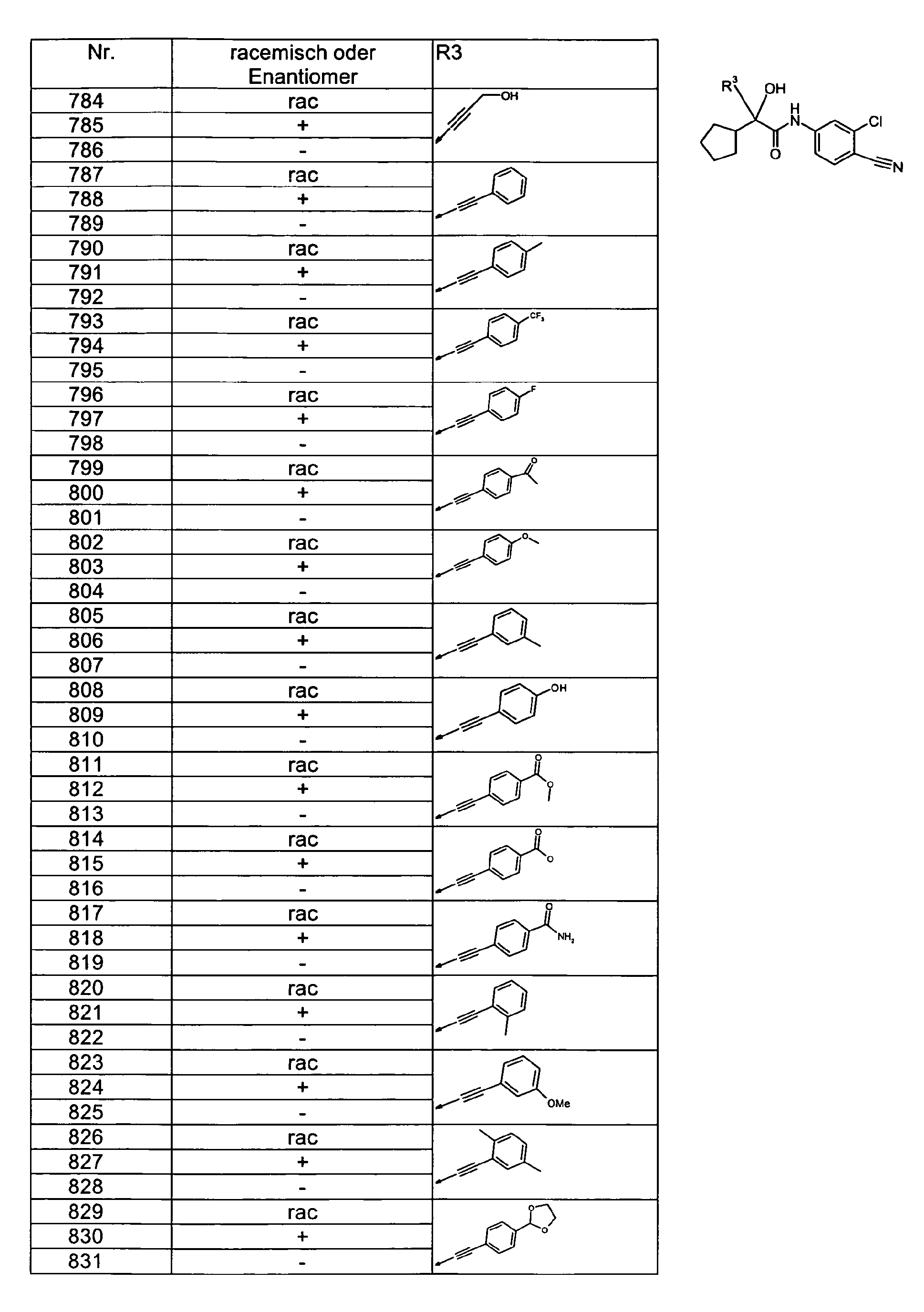

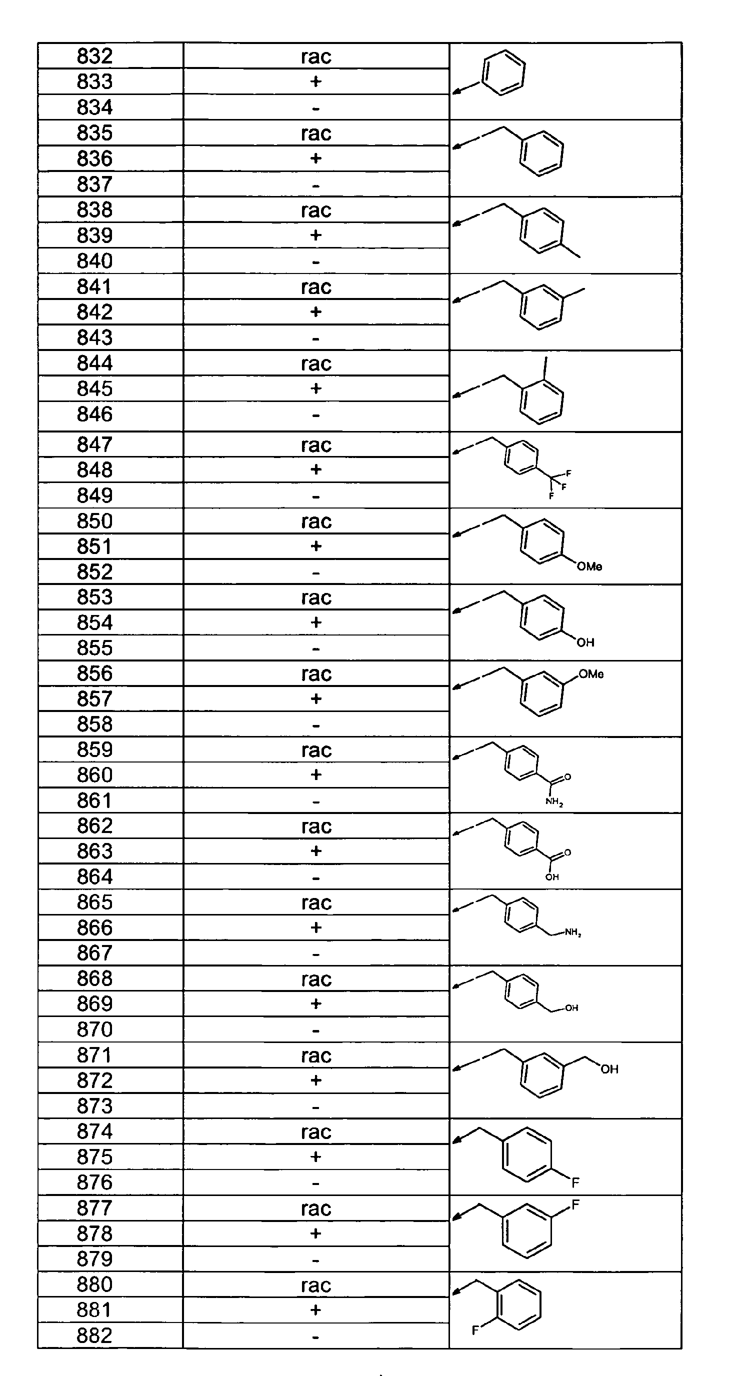

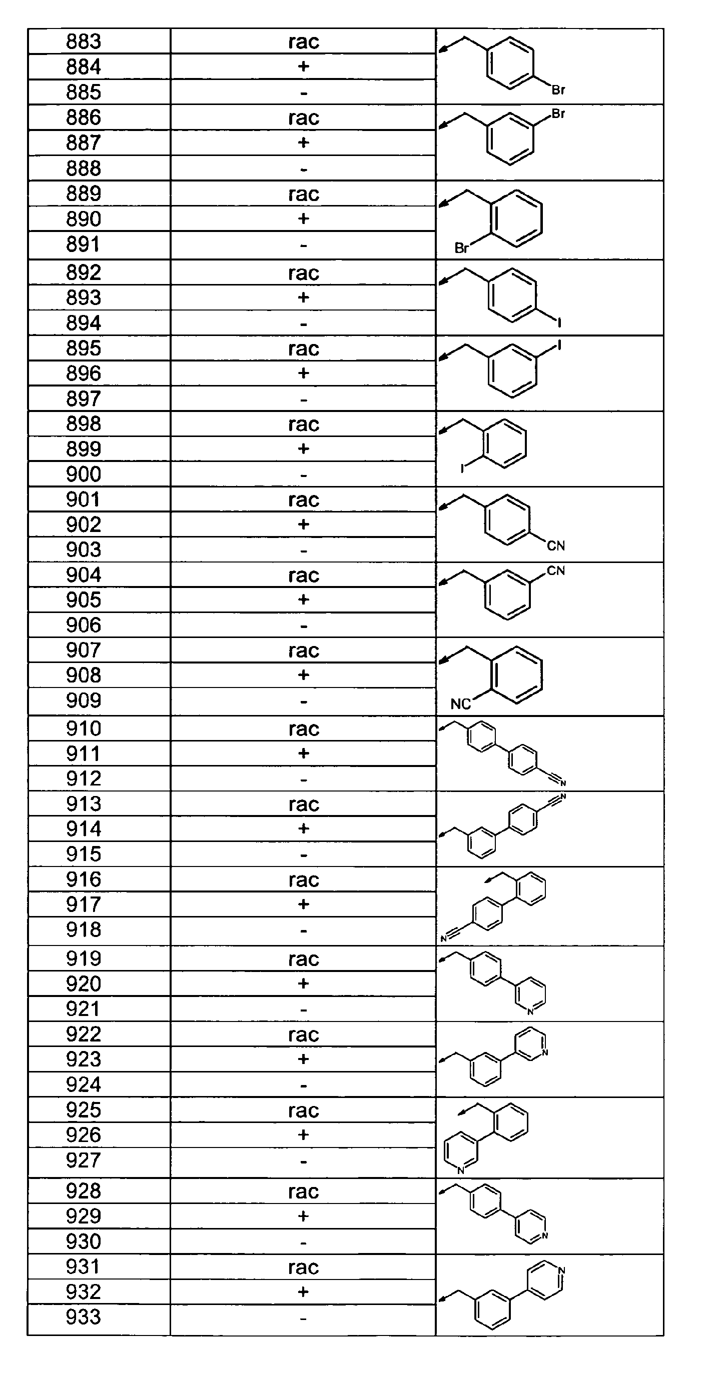

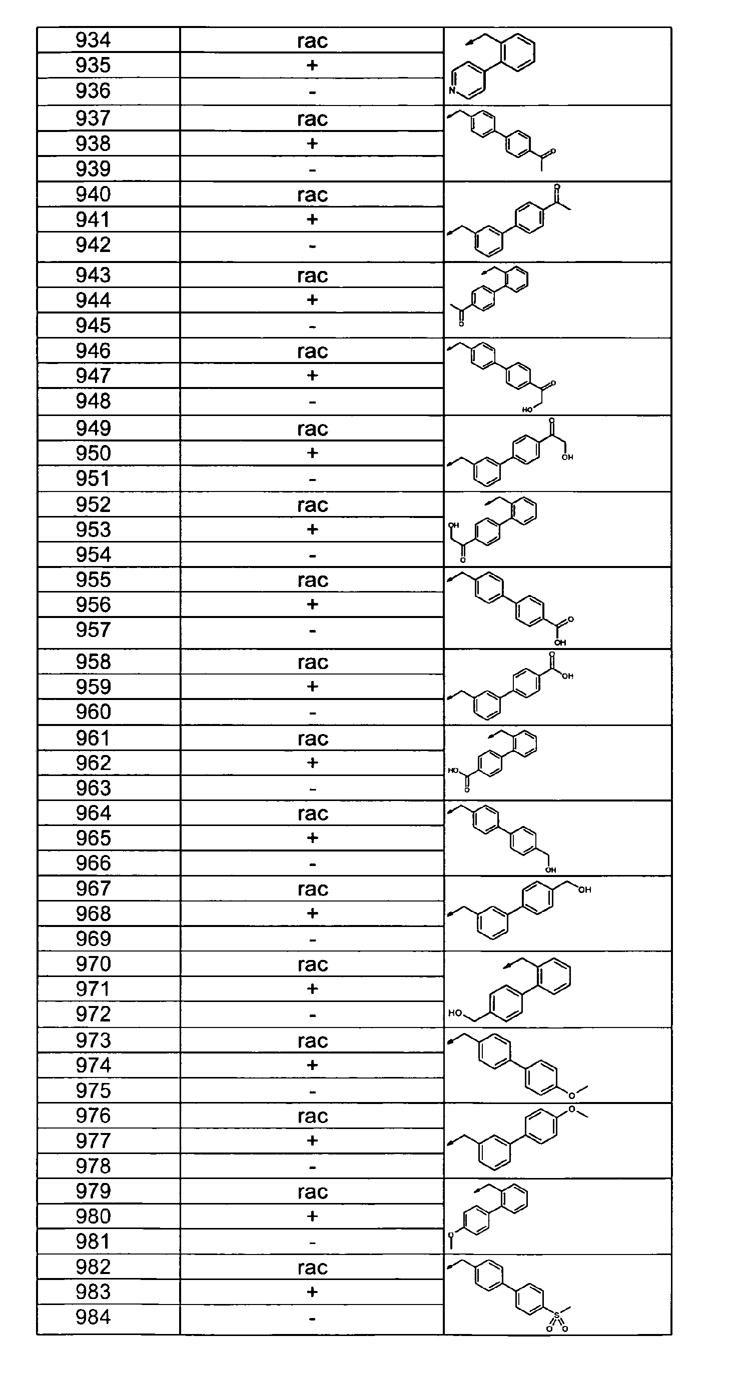

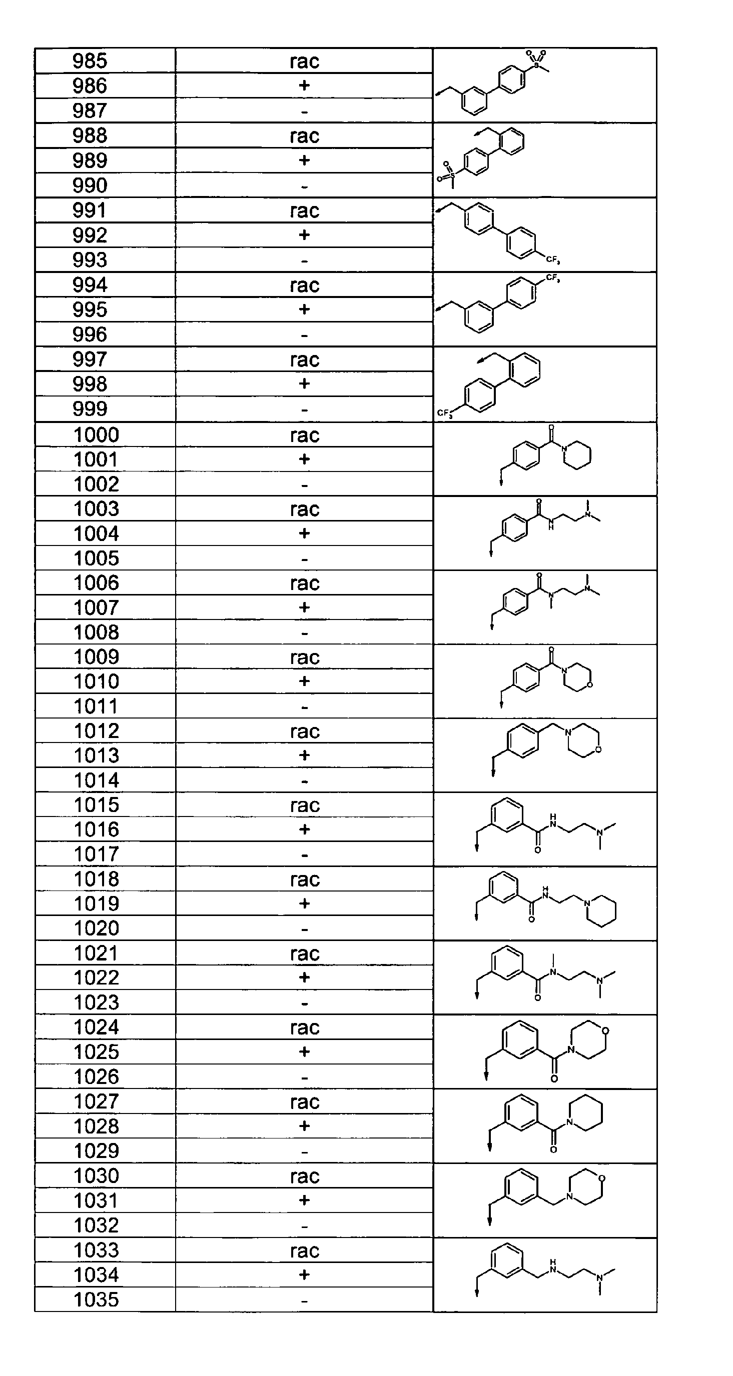

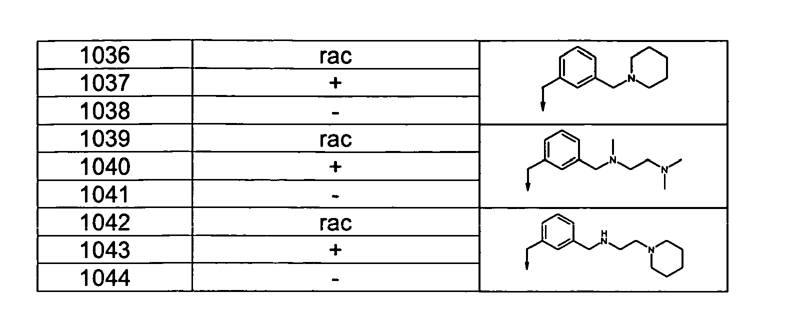

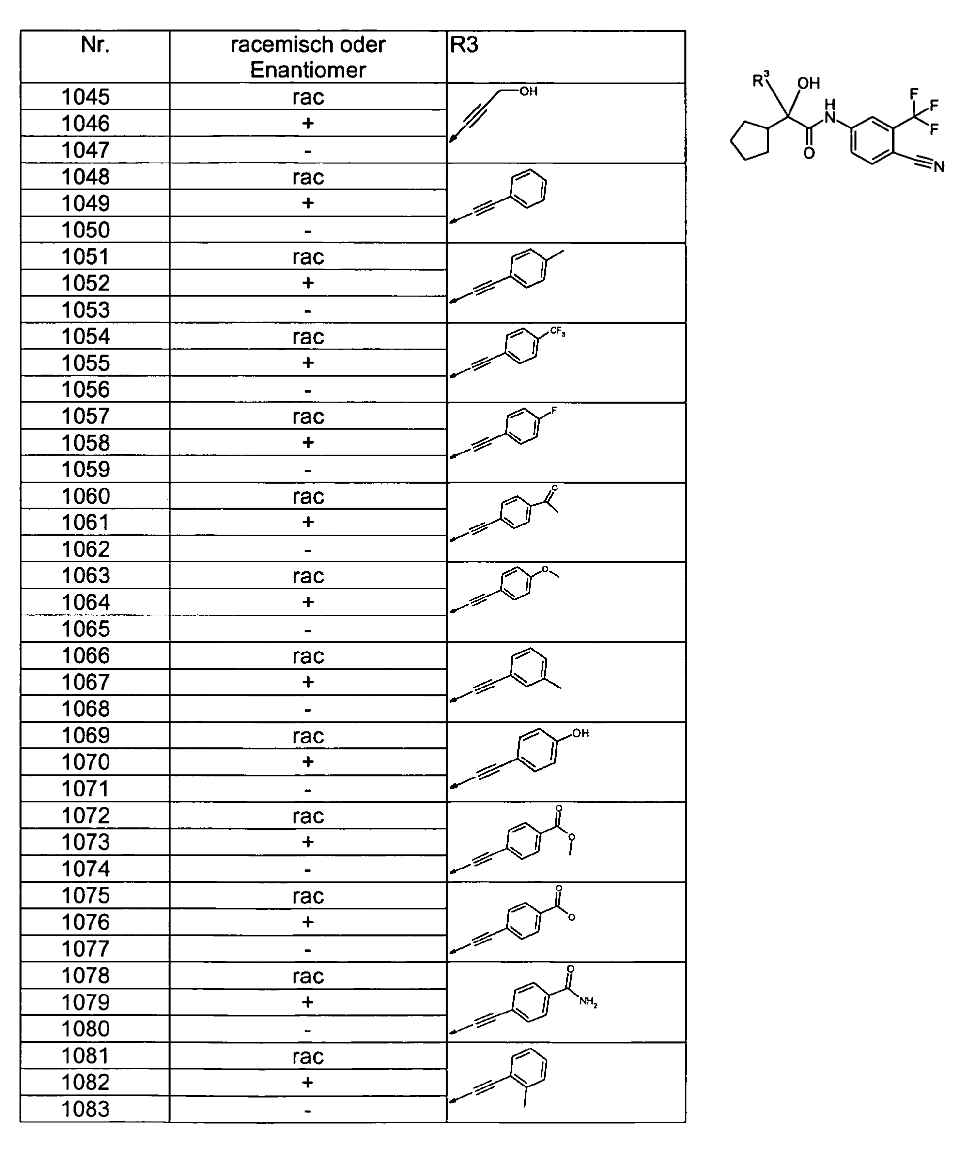

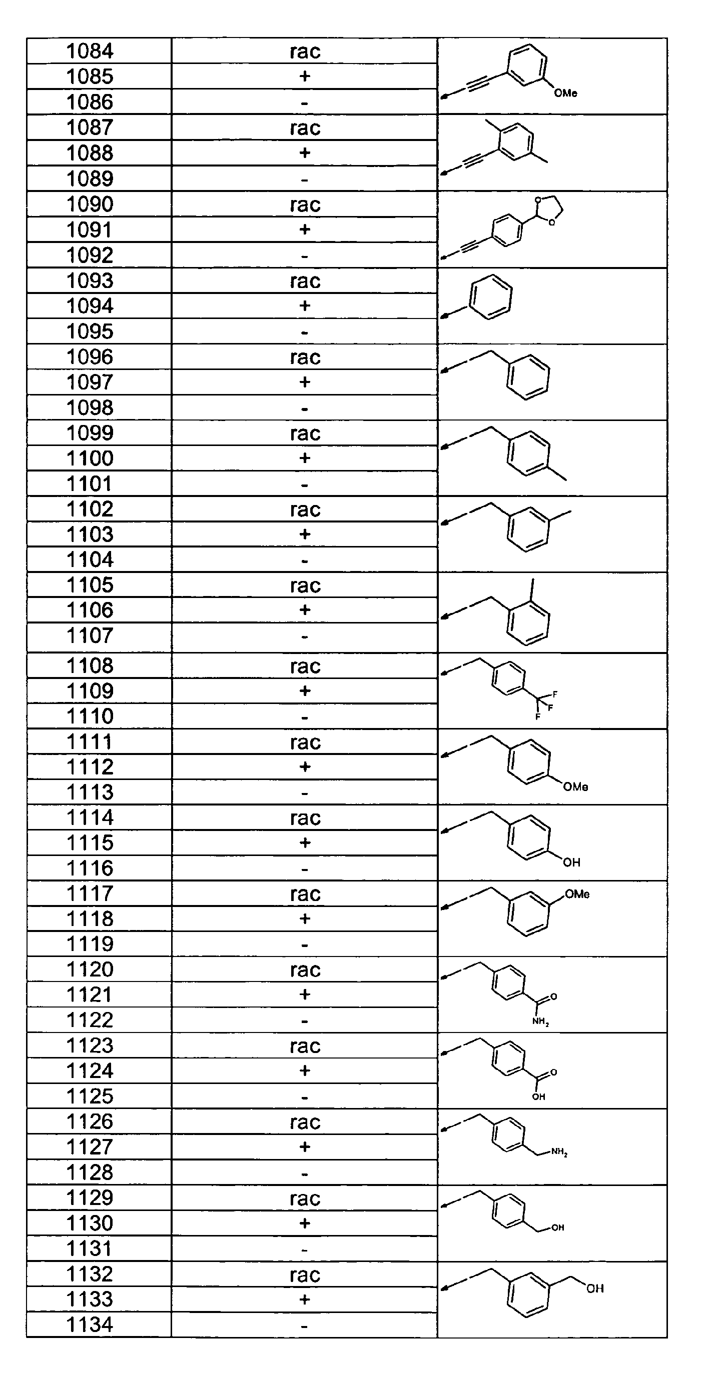

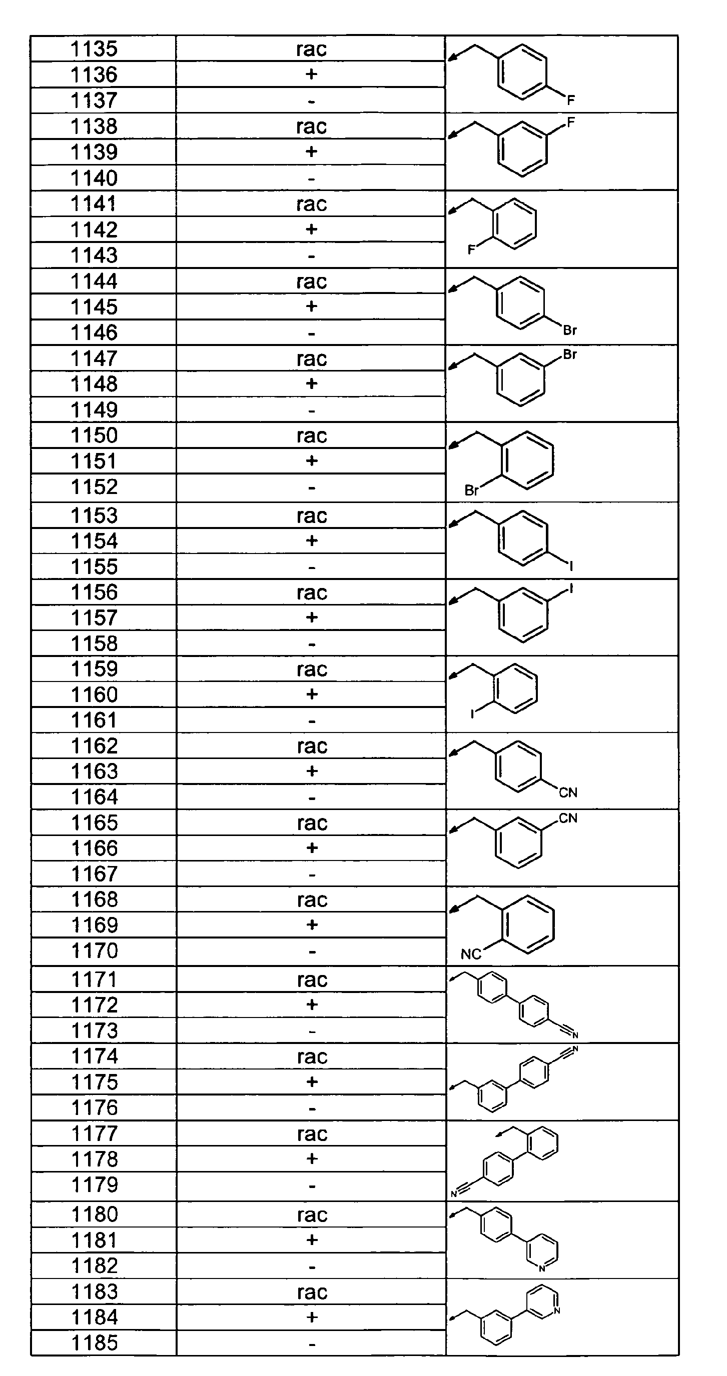

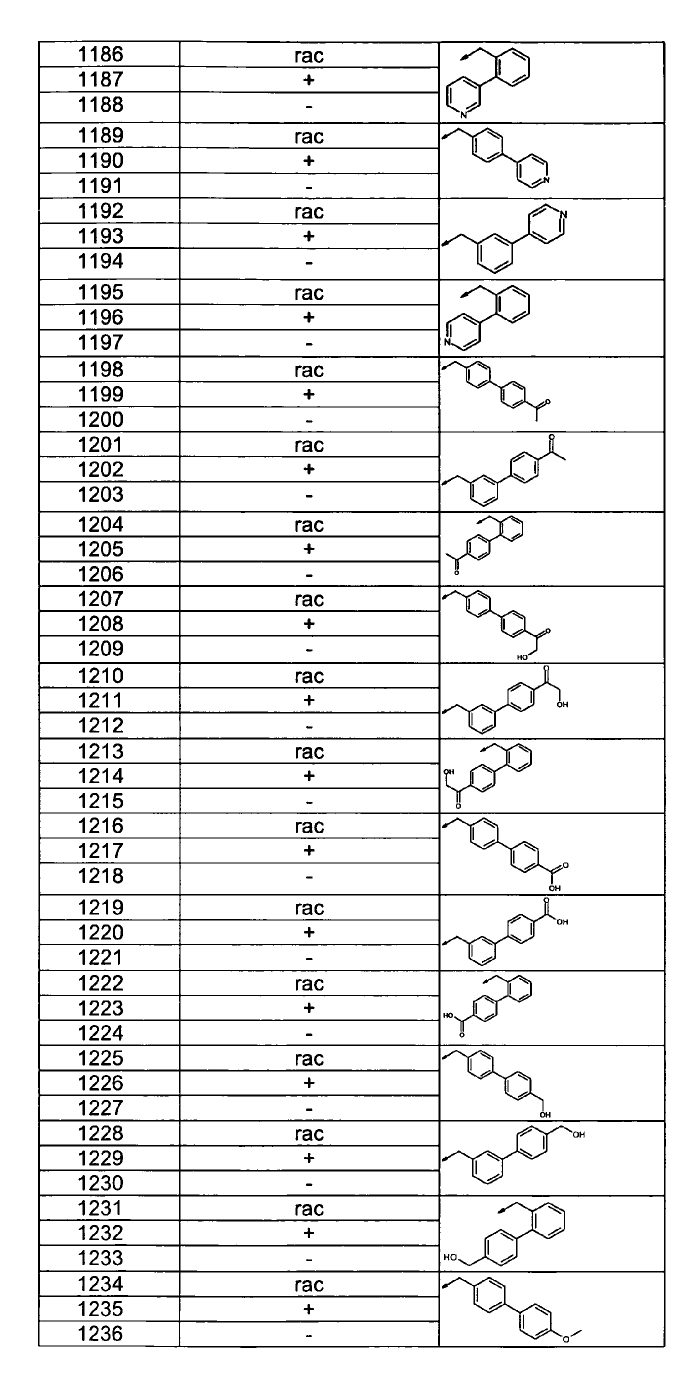

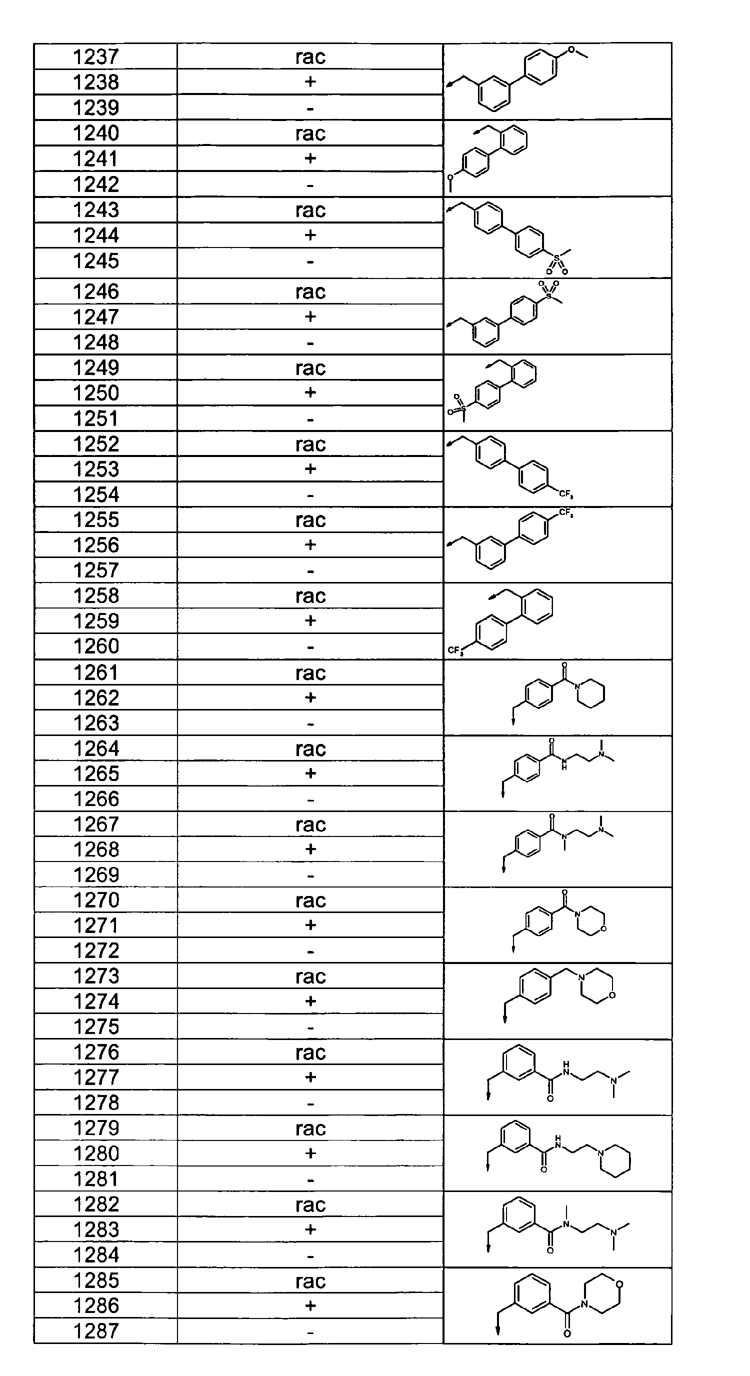

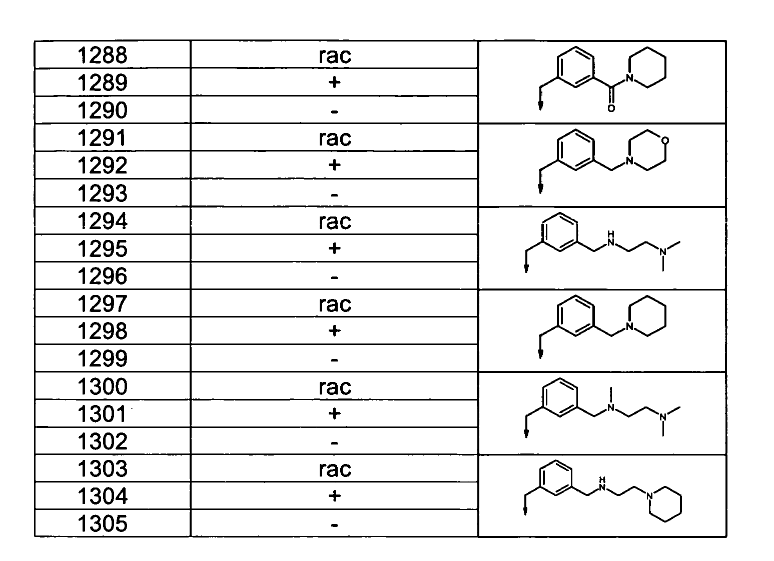

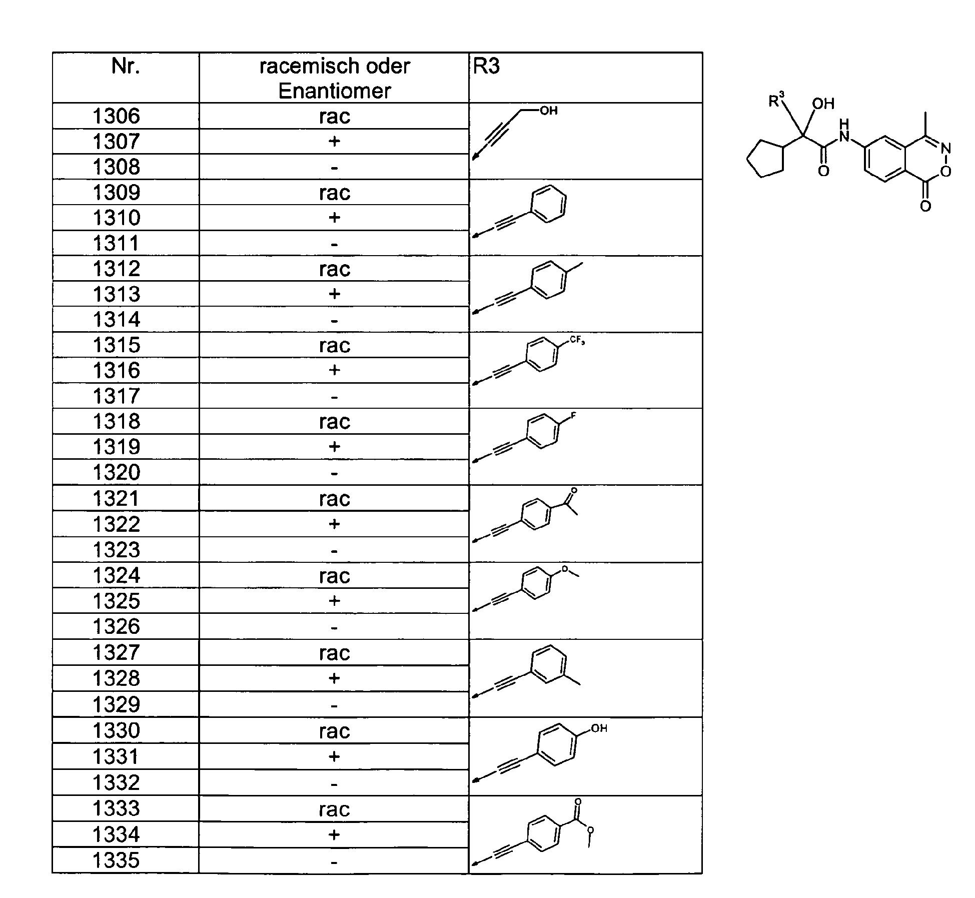

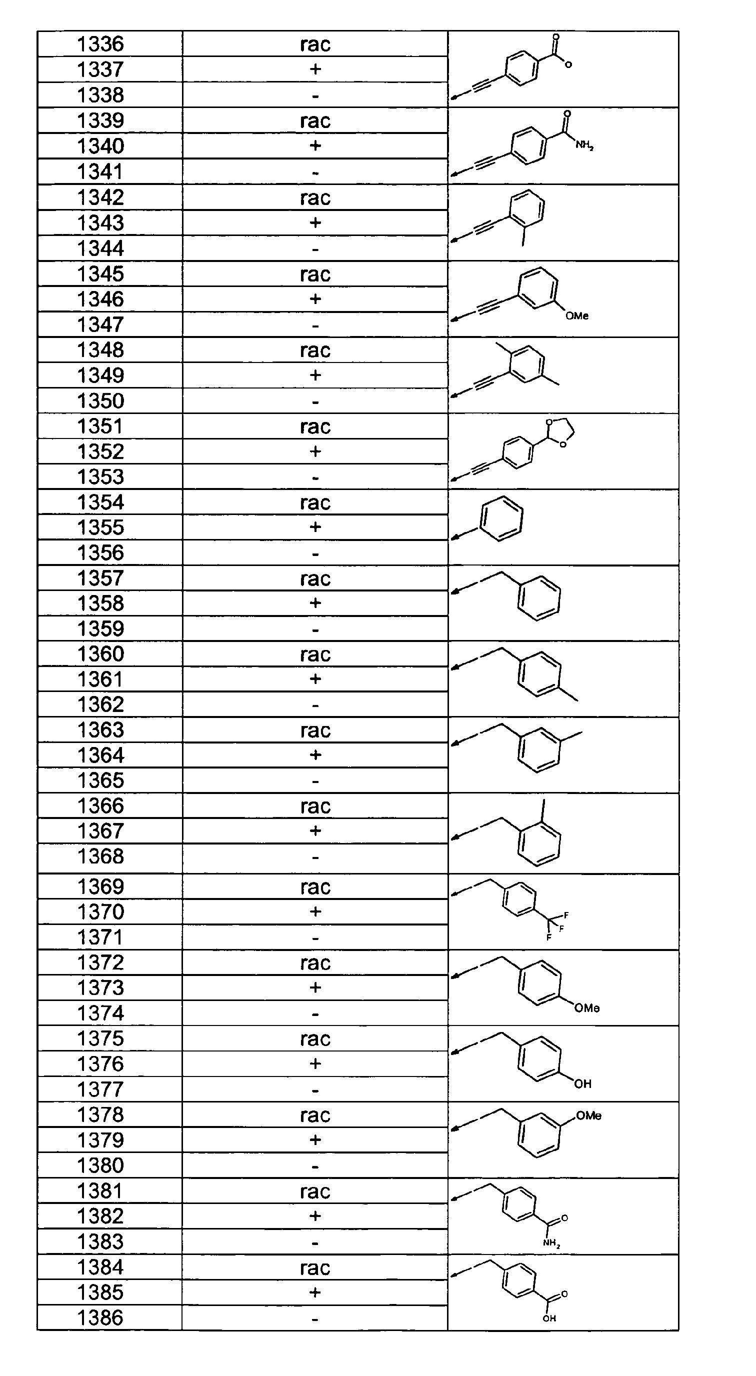

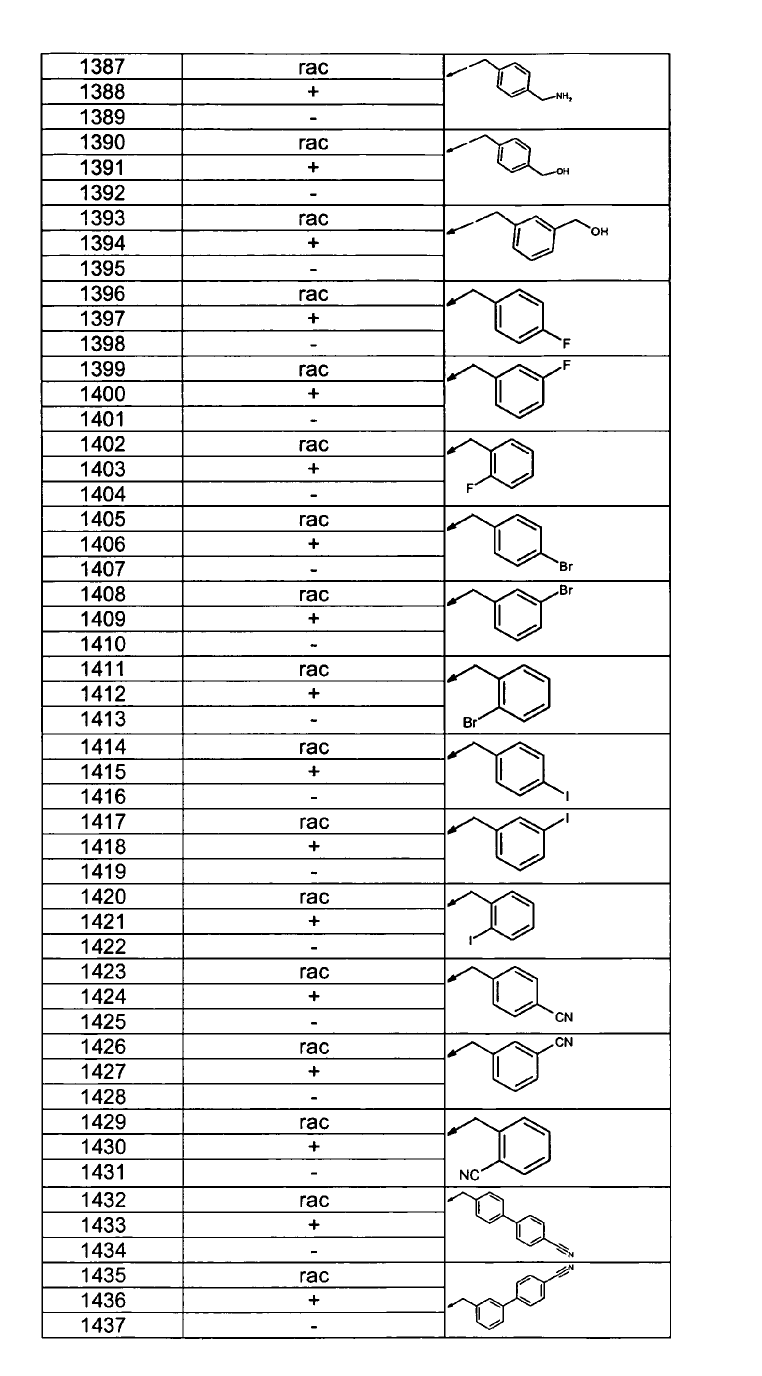

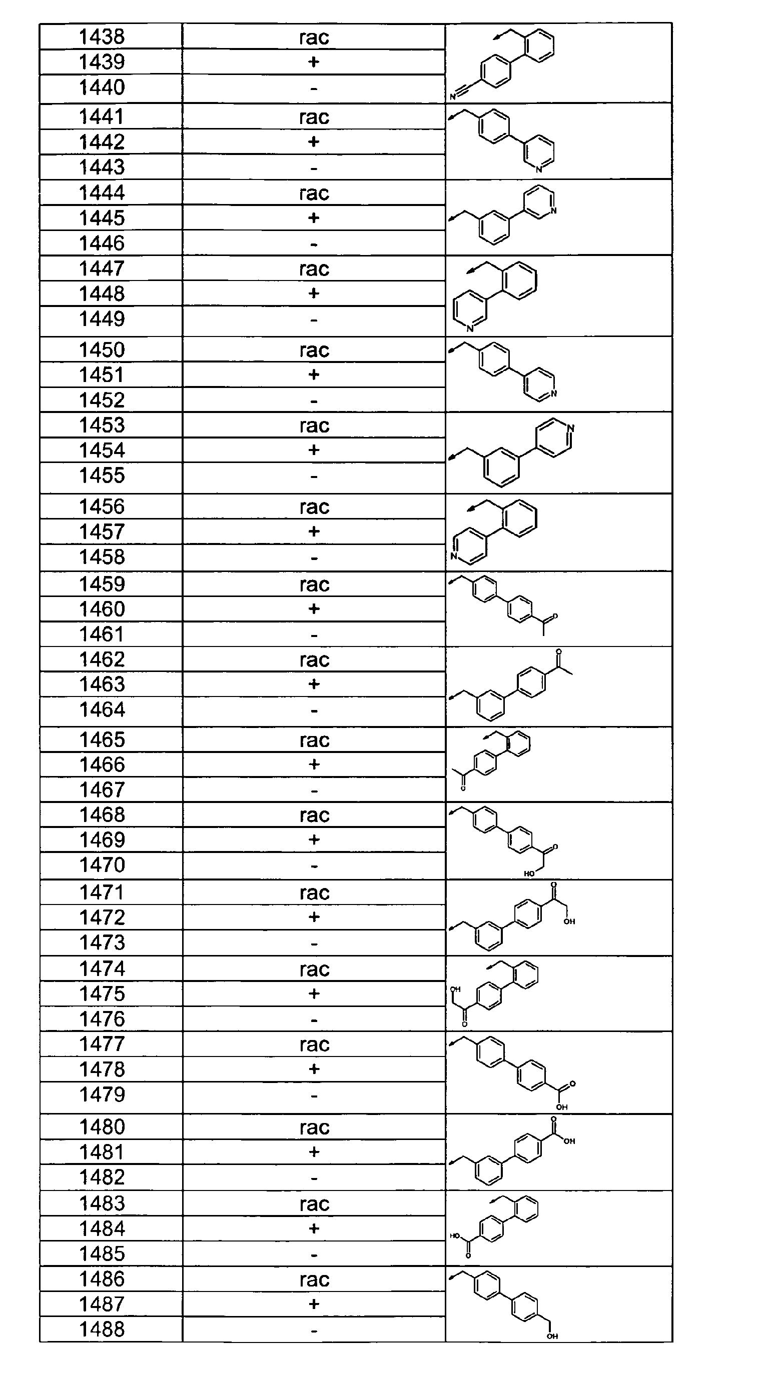

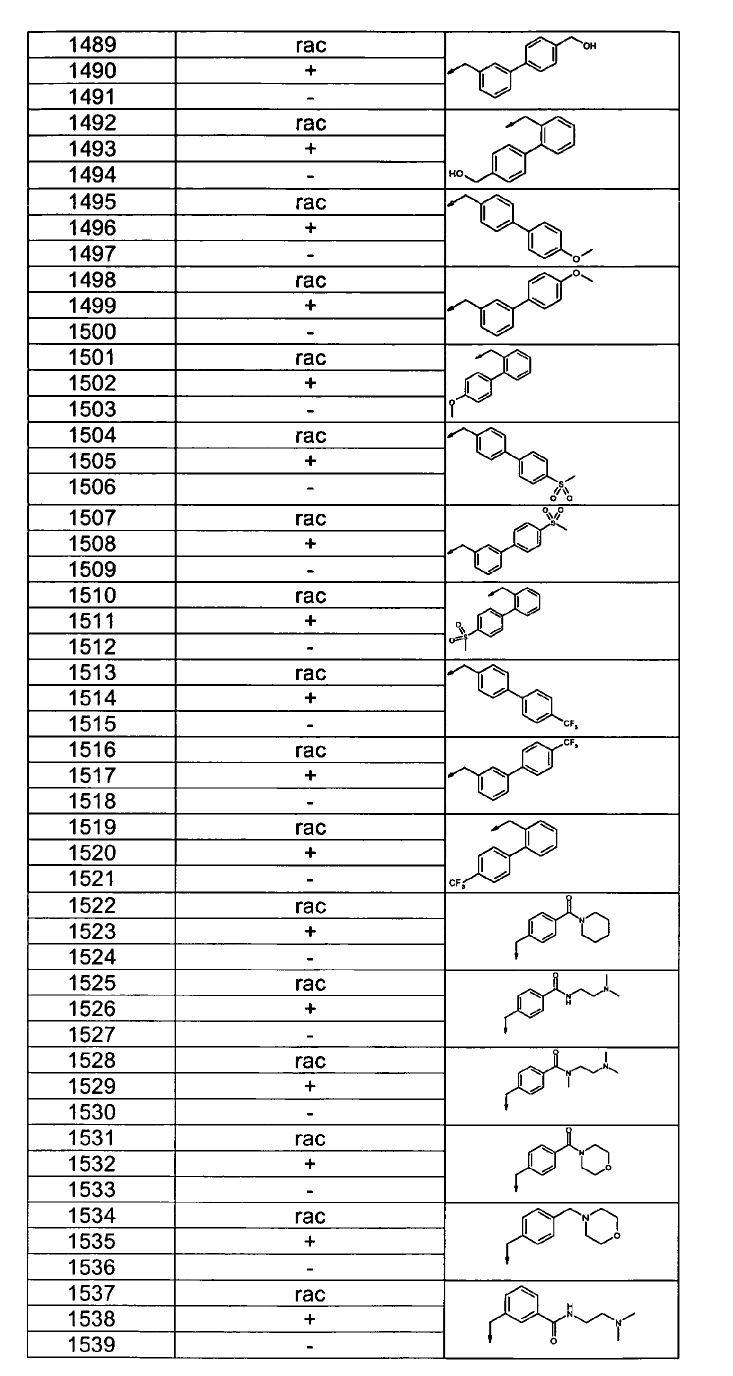

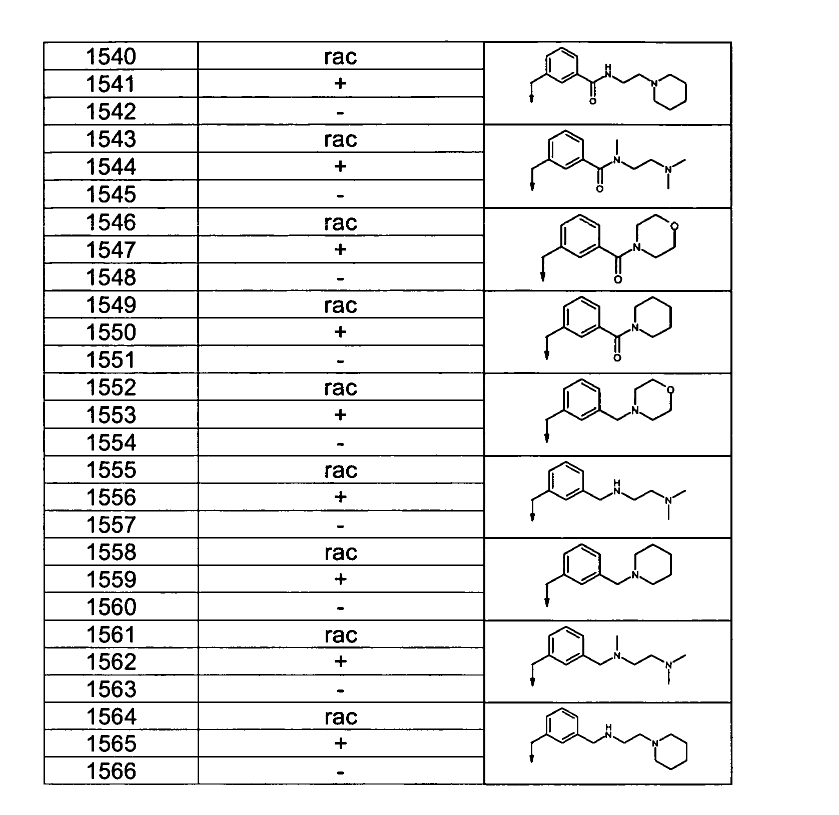

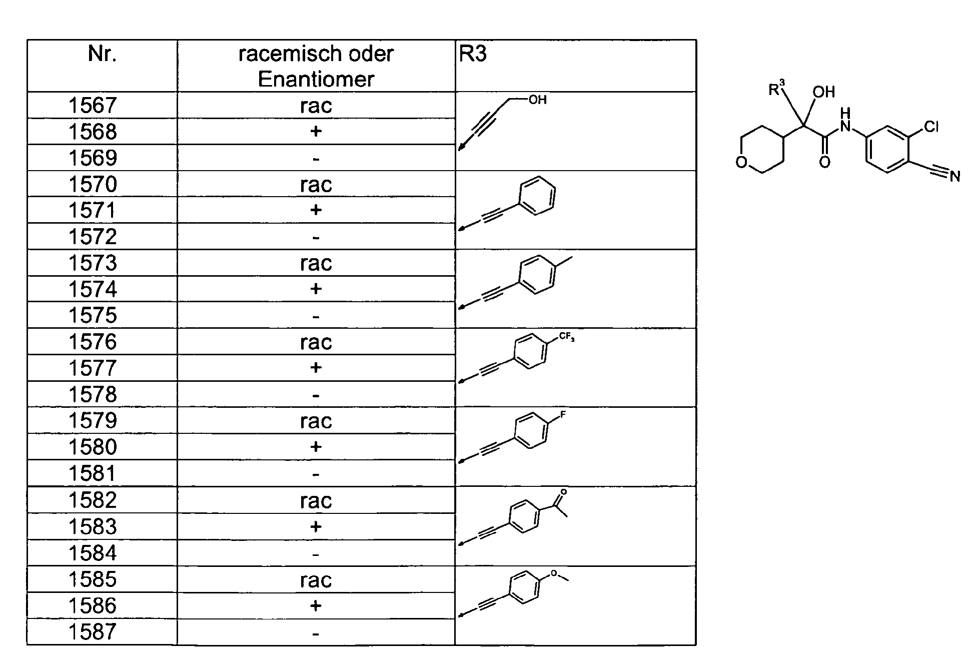

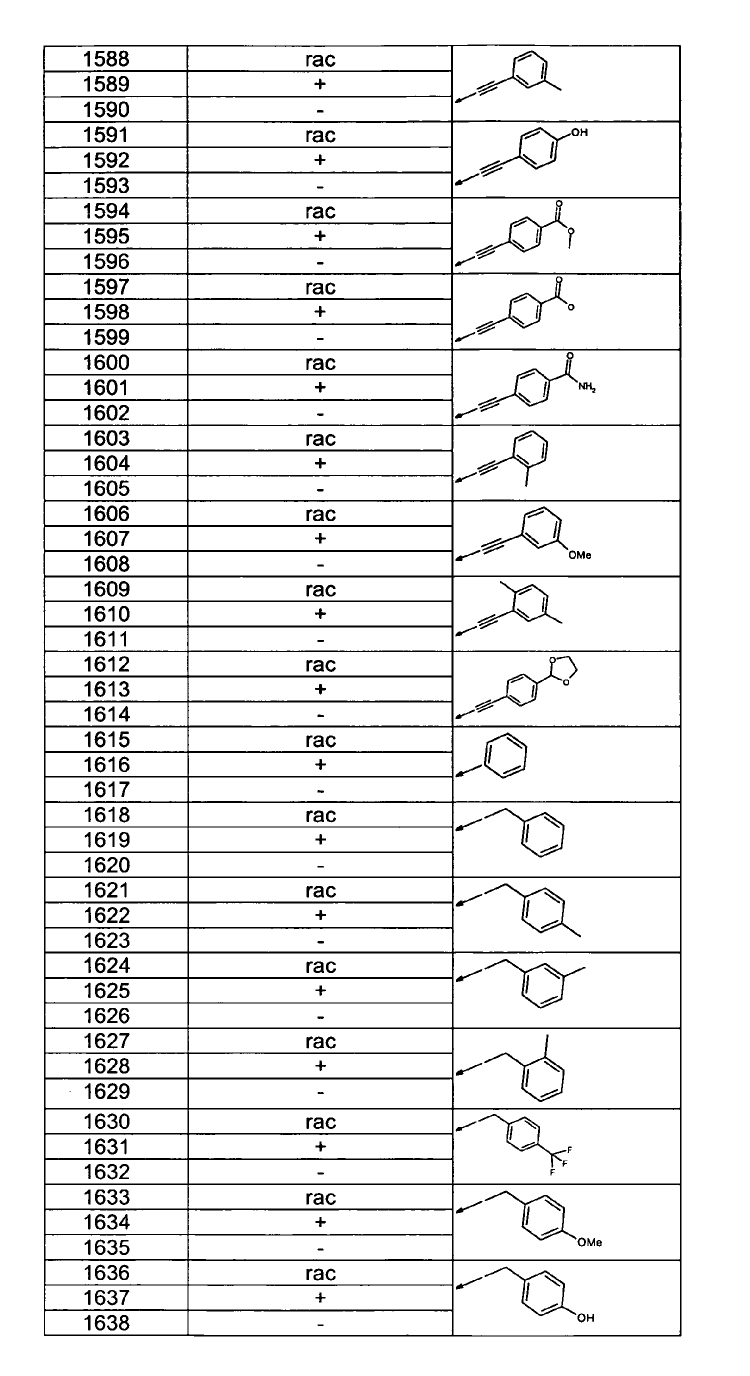

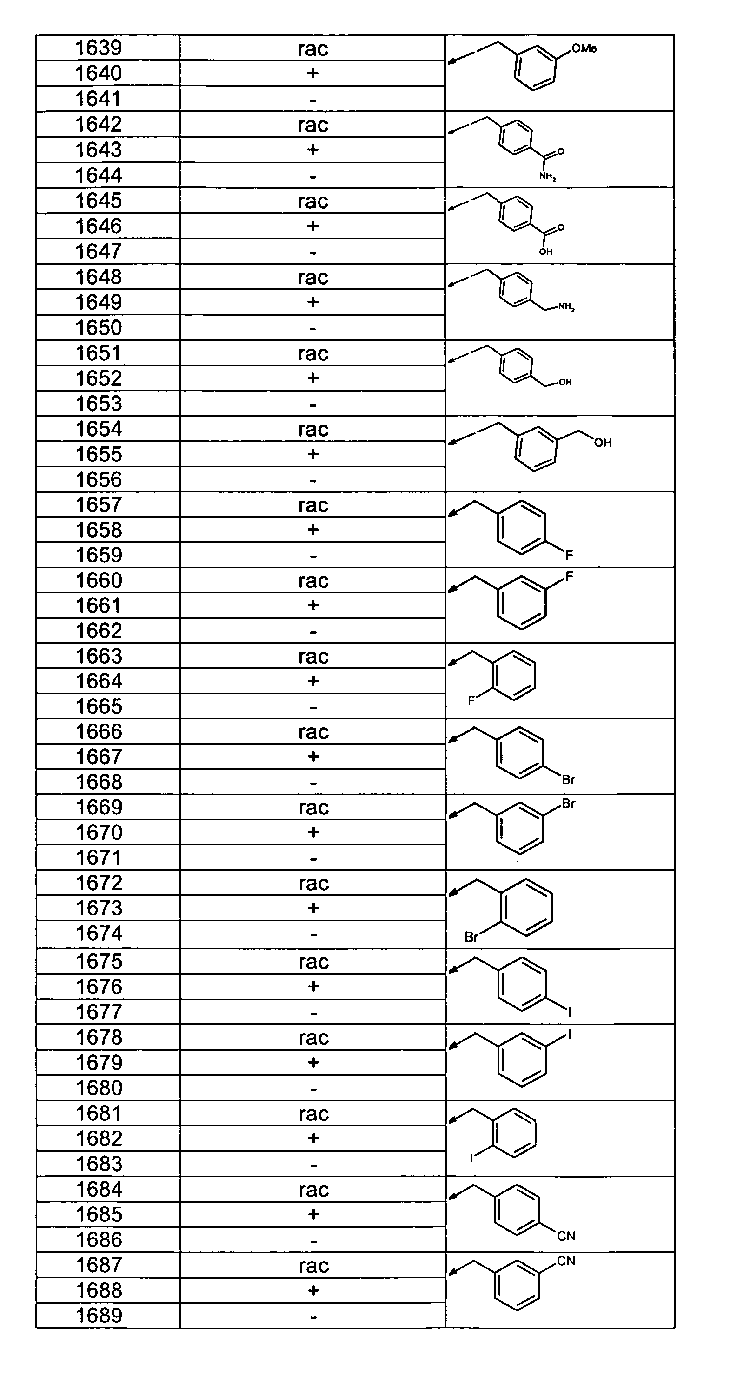

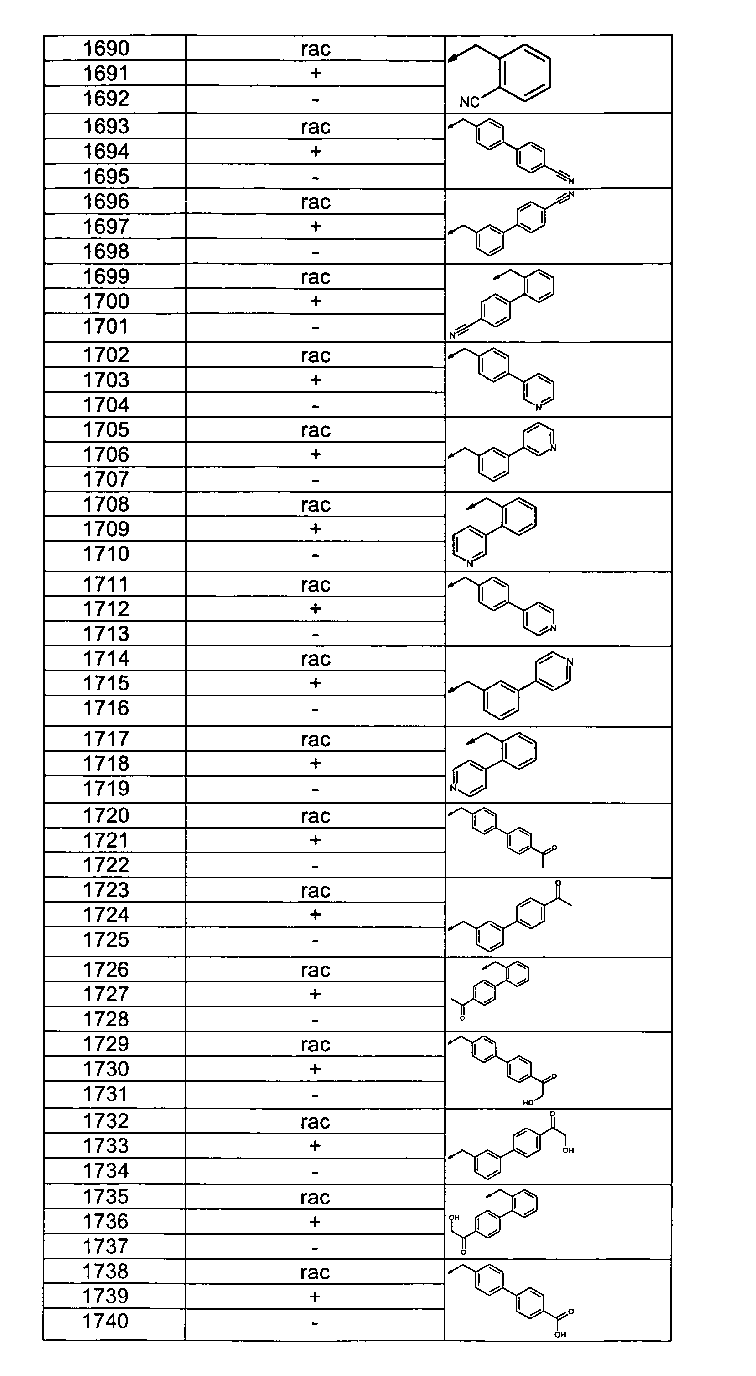

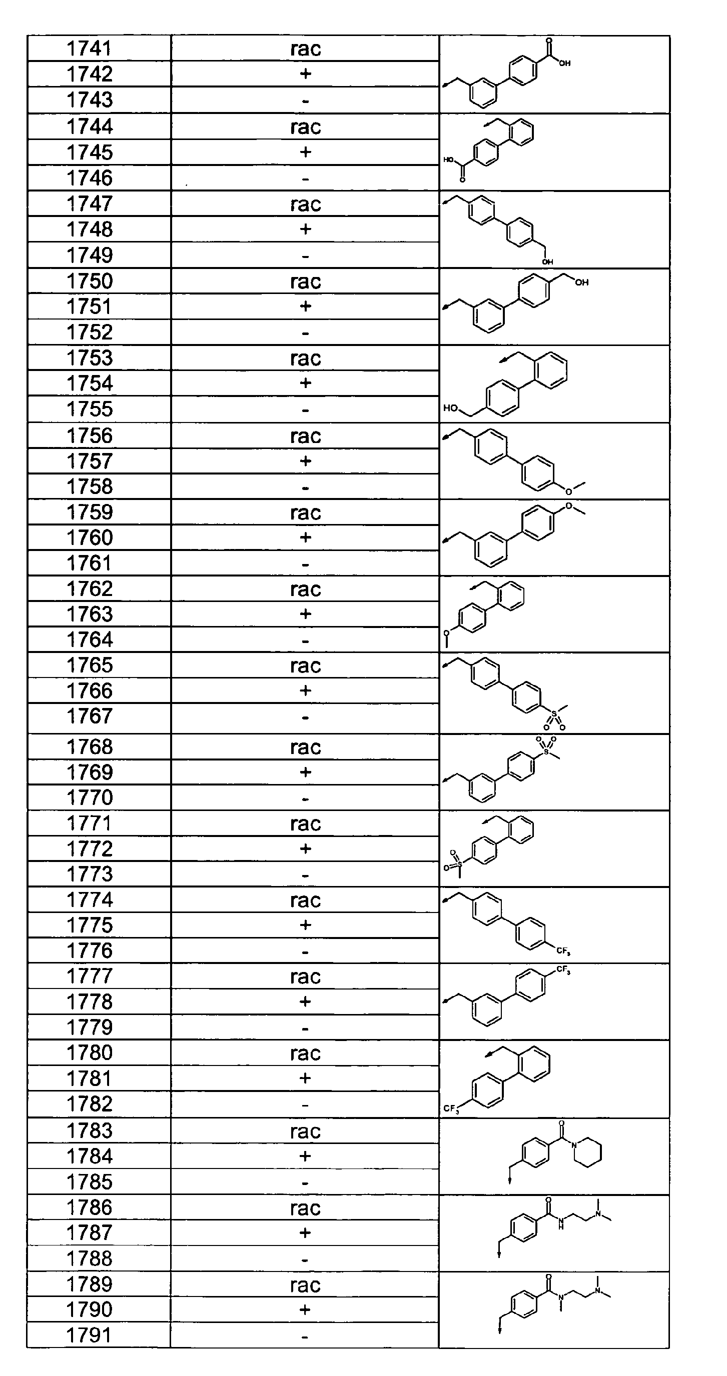

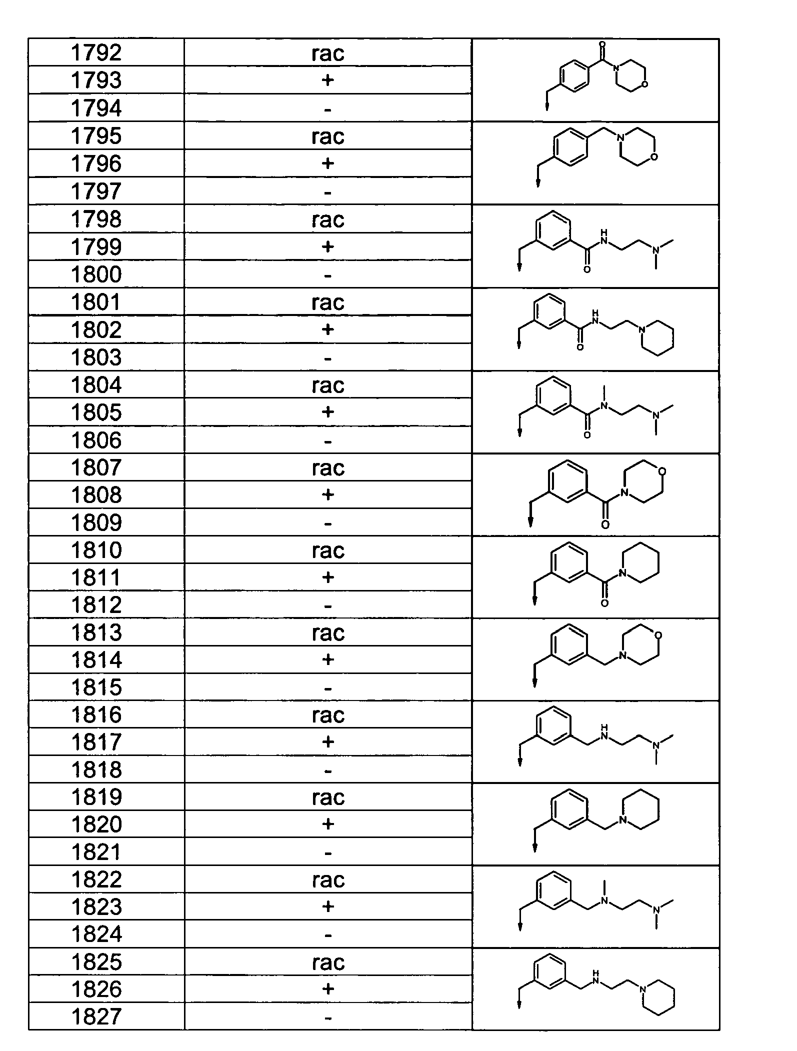

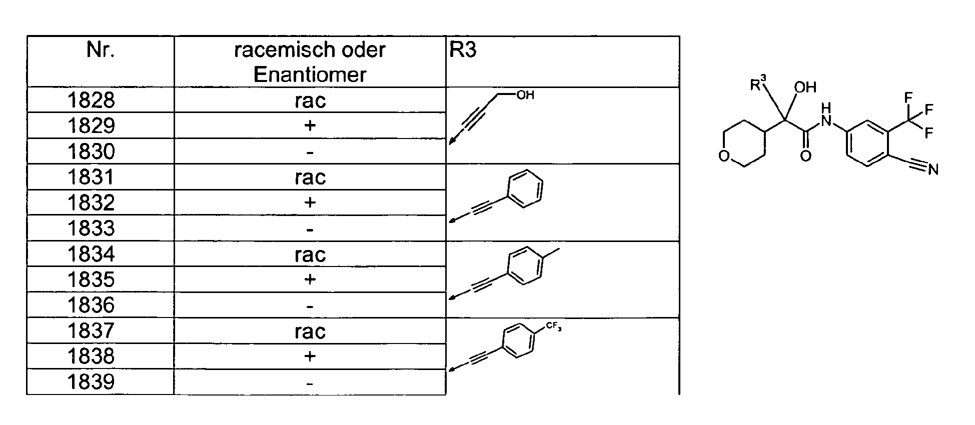

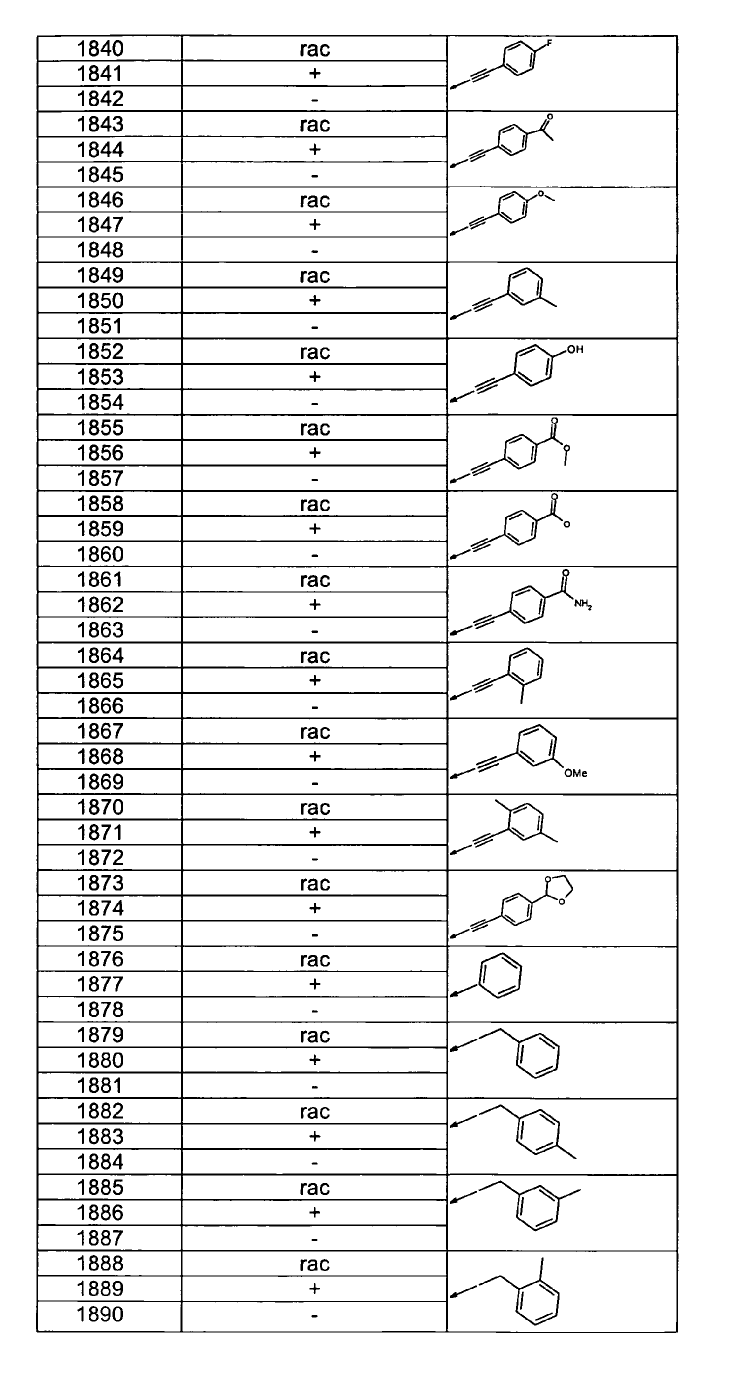

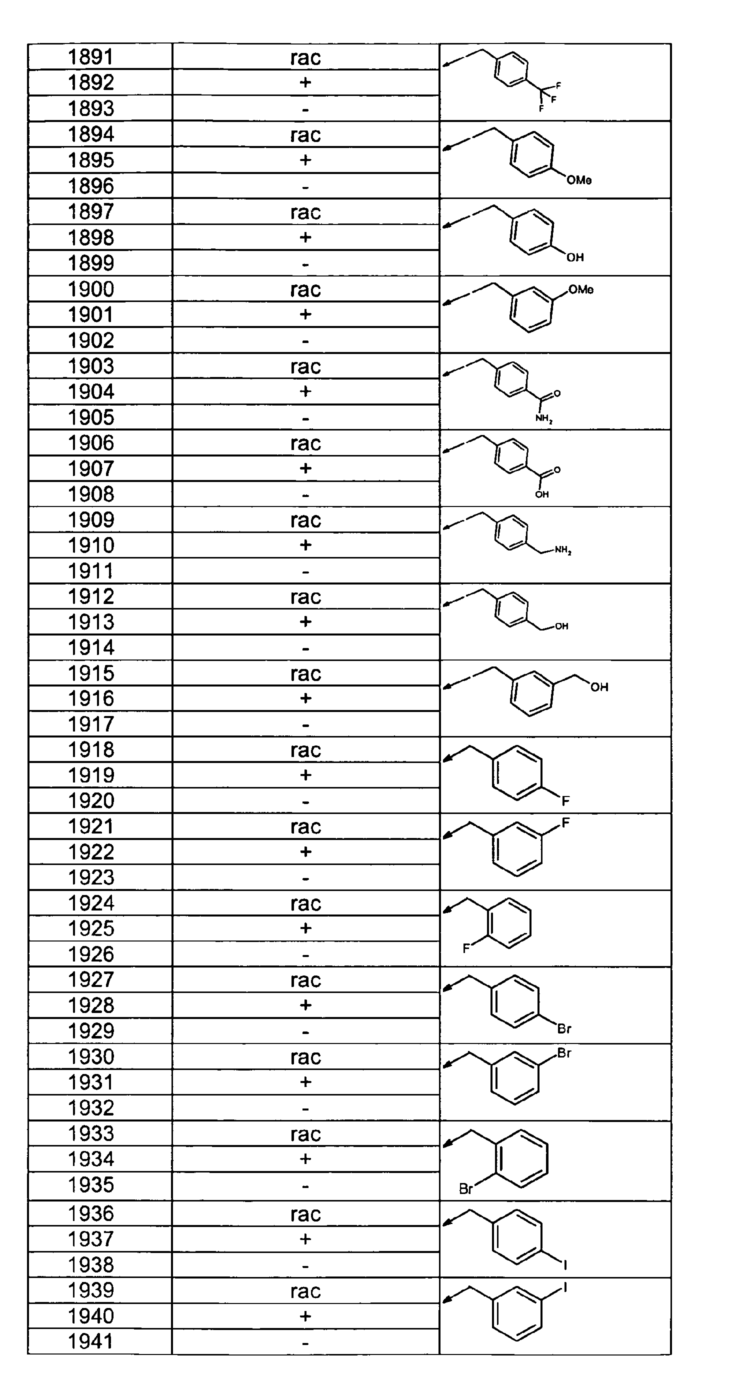

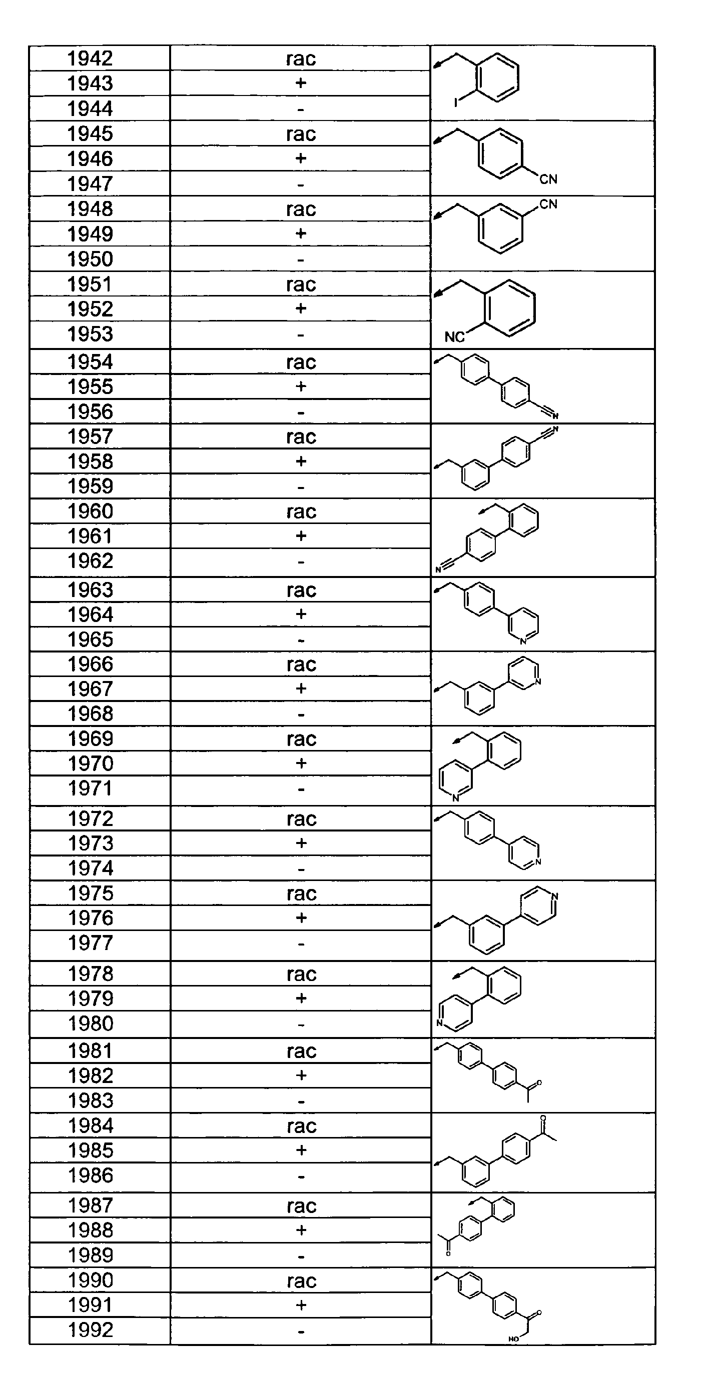

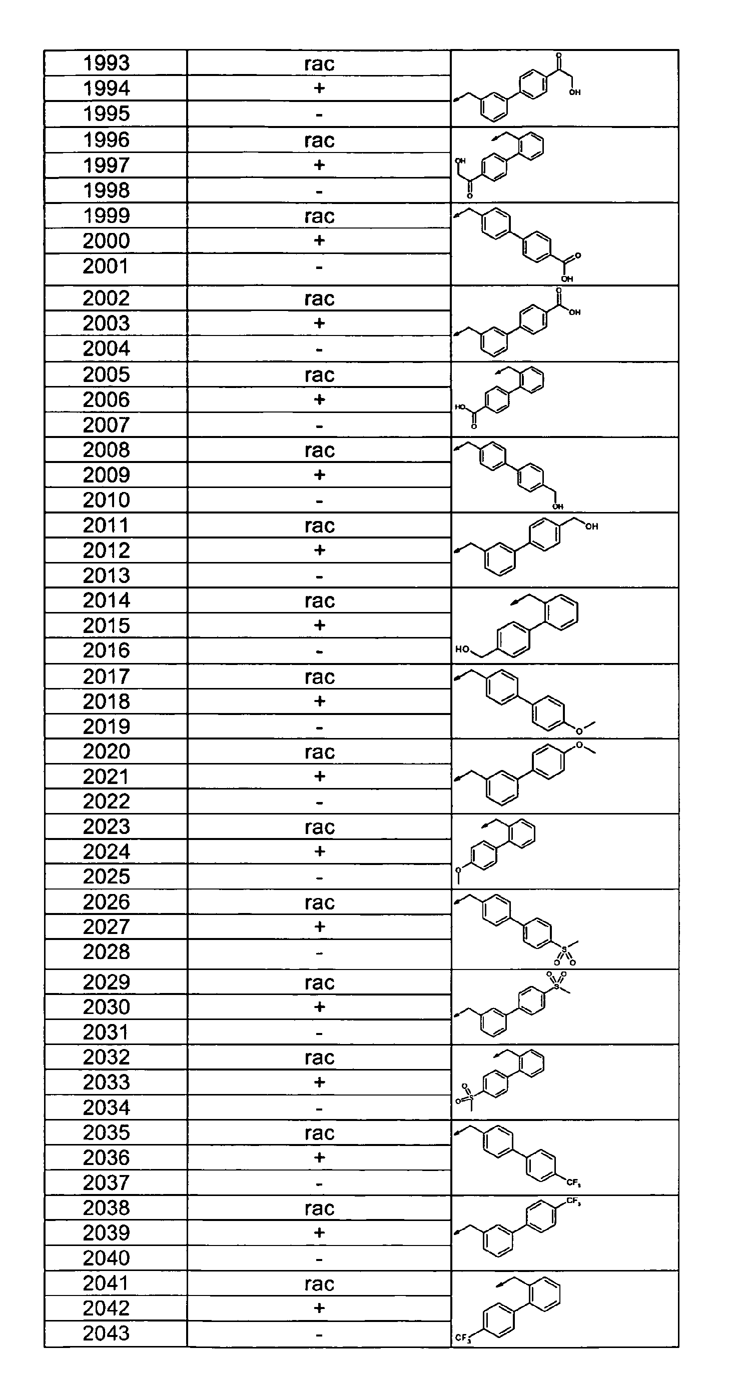

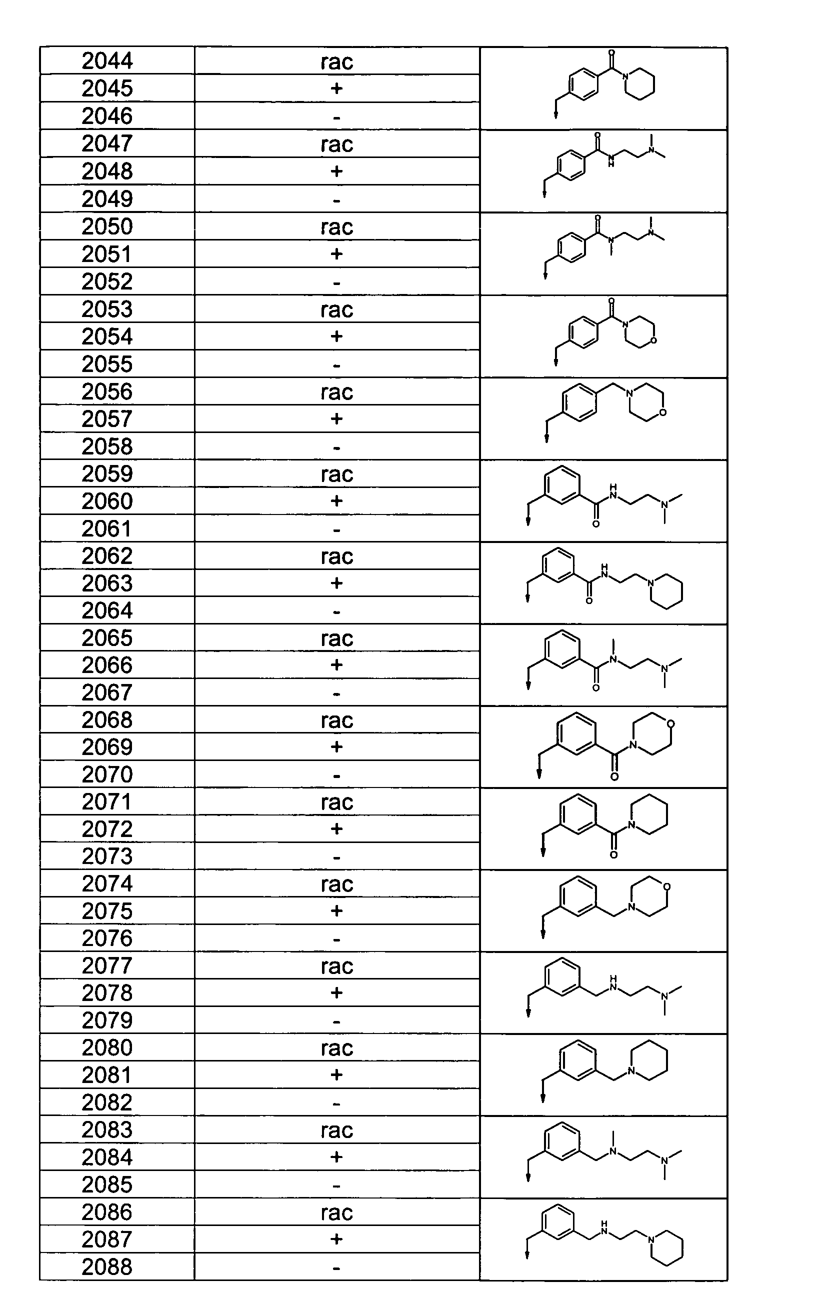

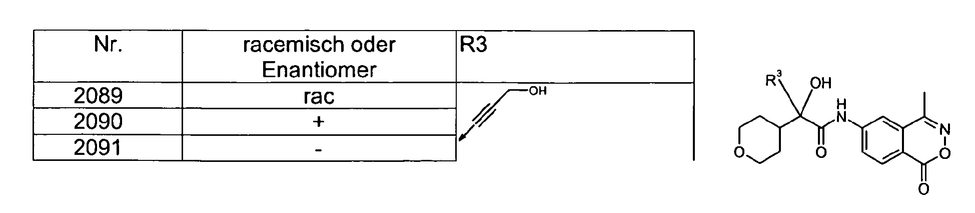

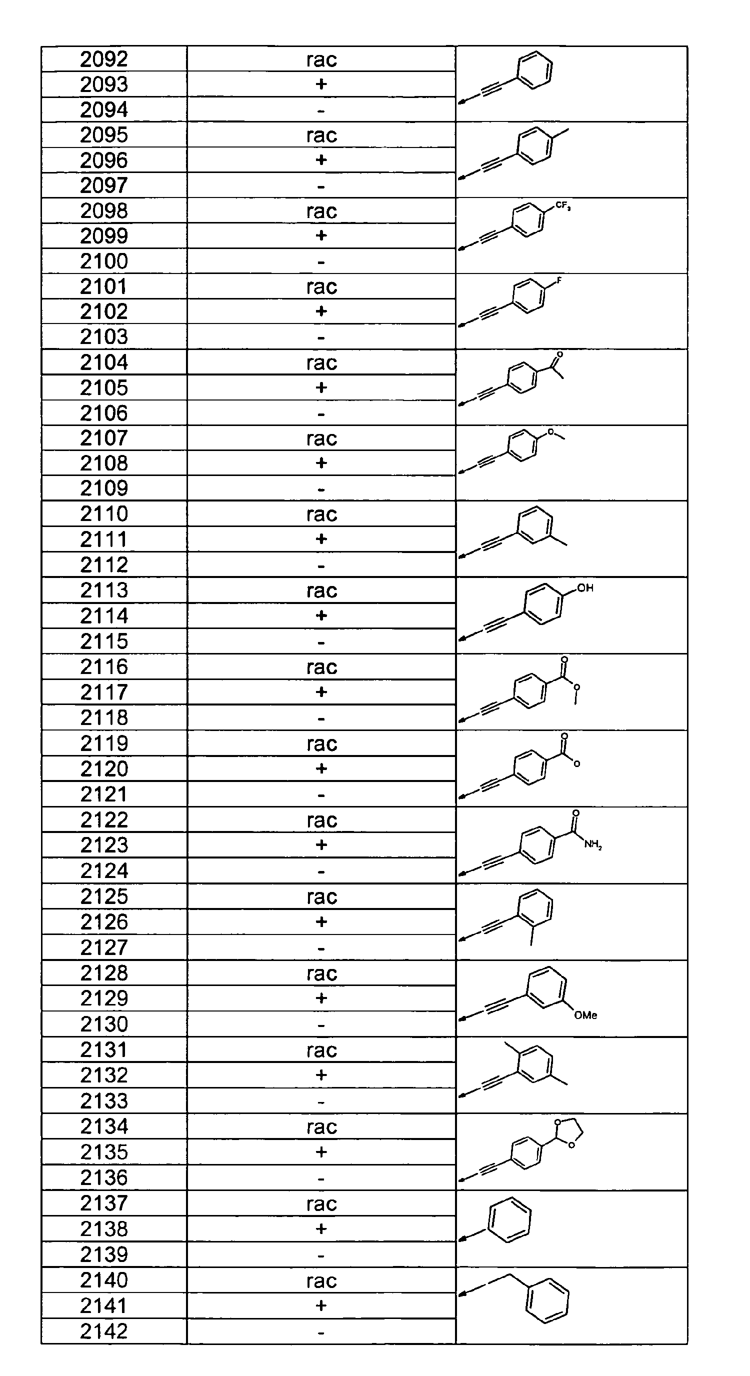

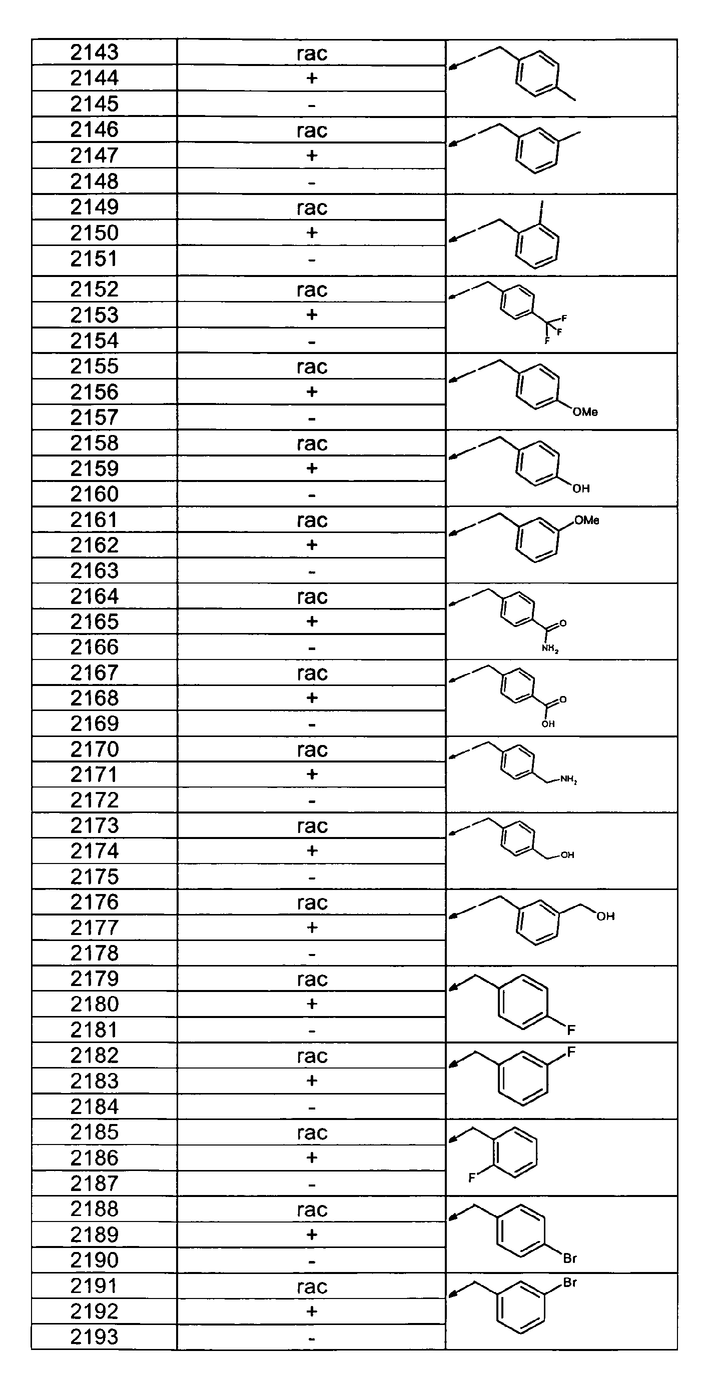

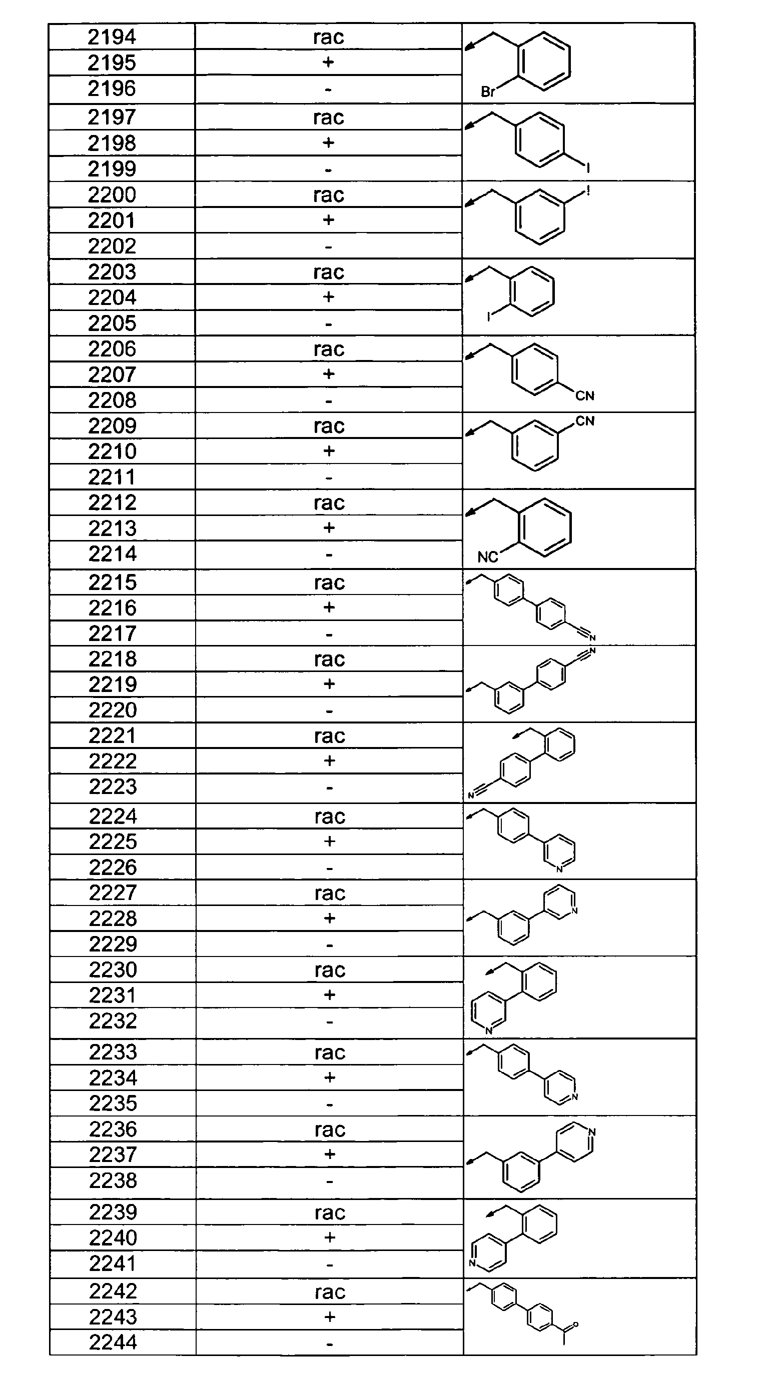

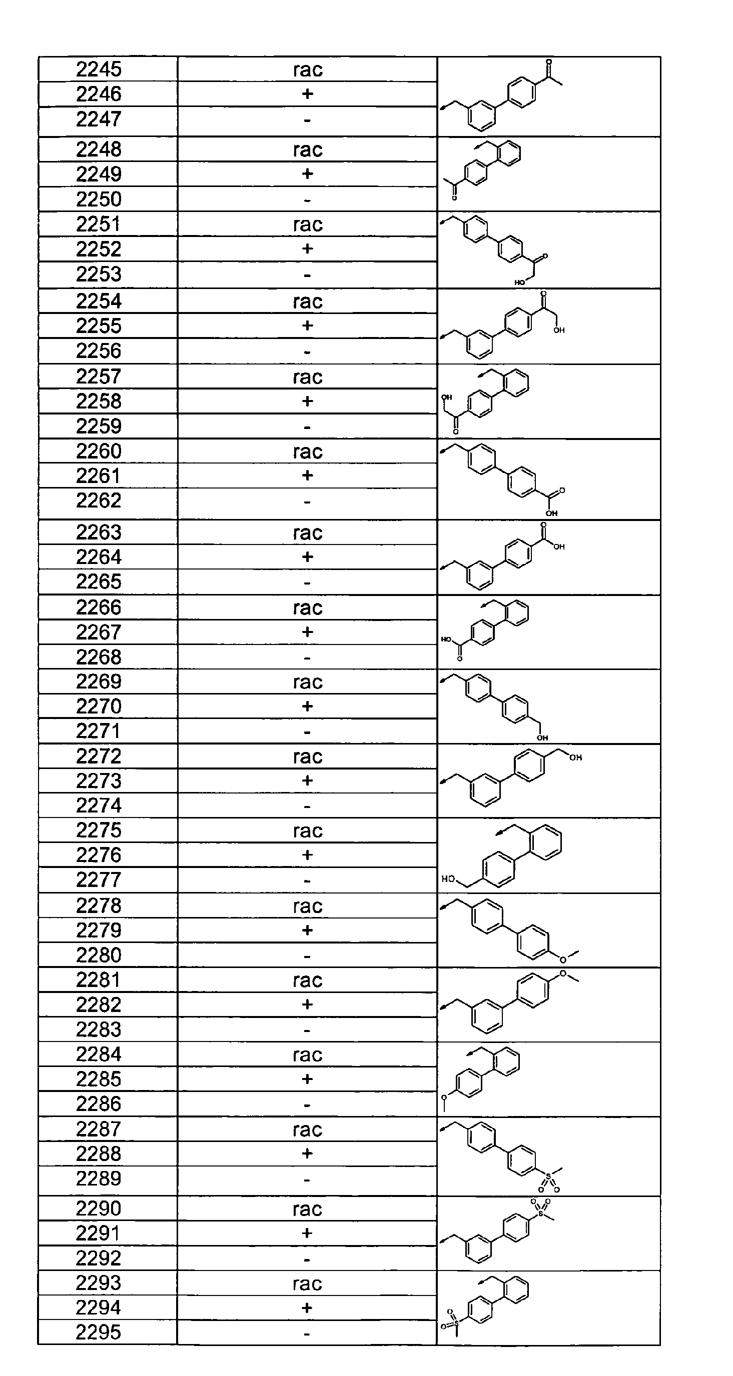

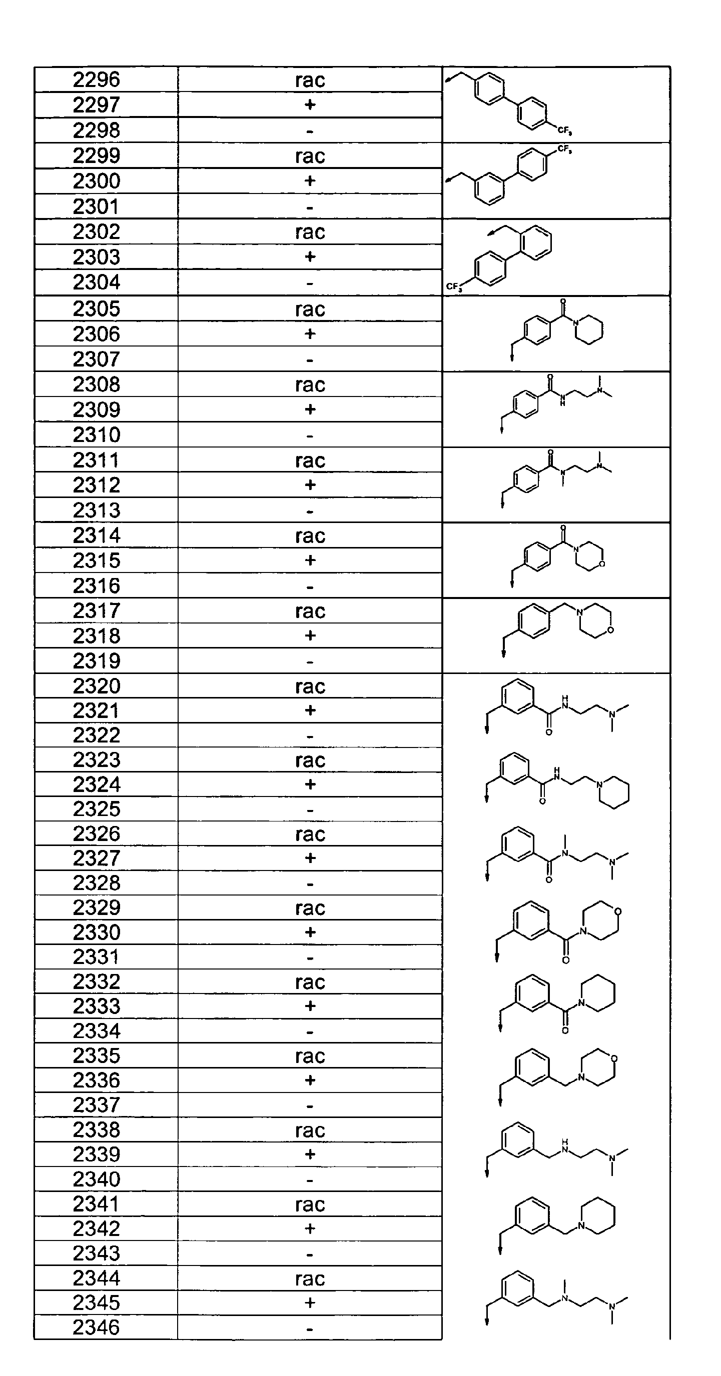

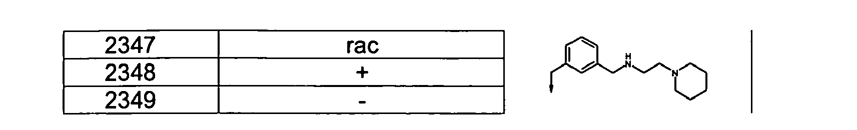

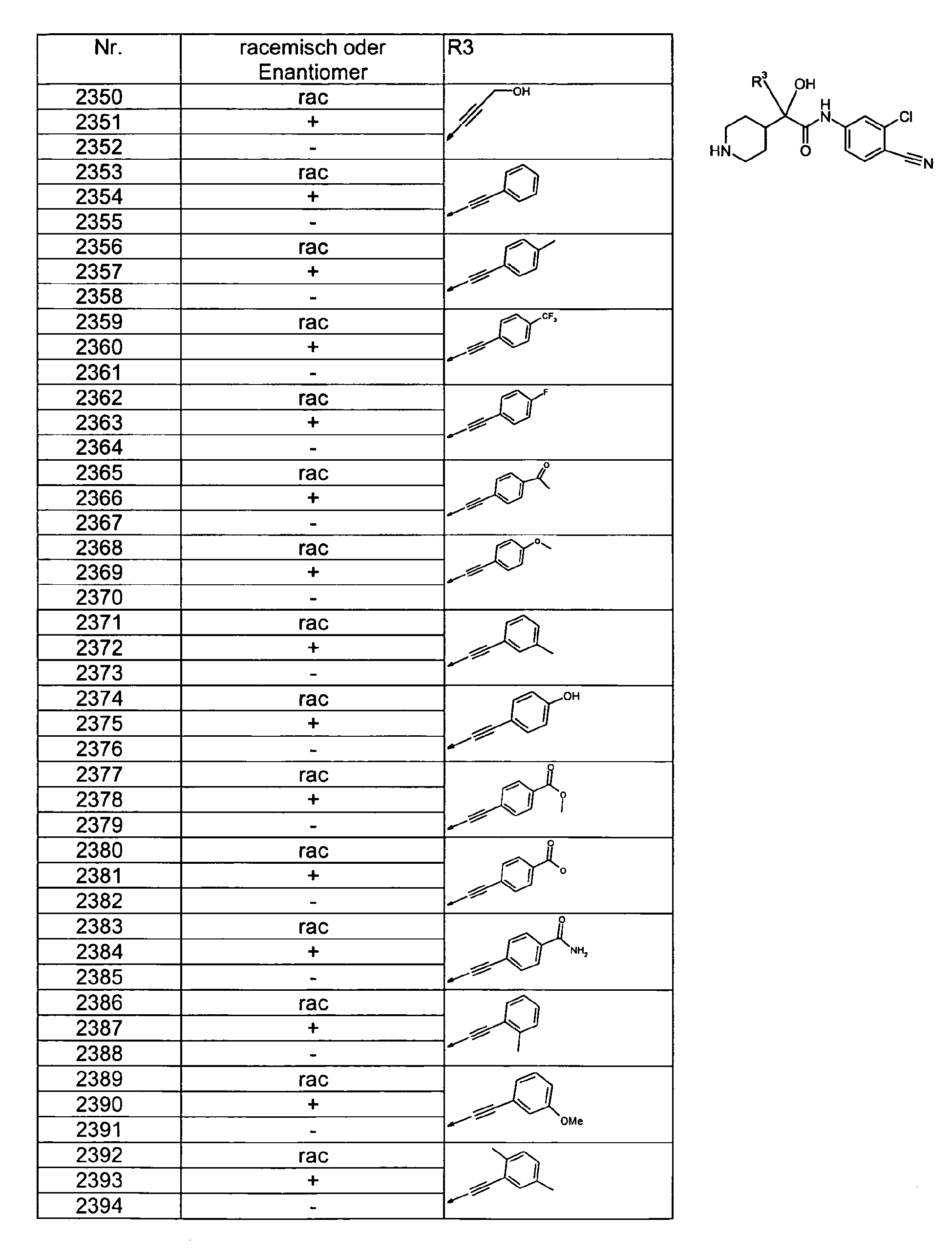

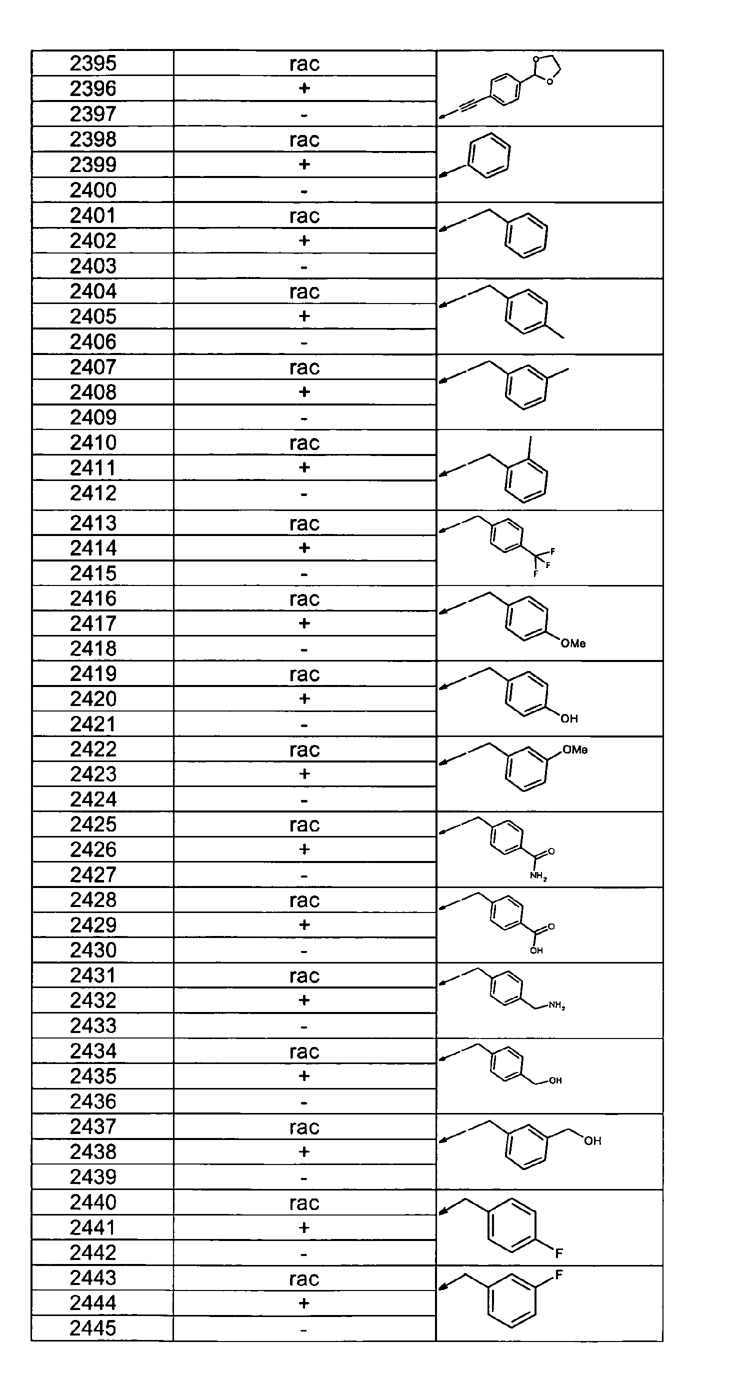

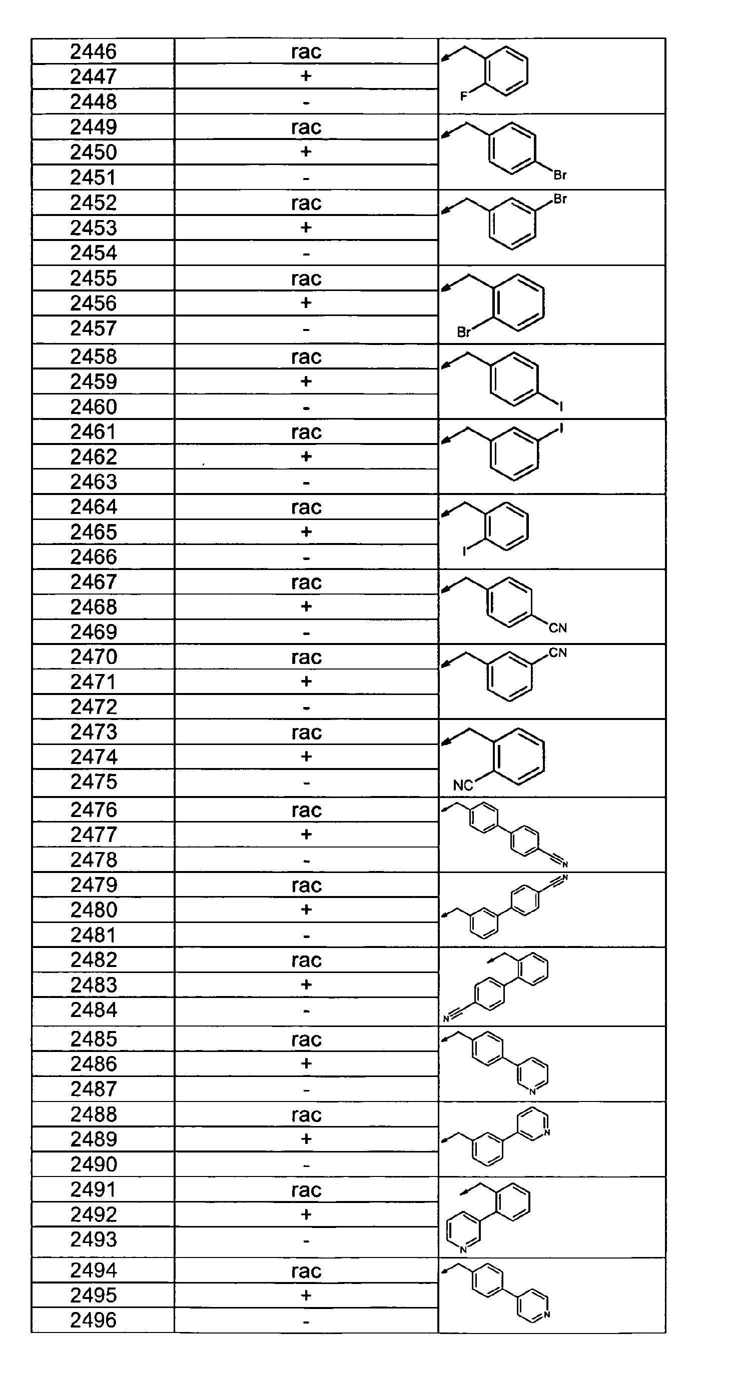

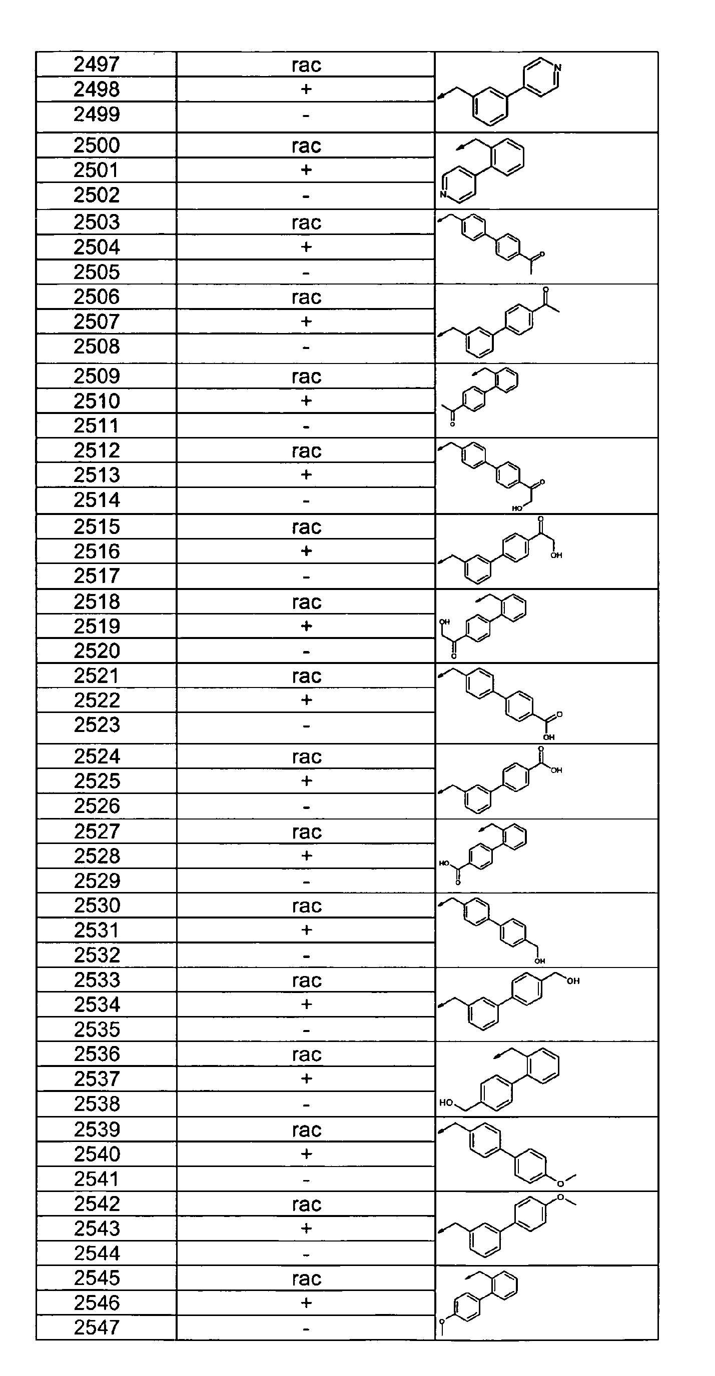

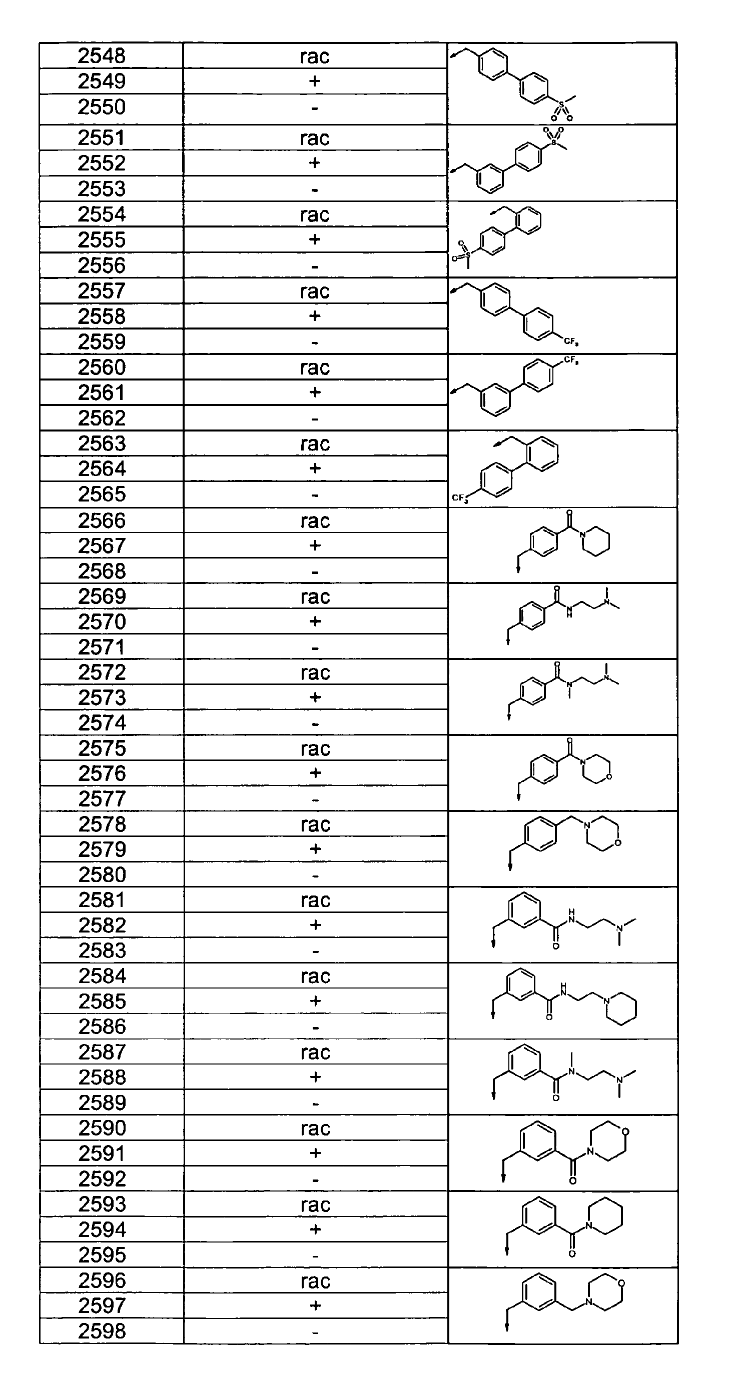

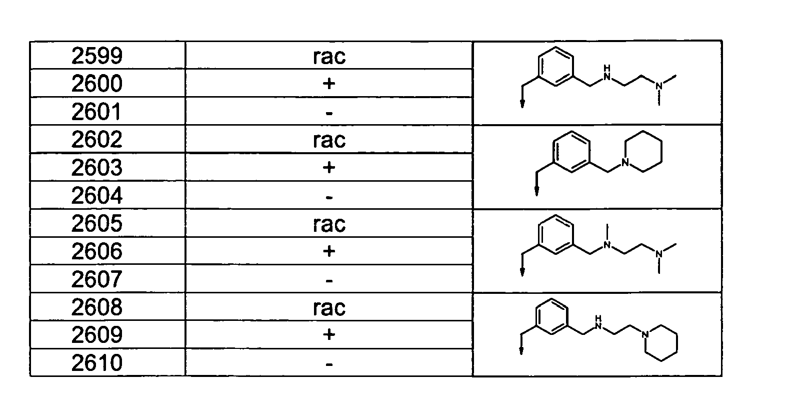

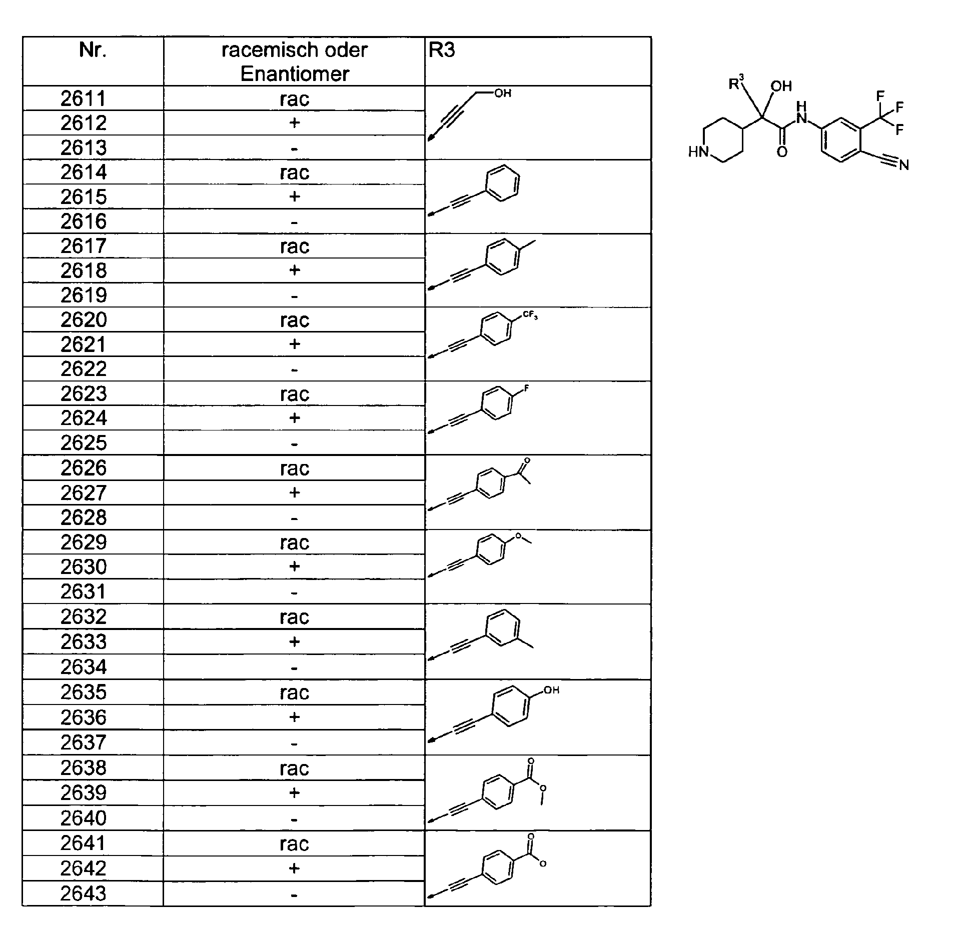

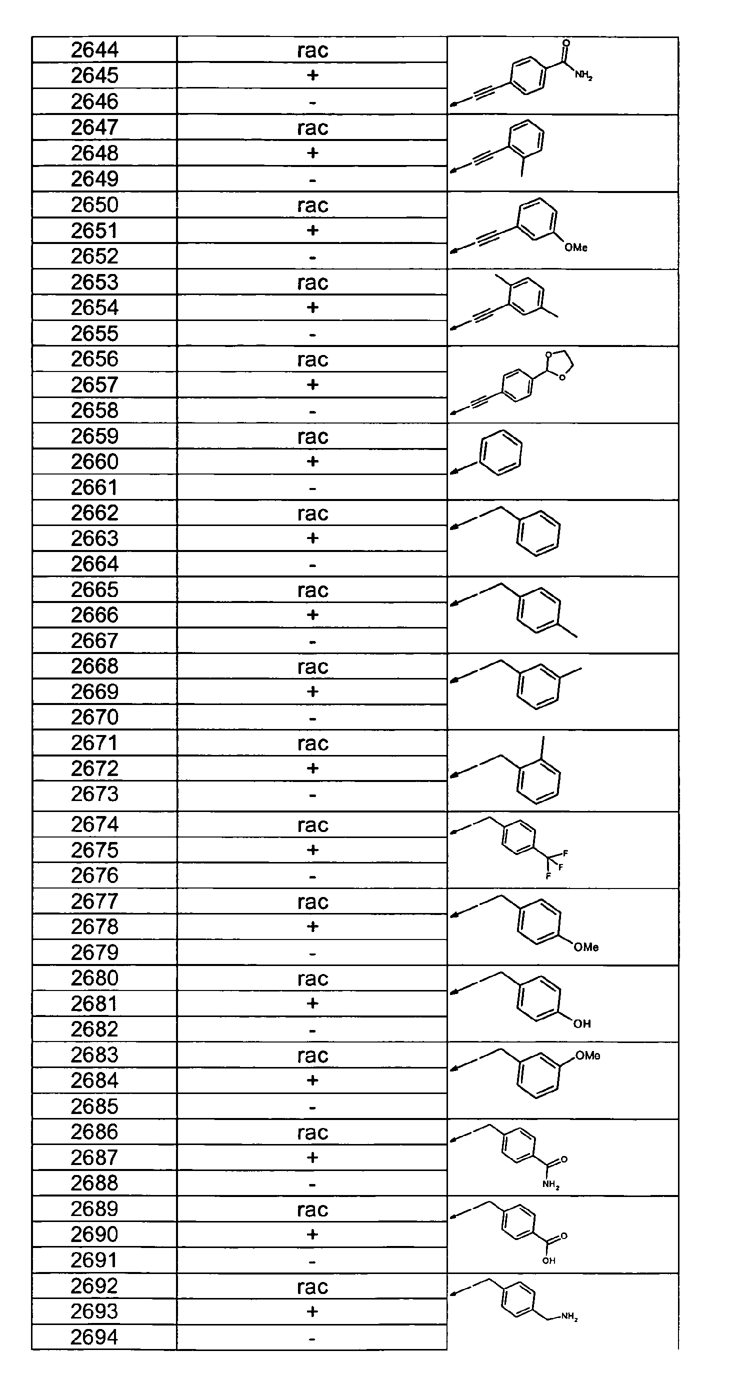

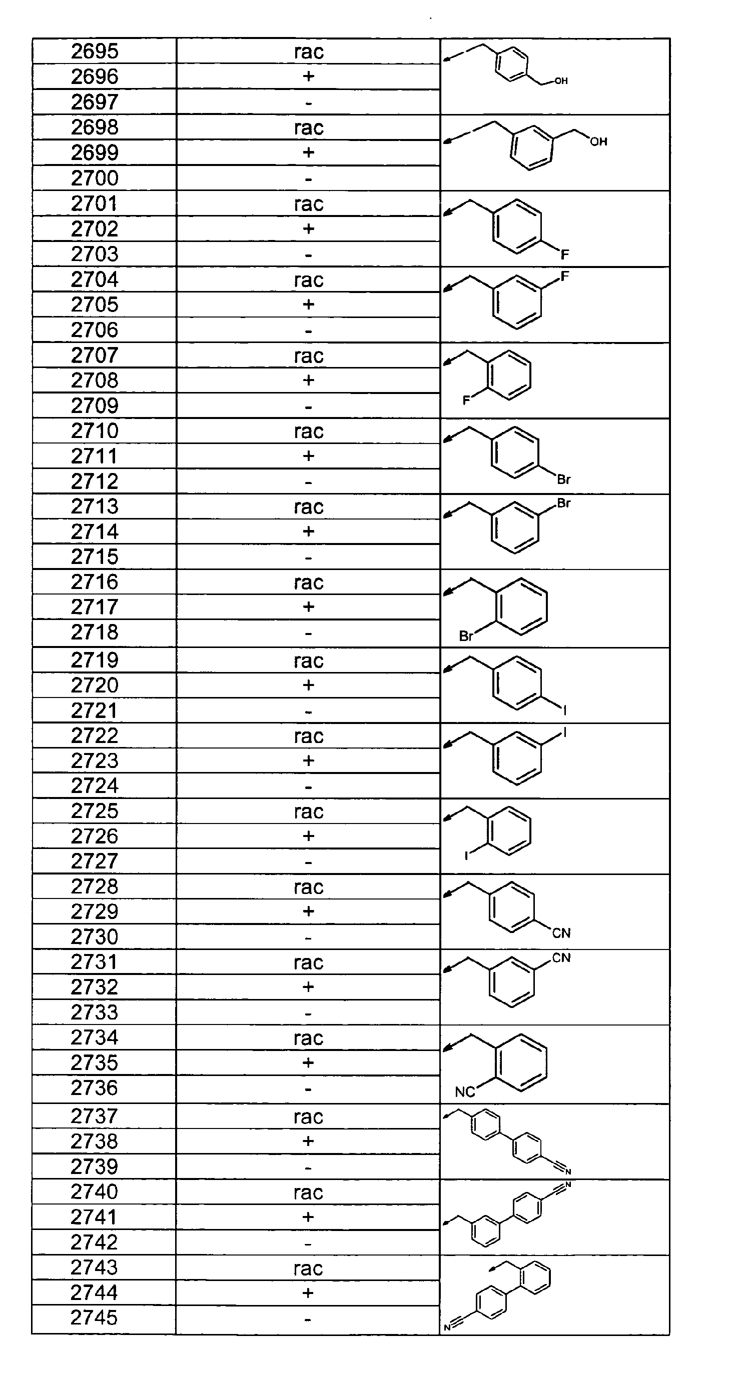

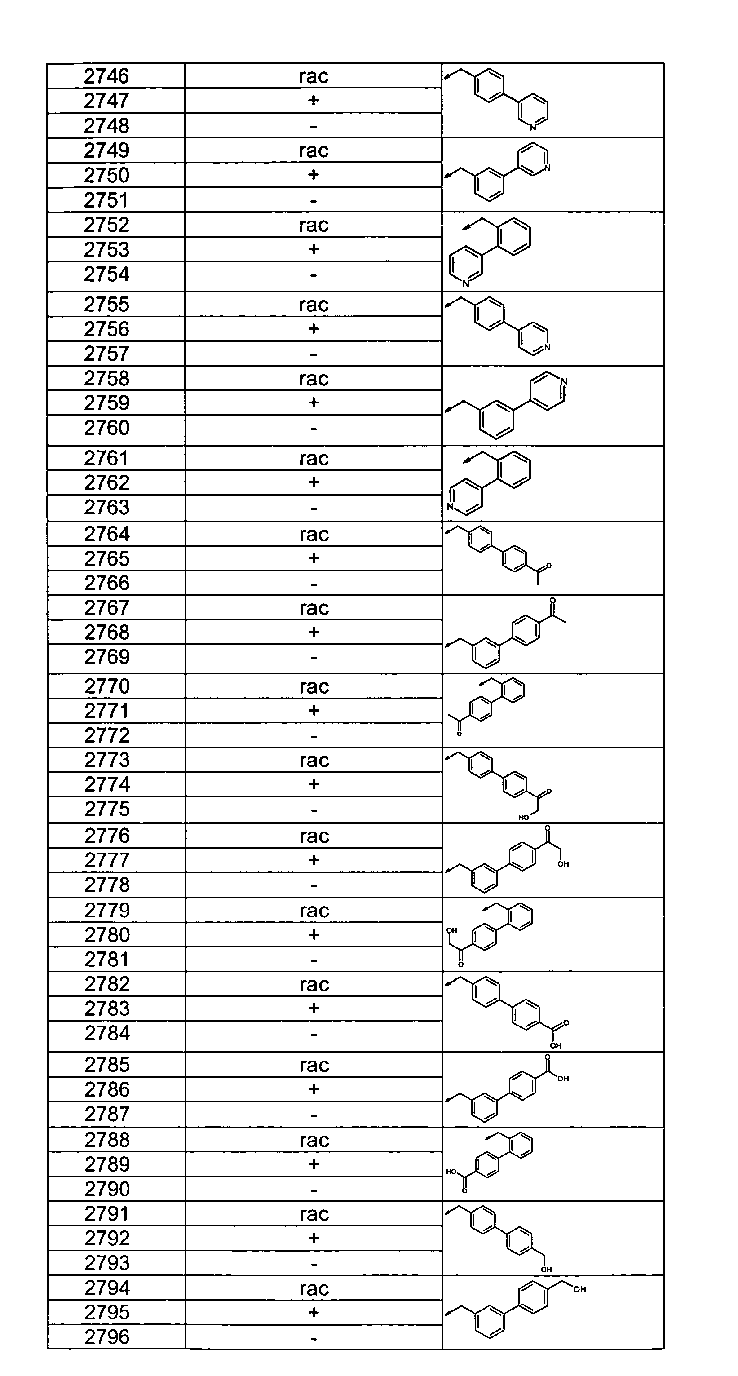

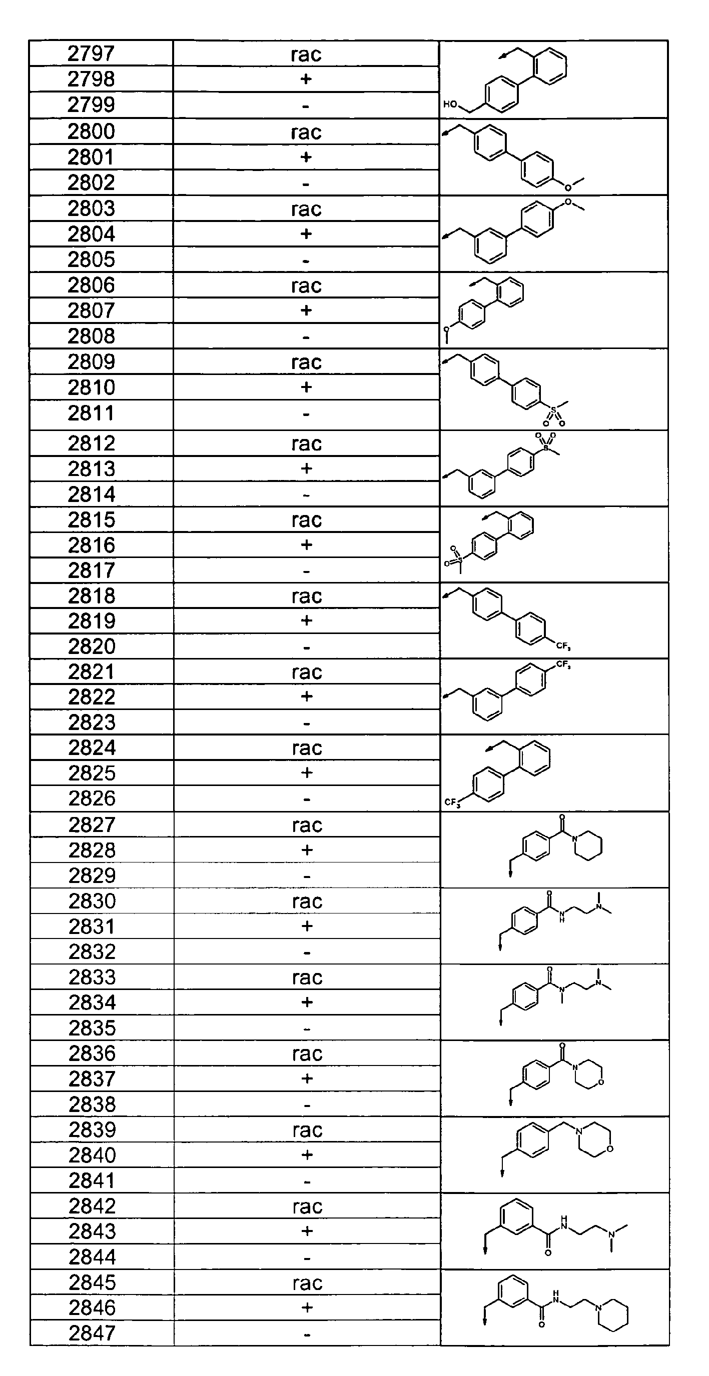

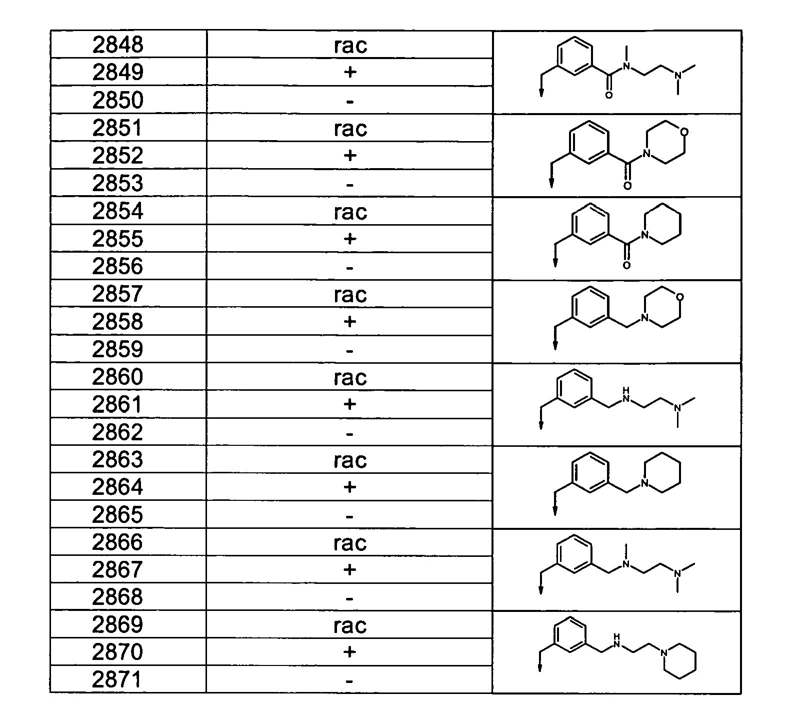

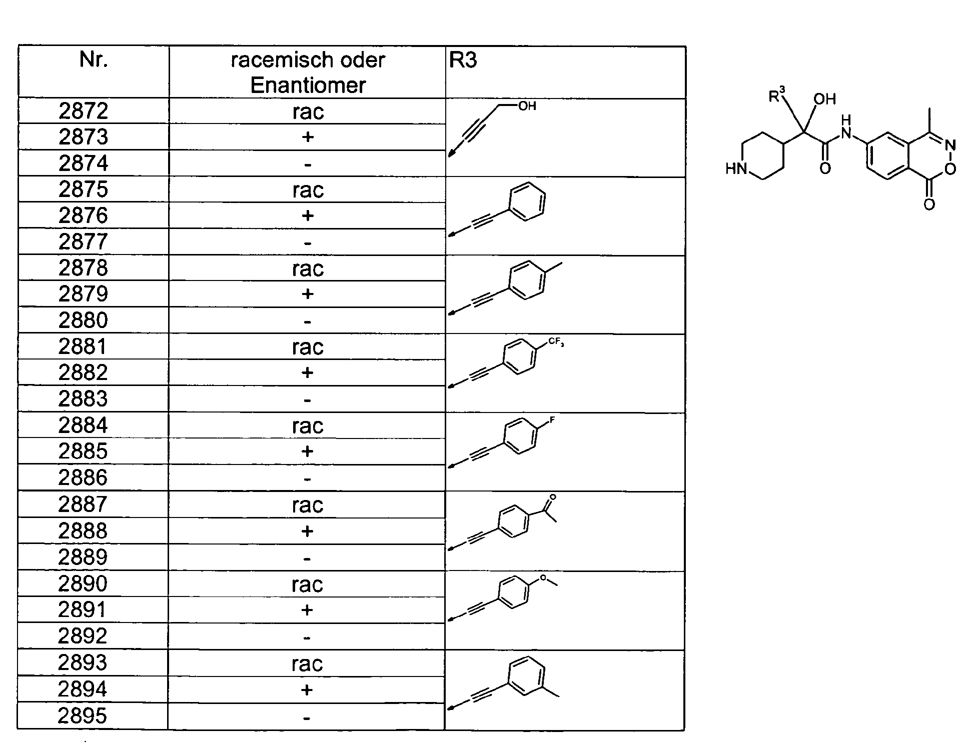

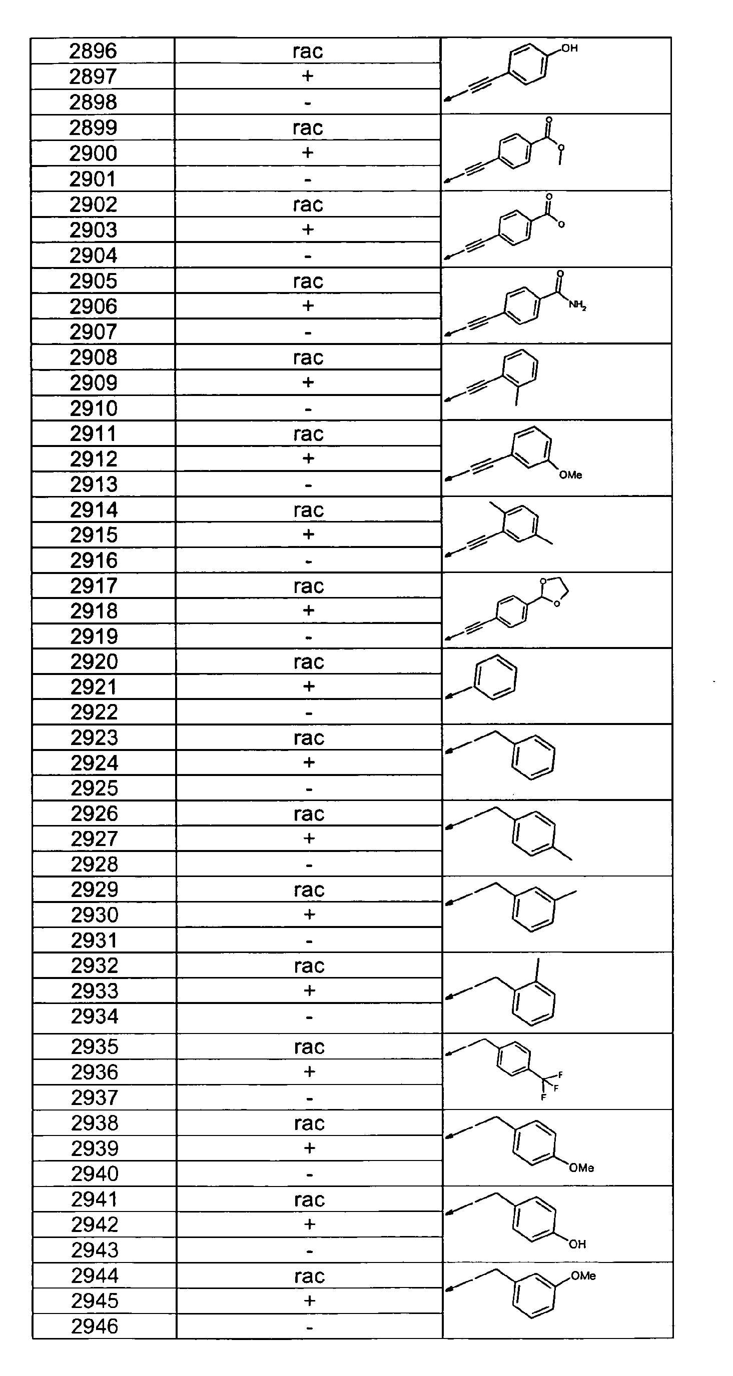

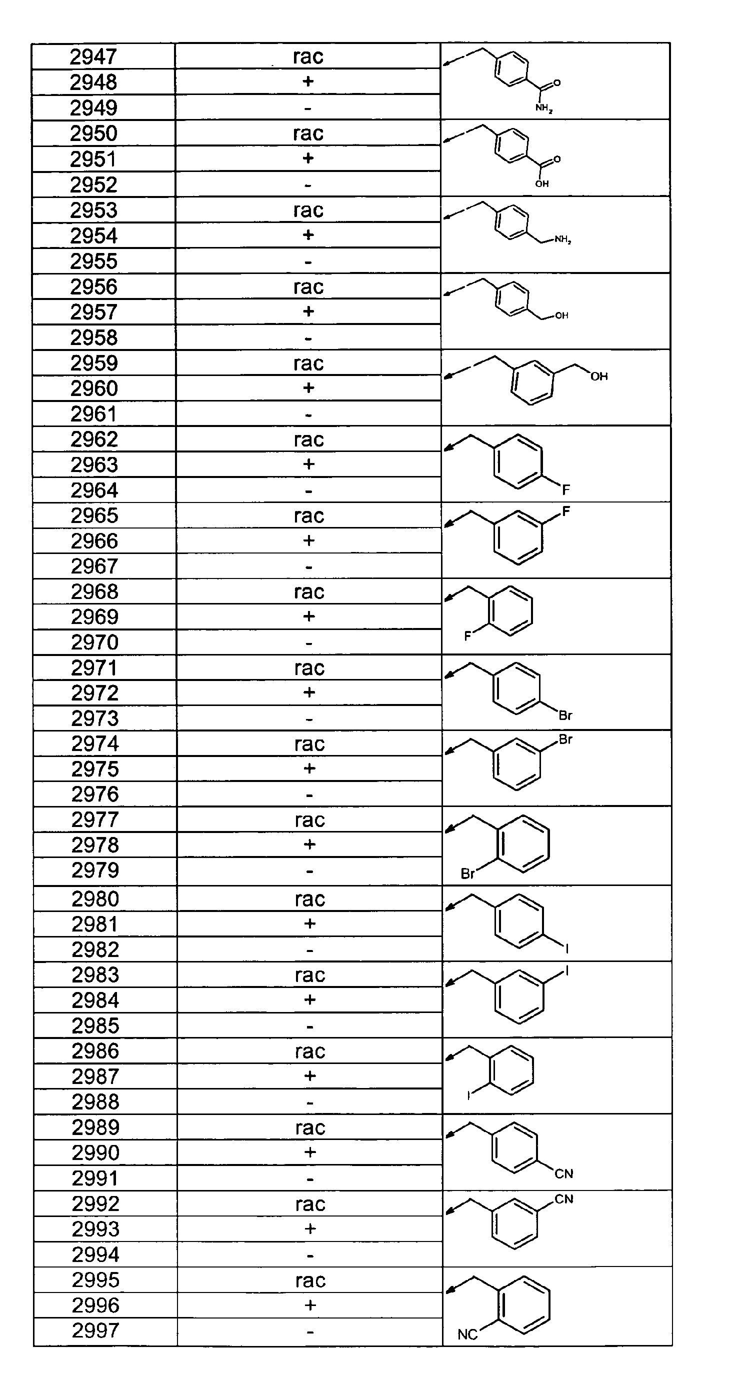

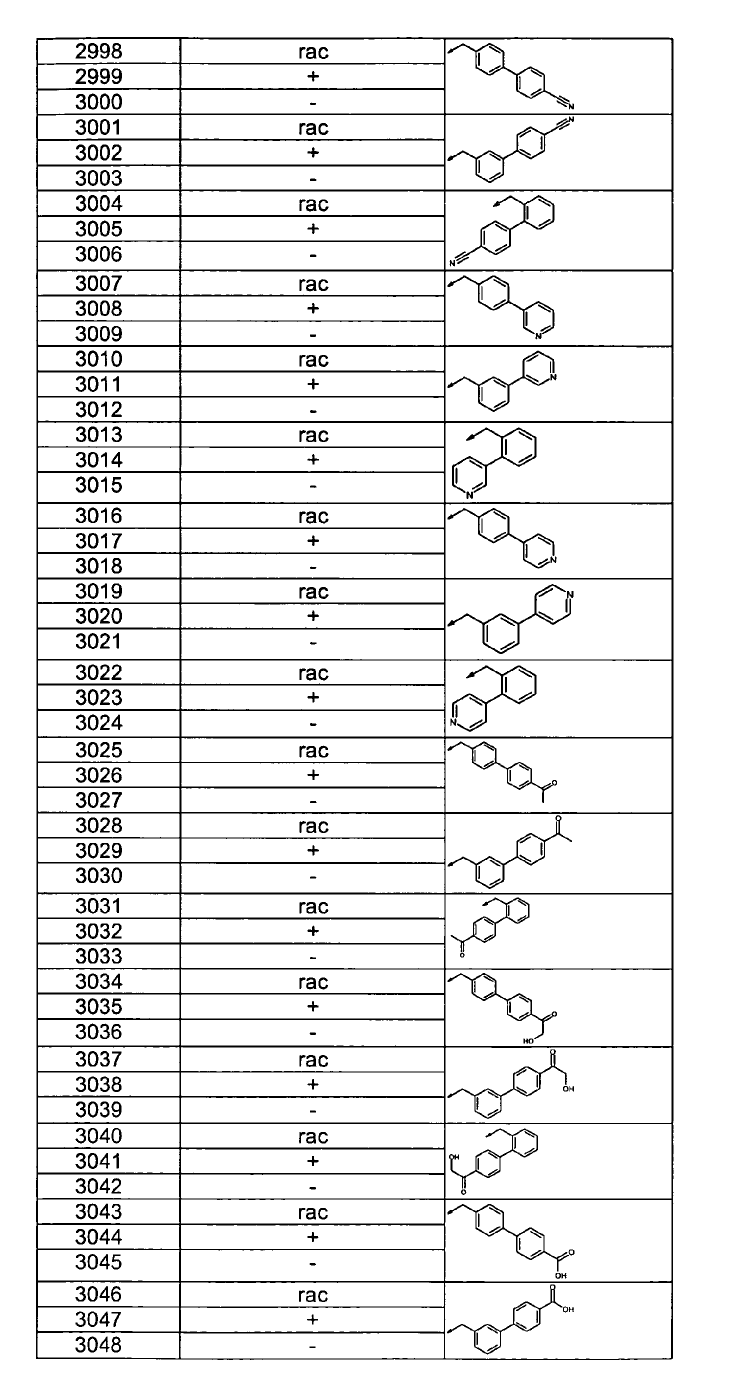

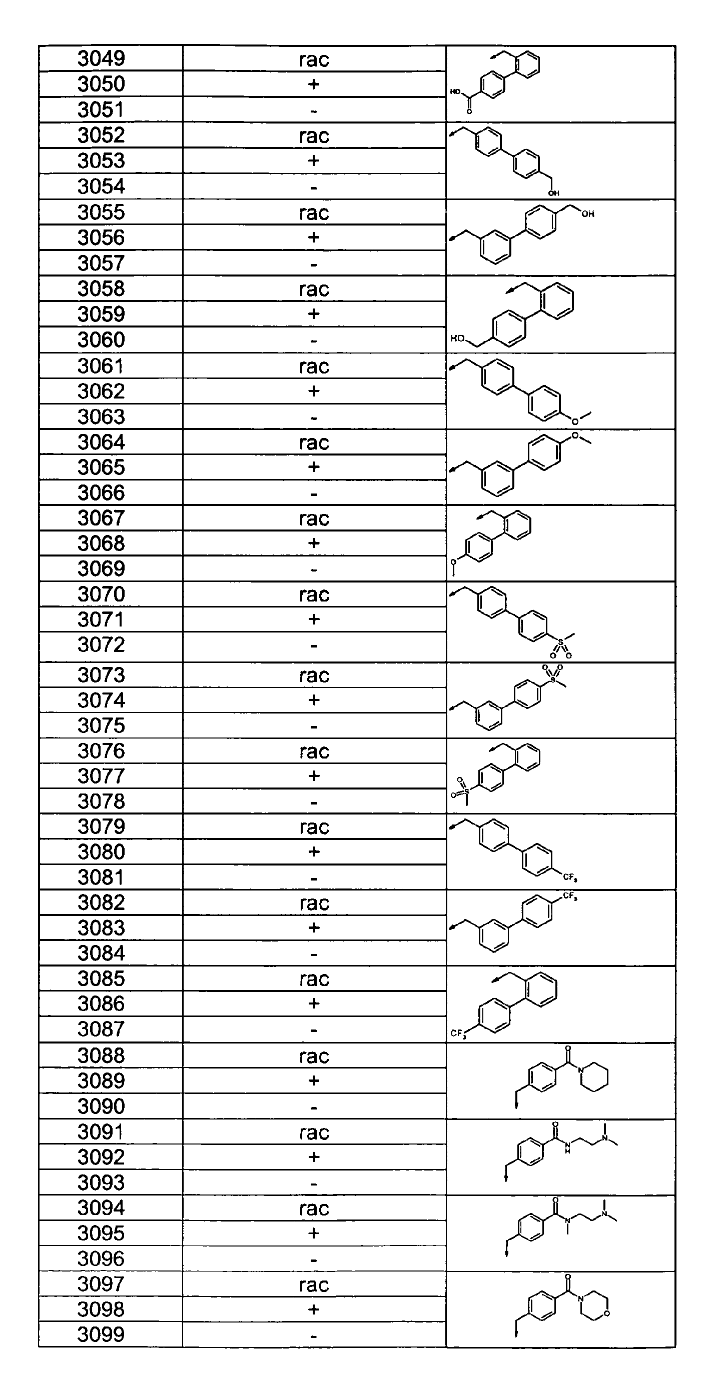

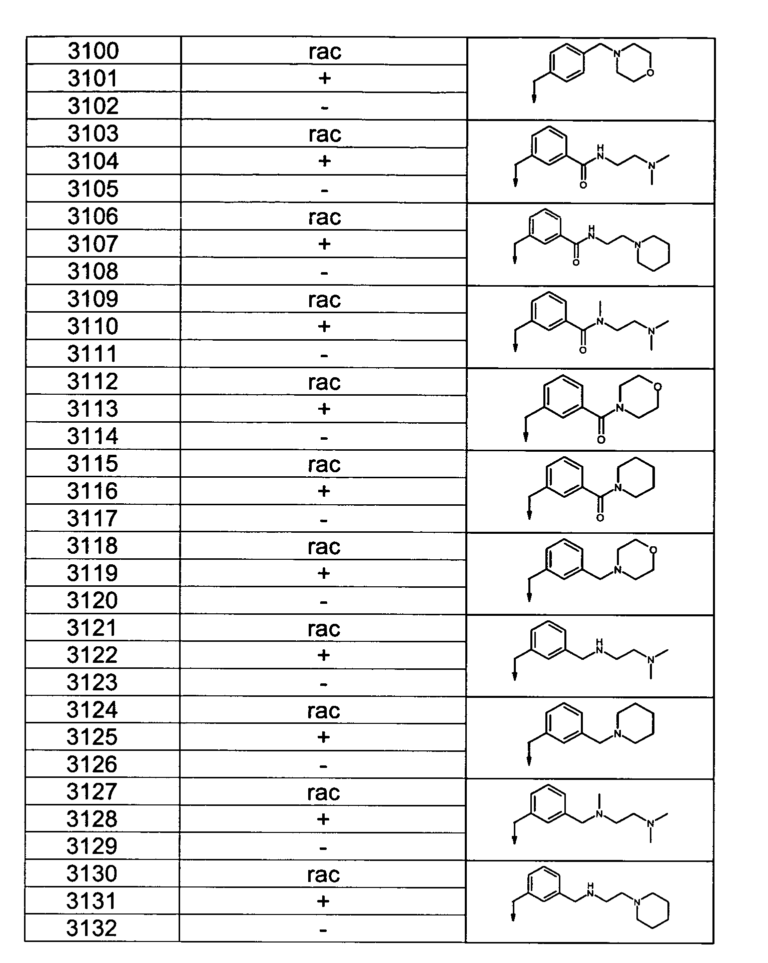

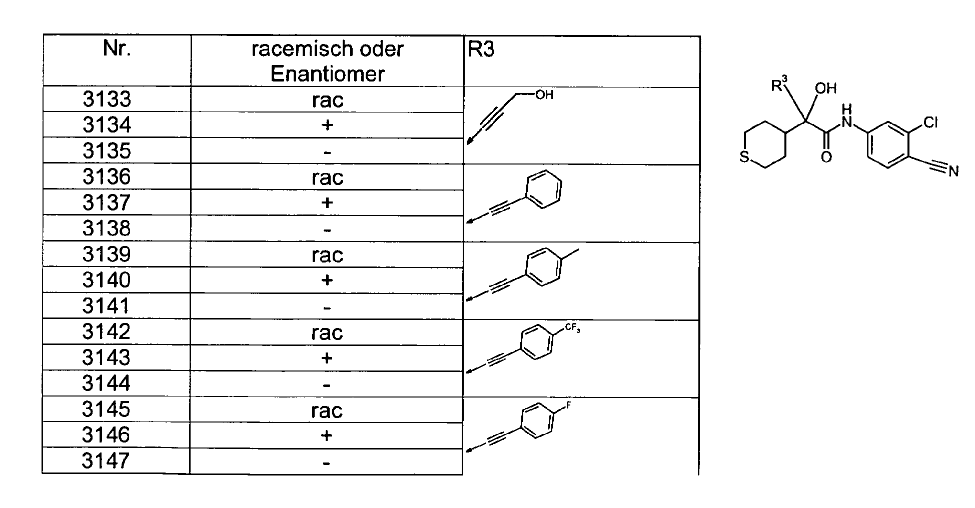

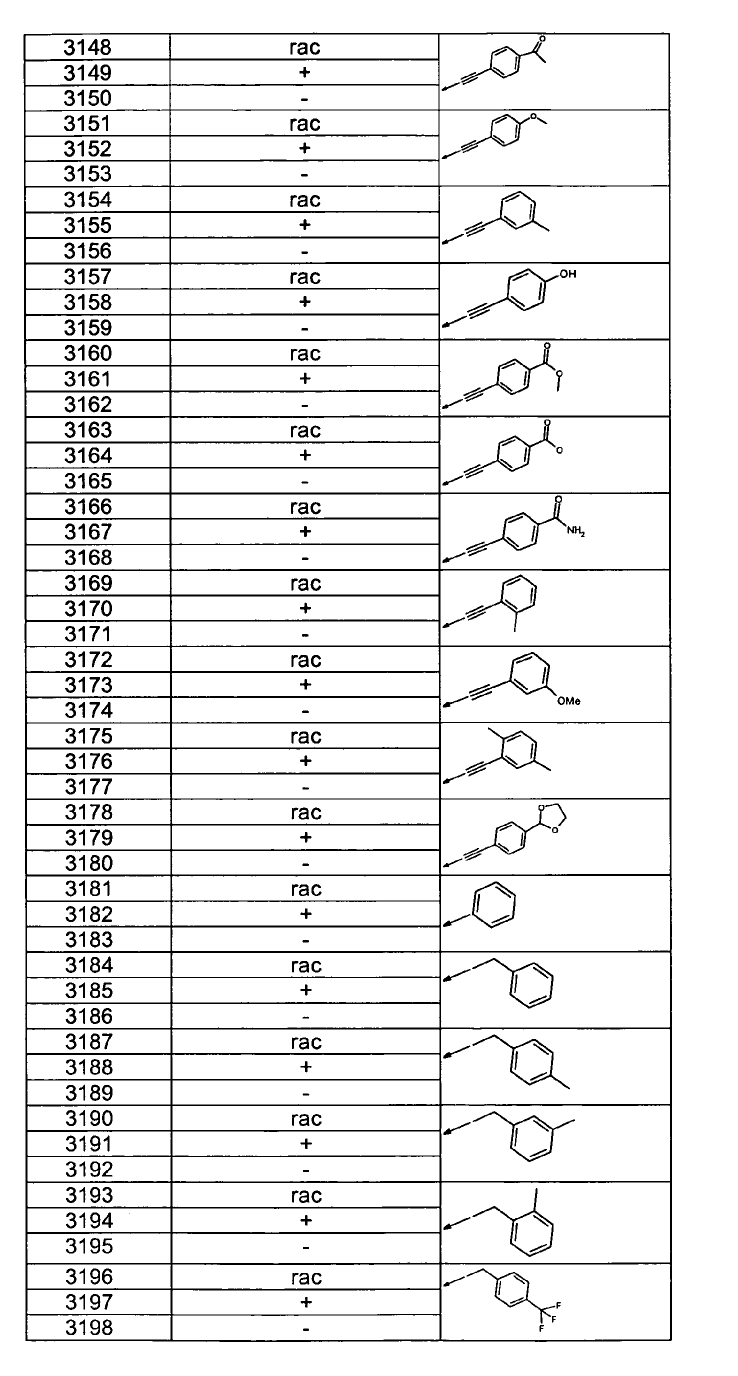

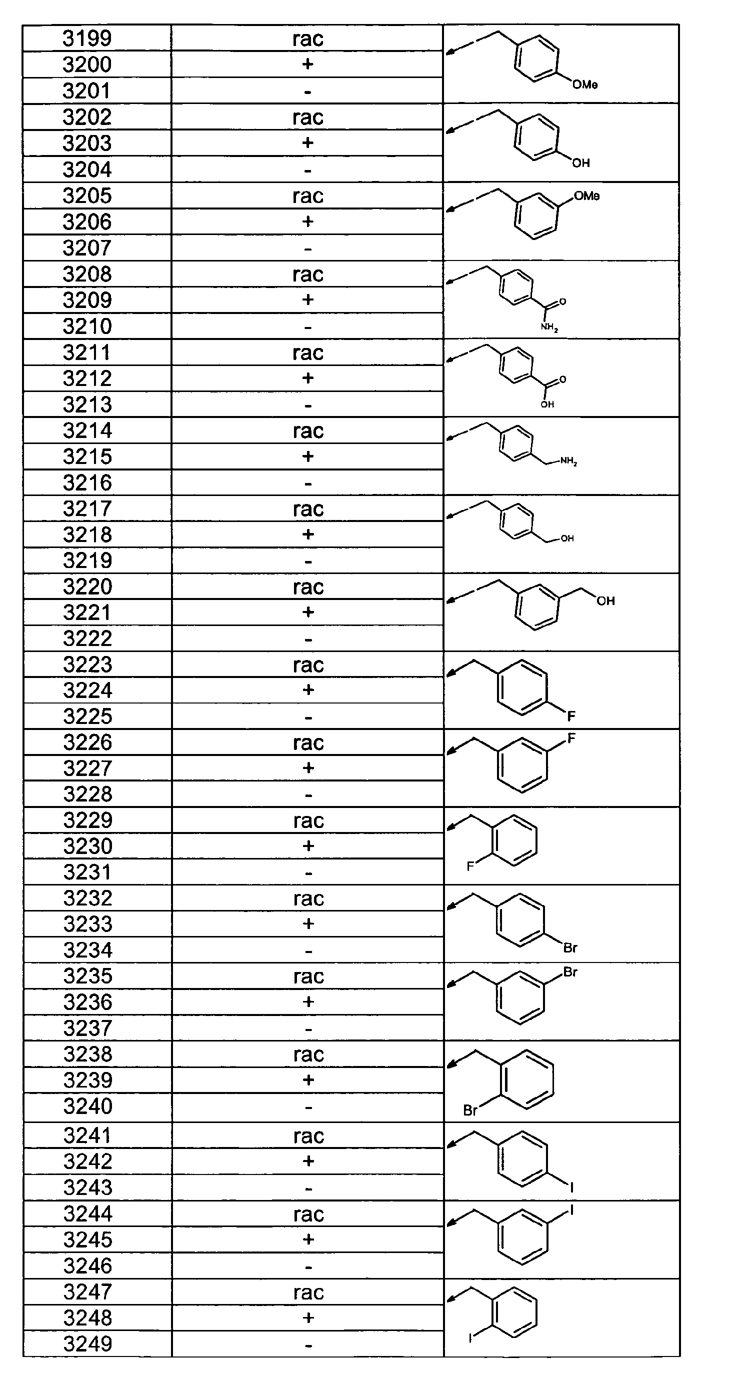

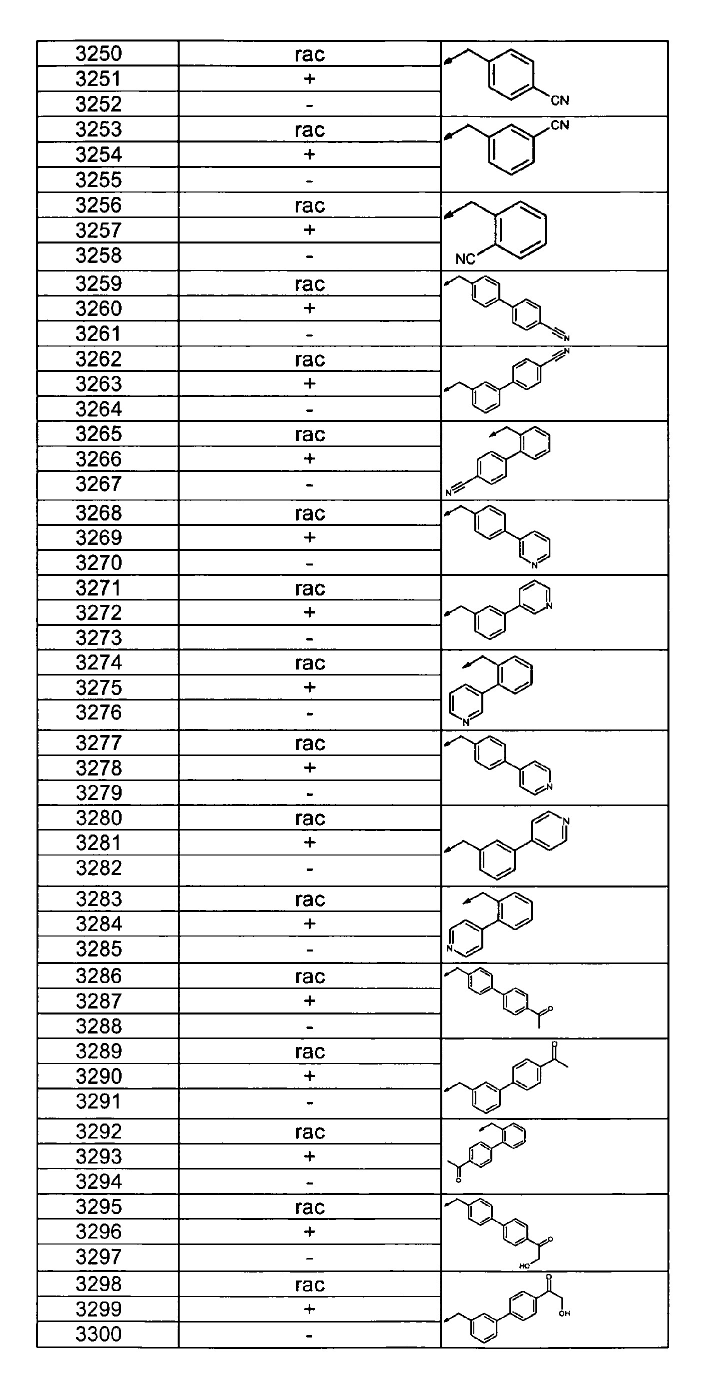

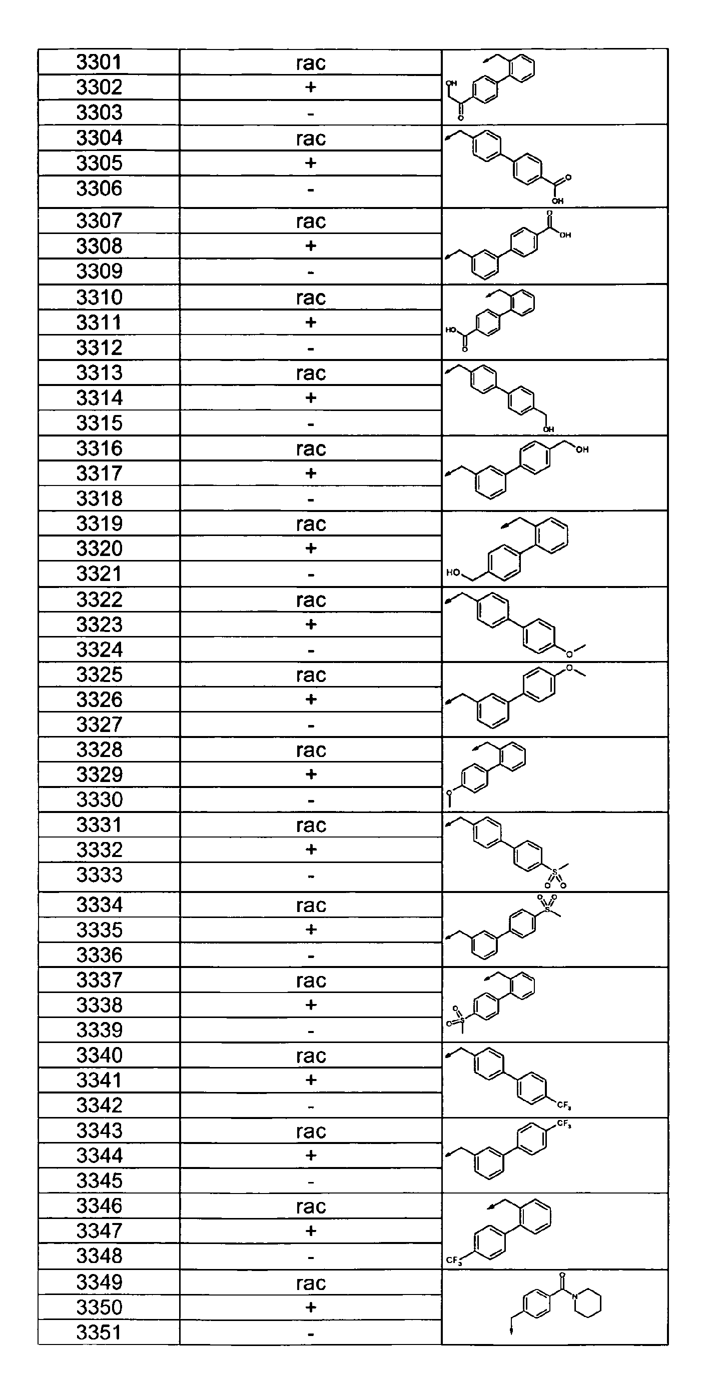

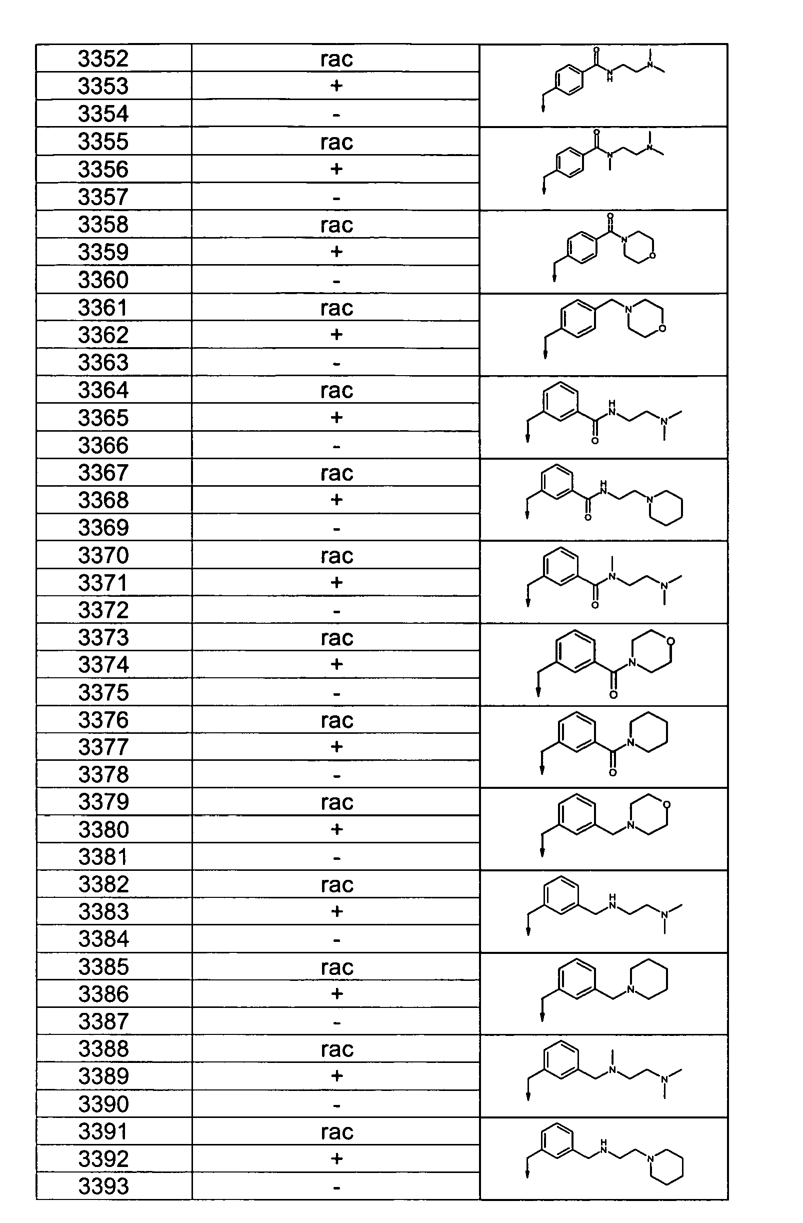

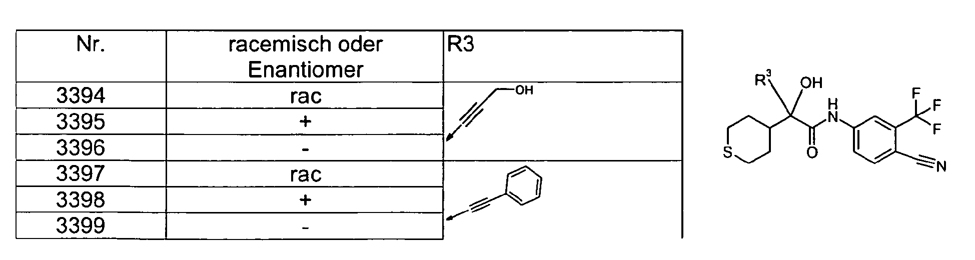

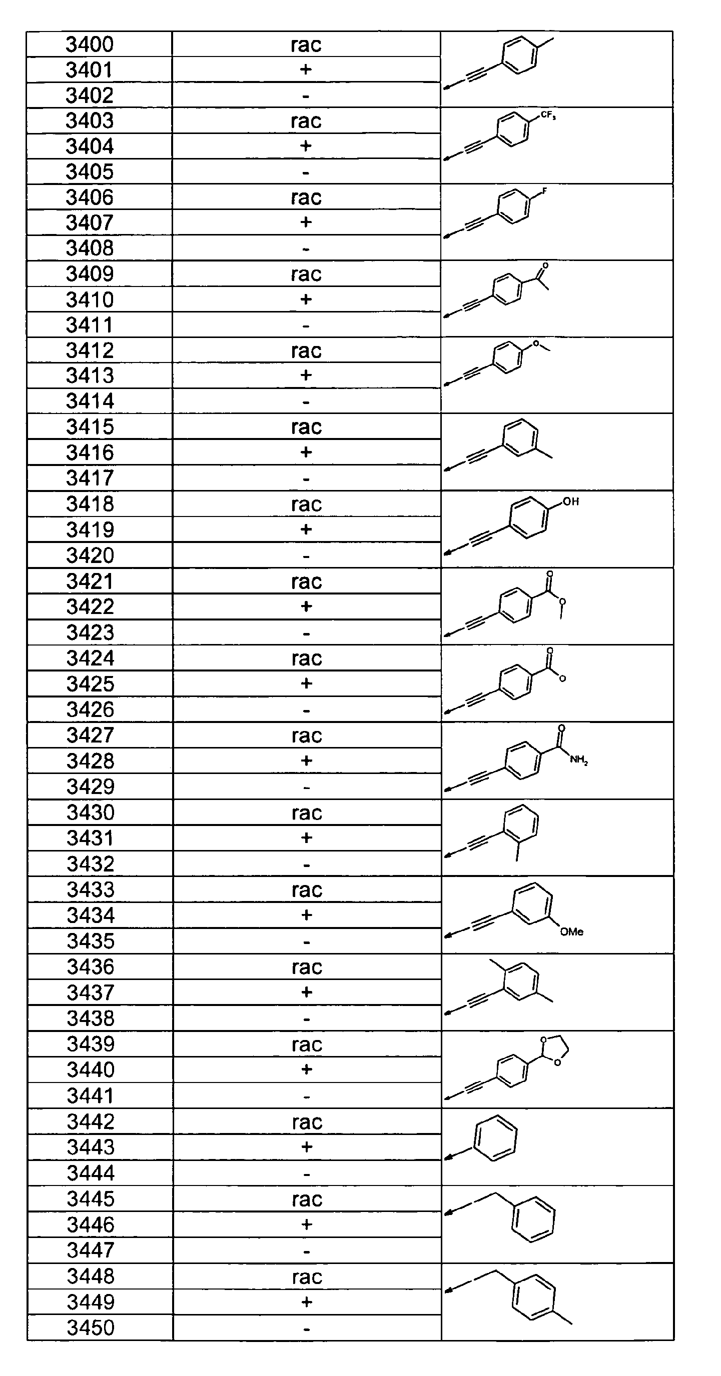

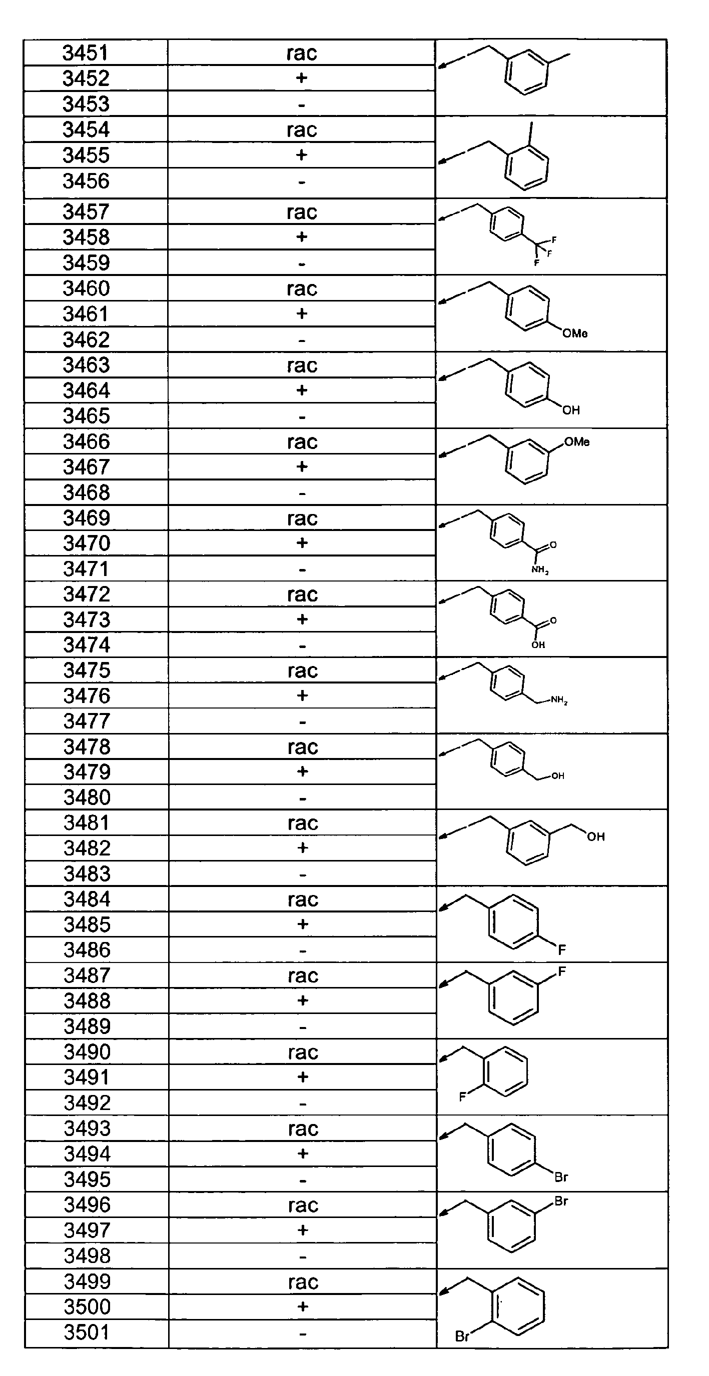

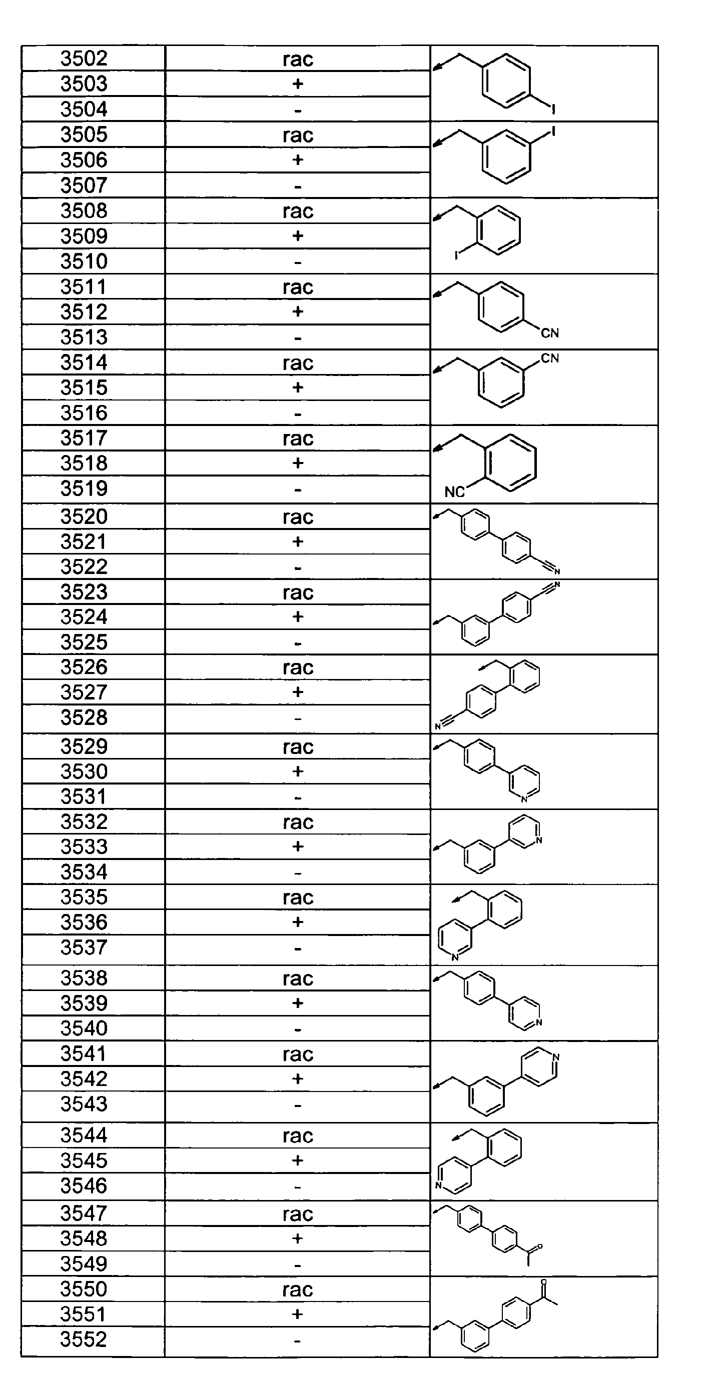

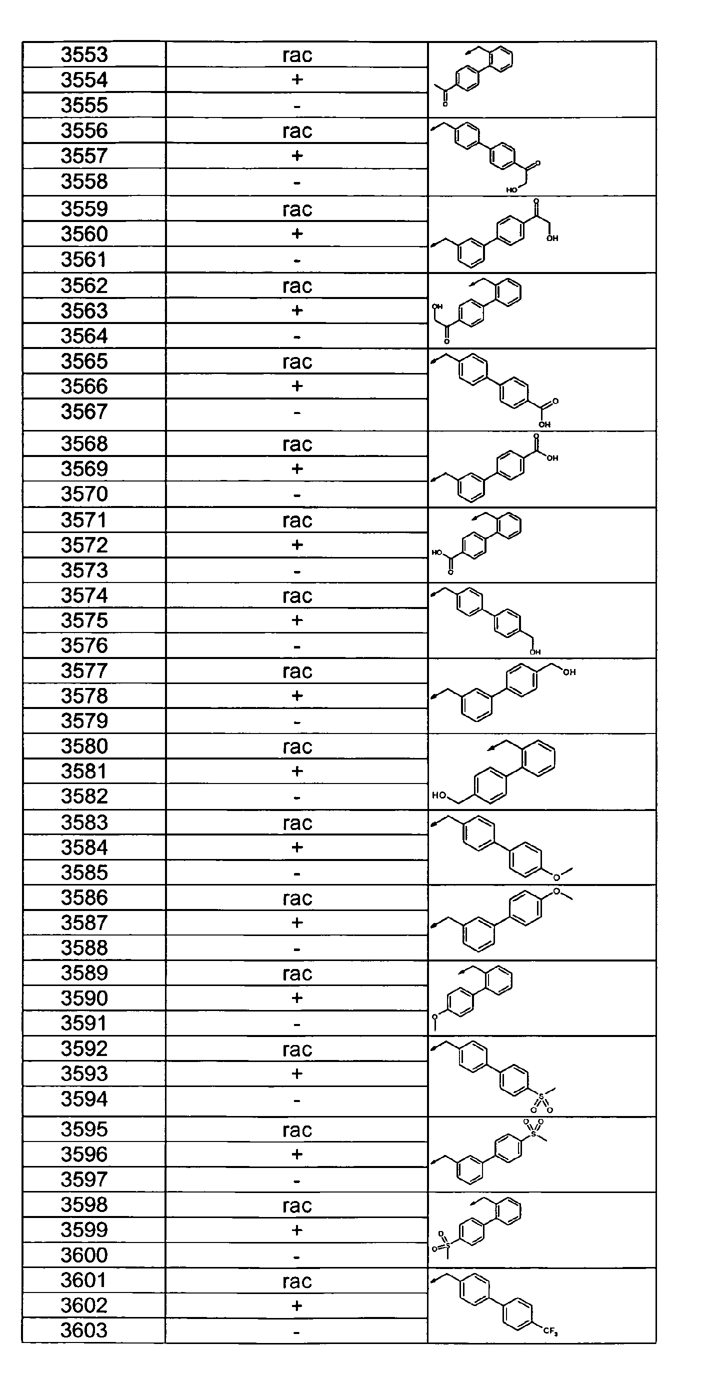

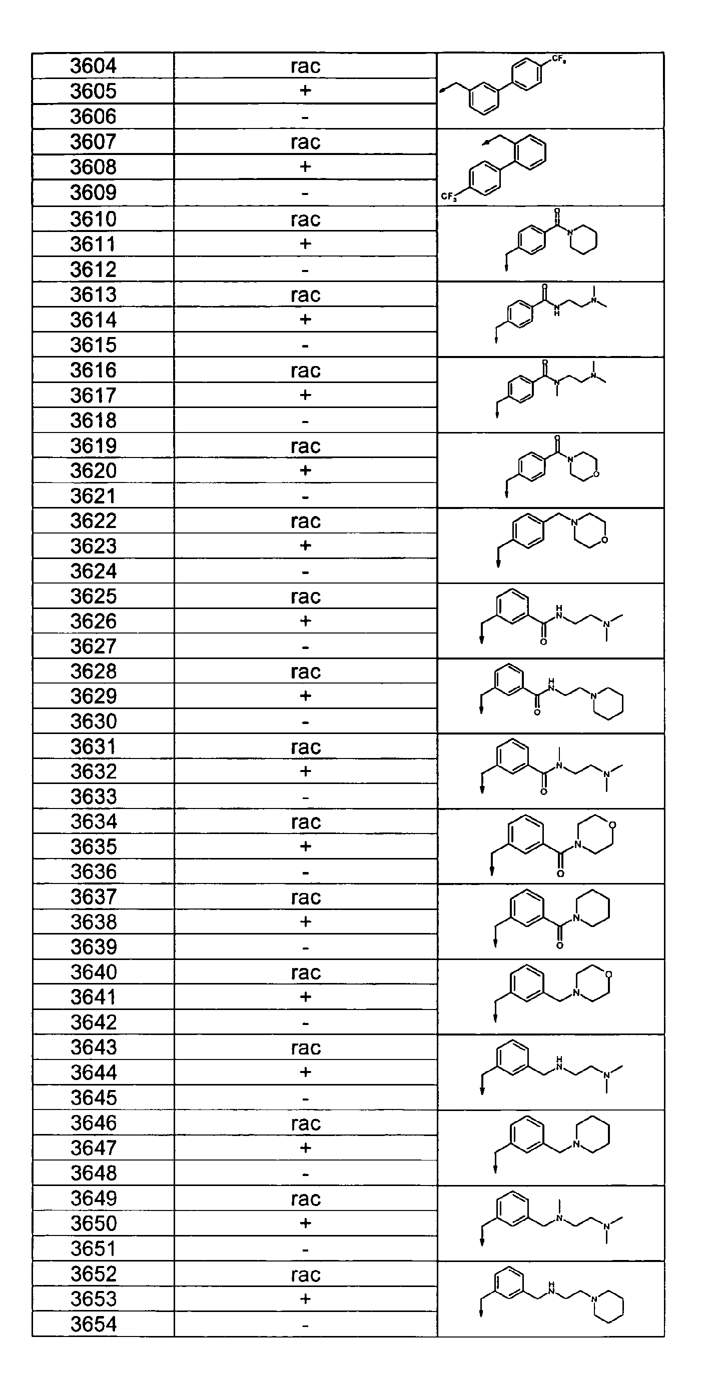

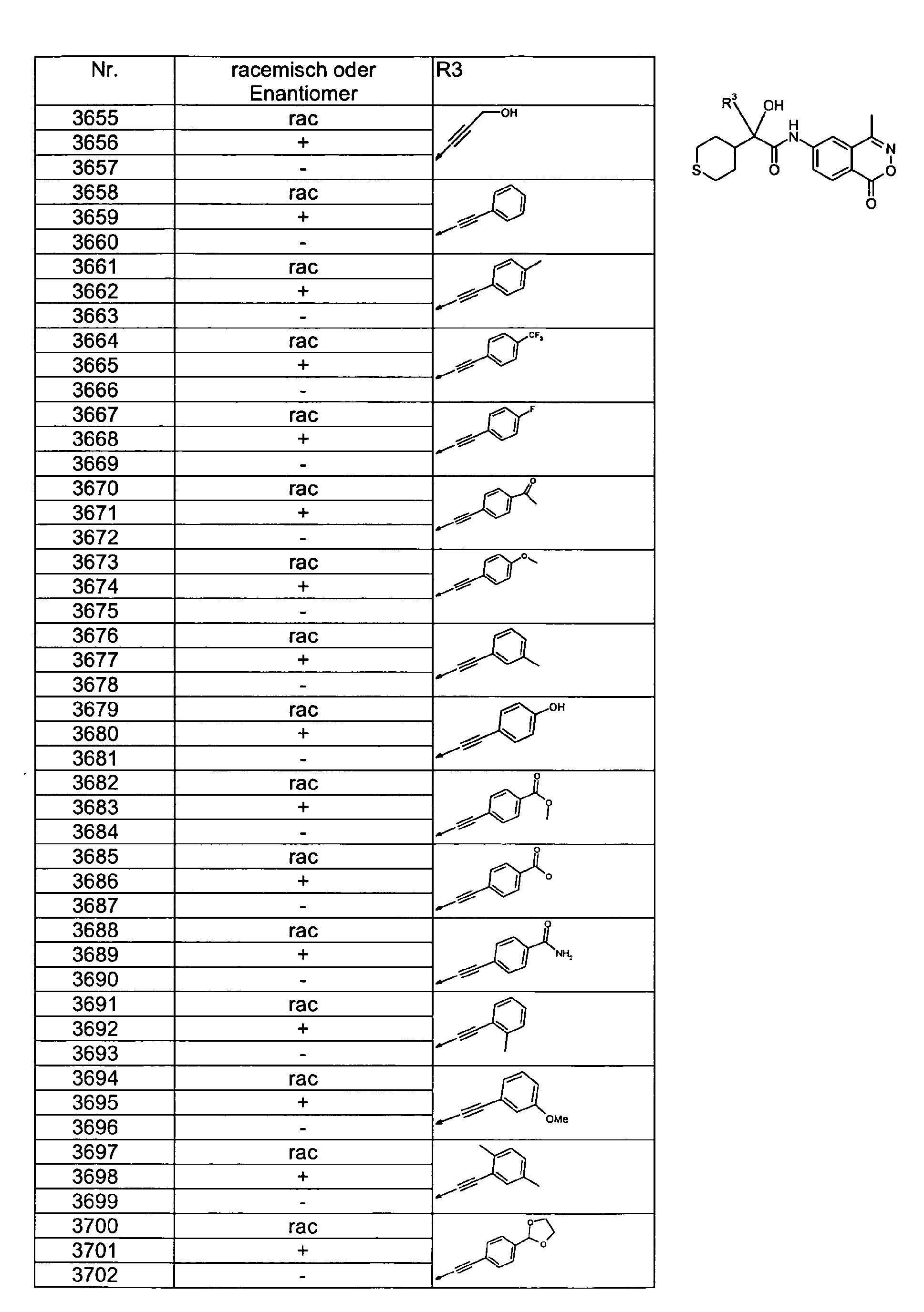

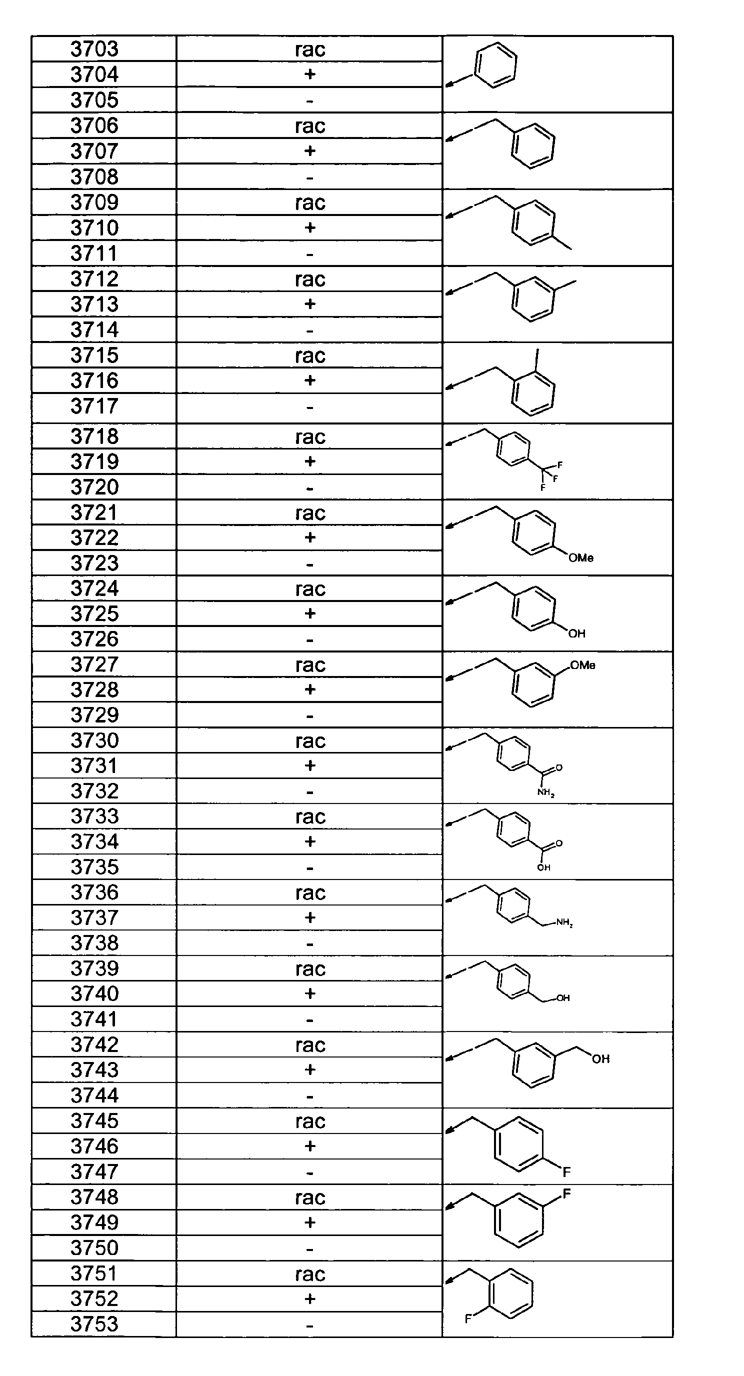

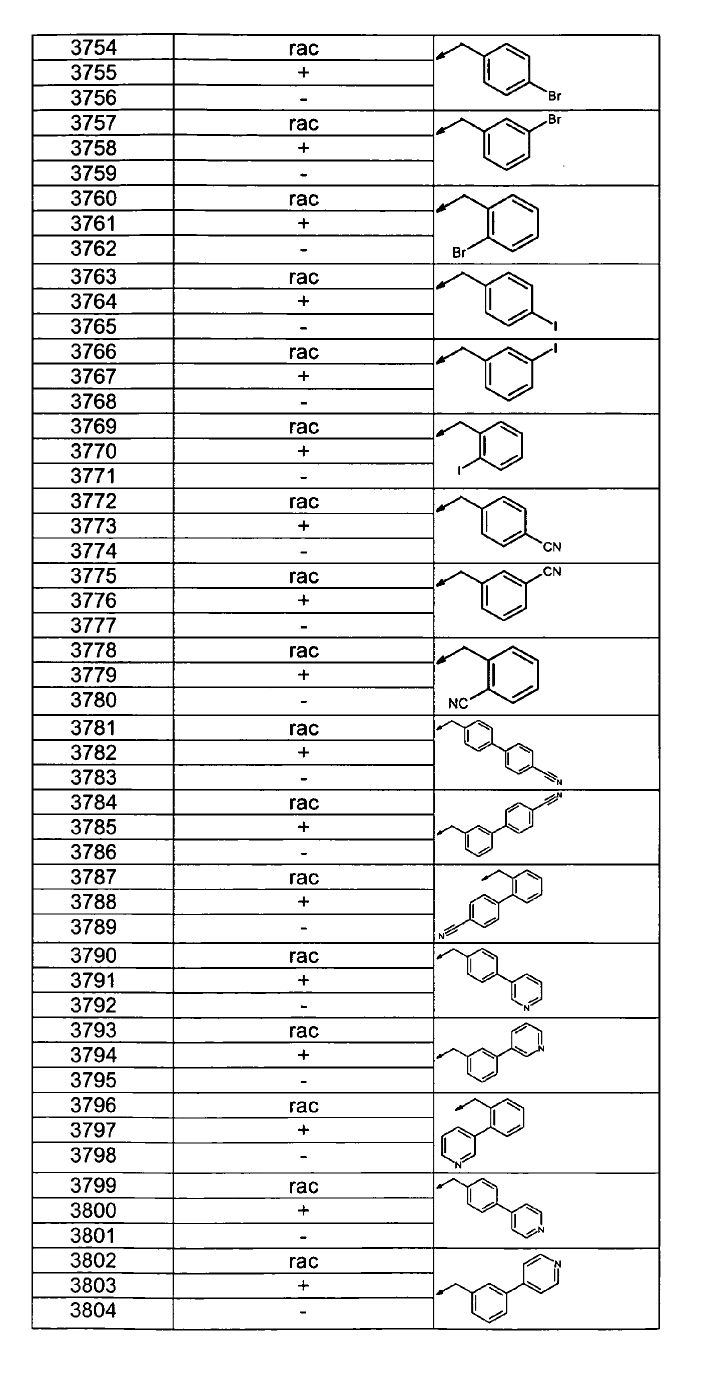

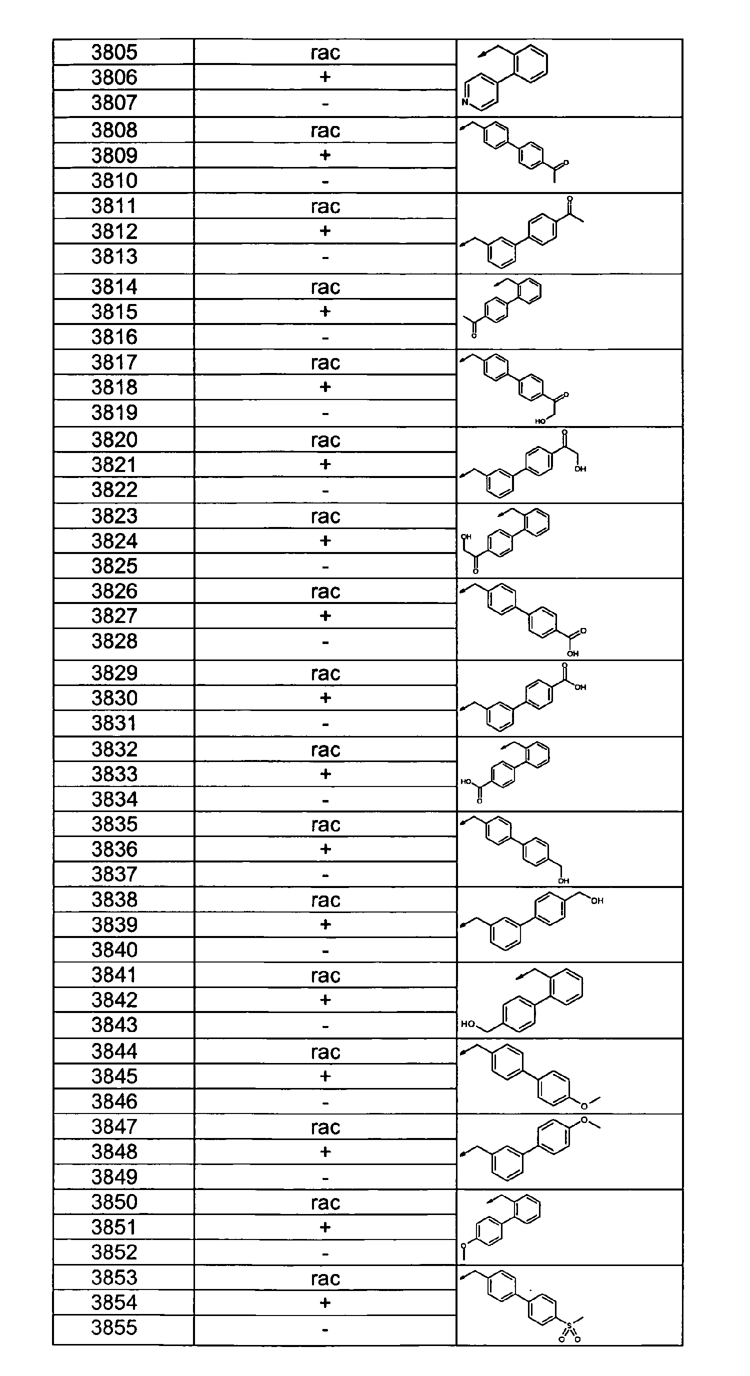

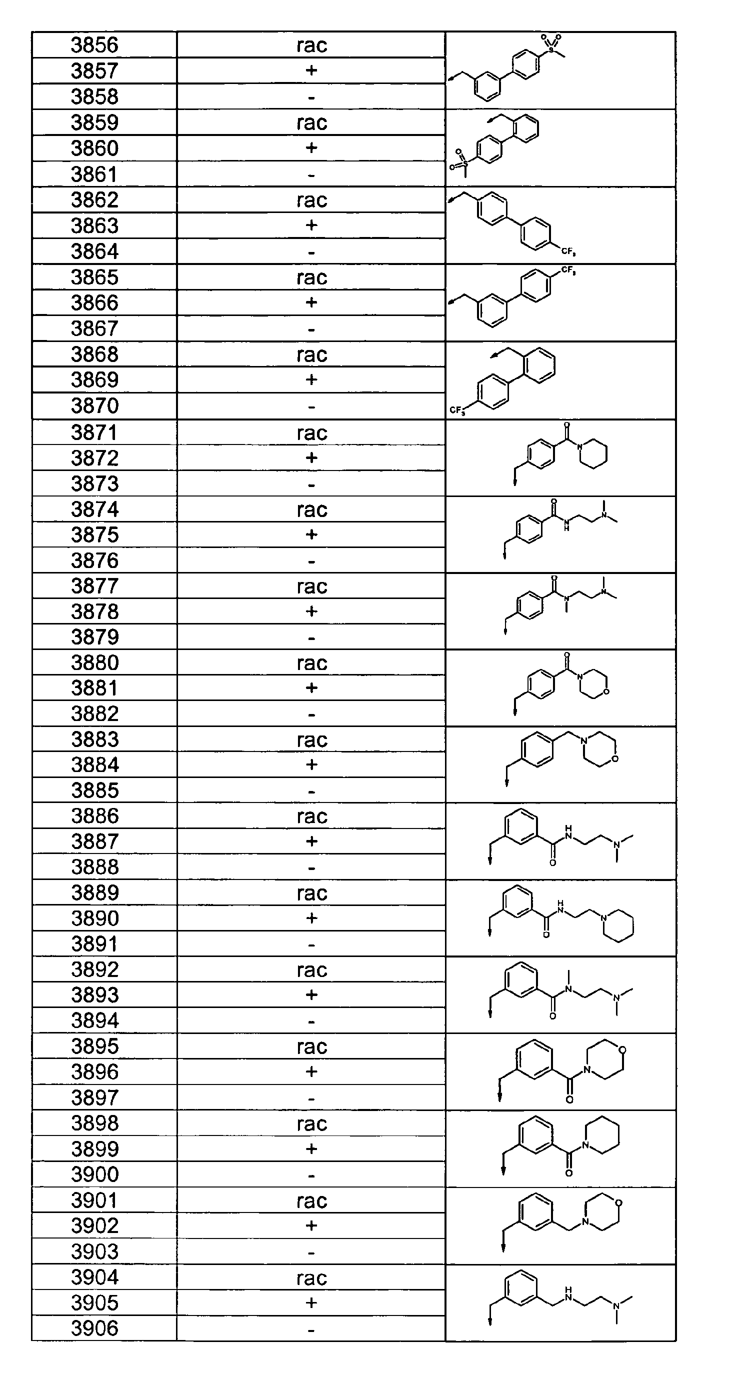

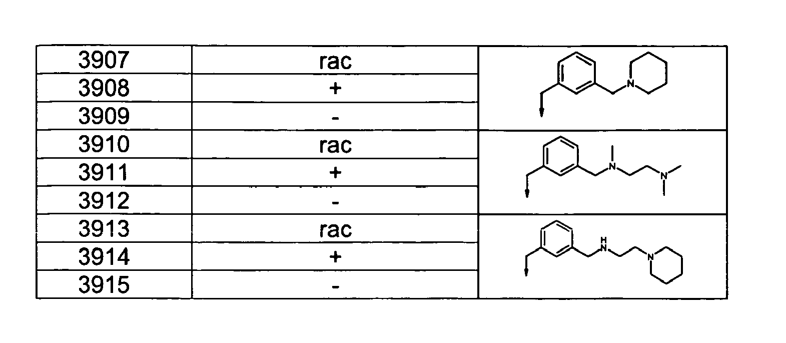

### Biologische Charakterisierung der erfindungsgemäßen Verbindungen

Die Identifizierung von Progesteronrezeptor-Modulatoren kann mit Hilfe einfacher Methoden, dem Fachmann bekannten Testprogrammen vorgenommen werden. Dazu kann beispielsweise eine zu testende Verbindung zusammen mit einem Gestagen in einem Testsystem für Progesteronrezeptorliganden inkubiert werden und geprüft werden, ob in diesem Testsystem die durch Progesteron vermittelte Wirkung in Anwesenheit des Modulatoren verändert wird.

Die erfindungsgemäßen Substanzen der allgemeinen Formel I wurden in folgenden Modellen getestet:

### Progesteronrezeptorbindungstest

Messung der Rezeptor-Bindungsaffinität:
Die Rezeptorbindungsaffinität wurde bestimmt durch kompetitive Bindung eines spezifisch bindenden ³H-markierten Hormons (Tracer) und der zu testenden Verbindung an Rezeptoren im Cytosol aus tierischen Target-Organen. Dabei wurden Rezeptorsättigung und Reaktionsgleichgewicht angestrebt.
   Der Tracer und steigende Konzentrationen der zu testenden Verbindung (Competitor) wurden bei 0-4°C über 18 h co-inkubiert mit der rezeptorhaltigen Cytosolfraktion. Nach Abtrennung des ungebundenen Tracers mit Kohle-Dextran-Suspension wurde für jede Konzentration der Rezeptor-gebundene Tracer-Anteil gemessen und aus der Konzentrationsreihe die IC₅₀ bestimmt. Als Quotient der IC₅₀-Werte von Referenzsubstanz und zu testender Verbindung (x 100%) wurde die relative molare Bindungsaffinität (RBA) errechnet (RBA der Referenzsubstanz = 100%).

### Für die Rezeptortypen wurden folgende Inkubationsbedingungen gewählt:

Progesteronrezeptor:
Uterus-Cytosol des Estradiol-geprimten Kaninchens, homogenisiert in TED-Puffer (20 mMTris/HCl, pH 7,4; 1 mM Ethylendiamintetraacetat, 2 mM Dithiothreitol) mit 250 mM Saccharose; aufbewahrt bei -30 °C. Tracer: ³H-ORG 2058, 5 nM; Referenzsubstanz: Progesteron.

### Glukokortikoidrezeptor:

Thymus-Cytosol der adrenalectomierten Ratte, Thymi aufbewahrt bei -30°C; Puffer:
TED. Tracer: ³H-Dexamethason, 20 nM; Referenzsubstanz: Dexamethason.
Die Kompetitionsfaktoren (KF-Werte) der erfindungsgemäßen Verbindungen der allgemeinen Formel (I) am Progesteronrezeptor liegen zwischen 0.2 und 35 bezogen auf Progesteron. Am Glukokortikoidrezeptor liegen die KF-Werte im Bereich von 3 bis 35 bezogen auf Dexamethason.

Die erfindungsgemäßen Verbindungen haben demnach eine hohe Affinität zum Progesteronrezeptor, aber nur eine geringe Affinität zum Glukokortikoidrezeptor.

### Antagonismus am Progesteronrezeptor PR

Der Transaktivierungsassay wird wie in WO 02/054064 beschrieben durchgeführt.
Die IC₅₀-Werte liegen im Bereich von 0.1 bis 150 nM. Die nachfolgende Tabelle zeigt beispielhaft Resultate aus dem Transaktivierungstest auf antagonistische Aktivität am (PR-B).

| Beispiel Nr | IC₅₀ [nM] |
|---|---|
| 2b | 1.8 |
| 3 | 5.6 |
| 4 | 2.0 |
| 5 | 7.9 |
| 6 | 5.0 |
| 10 | 7.0 |
| 12 | 1.8 |
| 16 | 17 |
| 32 | 27 |
| 39 | 17 |
| 44 | 8.0 |

### Agonismus am Progesteronrezeptor PR

Der Transaktivierungsassay wird wie in Fuhrmann et al. beschrieben durchgeführt (Fuhrmann U., Hess-Stump H., Cleve A., Neef G., Schwede W., Hoffmann J., Fritzemeier K.-H., Chwalisz K., Journal of Medicinal Chemistry, 43, 26, 2000, 5010-5016). Die EC₅₀-Werte liegen im Bereich von 0.01 bis 150 nM.

### Dosierung

Zur erfindungsgemäßen Verwendung können die Progesteronrezeptor-Modulatoren oral, enteral, parenteral oder transdermal verabreicht werden.

Im Allgemeinen sind zufriedenstellende Resultate bei der Behandlung der weiter oben genannten Indikationen zu erwarten, wenn die täglichen Dosen einen Bereich von 1 µg bis 1000 mg der erfindungsgemäßen Verbindung für gynäkologische Indikationen wie Behandlung von Endometriose, Leiomyome des Uterus und dysfunktionelle Blutungen sowie für die Verwendung in der Fertilitätskontrolle und für die Hormonersatztherapie umfassen. Für onkologische Indikationen sind tägliche Dosierungen im Bereich von 1 µg bis 2000 mg der erfindungsgemäßen Verbindung zu verabreichen.

Geeignete Dosierungen der erfindungsgemäßen Verbindungen am Menschen für die Behandlung der Endometriose, von Leiomyomen des Uterus und dysfunktionellen Blutungen sowie für die Verwendung in der Fertilitätskontrolle sowie für die Hormonersatztherapie betragen 50 µg bis 500 mg pro Tag, je nach Alter und Konstitution des Patienten, wobei die notwendige Tagesdosis durch Einmal- oder Mehrfachabgabe appliziert werden kann.

Für die Behandlung von Mammakarzinomen umfasst der Dosierungsbereich für die erfindungsgemäßen Verbindungen täglich 10 mg bis 2000 mg.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Filmtabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.
Weiterhin seien als Zubereitung auch Mittel zur vaginalen Anwendung genannt.

Zur pulmonalen Applikation der neuen Verbindungen können diese in Form von Aerosolen und Inhalaten verwendet werden.

Für die transdermale Applikation sind Pflaster bzw. für die topische Auftragung Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen, um eine ausreichende pharmakologische Wirkung zu erzielen.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen der erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltenden Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen der allgemeinen Formel (I) oder deren pharmazeutisch annehmbare Salze können aufgrund ihrer antagonistischen oder partialagonistischen Wirksamkeit für die Herstellung eines Arzneimittels, insbesondere zur Behandlung und Prophylaxe von gynäkologischen Erkrankungen wie Endometriose, Leiomyomen des Uterus, dysfunktionelle Blutungen und der Dysmenorrhoe verwendet werden. Weiterhin können sie gegen hormonelle Unregelmäßigkeiten, zur Menstruationsauslösung und allein oder in Kombination mit Prostaglandinen und/ oder Oxytocin zur Geburtseinleitung eingesetzt werden.

Die erfindungsgemäßen Verbindungen der allgemeinen Formel (I) oder deren pharmazeutisch annehmbare Salze sind weiterhin geeignet zur Herstellung von Präparaten für die Empfängnisverhütung für die Frau (siehe auch WO 93/23020, WO 93/21927).
Die erfindungsgemäßen Verbindungen oder deren pharmazeutisch annehmbare Salze können außerdem allein oder in Kombination mit einem Selektiven Estrogen-Rezeptor-Modulatoren (SERM) für die weibliche Hormonersatztherapie eingesetzt werden.

Weiterhin üben die genannten Verbindungen in hormonabhängigen Tumoren eine antiproliferative Wirkung aus. Sie sind daher für die Therapie von hormonabhängigen Karzinomen geeignet, wie beispielsweise für Mamma-, Prostata- und Endometriumskarzinome.

Die erfindungsgemäßen Verbindungen oder deren pharmazeutisch annehmbare Salze können für die Behandlung hormonabhängiger Karzinome sowohl in der first-line Therapie als auch in der second-line Therapie, insbesondere nach Tamoxifen-failure, zum Einsatz kommen.

Die erfindungsgemäßen, antagonistisch bzw. partialagonistisch wirksamen Verbindungen der allgemeinen Formel (I) oder deren pharmazeutisch annehmbare Salze können auch in Kombination mit antiestrogen wirksamen Verbindungen (Estrogenrezeptor-Antagonisten oder Aromatasehemmer) oder Selektiven Estrogen-Rezeptor-Modulatoren (SERM) zur Herstellung pharmazeutischer Präparate zur Behandlung hormonabhängiger Tumore verwendet werden. Für die Behandlung der Endometriose oder von Leiomyomen des Uterus können die erfindungsgemäßen Verbindungen ebenfalls in Kombination mit SERM's oder einem Antiestrogen (Estrogenrezeptor-Antagonisten oder Aromatasehemmer) verwendet werden Zur Kombination mit den erfindungsgemäßen nichtsteroidalen Progesteronrezeptor-Modulatoren kommen dabei beispielsweise die folgenden Antiestrogene (Estrogen-rezeptor-Antagonisten oder Aromatasehemmer) bzw. SERM's in Betracht: Tamoxifen, 5-(4-{5-[(RS)-(4,4,5,5,5-Pentafluorpentyl)sulfinyl]pentyloxy}phenyl)-6-phenyl-8,9-dihydro-7H-benzocyclohepten-2-ol (WO 00/03979), ICI 182 780 (7alpha-[9-(4,4,5,5-Pentafluorpentylsulfinyl)nonyl]estra-1,3,5(10)-trien-3,17-beta-diol), 11 beta-Fluor-7alpha-[5-(methyl{3-[(4,4,5,5,5-pentafluorpentyl)sulfanyl]propyl}amino)-pentyl]estra-1,3,5(10)-trien-3,17beta-diol (WO98/07740), 11 beta-Fluor-7alpha-{5-[methyl(7,7,8,8,9,9,10,10,10-nonafluordecyl)amino]pentyl}estra-1,3,5(10)-trien-3,17beta-diol (WO 99/33855), 11beta-Fluor-17alpha-methyl-7alpha-{5-[methyl-(8,8,9,9,9-pentafluornonyl)amino]pentyl}estra-1,3,5(10)-trien-3,17beta-diol (WO 03/045972), Clomifen, Raloxifen sowie weitere antiestrogen wirksame Verbindungen, und Aromataseinhibitoren wie beispielsweise Fadrozol, Formestan, Letrozol, Anastrozol oder Atamestan.

Schließlich betrifft die vorliegende Erfindung auch die Verwendung der Verbindungen der allgemeinen Formel I, gegebenenfalls zusammen mit einem Antiestrogen oder SERM, zur Herstellung eines Arzneimittels.

Ferner betrifft die vorliegende Erfindung pharmazeutische Zusammensetzungen, die mindestens eine erfindungsgemäße Verbindung, gegebenenfalls in Form eines pharmazeutisch/ pharmakologisch verträglichen Salzes.

Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/ oder Verdünnungsmitteln mindestens eine erfindungsgemäße Verbindung.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen, gegebenenfalls als Depotform.

Die pharmazeutischen Zusammensetzungen, die mindestens eine der erfindungsgemäßen Verbindungen enthalten, werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

### Herstellung der erfindungsgemäßen Verbindungen:

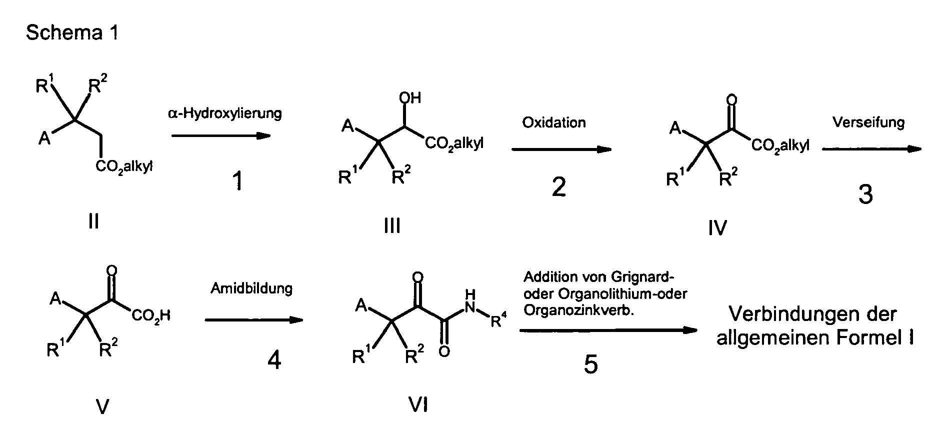

Die Verbindungen der allgemeinen Formel **I** können z. B. wie in Schema 1 gezeigt synthetisiert werden. Eine α-Hydroxylierung des Esters der allgemeinen Formel **II** und nachfolgende Oxidation des gebildeten Alkohols **III** zum Keton ergibt Verbindungen der allgemeinen Formel **IV.** Für die Darstellung von Verbindungen der allgemeinen Formel **III** durch α-Hydroxylierung von Estern kommen verschiedene aus der Literaturbekannte Verfahren in Frage (beispielsweise Davis et al. in J. Org. Chem, 1984, 49, 3241 unter Verwendung von 2-Sulfonyloxaziridin). Die Oxidation zu Verbindungen der allgemeinen Formel **IV** kann dann nach bekannten Standardmethoden erfolgen. Die Herstellung der Amide der allgemeinen Formel **VI** erfolgt beispielsweise über die Bildung der Säurechloride und anschließende Umsetzung mit den entsprechenden Aminen. Alternativ hierzu können je nach einzuführendem Amin aber auch andere Methoden zur Amidbildung genutzt werden. Aus den Amiden der allgemeinen Formel **VI** werden dann durch Addition von metallorganischen Verbindungen wie Magnesium-, Lithium- oder Zinkorganischen Verbindungen die Verbindungen der allgemeinen Formel **I** hergestellt. Die Schritte 1-5 können aber auch in veränderter Reihenfolge durchgeführt werden. Einige Zwischenverbindungen der allgemeinen Formeln **III-V** sind auch kommerziell erhältlich.

Die Substituenten A, X, Y, R¹, R², R³ und R⁴ können gegebenenfalls auch nach erfolgter Einführung weiter modifiziert werden. Hierfür kommen z.B. Oxidation, Reduktion, Alkylierungen, Acylierungen, nukleophile Additionen oder auch Übergangsmetall-katalysierte Kupplungsreaktionen in Frage. Funktionelle Gruppen in Verbindungen der allgemeinen Formeln **II-VI** werden gegebenenfalls zwischenzeitlich mit Schutzgruppen versehen, die dann auf einer geeigneten Stufe wieder abgespalten werden.

Die nachfolgenden Beispiele dienen der näheren Erläuterung des Erfindungsgegenstandes, ohne ihn auf diese beschränken zu wollen.

Die Herstellung von 6-Amino-4-methyl-2,3-benzoxazin-1-on wird in WO 199854159 beschrieben.

### Herstellung von 6-(4,4-Dimethyl-2-oxovaleroylamino)-4-methyl-2,3-benzoxazin-1-on:

a) 2-Hydroxy-2-cyclohexylessigsäure ethylester 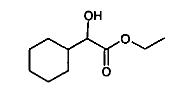 Zu einer Lösung von 2-Cyclohexylessigsäureethylester (1,2 g) in Tetrahydrofuran (25 mL) wurde eine Lösung Kaliumhexamethyldisilizan (20 mL, 0,5 M in Toluol) bei -70°C addiert. Man ließ 30 Minuten bei -70°C nachrühren und addierte dann eine Lösung von 3-Phenyl-2-phenylsulfonyloxaziridin (2,6 g) in Tetrahydrofuran (25 mL). Man ließ eine Stunde bei -70°C nachrühren. Danach wurde das Reaktionsgemisch auf gesättigte wässrige Ammoniumchloridlösung gegossen. Man rührte weitere 30 Minuten nach und trennte die Phasen. Die organische Phase wurde mit gesättiger wässriger Natriumchloridlösung gewaschen, über Natriumsulfat getrocknet und im Vakuum eingeengt. Das erhaltene Rohprodukt wurde an Kieselgel chromatographiert. Man erhielt 1,3 g Produkt. ¹H-NMR (ppm, CDCl₃, 400 MHz): 1.11-1.27 (6H), 1.31 (3H), 1.44 (1H), 1.61-1.79 (4H), 2.73 (1 H), 4.01 (1H), 4.25 (2H).
b) 2-Oxo-2-cyclohexylessigsäure ethylester 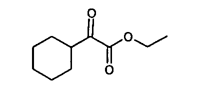 Zu einer Lösung von der unter a) beschriebenen Verbindung in 20 mL Dichlormethan wurden 20 ml einer 0,35 molaren Lösung von 1,1-Dihydro-1,1,1-triacetoxy-1,2-benziodoxol-3(1H)-on (Dess-Martin Periodan) in Dichlormethan addiert. Man ließ 14 Stunden bei 23°C nachrühren. Anschließend verdünnte man mit 500 ml Methyltertbutylether und goss dann auf 1 I einer wässrigen Lösung von 34 g Natriumhydrogencarbonat und 100 g Natriumthiosulfat. Man ließ 30 Minuten nachrühren, trennte dann die Phasen und extrahierte die wässrige Phase mit Methyltertbutylether. Die vereinigten organischen Phasen wurden mit gesättigter wässriger Natriumhydrogencarbonatlösung sowie gesättigter wässriger Natriumchloridlösung gewaschen und über Natriumsulfat getrocknet. Das erhaltene Rohprodukt wurde an Kieselgel chromatographiert. Man erhielt 0,8 g Produkt. ¹H-NMR (ppm, CDCl₃, 400 MHz): 1.15-1.29 (5H), 1.36 (3H), 1.67-1.91 (5H), 3.03 (1 H), 4.32 (2H).
c) 2-Oxo-2-cyclohexylessigsäure 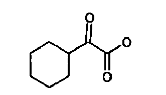 Zu einer Lösung der unter b) beschriebenen Verbindung in 13 mL Ethanol wurde eine Lösung von 0,5 g Natriumhydroxid in 8 ml Wasser addiert. Man ließ 14 Stunden bei 23°C nachrühren, verdünnte dann mit Wasser und extrahierte mit Ethylacetat. Anschließend wurde die wässrige Phase mit 2 normaler Salzsäure angesäuert (pH-Wert 4). Danach extrahierte man mit Ethylacetat und wusch die organische Phase mit gesättigter wässriger Natriumchloridlösung. Man trocknete dann über Natriumsulfat und engte im Vakuum ein. Das erhaltene Rohprodukt (0,7 g) wurde ohne Reinigung in die Folgestufe eingesetzt.
d) N-(3-Chlor-4-cyanophenyl)-2-oxo-2-cyclohexylacetamid 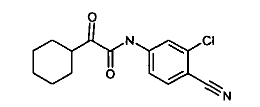 Die unter c) beschriebenen Carbonsäure wurden in 20 mL N,N-Dimetylacetamid gelöst. Man addierte bei -10°C 0,38 mL Thionylchlorid und ließ eine Stunde bei -10°C nachrühren. Anschließend wurden 0,92 g 3-Chlor-4-cyanoaniline portionsweise addiert. Danach wurde 3 Stunden nachgerührt (-10°C nach 0°C). Anschließend wurde das Reaktionsgemisch auf Eiswasser gegossen. Man extrahierte mit Ethylacetat. Die organische Phase wurde mit gesättigter wässriger Natriumchloridlösung gewaschen, über Natriumsulfat getrocknet und im Vakuum eingeengt. Das Rohprodukt wurde durch Säulenchromatographie an Kieselgel mit einem Gemisch aus Hexan/Ethylacetat gereinigt. Man erhielt 1,2 g Produkt. ¹H-NMR (ppm, CDCl₃, 400 MHz): 1.22-1.46 (5H), 1.72-1.96 (5H), 3.42-3.50 (1H), 7.59 (1H), 7.67 (1H), 8.02 (1H), 8.96 (1H).

### Beispiel 1: rac-N-(3-Chlor-4-cyanophenyl)-2-oxo-2-cyclohexyl-2-phenylacetamid

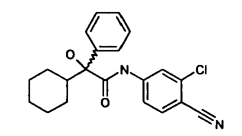

Eine 1 molare Lösung von Phenylmagnesiumbromid in Tetrahydrofuran (0,9 ml) wurde mit 10 ml absolutem Tetrahydrofuran verdünnt. Man kühlte auf -70°C und addierte dann eine Lösung von 100 mg N-(3-Chlor-4-cyanophenyl)-2-oxo-2-cyclohexylacetamid in 6 ml Tetrahydrofuran. Anschließend ließ man bei -70°C 2,5 Stunden nachrühren. Danach wurde das Reaktionsgemisch auf eiskalte gesättigte Ammoniumchloridlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 130 mg Produkt. ¹H-NMR (ppm, CDCl₃, 400 MHz): 0.84 (1H), 1.10-1.43 (5H), 1.71-1.84 (4H), 2.60 (2H), 7.29-7.42 (3H), 7.48 (1 H), 7.57 (1 H), 7.65 (2H), 7.96 (1 H), 8.95 (1 H).

### Beispiel 2: rac-N-(3-Chlor-4-cyanophenyl)-2-hydroxy-2-cyclohexyl-2-phenylethinylacetamid

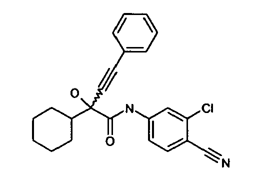

Zu einer Lösung von 94 µl Phenylacetylen in Tetrahydrofuran (5 mL) wurde bei -78°C n-Butyllithium (540 µl, 1,6 M in Hexan) addiert. Man ließ 30 Minuten bei dieser Temperatur nachrühren und tropfte dann eine Lösung von N-(3-Chlor-4-cyanophenyl)-2-oxo-2-cyclohexylacetamid (100 mg) in 5 ml Tetrahydrofuran hinzu. Anschließend ließ man über ca. 3 h auf 23°C kommen und rührte dann 10 h nach. Danach wurde das Reaktionsgemisch auf eiskalte gesättigte Ammoniumchloridlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 110 mg Produkt. ¹H-NMR (ppm, DMSO-d₆, 400 MHz): 0.84 (1H), 1.14-1.28 (5H), 1.60-1.98 (6H), 6.86 (1H), 7.36-7.46 (5H), 7.94 (2H), 8.27 (1 H), 10.41 (1 H).

### Beispiel 2a und 2b: (+)-N-(3-Chlor-4-cyanophenyl)-2-hydroxy-2-cyclohexyl-2-phenylethinylacetamid 2a und (-)-N-(3-Chlor-4-cyanophenyl)-2-hydroxy-2-cyclohexyl-2-phenylethinylacetamid 2b

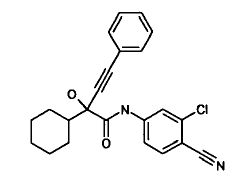

Das unter Beispiel 2 erhaltene racemische Gemisch wurde durch präparative chirale HPLC (Säule Chiralpak AD 250x10 mm) in die Enantiomeren 2a und 2b getrennt. **2a** : [α]^{D}₂₀: + 2.3° (CHCl₃, 11,3 mg/1 ml; λ=589 nM) **2b** : [α]^{D}₂₀: - 2.0° (CHCl₃, 11,0 mg/ 1ml; λ=589 nM)

### Beispiel 3: rac- N-(3-Chlor-4-cyanophenyl)-2-hydroxy-2-cyclohexyl-3-phenylpropanamid

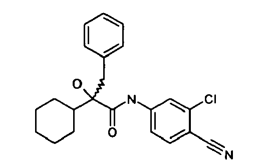

Eine 2 molare Lösung von Benzylmagnesiumchlorid in Tetrahydrofuran (430 µl) wurde mit 4 ml absolutem Tetrahydrofuran verdünnt. Man kühlte auf -70°C und addierte dann eine Lösung von 100 mg N-(3-Chlor-4-cyanophenyl)-2-oxo-2-cyclohexylacetamid in 6 ml Tetrahydrofuran. Anschließend ließ man bei -70°C 2 Stunden nachrühren. Danach wurde das Reaktionsgemisch auf eiskalte gesättigte Ammoniumchloridlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 78 mg Produkt. ¹H-NMR (ppm, DMSO-d₆, 400 MHz): 1.01-1.76 (10H), 1.93 (1H), 2.87 (1H), 3.02 (1H), 5.46 (1H), 7.05-7.28 (5H), 7.69-7.78 (2H), 8.01 (1H), 9.69 (1H).

### Beispiel 4: rac-N-(3-Chlor-4-cyanophenyl)-2-hydroxy-2-cyclohexyl-2-(4-methylphenyl)ethinylacetamid

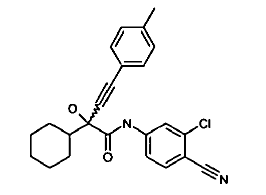

N-(3-Chlor-4-cyanophenyl)-2-oxo-2-cyclohexylacetamid (100 mg) wurde mit 4-Methylphenylethin und n-Butyllithium in Tetrahydrofuran in Analogie zu Beispiel 2 umgesetzt. Man erhielt nach Säulenchromatographie 45 mg Produkt. ¹H-NMR (ppm, CDCl₃, 400 MHz): 1.16-1.35 (5H), 1.66-1.88 (4H), 2.05-2.14 (2H), 2.35 (3H), 3.02 (1 H), 7.13 (2H), 7.35 (2H), 7.56 (1 H), 7.62 (1 H), 7.98 (1 H), 8.80 (1 H).

### Beispiel 5: rac-N-(3-Chlor-4-cyanophenyl)-2-hydroxy-2-cyclohexyl-2-(4-trifluormethylphenyl)ethinylacetamid

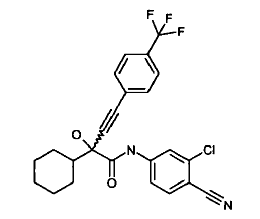

N-(3-Chlor-4-cyanophenyl)-2-oxo-2-cyclohexylacetamid (150 mg) wurde mit 4-Trifluormethylphenylethin und n-Butyllithium in Tetrahydrofuran in Analogie zu Beispiel 2 umgesetzt. Man erhielt nach Säulenchromatographie 140 mg Produkt. ¹H-NMR (ppm, CDCl₃, 400 MHz): 1.12-1.39 (5H), 1.64-1.90 (4H), 2.08-2.16 (2H), 3.08 (1H), 7.54-7.65 (6H), 7.99 (1H), 8.80 (1H).

### Beispiel 6: rac-N-(3-Chlor-4-cyanophenyl)-2-hydroxy-2-cyclohexyl-2-(4-fluorphenyl)ethinylacetamid

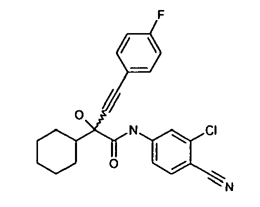

N-(3-Chlor-4-cyanophenyl)-2-oxo-2-cyclohexylacetamid (150 mg) wurde mit 4-Fluorphenylethin und n-Butyllithium in Tetrahydrofuran in Analogie zu Beispiel 2 umgesetzt. Man erhielt nach Säulenchromatographie 140 mg Produkt. ¹H-NMR (ppm, CDCl₃, 400 MHz): 1.21-1.37 (5H), 1.64-1.91 (4H), 2.05-2.16 (2H), 2.95 (1H), 7.02 (2H), 7.45 (2H), 7.56 (1H), 7.64 (1H), 7.98 (1H), 8.79 (1H).

### Beispiel 7: rac-N-(3-Chlor-4-cyanophenyl)-2-hydroxy-2-cyclohexyl-2-(4-formylphenyl)ethinylacetamid

7a) rac-N-(3-Chlor-4-cyanophenyl)-2-hydroxy-2-cyclohexyl-2-(4-(1,3-dioxolanyl)phenyl)-ethinylacetamid 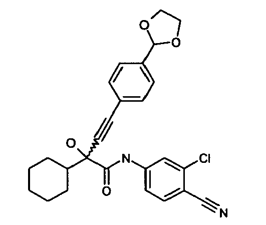 N-(3-Chlor-4-cyanophenyl)-2-oxo-2-cyclohexylacetamid (150 mg) wurde mit 4-(1-3-Dioxolanyl)phenylethin und n-Butyllithium in Tetrahydrofuran in Analogie zu Beispiel 2 umgesetzt. Man erhielt nach Säulenchromatographie 180 mg Produkt. ¹H-NMR (ppm, DMSO-d₆, 400 MHz): 1.07-1.25 (5H), 1.56-1.95 (6H), 3.88-4.03 (4H), 5.70 (1 H), 6.86 (1 H), 7.42 (4H), 7.91 (2H), 8.24 (1 H), 10.38 (1 H). 7b) rac-N-(3-Chlor-4-cyanophenyl)-2-hydroxy-2-cyclohexyl-2-(4-formylphenyl)-ethinylacetamid 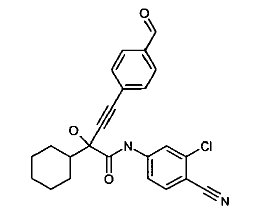 Zu einer Lösung von der unter 7a beschriebene Verbindung (50mg) in Methanol (2 mL) wurde HCl (1M in Wasser, 0.4 mL) bei 23°C addiert. Man ließ bei 23°C 24 Stunden nachrühren. Danach wurde das Reaktionsgemisch auf gesättigte Natriumhydrogencarbonatlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 20 mg Produkt 7b und 23 mg das folgende Produkt 7c. ¹H-NMR (ppm, DMSO-d₆, 400 MHz): 1.10-1.30 (5H), 1.64-1.79 (4H), 1.94-1.99 (2H), 7.66 (2H), 7.89-7.97 (4H), 8.26 (1 H), 10.02 (1H). 7c) rac-N-(3-Chlor-4-cyanophenyl)-2-hydroxy-2-cyclohexyl-2-(4-(1,1-dimethoxyacetyl)-phenyl)ethinylacetamid 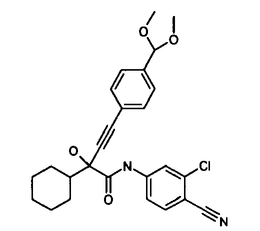 Verbindung 7c wurde als Nebenprodukt bei der Aufreinigung von 7b erhalten. ¹H-NMR (ppm, DMSO-d₆, 400 MHz): 1.11-1.29 (5H), 1.62-1.78 (4H), 1.92-1.99 (2H), 3.24 (6H), 5.40 (1H), 7.39 (2H), 7.46 (2H), 7.80 (1H), 7.96 (1H), 8.26 (1H).

### Beispiel 8: rac-N-(3-Chlor-4-cyanophenyl)-2-hydroxy-2-cyclohexyl-2-(3-methylphenyl)ethinyl-acetamid

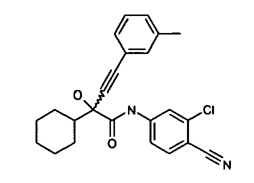

N-(3-Chlor-4-cyanophenyl)-2-oxo-2-cyclohexylacetamid (150 mg) wurde mit 3-Methylphenylethin und n-Butyllithium in Tetrahydrofuran in Analogie zu Beispiel 2 umgesetzt. Man erhielt nach Säulenchromatographie 140 mg Produkt. ¹H-NMR (ppm, CDCl₃, 400 MHz): 1.22-1.42 (5H), 1.73-1.94 (4H), 2.09-2.19 (2H), 2.37 (3H), 3.07 (1 H), 7.20-7.33 (4H), 7.60 (1 H), 7.67 (1 H), 8.02 (1 H), 8.84 (1 H).

### Beispiel 9: rac-N-(3-Chlor-4-cyanophenyl)-2-hydroxy-2-cyclohexyl-2-(2-methylphenyl)ethinyl-acetamid

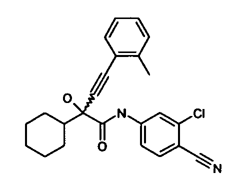

N-(3-Chlor-4-cyanophenyl)-2-oxo-2-cyclohexylacetamid (150 mg) wurde mit 2-Methylphenylethin und n-Butyllithium in Tetrahydrofuran in Analogie zu Beispiel 2 umgesetzt. Man erhielt nach Säulenchromatographie 160 mg Produkt. ¹H-NMR (ppm, CDCl₃, 400 MHz): 1.22-1.37 (5H), 1.70-1.90 (4H), 2.05-2.16 (2H), 2.45 (3H), 3.07 (1 H), 7.15 (1 H), 7.20-7.29 (3H), 7.43 (1 H), 7.56 (1 H), 7.64 (1 H), 7.98 (1 H), 8.81 (1 H).

### Beispiel 10: rac-N-(3-Chlor-4-cyanophenyl)-2-hydroxy-2-cyclohexyl-2-(4-hydroxyphenyl)-ethinylacetamid

10a) rac-N-(3-Chlor-4-cyanophenyl)-2-hydroxy-2-cyclohexyl-2-(4-methoxyphenyl)-ethinylacetamid 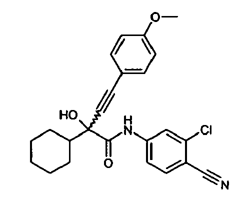 N-(3-Chlor-4-cyanophenyl)-2-oxo-2-cyclohexylacetamid (750 mg) wurde mit 4-Methoxyphenylethin und n-Butyllithium in Tetrahydrofuran in Analogie zu Beispiel 2 umgesetzt. Man erhielt nach Säulenchromatographie 1 g Produkt. ¹H-NMR (ppm, CDCl₃, 400 MHz): 1.13-1.37 (5H), 1.66-1.89 (4H), 2.05-2.16 (2H), 3.08 (1 H), 3.82 (3H), 6.85 (2H), 7.40 (2H), 7.56 (1 H), 7.63 (1 H), 7.98 (1 H), 8.83 (1H). 10b) rac-N-(3-Chlor-4-cyanophenyl)-2-hydroxy-2-cyclohexyl-2-(4-hydroxyphenyl)ethinyl-acetamid 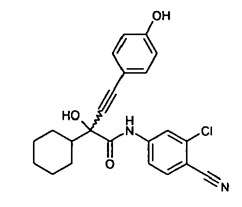 Zu einer Lösung von der unter 10a beschriebene Verbindung (200mg) in Dichlormethan (6 mL) wurde Bortribromid (1 M in Dichlormethan, 2.8 mL) bei -15°C addiert. Man ließ das Reaktionsgemisch bei 23°C über 3 Stunden aufwärmen und man ließ 24 Stunden nachrühren. Danach wurde das Reaktionsgemisch auf eiskalte gesättigte Natriumhydrogencarbonatlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 81 mg Produkt. ¹H-NMR (ppm, CDCl₃, 400 MHz): 1.16-1.43 (5H), 1.65-1.98 (5H), 2.44 (1H), 3.20 (1H), 5.22 (1 H), 6.55 (1 H), 6.78 (2H), 7.19 (2H), 7.44 (1 H), 7.70 (1 H), 8.14 (1H).

### Beispiel 11: rac-N-(3-Chlor-4-cyanophenyl)-2-hydroxy-2-cyclohexyl-2-(1-hydroxypropinyl)-acetamid

11a) rac-N-(3-Chlor-4-cyanophenyl)-2-hydroxy-2-cyclohexyl-2-(1-(2-tetrahydro-pyranyloxy)propinyl)acetamid 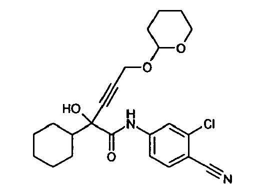 N-(3-Chlor-4-cyanophenyl)-2-oxo-2-cyclohexylacetamid (150 mg) wurde mit Tetrahydro-2-(2-propinyloxy)-2H-pyran und n-Butyllithium in Tetrahydrofuran in Analogie zu Beispiel 2 umgesetzt. Man erhielt nach Säulenchromatographie 190 mg Produkt. ¹H-NMR (ppm, DMSO-d₆, 400 MHz): 1.06-1.20 (5H), 1.43-1.87 (12H), 3.39-3.44 (1H), 3.66-3.73 (1 H), 4.25 (2H), 4.74 (1 H), 7.89 (2H), 8.19 (1 H). 11b) rac-N-(3-Chlor-4-cyanophenyl)-2-hydroxy-2-cyclohexyl-2-(1-hydroxypropinyl)-acetamid 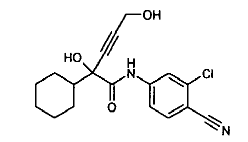 Zu einer Lösung der unter 11a beschriebene Verbindung (50mg) in Aceton (2 mL) wurde HCl (1 M in Wasser, 0.4 mL) bei 23°C addiert. Man ließ bei 23°C 24 Stunden nachrühren. Danach wurde das Reaktionsgemisch auf gesättigte Natriumhydrogencarbonatlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 40 mg Produkt. ¹H-NMR (ppm, DMSO-d₆, 400 MHz): 1.05-1.23 (5H), 1.49-1.93 (6H), 4.13 (2H), 5.22 (1 H), 6.66 (1 H), 7.89-7.97 (2H), 8.24 (1 H), 10.30 (1 H).

### Beispiel 12: rac-N-(3-Chlor-4-cyanophenyl)-2-hydroxy-2-cyclohexyl-2-(4-carboxyphenyl)ethinylacetamid

12a) rac-N-(3-Chlor-4-cyanophenyl)-2-hydroxy-2-cyclohexyl-2-(4-carbomethoxyphenyl)-ethinylacetamid 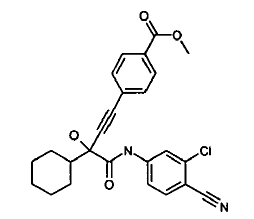 N-(3-Chlor-4-cyanophenyl)-2-oxo-2-cyclohexylacetamid (140 mg) wurde mit 4-Ethinylbenzoesäuremethylester und Lithiumdiisopropylamid in Tetrahydrofuran in Analogie zu Beispiel 2 umgesetzt. Man erhielt nach Säulenchromatographie 130 mg Produkt. ¹H-NMR (ppm, DMSO-d₆, 400 MHz): 1.07-1.26 (5H), 1.58-1.72 (4H), 1.87-1.95 (2H), 3.82 (3H), 6.93 (1H), 7.55 (2H), 7.86-7.95 (4H), 8.23 (1 H), 10.41 (1 H). 12b) rac-N-(3-Chlor-4-cyanophenyl)-2-hydroxy-2-cyclohexyl-2-(4-carboxyphenyl)ethinyl-acetamid 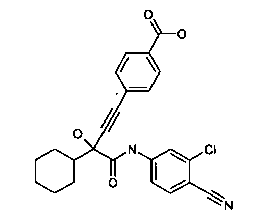 Zu einer Lösung von der unter 12a beschriebene Verbindung (50mg) in Methanol (1.5 mL) wurde eine Lösung Kaliumcarbonat (100mg) im Wasser (50 µL) bei 23°C addiert.

Man ließ 4 Tage bei 23°C nachrühren. Danach wurde das Reaktionsgemisch auf 40°C erwärmt und man ließ 4 Stunden nachrühren. Danach wurde das Reaktionsgemisch auf gesättigte Ammoniumchloridlösung gegossen. Es wurde mit Ethylacetat extrahiert. Die vereinigten organischen Phasen wurden mit gesättigter Natriumchloridlösung gewaschen und über Natriumsulfat getrocknet. Das Rohprodukt wurde an Kieselgel chromatographiert. Man erhielt 28 mg Produkt. ¹H-NMR (ppm, DMSO-d₆, 400 MHz): 1.02-1.31 (5H), 1.58-1.76 (4H), 1.87-1.95 (2H), 6.93 (1H), 7.53 (2H), 7.86-7.95 (4H), 8.23 (1H), 10.41 (1H), 13.11 (1H).

### Beispiel 13: rac-N-(3-Chlor-4-cyanophenyl)-2-hydroxy-2-cyclohexyl-2-(2,5-dimethylphenyl)-ethinylacetamid

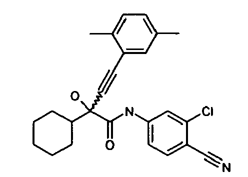

N-(3-Chlor-4-cyanophenyl)-2-oxo-2-cyclohexylacetamid (150 mg) wurde mit 2,5-Dimethylphenylethin und n-Butyllithium in Tetrahydrofuran in Analogie zu Beispiel 2 umgesetzt. Man erhielt nach Säulenchromatographie 190 mg Produkt. ¹H-NMR (ppm, CDCl₃, 400 MHz): 1.14-1.38 (5H), 1.70-1.90 (4H), 2.05-2.16 (2H), 2.29 (3H), 2.40 (3H), 3.07 (1 H), 7.09 (2H), 7.25 (1 H), 7.56 (1 H), 7.63 (1 H), 7.98 (1 H), 8.82 (1H).

### Beispiel 14: rac-N-(3-Chlor-4-cyanophenyl)-2-hydroxy-2-cyclohexyl-2-(3-methoxyphenyl)-ethinylacetamid

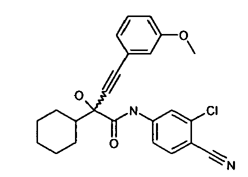

N-(3-Chlor-4-cyanophenyl)-2-oxo-2-cyclohexylacetamid (750 mg) wurde mit 3-Methoxyphenylethin und n-Butyllithium in Tetrahydrofuran in Analogie zu Beispiel 2 umgesetzt. Man erhielt nach Säulenchromatographie 1.0 g Produkt. ¹H-NMR (ppm, CDCl₃, 400 MHz): 1.14-1.38 (5H), 1.66-1.90 (4H), 2.10-2.18 (2H), 3.15 (1H), 3.80 (3H), 6.92 (1H), 6.98 (1H), 7.06 (1H), 7.22 (1H), 7.56 (1H), 7.63 (1H), 7.98 (1H), 8.83 (1H).

### Beispiel 15: rac-N-(3-Chlor-4-cyanophenyl)-2-cyclohexyl-2-hydroxy-3-m-tolyl-propionamid

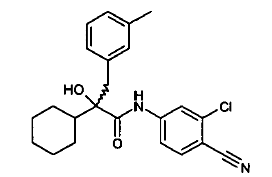

Die Herstellung erfolgte analog Beispiel 3 mit 3-Methyl-benzylmagnesiumchlorid. ¹H-NMR (ppm, CDCl₃, 400 MHz): 1.0 - 2.2 (m, 11 H), 2.20 (s, 1 H), 2.29 (s, 3H), 2.90 (d, 1 H), 3.45 (d, 1 H), 6.97 - 7.33 (m, 4H), 7.41 (dd, 1H), 7.60 (d, 1 H), 7.85 (d, 1 H), 8.56 (s, 1 H).

### Beispiel 16: rac-N-(3-Chlor-4-cyanophenyl)-2-cyclohexyl-2-hydroxy-3-(4-methoxy-phenyl)-propionamid

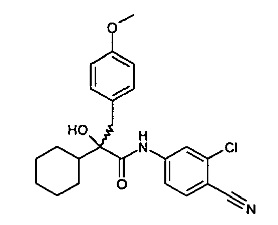

Die Herstellung erfolgte analog Beispiel 3 mit 4-Methoxy-benzylmagnesiumchlorid. ¹H-NMR (ppm, CDCl₃, 400 MHz): 1.09 - 1.40 (m, 5H), 1.63 - 2.04 (m, 6H), 2.11 (s, 1H), 2.83 (d, 1 H), 3.38 (d, 1 H), 3.75 (s, 3H), 6.80 (d, 2H), 7.07 (d, 2H), 7.37 (dd, 1 H), 7.55 (d, 1 H), 7.84 (d, 1 H), 8.54 (s, 1 H).

### Beispiel 17: rac-N-(3-Chlor-4-cyanophenyl)-2-cyclohexyl-2-hydroxy-3-(3-methoxy-phenyl)-propionamid

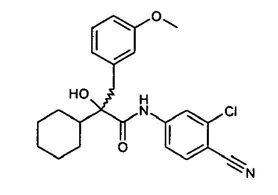

Die Herstellung erfolgte analog Beispiel 3 mit 3-Methoxy-benzylmagnesiumchlorid. ¹H-NMR (ppm, CDCl₃, 400 MHz): 1.05 - 1.40 (m, 5H), 1.60 - 2.05 (m, 6H), 2.88 (d, 1 H), 3.30 (d, 1H), 3.70 (s, 3H), 6.75 (m, 3H), 7.16 (dd, 1 H), 7.40 (dd, 1 H), 7.55 (d, 1 H), 7.82 (d, 1 H), 8.75 (s, 1 H).

### Beispiel 18: rac-N-(3-Chlor-4-cyanophenyl)-2-cyclohexyl-2-hydroxy-3-(3-iod-phenyl)-propionamid

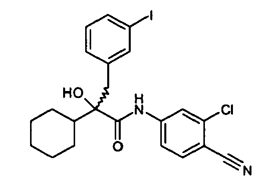

35 ml 3-lodbenzylzinkbromidlösung (0.5 M in THF) wurden in 15 nl THF vorgelegt und auf -75°C gekühlt. 1.0 g N-(3-Chlor-4-cyanophenyl)-2-cyclohexyl-2-oxo-acetamid, gelöst in 15 ml THF wurden zugetropft. Es wurde 4 h bei -75°C und eine weitere Stunde bei Raumtemperatur gerührt, dann auf ges. Ammoniumchloridlösung gegeben und mit Ethylacetat extrahiert. Die organischen Phasen wurden mit ges. NaCl-Lösung gewaschen und mit Natriumsulfat getrocknet. Das Rohprodukt wurde chromatographisch gereinigt und anschließend aus Hexan/Diisopropylether umkristallisiert. Man erhielt 338 mg des gewünschten Produkts als farblosen Feststoff. ¹H-NMR (ppm, CDCl₃, 400 MHz): 1.0 - 1.4 (m, 5H), 1.6 - 2.0 (m, 6H), 2.03 (s, 1 H), 2.85 (d, 1 H), 3.27 (d, 1 H), 6.97 (dd, 1 H), 7.12 (d, 1 H), 7.36 (dd, 1 H), 7.55 (m, 3H), 7.79 (s, 1 H), 8.46 (s, 1 H).

### Beispiel 19: rac-3-(4'-Acetyl-biphenyl-2-yl)-N-(3-chlor-4-cyano-phenyl)-2-cyclohexyl-2-hydroxy-propionamid

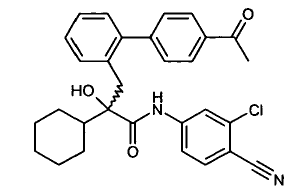

rac-N-(3-Chlor-4-cyanophenyl)-2-cyclohexyl-2-hydroxy-3-(2-iodo-phenyl)-propionamid wurde analog Beispiel 18 hergestellt. 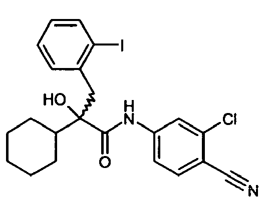 390 mg der lodverbindung und 197 mg 4-Acetylphenylboronsäure wurden in 6 ml Toluol/Ethanol 1:1 vorgelegt und 1.5 ml 1M Natriumcarbonatlösung und 90 mg Tetrakistriphenylphosphin-palladium zugegeben. Die Mischung wurde 25 min bei 120°C in der Mikrowelle erhitzt, dann über Celite abfiltriert und mit Ethylacetat gespült. Die Lösung wurde mit ges. NaCl-Lösung gewaschen, mit atriumsulfat getrocknet und eingeengt. Das Rohprodukt wurde chromatographisch gereinigt. Man erhielt 137 mg des gewünschten Produkts als farblosen Schaum. ¹H-NMR (ppm, DMSO-D₆, 400 MHz): 0-95 - 1.35 (m, 5H), 1.50 - 1.80 (m, 6H), 2.63 (s, 3H), 3.03 (d, 1 H), 3.16 (d, 1 H), 7.09 (m, 1 H), 7.23 (m, 2H), 7.46 (d, 2H), 7.59 (m, 1 H), 7.76 (dd, 1 H), 7.84 (d, 1 H), 7.97 (d, 2H), 8.05 (d, 1H), 9.91 (s, 1 H).

### Beispiel 20: rac-N-(4-Cyano-3-trifluormethyl-phenyl)-2-cyclohexyl-2-hydroxy-3-phenyl-propionamid

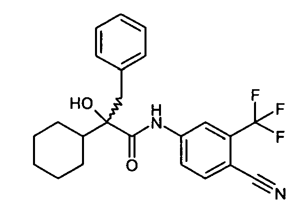

Die Herstellung erfolgte analog Beispiel 3 aus N-(4-cyano-3-trifluormethyl-phenyl)-2-cyclohexyl-2-oxo-acetamid (hergestellt analog N-(3-Chlor-4-cyano-phenyl)-2-cyclohexyl-2-oxo-acetamid, siehe oben) und Benzylmagnesiumchlorid. ¹H-NMR (ppm, CDCl₃, 400 MHz): 1.05 - 1.40 (m, 5H), 1.65 - 2.05 (m, 6H), 2.15 (s, 1 H), 2.92 (d, 1 H), 3.41 (d, 1H), 7.16 (m, 2H), 7.24 (m, 3H), 7.73 (d, 1 H), 7.81 (dd, 1 H), 7.86 (d, 1 H), 8.63 (s, 1H). Das erhaltene racemische Gemisch wurde durch präparative chirale HPLC (Säule Chiralpak AD 250x10 mm) in die Enantiomeren 20a und 20b getrennt.

### Beispiel 20a: [α]^{D}₂₀ = -129.4° (MeOH, c = 1.01) Beispiel 20b: [α]^{D}₂₀ = +132.7° (MeOH, c = 1.00)

### Beispiel 21: rac-N-(4-Cyano-3-trifluormethyl-phenyl)-2-cyclohexyl-2-hydroxy-3-m-tolyl-propionamid

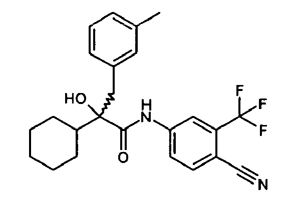

Die Herstellung erfolgte analog Beispiel 20 mit 3-Methyl-benzylmagnesiumchlorid. ¹H-NMR (ppm, CDCl₃, 400 MHz): 1.2 - 1.5 (m, 5H), 1.6 - 2.1 (m, 6H), 2.20 (s, 1 H), 2.28 (s, 3H), 2.94 (d, 1 H), 3.42 (d, 1 H), 7.0 - 7.3 (m, 4H), 7.85 (m, 3H), 8.68 (s, 1 H).

### Beispiel 22: rac-N-(4-Cyano-3-trifluormethyl-phenyl)-2-cyclohexyl-2-hydroxy-3-p-tolyl-propionamid

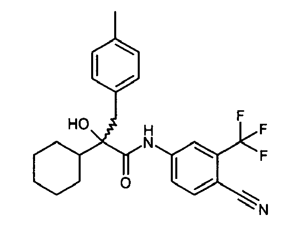

Die Herstellung erfolgte analog Beispiel 20 mit 4-Methyl-benzylmagnesiumchlorid. ¹H-NMR (ppm, DMSO-D₆, 400 MHz): 1.0 - 1.3 (m, 5H), 1.4 - 1.8 (m, 6H), 2.12 (s, 1 H), 2.23 (s, 3H), 2.84 (d, 1 H), 2.98 (d, 1 H), 7.0 - 7.3 (m, 4H), 7.97 (d, 1 H), 8.11 (dd, 1 H), 8.23 (d, 1 H), 9.90 (s, 1 H).

### Beispiel 23: rac-N-(4-Cyano-3-trifluormethyl-phenyl)-2-cyclohexyl-2-hydroxy-3-(4-methoxy-phenyl)-propionamid

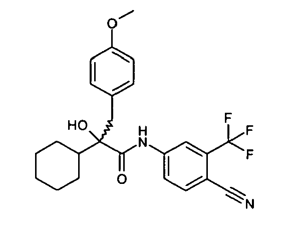

Die Herstellung erfolgte analog Beispiel 20 mit 4-Methoxy-benzylmagnesiumchlorid. ¹H-NMR (ppm, CDCl₃, 400 MHz): 1.10 - 1.45 (m, 5H), 1.65 - 2.05 (m, 6H), 2.19 (s, 1 H), 2.90 (d, 1 H), 3.42 (d, 1 H), 3.79 (s, 3H), 6.84 (d, 2H), 7.12 (d, 2H), 7.79 (d, 1 H), 7.88 (dd, 1 H), 7.94 (d, 1 H), 8.71 (s, 1 H).

Das erhaltene racemische Gemisch wurde durch präparative chirale HPLC (Säule Chiralpak AD 250x10 mm) in die Enantiomeren 23a und 23b getrennt.

### Beispiel 23a: Rₜ = 5.41 min (Chiralpak IA 5µ 150x4.6, 80% Hexan/20%2-Propanol, 1 ml/min)

### Beispiel 23b: Rₜ = 6.36 min (Chiralpak IA 5µ 150x4.6, 80% Hexan/20%2-Propanol, 1 ml/min)

### Beispiel 24: rac-N-(4-Cyano-3-trifluormethyl-phenyl)-2-cyclohexyl-2-hydroxy-3-(3-methoxyphenyl)-propionamid

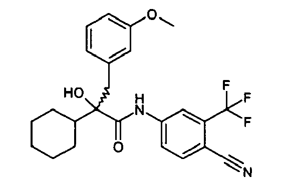

Die Herstellung erfolgte analog Beispiel 3 mit 4-Methoxy-benzylmagnesiumchlorid. ¹H-NMR (ppm, CDCl₃, 400 MHz): 1.0 - 2.0 (m, 11 H), 2.88 (d, 1H), 2.97 (d, 1 H), 3.56 (s, 3H), 5.44 (s, 1 H), 6.58 (m, 1 H), 6.74 (m, 2H), 7.00 (dd, 1 H), 8.17 (dd, 1 H), 8.27 (d, 1 H), 9.92 (s, 1 H).

### Beispiel 25: rac-N-(4-Cyano-3-trifluormethyl-phenyl)-2-cyclohexyl-2-hydroxy-3-(2-iodo-phenyl)-propionamid

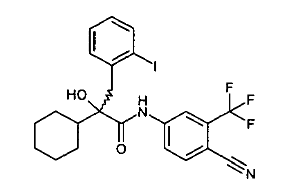

Die Herstellung erfolgte analog Beispiel 18 unter Verwendung von 2-lodbenzylzinkbromid. ¹H-NMR (ppm, CDCl₃, 400 MHz): 1.0 - 1.4 (m, 5H), 1.6 - 2.0 (m, 6H), 2.03 (s, 1 H), 2.85 (d, 1 H), 3.27 (d, 1 H), 6.97 (dd, 1 H), 7.12 (d, 1 H), 7.36 (dd, 1 H), 7.55 (m, 3H), 7.79 (s, 1 H), 8.46 (s, 1 H).

### Beispiel 26: rac-N-(4-Cyano-3-trifluormethyl-phenyl)-2-cyclohexyl-2-hydroxy-3-(3-iodo-phenyl)-propionamid

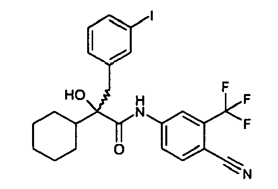

Die Herstellung erfolgte analog Beispiel 25 unter Verwendung von 3-lodbenzylzinkbromid. ¹H-NMR (ppm, CDCl₃, 400 MHz): 1.05 - 1.40 (m, 5H), 1.60 - 2.05 (m, 6H), 2.06 (s, 1H), 2.86 (d, 1 H), 3.27 (d, 1 H), 6.96 (m, 1H), 7.12 (m, 1 H), 7.55 (d, 2H), 7.76 (m, 2H), 7.87 (s, 1 H), 8.58 (s, 1 H).

### Beispiel 27: rac-3-(4'-Acetyl-biphenyl-2-yl)-N-(4-cyano-3-trifluormethyl-phenyl)-2-cyclohexyl-2-hydroxy-propionamid

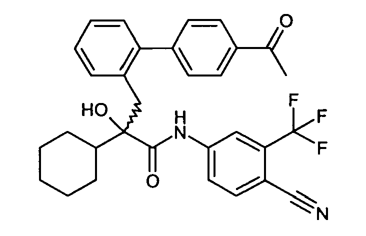

Die Herstellung erfolgte analog Beispiel 19 unter Verwendung der unter Beispiel 25 hergestellten Verbindung. ¹H-NMR (ppm, DMSO-D₆, 400 MHz): 0.90 - 1.25 (m, 5H), 1.45 - 1.80 (m, 6H), 2.57 (s, 3H), 3.01 (d, 1H), 3.13 (d, 1 H), 5.49 (s, 1H), 7.04 (dd, 1 H), 7.17 (m, 2H), 7.39 (d, 2H), 7.91 (d, 2H), 8.00 (m, 2H), 8.05 (m, 1H), 8.26 (d, 1H), 10.07 (s, 1 H).

### Beispiel 28: rac-3-(4'-Acetyl-biphenyl-3-yl)-N-(4-cyano-3-trifluormethyl-phenyl)-2-cyclohexyl-2-hydroxy-propionamid

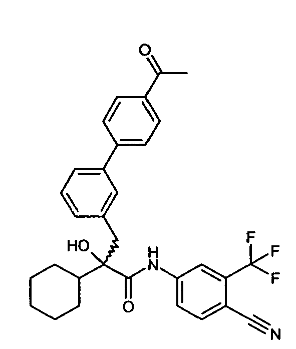

Die Herstellung erfolgte analog Beispiel 19 unter Verwendung der unter Beispiel 26 hergestellten Verbindung. ¹H-NMR (ppm, CDCl₃, 400 MHz): 1.05 - 1.45 (m, 5H), 1.65 - 2.10 (m, 6H), 2.22 (s, 1 H), 2.63 (s, 3H), 3.01 (d, 1 H), 3.44 (d, 1 H), 7.20 (m, 1H), 7.35 (m, 1 H), 7.44 (m, 1H), 7.50 (d, 2H), 7.69 (m, 3H), 7.90 (m, 1 H), 7.95 (d, 2H), 8. 64 (s, 1 H).

### Beispiel 29: rac-6-[2-cyclohexyl-2-hydroxy-2-(phenylethinyl)ethanoylamino]-4-methyl-2,3-benzoxazin-1-on

29a) rac-6-[2-cyclohexyl-2-oxo-ethanoylamino]-4-methyl-2,3-benzoxazin-1-on 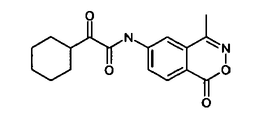 2-Oxo-2-cyclohexylessigsäure (200 mg) wurde mit 6-Amino-4-methyl-2,3-benzoxazin-1-on und Thionylchlorid in N,N-Dimethylacetamid in Analogie zu Beispiel d umgesetzt. Man erhielt nach Säulenchromatographie 360 mg Produkt. ¹H-NMR (ppm, CDCl₃, 400 MHz): 1.23-1.51 (5H), 1.73-1.98 (5H), 2.61 (3H), 3.45-3.53 (1H), 7.84 (1H), 8.36 (1H), 8.39 (1H), 9.19 (1H). 29b) rac-6-[2-cyclohexyl-2-hydroxy-2-(phenylethinyl)ethanoylamino]-4-methyl-2,3-benz-oxazin-1-on 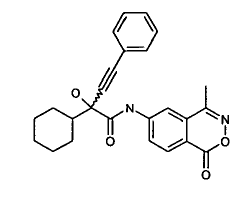 6-[2-Cyclohexyl-2-oxo-ethanoylamino]-4-methyl-2,3-benzoxazin-1-on (200 mg) wurde mit Phenylethin und n-Butyllithium in Tetrahydrofuran in Analogie zu Beispiel 2 umgesetzt. Man erhielt nach Säulenchromatographie 160 mg Produkt. ¹H-NMR (ppm, DMSO-d₆, 400 MHz): 1.00-1.32 (5H), 1.58-1.77 (4H), 1.93-2.02 (2H), 2.47 (3H), 6.86 (1 H), 7.34-7.43 (5H), 8.19 (1 H), 8.37-8.41 (2H), 10.50 (1 H).

### Beispiel 30: rac-2-Cyclohexyl-2-hydroxy-N-(4-methyl-1-oxo-1 H-benzo[d][1,2]oxazin-6-yl)-3-phenyl-propionamid

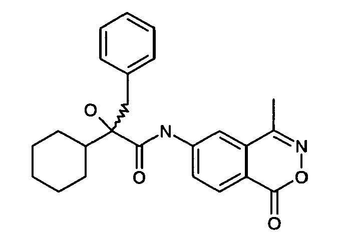

Die Herstellung erfolgte analog Beispiel 3 unter Verwendung des unter Beispiel 29a beschriebenen Ketoamids und Benzylmagnesiumchlorid. ¹H-NMR (ppm, DMSO-D₆, 400 MHz): 1.00 - 1.35 (m, 5H), 1.45 - 1.85 (m, 5H), 1.95 (m, 1 H), 2.40 (s, 3H), 2.90 (d, 1 H), 3.06 (d, 1H), 5.48 (s, 1 H), 7.00 - 7.25 (m, 5H), 8.06 (m, 1H), 8.10 (s, 1H), 8.25 (dd, 1 H), 9.81 (s, 1 H). Das erhaltene racemische Gemisch wurde durch präparative chirale HPLC (Säule Chiralpak AD 250x10 mm) in die Enantiomeren 30a und 30b getrennt.

### Beispiel 30a: [α]^{D}₂₀ = -119.4° (DMSO, c = 0.54)

### Beispiel 30b: [α]^{D}₂₀ = +113.5° (DMSO, c = 0.57)

### Beispiel 31: rac-2-Cyclohexyl-2-hydroxy-3-(4-methoxy-phenyl)-N-(4-methyl-1-oxo-1 H-benzo[d][1,2]oxazin-6-yl)-propionamid

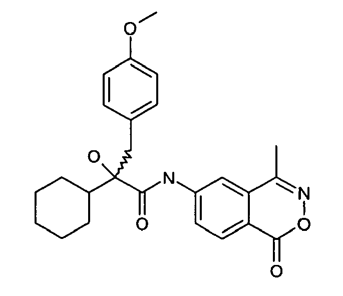

Die Herstellung erfolgte analog Beispiel 30 unter Verwendung von 4-Methoxybenzylmagnesiumchlorid. ¹H-NMR (ppm, CDCl₃, 400 MHz): 1.12 - 1.47 (m, 5H), 1.55 - 2.14 (m, 6H), 2.23 (s, 1 H), 2.64 (s, 3H), 2.92 (d, 1H), 3.44 (d, 1 H), 3.86 (s, 3H), 6.83 (d, 2H), 7.14 (d, 2H), 7.62 (dd, 1 H), 8.25 (d, 1H), 8.31 (d, 1 H), 8.81 (s, 1 H).

### Beispiel 32: rac-N-(3-Chlor-4-Cyano-phenyl)-2-cyclopentyl-2-hydroxy-3-phenyl-propionamid

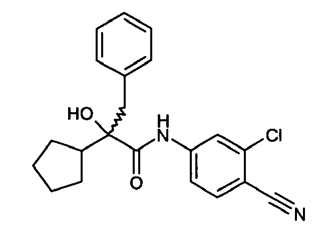

Die Verbindung wurde analog Beispiel 3 durch Umsetzung von N-(3-Chloro-4-cyano-phenyl)-2-cyclopentyl-2-oxo-acetamid (erhalten analog zur entsprechenden Cyclohexylverbindung, ¹H-NMR (ppm, CDCl₃, 400 MHz): 1.71 (m, 4H), 1.82 (m, 2H), 2.01 (m, 2H), 3.85 (m, 1 H), 7.60 (dd, 1 H), 7.68 (d, 1 H), 8.03 (d, 1H), 9.01 (s, 1H)) mit Benzylmagnesiumchlorid erhalten. ¹H-NMR (ppm, CDCl₃, 400 MHz): 1.45 - 1.95 (m, 8H), 2.50 (m, 1 H), 2.84 (d, 1 H), 3.50 (d, 1 H), 7.16 (m, 2H), 7.27 (m, 3H), 7.37 (dd, 1 H), 7.55 (d, 1 H), 7.82 (d, 1 H), 8.52 (s, 1 H).

### Beispiel 33: rac-N-(3-Chlor-4-cyano-phenyl)-2-cyclopentyl-2-hydroxy-3-p-tolyl-propionamid

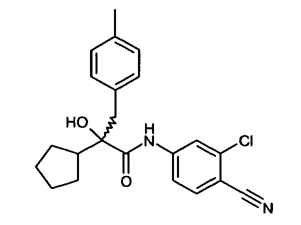

Die Herstellung erfolgte analog Beispiel 32 mit 4-Methyl-benzylmagnesiumchlorid. ¹H-NMR (ppm, CDCl₃, 400 MHz): 1.45 - 1.75 (m, 7H), 1.87 (m, 1 H), 2.15 (s, 1 H), 2.29 (s, 3H), 2.48 (m, 1 H), 2.79 (d, 1 H), 3.48 (d, 1 H), 7.06 (m, 4H), 7.39 (dd, 1 H), 7.56 (d, 1 H), 7.83 (d, 1 H), 8.55 (s, 1 H).

### Beispiel 34: rac-N-(3-Chlor-4-cyano-phenyl)-2-cyclopentyl-2-hydroxy-3-m-tolyl-propionamid

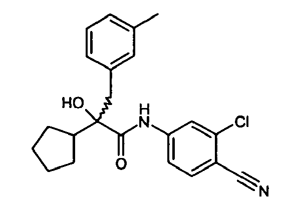

Die Herstellung erfolgte analog Beispiel 32 mit 3-Methyl-benzylmagnesiumchlorid. ¹H-NMR (ppm, CDCl₃, 400 MHz): 1.45 - 1.73 (m, 7H), 1.85 (m, 1 H), 2.17 (s, 1 H), 2.25 (s, 3H), 2.49 (m, 1 H), 2.79 (d, 1 H), 3.48 (d, 1 H), 6.95 (m, 2H), 7.05 (d, 1 H), 7.16 (m, 1 H), 7.38 (dd, 1 H) 7.56 (d, 1 H), 7.82 (d, 1 H), 8.53 (s, 1 H).

### Beispiel 35: rac-N-(3-Chlor-4-cyanophenyl)-2-cyclopentyl-2-hydroxy-3-(4-methoxy-phenyl)-propionamid

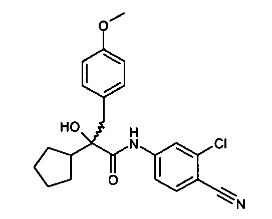

Die Herstellung erfolgte analog Beispiel 32 mit 4-Methoxy-benzylmagnesiumchlorid. ¹H-NMR (ppm, CDCl₃, 400 MHz): 1.46 - 1.79 (m, 7H), 1.91 (m, 1 H), 2.17 (s, 1 H), 2.52 (m, 1 H), 2.81 (d, 1 H), 3.50 (d, 1H), 3.80 (s, 3H), 6.85 (m, 2H), 7.12 (m, 2H), 7.44 (dd, 1 H), 7.61 (d, 1 H), 7.89 (d, 1 H), 8.61 (s, 1 H).

### Beispiel 36: rac-N-(3-Chlor-4-cyanophenyl)-2-cyclopentyl-2-hydroxy-3-(3-methoxy-phenyl)-propionamid

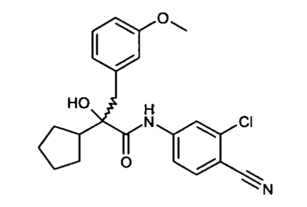

Die Herstellung erfolgte analog Beispiel 32 mit 3-Methoxy-benzylmagnesiumchlorid. ¹H-NMR (ppm, CDCl₃, 400 MHz): 1.46 - 1.72 (m, 7H), 1.87 (m, 1 H), 2.20 (s, 1 H), 2.49 (m, 1 H), 2.80 (d, 1 H), 3.50 (d, 1H), 3.69 (s, 3H), 6.75 (m, 3H), 7.19 (m, 1 H), 7.40 (dd, 1 H), 7.56 (d, 1 H), 7.84 (d, 1 H), 8.58 (s, 1 H).

### Beispiel 37: rac-N-(3-Chtor-4-cyanophenyl)-2-cyctopentyl-2-hydroxy-3-(3-fluor-phenyl)-propionamid

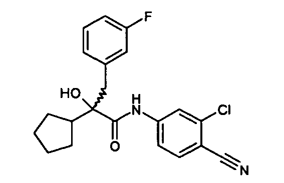

Die Herstellung erfolgte analog Beispiel 18, jedoch unter Verwendung von 3-Fluorbenzylzinkchloridlösung und N-(3-Chlor-4-cyanophenyl)-2-cyclopentyl-2-oxo-acetamid (siehe Beispiel 32) ¹H-NMR (ppm, CDCl₃, 400 MHz): 1.40 - 1.75 (m, 7H), 1.87 (m, 1 H), 2.10 (s, 1 H), 2.51 (m, 1 H), 2.84 (d, 1 H), 3.45 (d, 1 H), 6.93 (m, 3H), 7.21 (m, 1 H), 7.38 (m, 1H), 7.56 (d, 1 H), 7.81 (d, 1H), 8.53 (s, 1 H).

### Beispiel 38: rac-N-(3-Chlor-4-cyanophenyl)-2-cyclopentyl-2-hydroxy-3-(3-chlor-phenyl)-propionamid

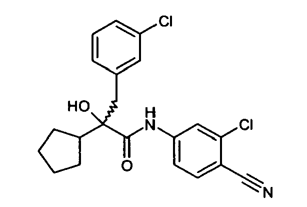

Die Herstellung erfolgte analog Beispiel 37 unter Verwendung von 3-Chlorbenzylzinkchloridlösung ¹H-NMR (ppm, CDCl₃, 400 MHz): 1.41 - 1.75 (m, 7H), 1.89 (m, 1 H), 2.09 (s, 1 H), 2.52 (m, 1 H), 2.81 (d, 1 H), 3.40 (d, 1 H), 7.04 (m, 1 H), 7.19 (m, 3H), 7.39 (m, 1 H), 7.56 (m, 1 H), 7.80 (d, 1 H), 8.51 (s, 1 H).

### Beispiel 39: rac-N-(4-Cyano-3-trifluormethyl-phenyl)-2-cyclopentyl-2-hydroxy-3-phenyl-propionamid

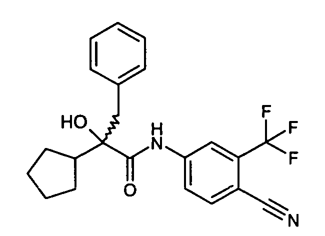

Die Verbindung wurde analog Beispiel 32 durch Umsetzung von N-(4-Cyano-3-trifluormethyl-phenyl)-2-cyclopentyl-2-oxo-acetamid (erhalten analog zur entsprechenden Verbindung mit Cl-Substituenten, ¹H-NMR (ppm, CDCl₃, 400 MHz): 1.60 - 1.87 (m, 7H), 1.99 (m, 1 H), 3.84 (m, 1H), 7.84 (d, 1 H), 7.99 (dd, 1 H), 8.16 (d, 1 H), 9.12 (s, 1H)) mit Benzylmagnesiumchlorid erhalten. ¹H-NMR (ppm, CDCl₃, 400 MHz): 1.45 - 1.75 (m, 7H), 1.89 (m, 1 H), 2.16 (s, 1 H), 2.52 (m, 1 H), 2.85 (d, 1 H), 3.50 (d, 1 H), 7.17 (dd, 2H), 7.28 (m, 3H), 7.74 (d, 1 H), 7.82 (dd, 1 H), 7.88 (d, 1 H), 8.65 (s, 1 H).

### Beispiel 40: rac-N-(4-Cyano-3-trifluormethyl-phenyl)-2-cyclopentyl-2-hydroxy-3-p-tolyl-propionamid

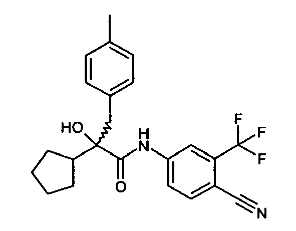

Die Herstellung erfolgte analog Beispiel 33. ¹H-NMR (ppm, CDCl₃, 400 MHz): 1.48 - 1.72 (m, 7H), 1.89 (m, 1H), 2.18 (s, 1 H), 2.28 (s, 3H), 2.49 (m, 1 H), 2.81 (d, 1 H), 3.48 (d, 1 H), 7.06 (m, 4H), 7.75 (d, 1 H), 8.86 (m, 2H), 8.67 (s, 1 H).

### Beispiel 41: rac-N-(4-Cyano-3-trifluormethyl-phenyl)-2-cyclopentyl-2-hydroxy-3-m-tolyl-propionamid

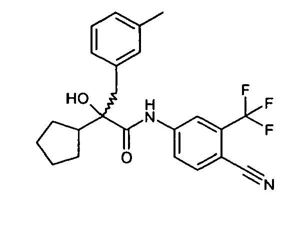

Die Herstellung erfolgte analog Beispiel 34. ¹H-NMR (ppm, CDCl₃, 400 MHz): 1.45 - 1.75 (m, 7H), 1.89 (m, 1 H), 2.19 (s, 1 H), 2.24 (s, 3H), 2.51 (m, 1 H), 2.80 (d, 1 H), 3.47 (d, 1 H), 6.96 (m, 2H), 7.05 (d, 1 H), 7.16 (m, 1H), 7.75 (d, 1 H) 7.83 (dd, 1 H), 7.88 (d, 1 H), 8.66 (s, 1 H).

### Beispiel 42: rac-N-(4-Cyano-3-trifluormethyl-phenyl)-2-cyclopentyl-2-hydroxy-3-(4-methoxy-phenyl)-propionamid

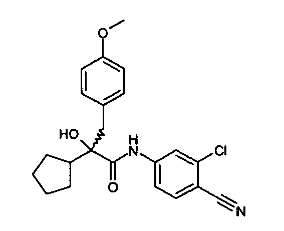

Die Herstellung erfolgte analog Beispiel 35. ¹H-NMR (ppm, CDCl₃, 400 MHz): 1.50 - 1.75 (m, 7H), 1.93 (m, 1 H), 2.21 (s, 1 H), 2.53 (m, 1 H), 2.83 (d, 1 H), 3.51 (d, 1H), 3.79 (s, 3H), 6.85 (m, 2H), 7.13 (d, 2H), 7.79 (d, 1H), 7.90 (dd, 1 H), 7.95 (d, 1 H), 8.73 (s, 1H).

### Beispiel 43: rac-N-(4-Cyano-3-trifluormethyl-phenyl)-2-cyclopentyl-2-hydroxy-3-(3-methoxy-phenyl)-propionamid

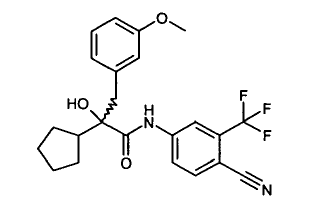

Die Herstellung erfolgte analog Beispiel 36. ¹H-NMR (ppm, CDCl₃, 400 MHz): 1.46 - 1.73 (m, 7H), 1.88 (m, 1 H), 2.23 (s, 1 H), 2.50 (m, 1 H), 2.81 (d, 1 H), 3.49 (d, 1 H), 3.68 (s, 3H), 6.69 - 6.80 (m, 3H), 7.19 (dd, 1 H), 7.74 (d, 1 H), 7.84 (dd, 1 H), 7.91 (d, 1 H), 8.70 (s, 1H).

### Beispiel 44: rac-N-(4-Cyano-3-trifluormethyl-phenyl)-2-cyclopentyl-2-hydroxy-3-phenyl-propionamid

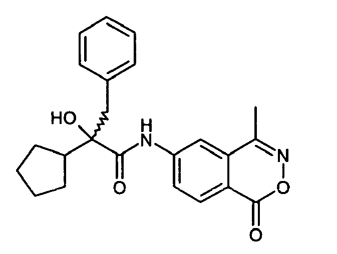

Die Verbindung wurde analog Beispiel 32 durch Umsetzung von 2-Cyclopentyl-N-(4-methyl-1-oxo-1H-benzo[d][1,2]oxazin-6-yl)-2-oxo-acetamid (erhalten analog zur entsprechenden Cyclohexylverbindung aus Beispiel 15^{a}), ¹H-NMR (ppm, CDCl₃, 400 MHz): 1.71 (m, 4H), 1.82 (m, 2H), 2.01 (m, 2H), 2.61 (s, 3H), 3.86 (m, 1 H), 7.84 (dd, 1 H), 8.35 (d, 1 H), 8.38 (d, 1 H), 9.21 (s, 1 H)) mit Benzylmagnesiumchlorid erhalten. ¹H-NMR (ppm, CDCl₃, 400 MHz): 1.50 - 1.80 (m, 7H), 1.96 (m, 1 H), 2.25 (s, 1 H), 2.59 (m, 1 H), 2.63 (s, 3H), 2.93 (d, 1 H), 3.57 (d, 1 H), 7.21 - 7.43 (m, 5H), 7.61 (dd, 1 H), 8.25 (d, 1H), 8.32 (d, 1 H), 8.79 (s, 1 H).

### Beispiel 45: rac-N-(4-Cyano-3-trifluormethyl-phenyl)-2-hydroxy-2-hydroxy-3-phenyl-2-(tetrahydropyran-4-yl)-propionamid

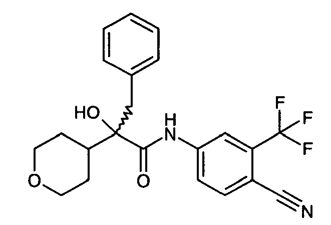

a) Acetoxy-(tetrahydropyran-4-yliden)-essigsäureethylester 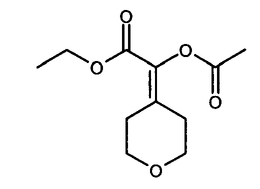 7.0 g Ethyl-acetoxy(diethoxyphosphoryl)acetat wurden in 25 ml THF vorgelegt, 1.06 g Lithiumchlorid zugegeben und die Mischung auf 0°C gekühlt. 3.1 ml N,N.N',N'-Tetramethylguanidin wurden zugetropft und 15 min gerührt. Dann wurden 2.46 g Tetrahydro-4H-pyran-4-on, gelöst in 10 ml THF zugetropft. Man ließ auf Raumtemperatur kommen und 18 h rühren. Die Mischung wurde zwischen Ethylacetat und Wasser verteilt, die Phasen getrennt und mit Ethylacetat extrahiert. Die vereinigten organischen Phasen wurden mit Natriumsulfat getrocknet und eingeengt. Der Rückstand wurde an Kieselgel chromatographiert. Man erhielt 3.37 g des gewünschten Zwischenprodukts als gelbliche Flüssigkeit. ¹H-NMR (ppm, CDCl₃, 400 MHz): 1.28 (t, 3H), 2.21 (s, 3H), 2.36 (t, 2H), 2.98 (t, 2H), 3.75 (m, 4H), 4.21 (q, 2H).
b) Oxo-(tetrahydropyran-4-yl)-essigsäure 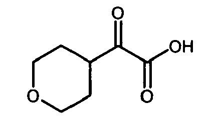 870 mg Acetoxy-(tetrahydropyran-4-yliden)-essigsäureethylester wurden in 8 ml 1 M Natronlauge in Ethanol/Wasser 2:1 gegeben. Man ließ 15 min bei Raumtemperatur rühren, verdünnte dann mit kaltem Wasser, säuerte mit Salzsäure an und extrahierte mit Ethylacetat. Die vereinigten organischen Phasen wurden mit Natriumsulfat getrocknet und eingeengt. Man erhielt 540 mg Oxo-(tetrahydropyran-4-yl)-essigsäure als farblosen kristallinen Feststoff. ¹H-NMR (ppm, CDCl₃, 400 MHz): 1.77 (m, 2H), 1.89 (m, 2H), 3.45 (m, 1 H), 3.56 (m, 2H), 4.07 (m, 2H), 8.76 (br s, 1 H).
c) N-(4-Cyano-3-trifluormethyl-phenyl)-2-oxo-2-(tetrahydropyran-4-yl)-acetamid 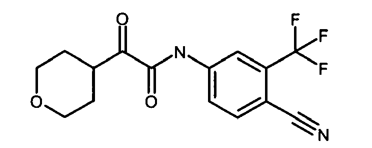 Die Herstellung erfolgte analog der entsprechenden Cyclohexylverbindung (siehe oben) aus Oxo-(tetrahydropyran-4-yl)-essigsäure, 4-Cyano-3-trifluormethyl-anilin und Thionylchlorid. ¹H-NMR (ppm, CDCl₃, 400 MHz): 1.75 (m, 2H), 1.85 (m, 2H), 3.55 (m, 2H), 3.68 (m, 1 H), 4.05 (m, 2H), 7.86 (d, 1 H), 7.98 (dd, 1 H), 8.17 (d, 1 H), 9.08 (s, 1 H).
d) rac-N-(4-Cyano-3-trifluormethyl-phenyl)-2-hydroxy-2-hydroxy-3-phenyl-2-(tetrahydropyran-4-yl)-propionamid Die Herstellung erfolgte analog Beispiel 20 aus N-(4-Cyano-3-trifluormethyl-phenyl)-2-oxo-2-(tetrahydropyran-4-yl)-acetamid und Benzylmagnesiumchlorid. ¹H-NMR (ppm, CDCl₃, 400 MHz): 1.45 - 1.87 (m, 4H), 2.19 (m, 1H), 2.25 (s, 1 H), 2.88 (d, 1 H), 3.40 (m, 2H), 3.46 (d, 1 H), 4.01 (dd, 1 H), 4.10 (dd, 1 H), 7.17 (m, 2H), 7.28 (m, 3H), 7.75 (d, 1 H), 7.82 (d, 1 H), 7.88 (d, 1 H), 8.64 (s, 1 H).

## Patentansprüche

1. Verbindungen der allgemeinen Formel (I) 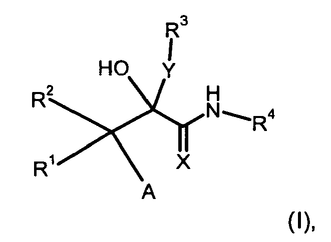 worin
A ein Wasserstoff oder ein gegebenenfalls ein- oder mehrfach, gleich oder verschieden mit Z substituierter C₁-C₈-Alkyl-, C₂-C₈-Alkenyl-, C₂-C₈-Alkinylrest ist oder aber direkt für Z steht, wobei Z folgende Bedeutung besitzt: Cyano, Halogen, Hydroxy, Nitro, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d}, -C=NOR^{b}, -NR^{c}R^{d}, -PO₃(R^{b})₂, -NR^{e}COR^{b}, -NR^{e}CSR^{b}, -NR^{e}S(O)R^{b}, -NR^{e}S(O)₂R^{b}, -NR^{e}CONR^{c}R^{d}, -NR^{e}COOR^{b}, -NR^{e}C(NH)NR^{c}R^{d}, -NR^{e}CSNR^{c}R^{d}, -NR^{e}S(O)NR^{c}R^{d}, -NR^{e}S(OhNR^{c}R^{d}, -S(O)R^{b}, -S(O)NR^{c}R^{d}, -S(O)₂R^{b}, -SO₂OR^{b}, -CSNR^{c}R^{d}, -CR^{b}(OH)-R^{b} mit
R^{b} ein Wasserstoff, ein C₁-C₆-Alkyl-, Hydroxy-C₁-C₃-alkyl-, C₁-C₃-Alkoxy-C₁-C₃-alkyl-, C₂-C₈-Alkenyl-, C₂-C₈-Alkinyl-, C₃-C₁₀-Cycloalkyl-, C₆-C₁₂-Aryl- oder ein teilweise oder vollständig fluorierter C₁-C₃-Fluoralkylrest und
R^{c} und R^{d} unabhängig voneinander ein Wasserstoff, ein gegebenenfalls mit W substituiertes C₁-C₆-Alkyl-, C₂-C₈-Alkenyl-, C₂-C₈-Alkinyl-, C₃-C₁₀-Cycloalkyl-, C₆-C₁₂-Aryl-, ein 5- bis 12-gliedriger Heteroarylrest, eine Gruppe C(O)R^{b} mit der weiter oben angegebenen Bedeutung für R^{b} oder eine Hydroxygruppe bedeuten oder gemeinsam unter Einschluss des Stickstoffs einen 3- bis 7-gliedrigen Ring bilden, der gegebenenfalls um O, S oder NR^{f} erweitert ist, wobei
R^{f} Wasserstoff, C₁-C₃-Alkyl, C₁-C₃-Alkanoyl, C₁-C₃- Alkylsulfonyl oder C₁-C₃-Alkoxycarbonyl ist, und W -NR⁹R^{h} mit
R⁹ Wasserstoff oder C₁-C₃-Alkyl und R^{h} Wasserstoff oder C₁-C₃-Alkyl oder
R⁹ und R^{h} gemeinsam unter Einschluss des Stickstoffs einen 3- bis 7-gliedrigen Ring bilden, der gegebenenfalls um O, S oder NR^{f} erweitert ist, wobei
R^{f} Wasserstoff, C₁-C₃-Alkyl, C₁-C₃-Alkanoyl, C₁-C₃- Alkylsulfonyl oder C₁-C₃-Alkoxycarbonyl ist,
und wobei, wenn
R^{c} eine Hydroxygruppe ist, R^{d} nur ein Wasserstoff, ein gegebenenfalls mit W substituiertes C₁-C₆-Alkyl, C₂-C₈-Alkenyl, C₂-C₈-Alkinyl, C₃-C₁₀-Cycloalkyl oder C₆-C₁₂-Aryl sein kann und umgekehrt sowie weiterhin
R^{e} ein Wasserstoff, ein gegebenenfalls mit W substituiertes C₁-C₆- Alkyl, C₂-C₈-Alkenyl, C₂-C₈-Alkinyl, C₃-C₁₀-Cyclo-alkyl oder C₆-C₁₂-Aryl bedeutet
oder
A ein gegebenenfalls ein- oder mehrfach, gleich oder verschieden mit M substituierter C₃-C₁₀-Cycloalkyl- oder 3-12-gliedriger Hetero-cycloalkylrest bedeutet und
M ein C₁-C₆-Alkyl oder eine Gruppe -COR^{b}, CO₂R^{b}, - O-R^{b}, oder -NR^{c}R^{d} ist, wobei R^{b}, R^{c} und R^{d} die weiter oben angegebenen Bedeutungen haben und
R¹ und R² unabhängig voneinander eine unverzweigte oder verzweigte, gegebenenfalls mit Z substituierte C₁-C₅-Alkylgruppe oder gemeinsam mit dem C-Atom der Kette einen gegebenenfalls mit Z substituierten carbocyclischen oder heterocyclischen Ring mit insgesamt 3-7 Gliedern bildend,
wobei, wenn
A ein Wasserstoff ist und R¹ ein Methylrest ist, R² nicht ein Methylrest oder ein Ethylrest sein können,
A ein Wasserstoff ist R¹ und R² nicht gemeinsam einen Ring mit 3-4 Gliedern sein können
A ein Methylrest ist R¹ und R² nicht gleichzeitig ein Methylrest sein oder gemeinsam mit dem C-Atom der Kette einen Cyclopropylring bilden können,
R³ ein Wasserstoff oder ein gegebenenfalls ein- oder mehrfach, gleich oder verschieden mit K substituierter C₁-C₈-Alkyl-, C₂-C₈-Alkenyl-, C₂-C₈-Alkinyl-, C₃-C₁₀-Cycloalkyl-, 3-12-gliedriger Heterocycloalkylrest oder ein gegebenenfalls ein- oder mehrfach, gleich oder verschieden mit L substituierter Aryl- oder 3-12-gliedriger Heteroarylrest bedeutet,
und
K ein Cyano, Halogen, Hydroxy, Nitro, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d}, -C=NOR^{b} -NR^{c}R^{d} oder ein gegebenenfalls ein- oder mehrfach, gleich oder verschieden mit M substituierter C₃-C₁₀-Cycloalkyl-, 3-12-gliedriger Heterocycloalkylrest oder ein gegebenenfalls ein- oder mehrfach mit L substituierter Aryl- oder 3-12-gliedriger Heteroarylrest ist, mit der für M unter A angegebenen Bedeutung und
L C₁-C₈-Alkyl, C₂-C₈-Alkenyl, C₂-C₈-Alkinyl, ein teilweise oder vollständig fluoriertes C₁-C₆-Fluoralkyl, ein teilweise oder vollständig fluoriertes C₁-C₆-Fluoralkoxy, C₁-C₆-Alkoxy-C₁-C₆-alkyl, C₁-C₆-Alkoxy-C₁-C₆-alkoxy, ein mono- oder bicyclischer (CH₂)ₚ-C₃-C₁₀-Cycloalkyl, ein mono- oder bicyclischer 3-12-gliedriger (CH₂)ₚ-Heterocycloalkylrest, (CH₂)ₚCN, (CH₂)ₚHal, (CH₂)ₚNO₂, ein mono- oder bicyclischer gegebenenfalls mit V substituierter (CH₂)ₚ-C₆-C₁₂-Arylrest, ein mono- oder bicyclischer gegebenenfalls mit V substituierter 3-12-gliedriger (CH₂)ₚ-Heteroarylrest, oder -(CH₂)ₚPO₃(R^{b})₂, -(CH₂)ₚNR^{c}R^{d}, -(CH₂)ₚNR^{e}COR^{b}, -(CH₂)ₚNR^{e}CSR^{b}, -(CH₂)ₚNR^{e}S(O)R^{b}, -(CH₂)ₚNR^{e}S(O)₂R^{b}, -(CH₂)ₚNR^{e}CONR^{c}R^{d}, -(CH₂)ₚNR^{e}COOR^{b}, -(CH₂)ₚNR^{e}C(NH)NR^{c}R^{d}, -(CH₂)ₚNR^{e}CSNR^{c}R^{d}, -(CH₂)ₚNR^{e}S(O)NR^{c}R^{d}, -(CH₂)ₚNR^{e}S(O)₂NR^{c}R^{d}, -(CH₂)ₚCOR^{b}, -(CH₂)ₚCSR^{b}, -(CH₂)ₚ S(O)R^{b}, -(CH₂)ₚS(O)(NH)R^{b}, -(CH₂)ₚS(O)₂R^{b}, -(CH₂)ₚS(O)₂NR^{c}R^{d}, -(CH₂)ₚSO₂OR^{b}, -(CH₂)ₚCO₂R^{b}, -(CH₂)ₚCONR^{c}R^{d}, -(CH₂)ₚCSNR^{c}R^{d}, -(CH₂)ₚOR^{b}, -(CH₂)ₚSR^{b}, -(CH₂)ₚCR^{b}(OH)-R^{b}, -(CH₂)ₚ-C=NOR^{b}, -O-(CH₂)ₙ-O-, -O-(CH₂)ₙ-CH₂-, -O-CH=CH- oder -(CH₂)ₙ₊₂- ist und die end-ständigen Sauerstoffatome und/oder Kohlenstoffatome mit direkt benachbarten Ringkohlenstoffatomen verknüpft sind und wobei
n 1 oder 2 und
p eine Zahl 0, 1, 2, 3, 4, 5 oder 6 ist, sowie
V ein Cyano, Halogen, Nitro, -(CH₂)ₚOR^{b}, -(CH₂)ₚS(O)₂R^{b}, -C(O)R^{b}, CO₂R^{b}, -O-R^{b}, -S-R^{b}, SO₂NR^{c}R^{d}, -C(O)-NR^{c}R^{d}, -OC(O)-NR^{c}R^{d},-C=NOR^{b} -NR^{c}R^{d}, ein teilweise oder vollständig fluoriertes C₁-C₆-Fluoralkyl oder ein teilweise oder vollständig fluoriertes C₁-C₆-Fluoralkoxy,
X ein Sauerstoff- oder zwei Wasserstoffatome
Y für (CH₂)ₘ, -C≡C- oder -CH=CH- mit m = 0 oder 1 steht, und
R⁴ ein gegebenenfalls gleich oder verschieden mit 1 bis 3 der unter L genannten Reste substituierter aromatischer oder heteroaromatischer 3 bis 12-gliedriger Mono- oder Bicyclus, oder für eine der folgenden unter B oder C genannten Gruppen steht:
B: 6-Ring/6-Ring-Systeme: 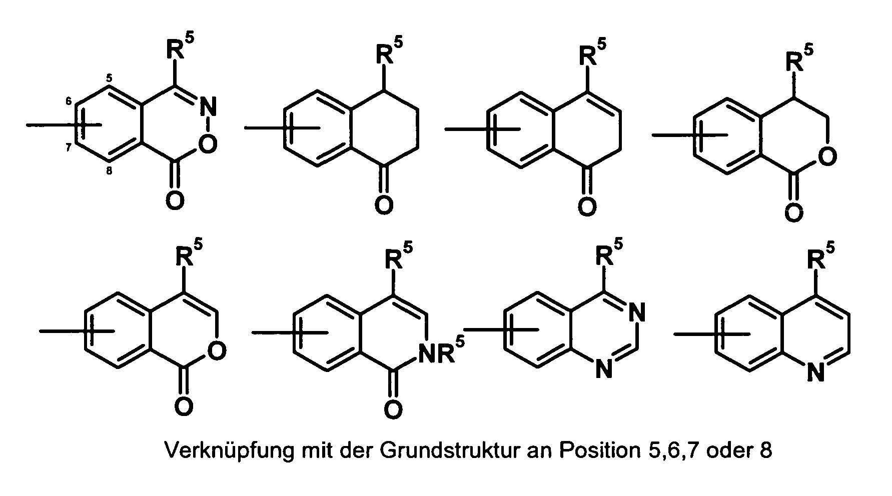 C: 6-Ring/5-Ring-Systeme: 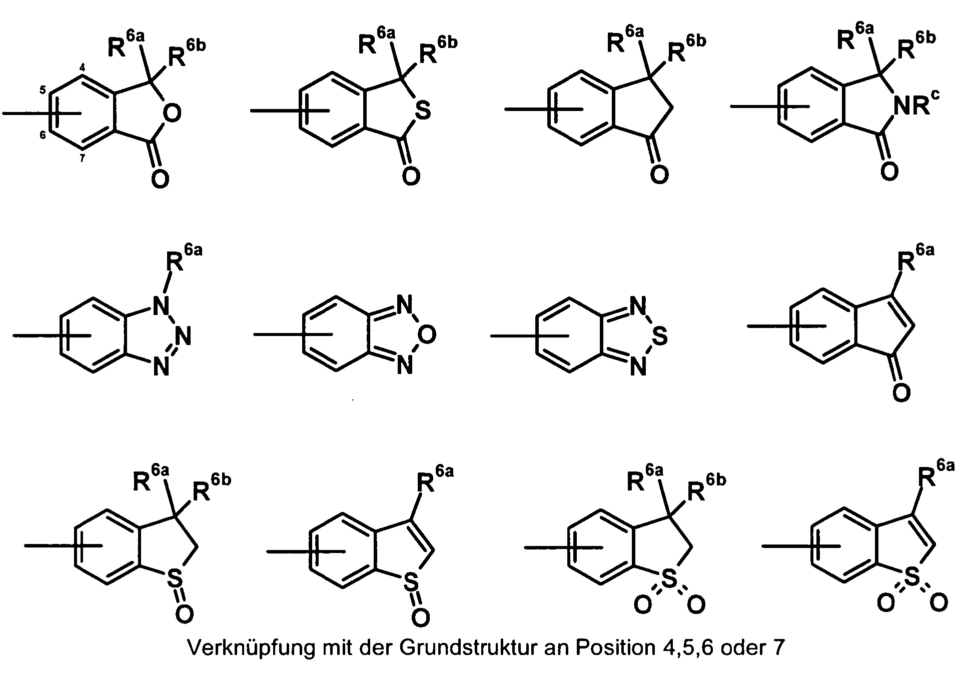 wobei
R⁵ Wasserstoff oder C₁-C₄ Alkyl, oder ein teilweise oder vollständig fluoriertes C₁-C₄ Fluoralkyl,
R^{6a} und R^{6b} unabhängig voneinander Wasserstoff, C₁-C₄ Alkyl oder ein teilweise oder vollständig fluoriertes C₁-C₄-Fluoralkyl sind oder gemeinsam mit dem Ringkohlenstoffatom einen 3-bis 6-gliedrigen Ring bilden,
sowie ihre pharmazeutisch annehmbaren Salze.

2. Verbindungen nach Anspruch 1, worin A ein Wasserstoff ist.

3. Verbindungen nach Anspruch 2, worin Y für -C≡C- steht, R¹ und R² gemeinsam mit dem C-Atom der Kette einen carbocyclischen oder heterocyclischen 3-6-gliedrigen Ring bilden und R³ ein gegebenenfalls mit K substituiertes C₁-C₈-Alkyl, ein gegebenenfalls ein- oder mehrfach, gleich oder verschieden mit L substituiertes Aryl oder ein 3- bis 12-gliedriges Heteroaryl bedeutet.

4. Verbindungen nach Anspruch 2, worin Y für (CH₂)ₘ steht und R³ ein gegebenenfalls ein- oder mehrfach, gleich oder verschieden mit L substituiertes Aryl oder ein 3- bis 12-gliedriges Heteroaryl ist und R⁴ ein gegebenenfalls 1- oder 2-fach gleich oder verschieden mit L substituiertes mono- oder bicyclisches Aryl oder eine der unter R⁴ genannten Gruppen B mit Verknüpfung an Position 6 oder C mit Verknüpfung an Position 5 ist.Verbindungen nach Anspruch 4, worin m = 1 ist.

5. Verbindungen nach Anspruch 4, worin R⁴ ein mit bis zu 2 der unter L genannten Reste substituierter Phenylring ist.

6. Verbindungen nach Anspruch 5, worin der Phenylring mit einem Cyanorest, mit Chlor und/ oder mit einem Trifluormethylrest substituiert ist.

7. Verbindungen nach Anspruch 4, worin R⁴ folgende Bedeutung hat:

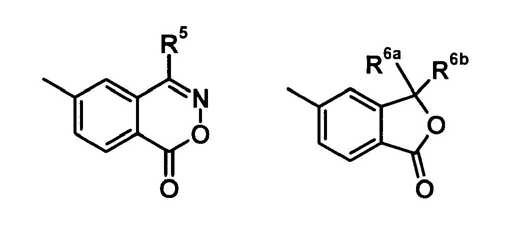

8. Verbindungen nach Anspruch 8, worin
R⁵ ein Methyl oder Ethyl,
R⁶ ein Wasserstoff,

9. Verbindungen nach Anspruch 2, worin
p 0, 1 oder 2 sowie
L ein C₁-C₈-Alkyl, C₂-C₈-Alkenyl, C₂-C₈-Alkinyl, ein teilweise oder vollständig fluoriertes C₁-C₆-Fluoralkyl, -(CH₂)ₚCN, (CH₂)ₚHal, (CH₂)ₚNO₂, (CH₂)ₚ-C₆-C₁₂-Aryl, -(CH₂)ₚ-Heteroaryl, -(CH₂)ₚNR^{c}R^{d}, -(CH₂)ₚNR^{e}COR^{b}, -(CH₂)ₚNR^{e}S(O)₂R^{b}, -(CH₂)ₚNR^{e}CONR^{c}R^{d}, -(CH₂)ₚNR^{e}S(O)NR^{c}R^{d}, -(CH₂)ₚNR^{e}S(O)₂NR^{c}R^{d}, -(CH₂)ₚCOR^{b}, -(CH₂)ₚS(O)R^{b}, -(CH₂)ₚS(O)₂R^{b}, -(CH₂)ₚS(O)₂NR^{c}R^{d}, -(CH₂)ₚCO₂R^{b}, -(CH₂)ₚCONR^{c}R^{d}, -(CH₂)ₚOR^{b}, -(CH₂)ₚCR^{b}(OH)-R^{b} und
Z ein Cyano, Halogen, Hydroxy, Nitro, -C(O)R^{b}, CO₂R^{b}, -O-R^{b},-SO₂NR^{c}R^{d}, -C(O)-NR^{c}R^{d}, -NR^{c}R^{d}, -NR^{e}COR^{b}, -NR^{e}S(O)R^{b}, -NR^{e}S(O)₂R^{b}, -NR^{e}CONR^{c}R^{d}, -S(O)R^{b}, -S(O)NR^{c}R^{d}, -S(O)₂R^{b}, -CR^{b}(OH)-R^{b}, oder ein gegebenenfalls ein- oder mehrfach, gleich oder verschieden mit M substituiertes C₃-C₁₀-Cycloalkyl oder Heterocycloalkyl ist.

10. Verbindungen gemäß Anspruch 2, worin R¹ und R² gemeinsam mit dem C-Atom der Kette einen Cyclopropyl-, Cyclopentyl- oder Cyclohexylring bilden.

11. Verbindungen gemäß Anspruch 2, worin R¹ und R² gemeinsam mit dem C-Atom der Kette einen Tetrahydropranyl-, Piperidinyl- oder Tetrahydro-thiopyranylring bilden.

12. Verbindungen gemäß einem der voranstehenden Ansprüche, nämlich

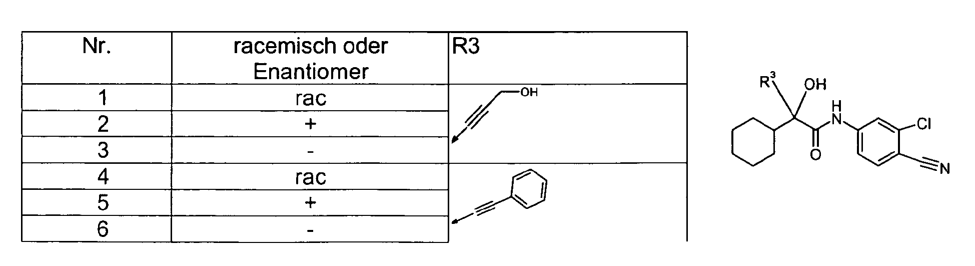

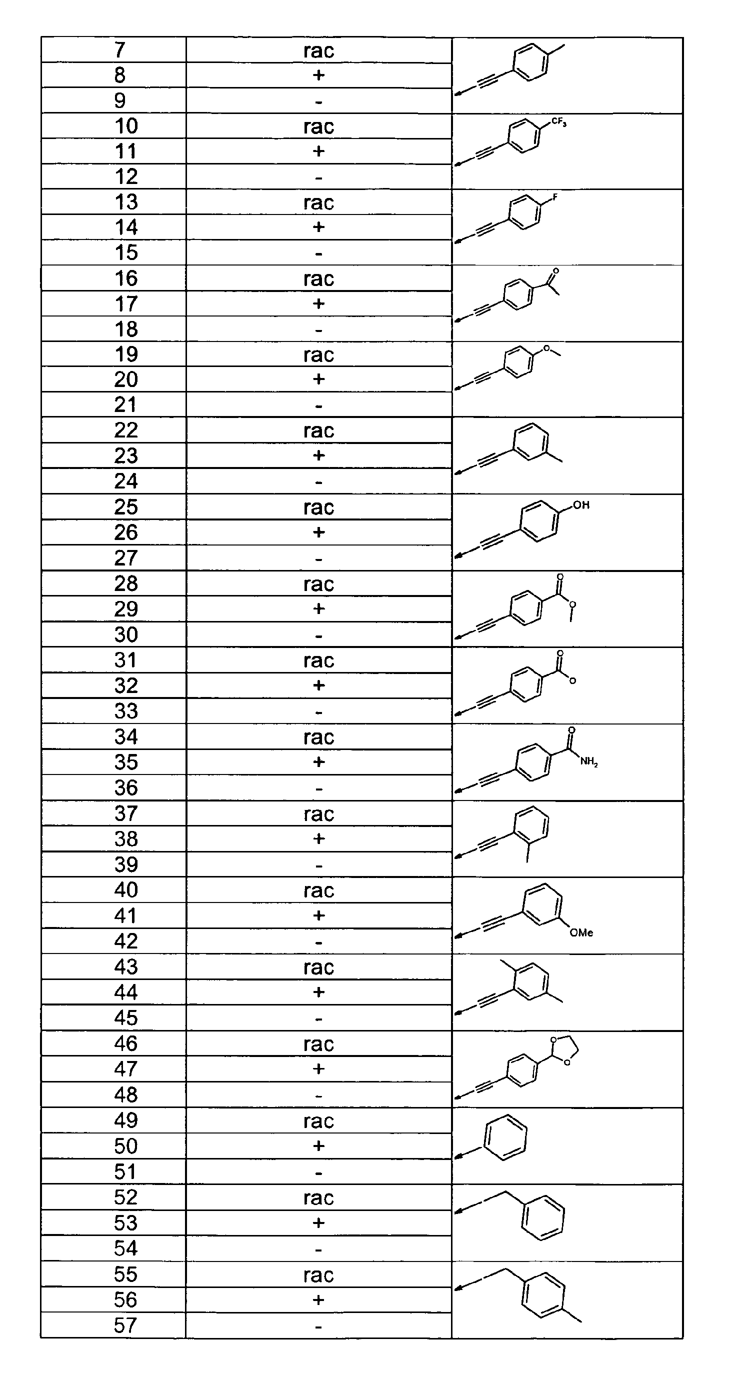

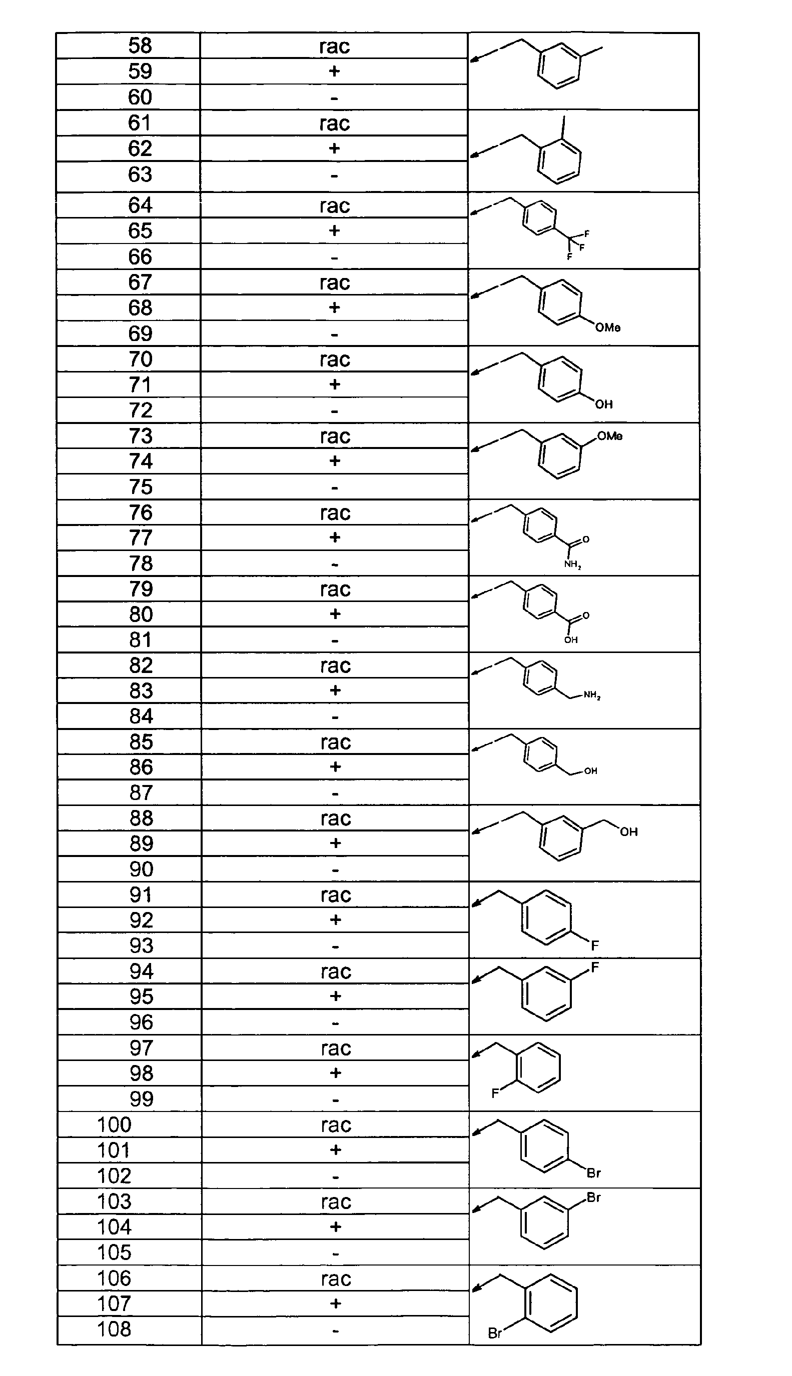

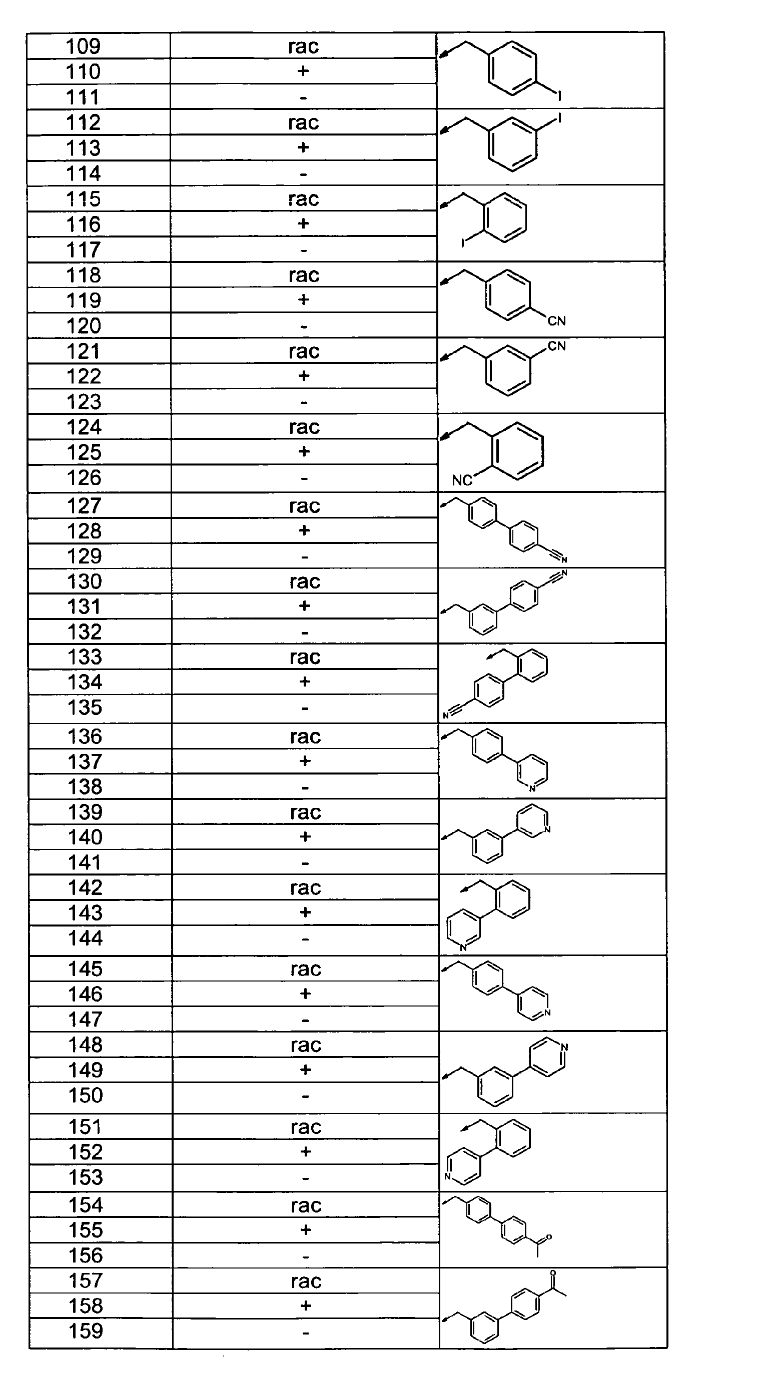

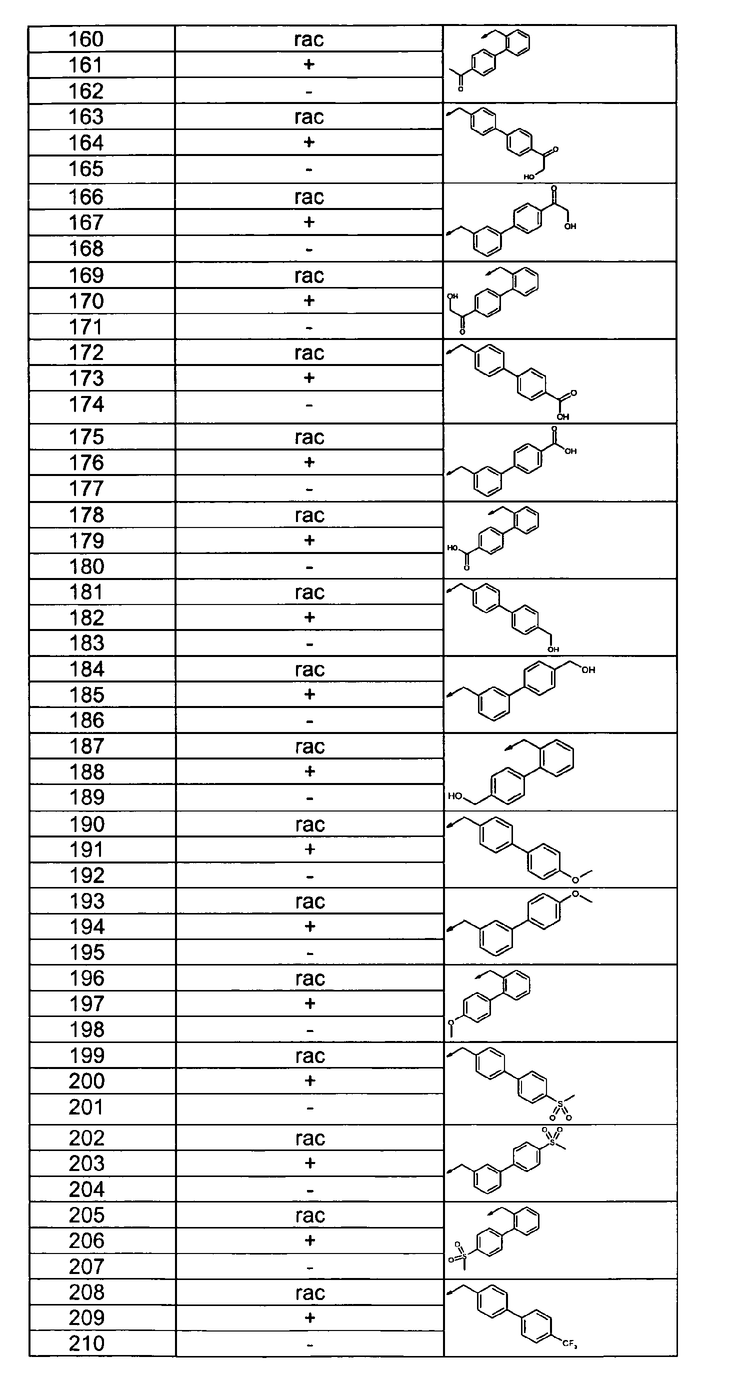

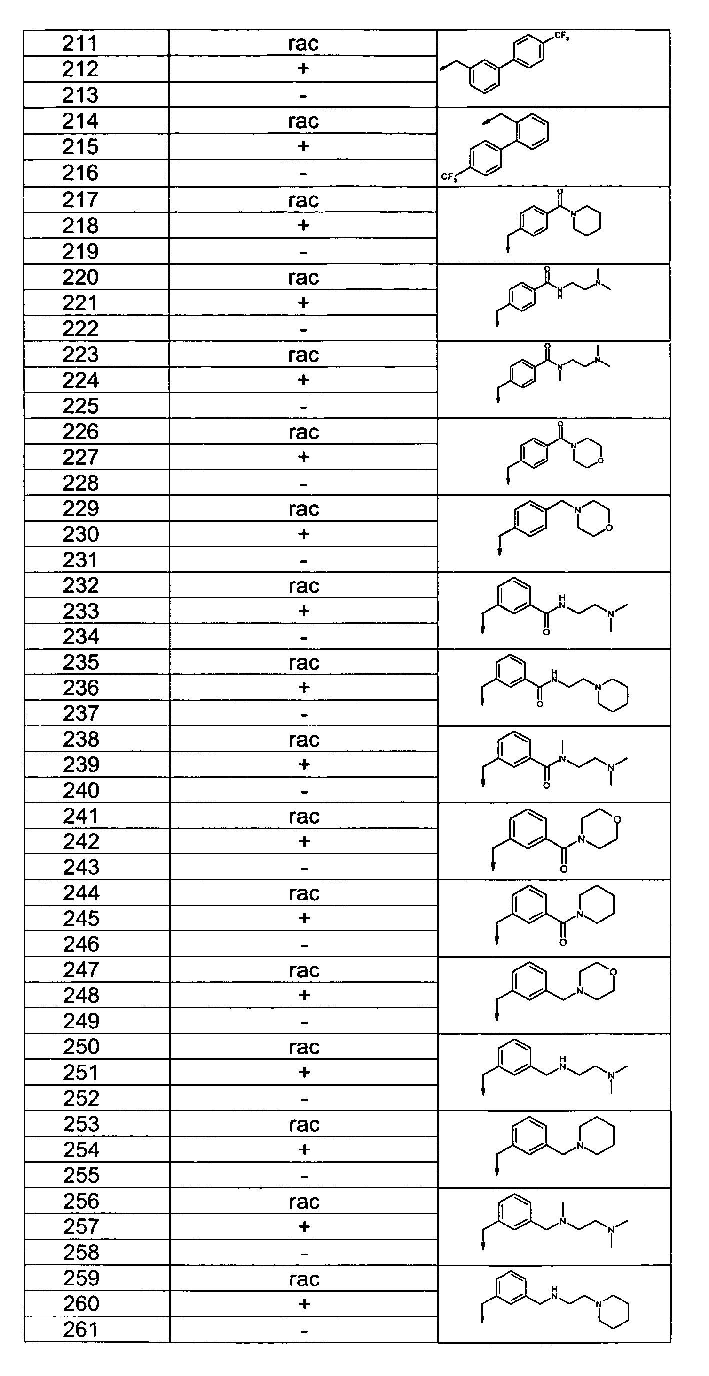

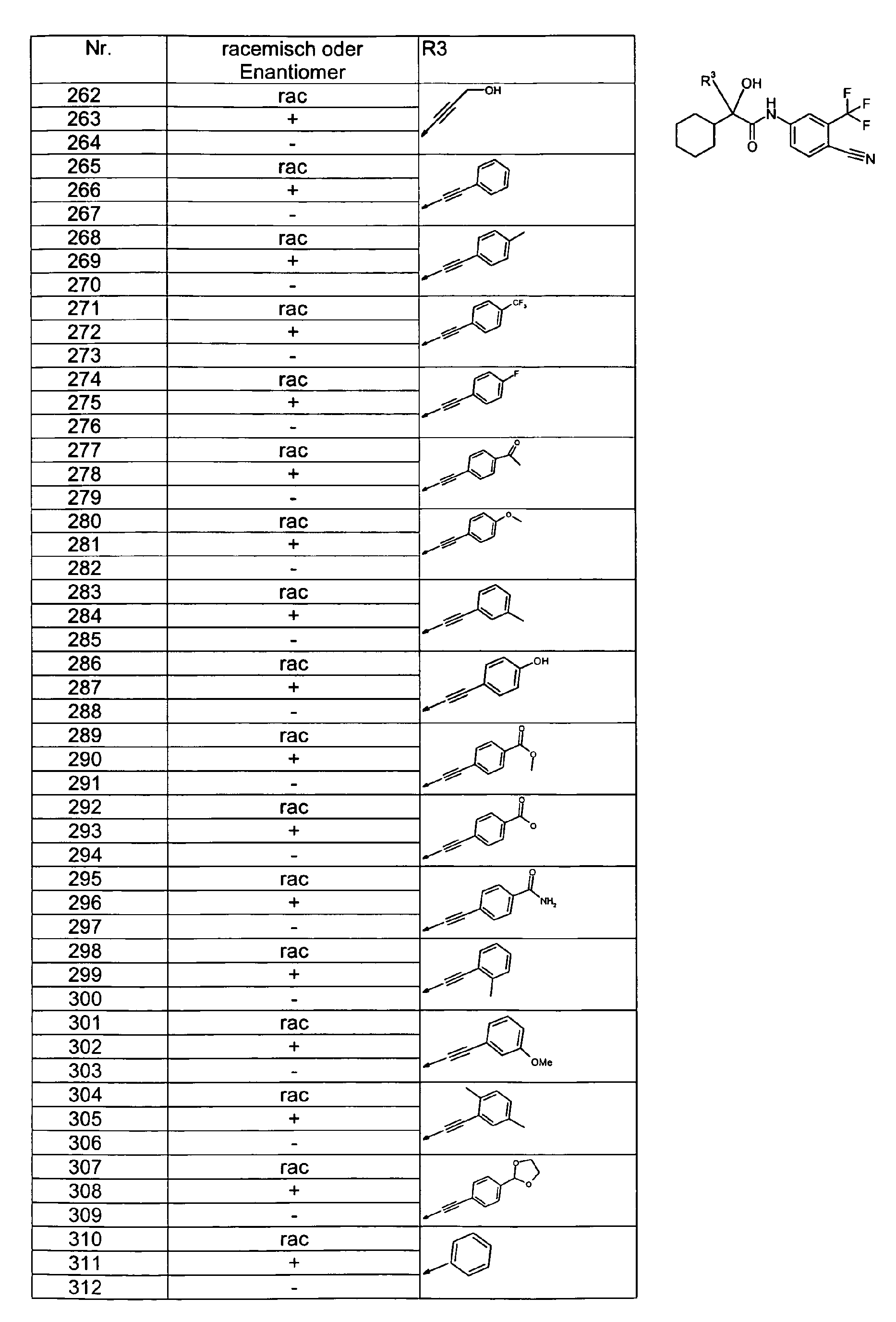

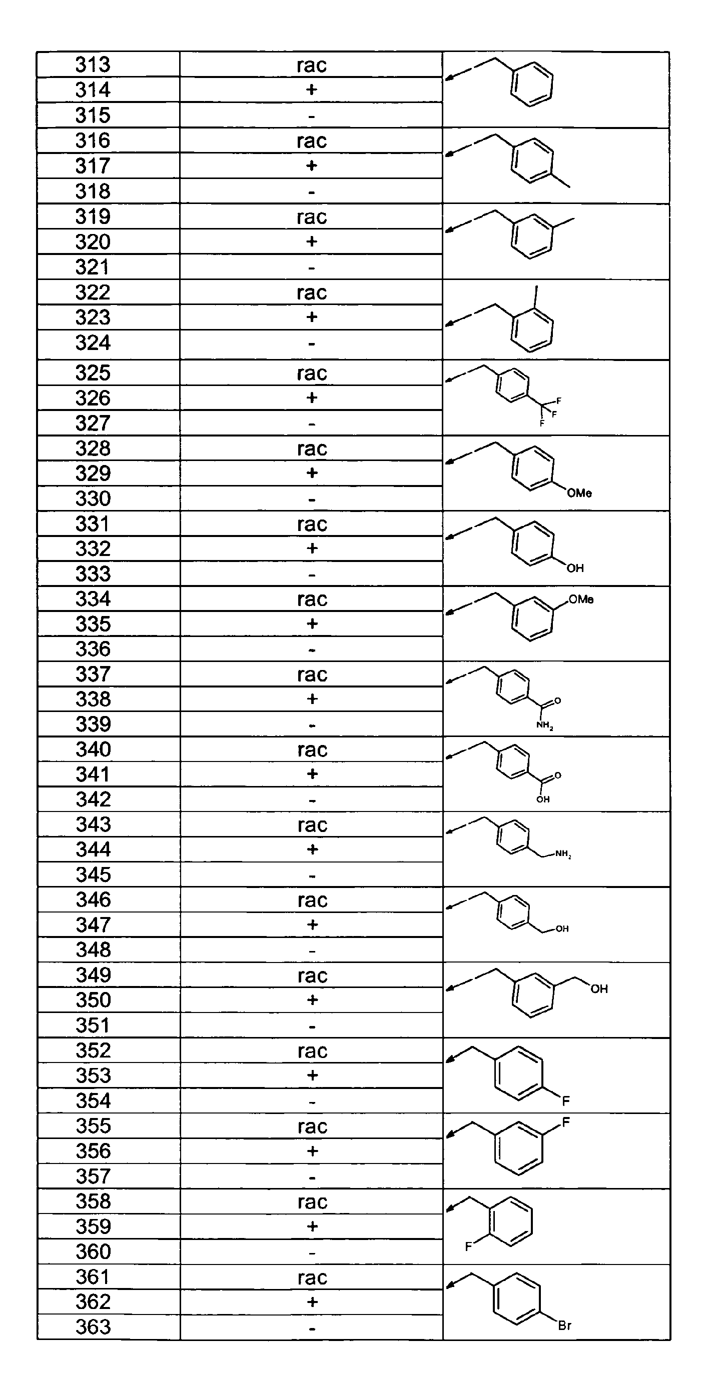

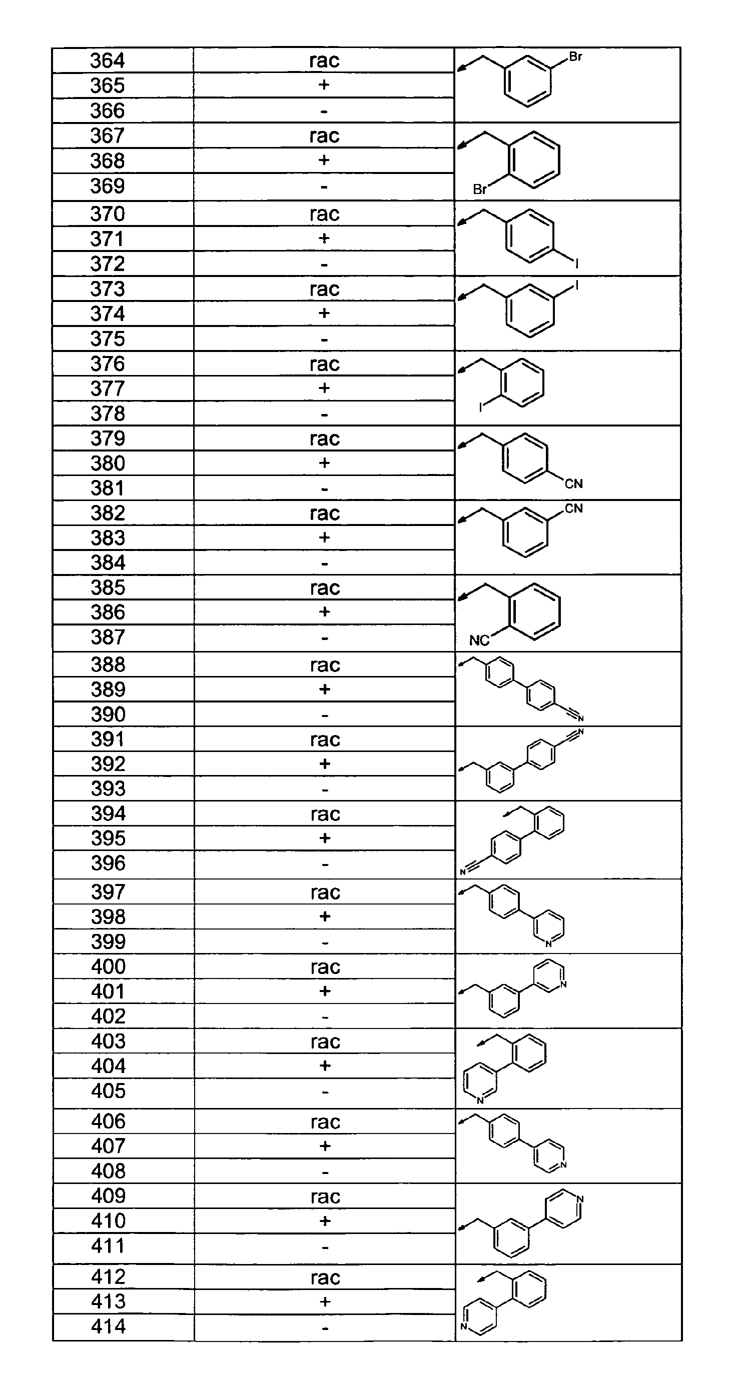

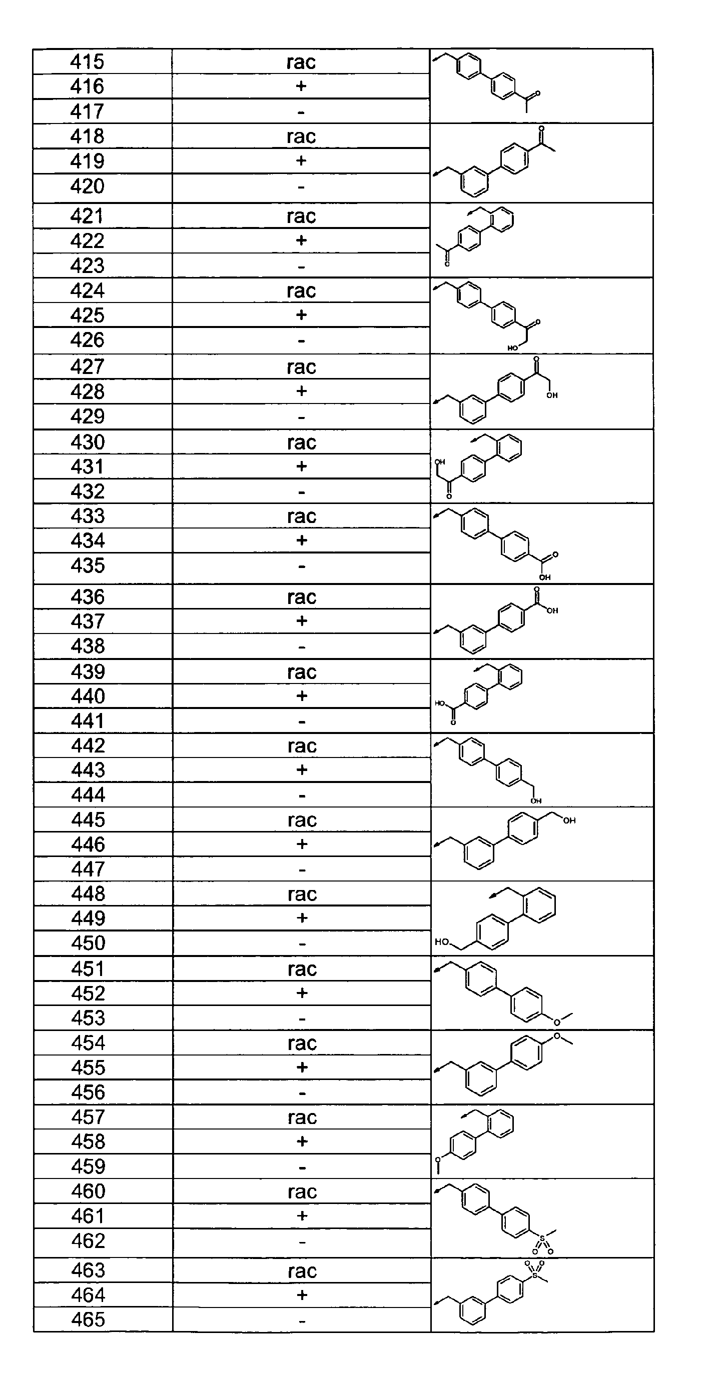

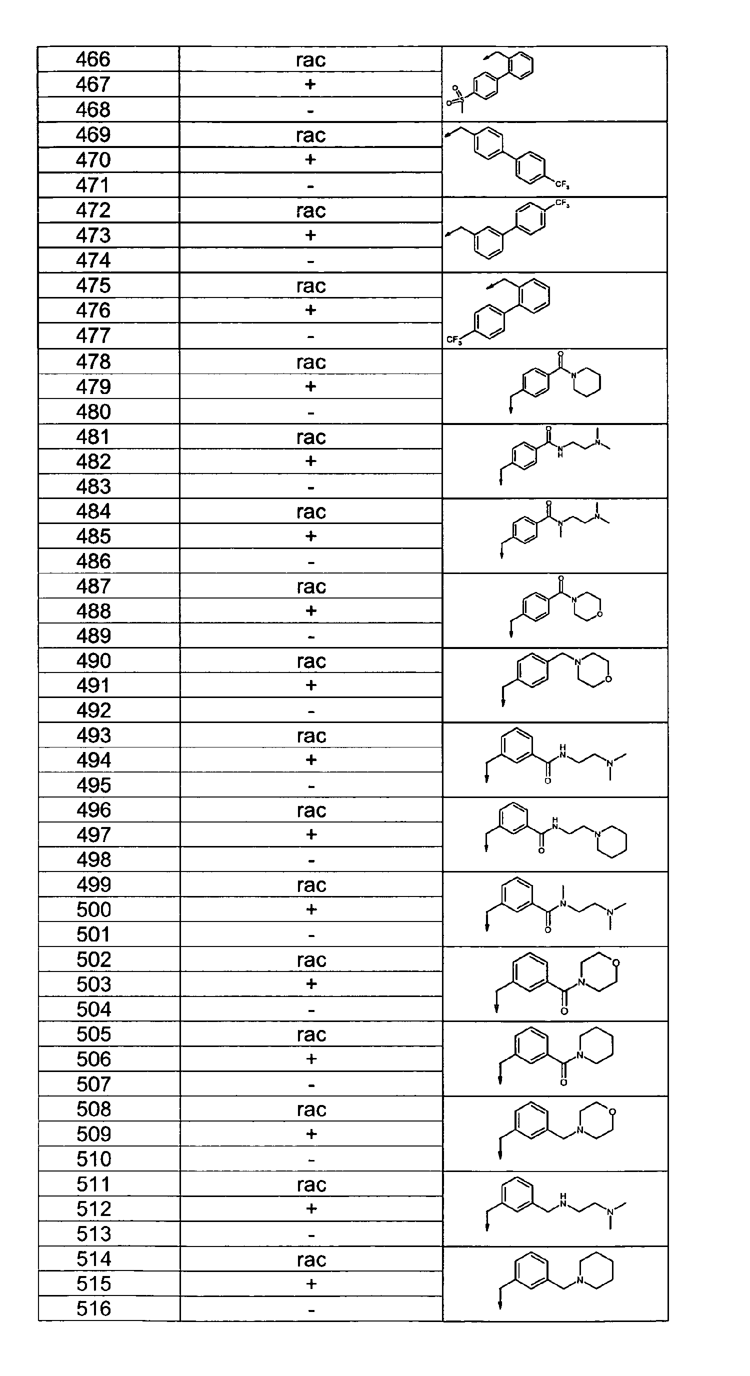

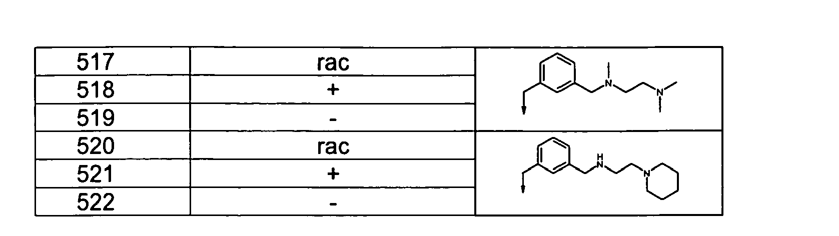

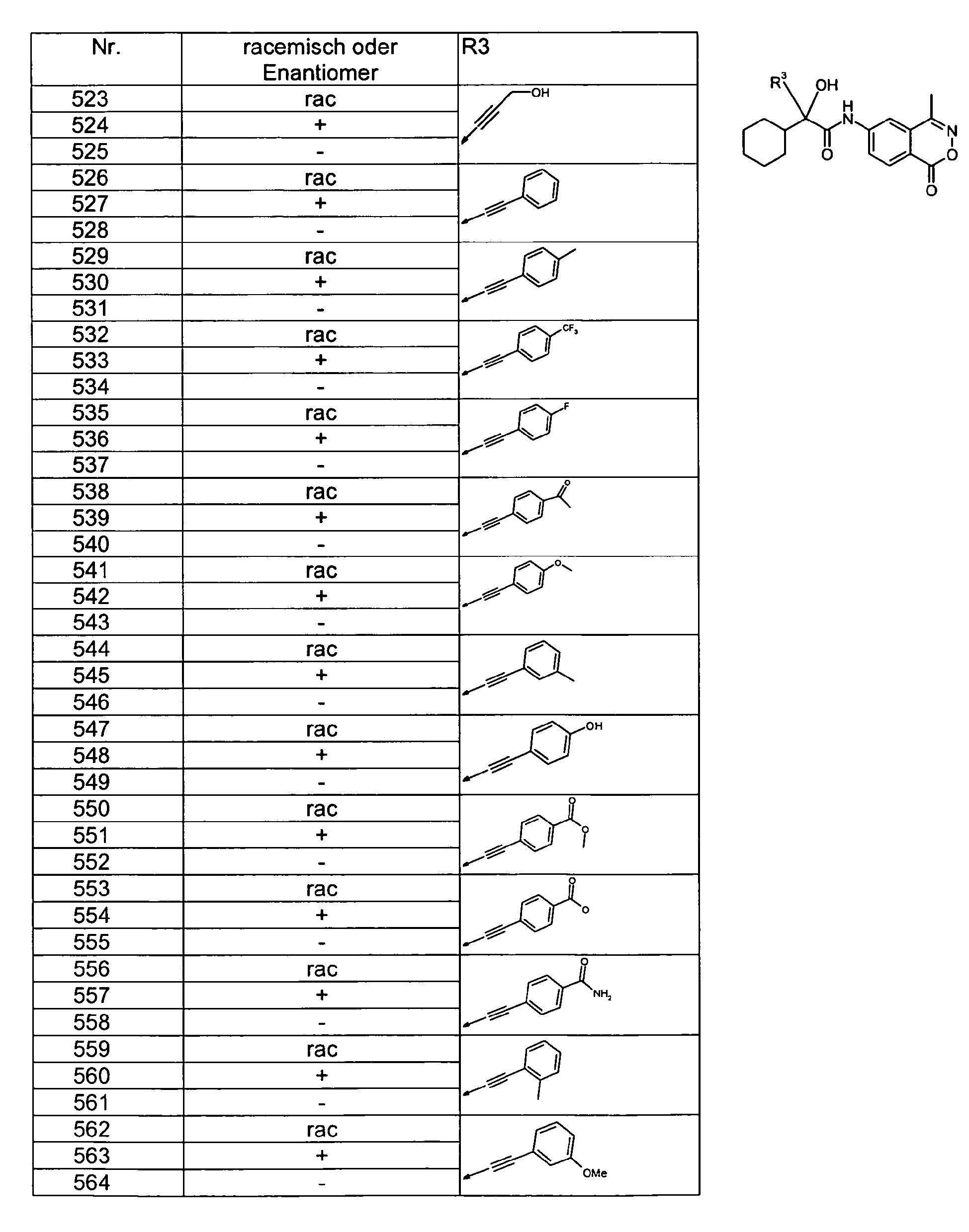

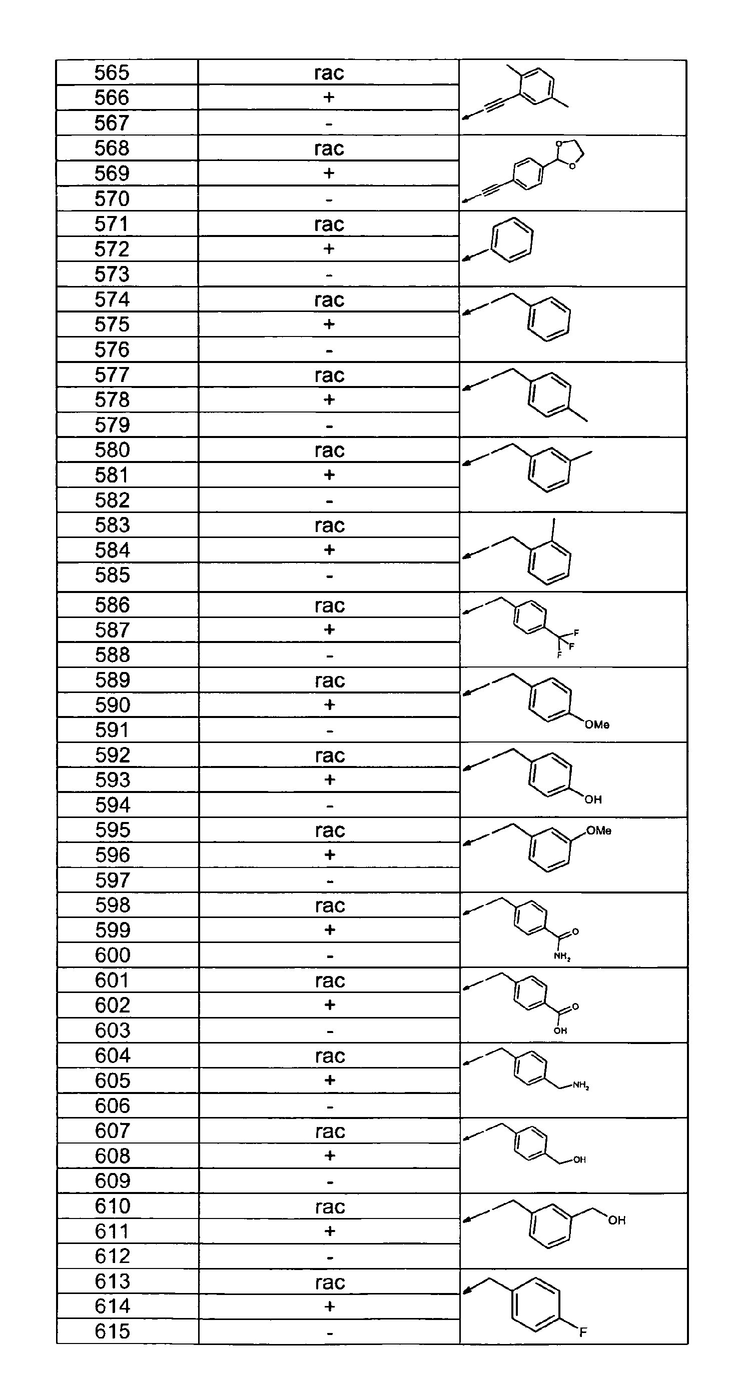

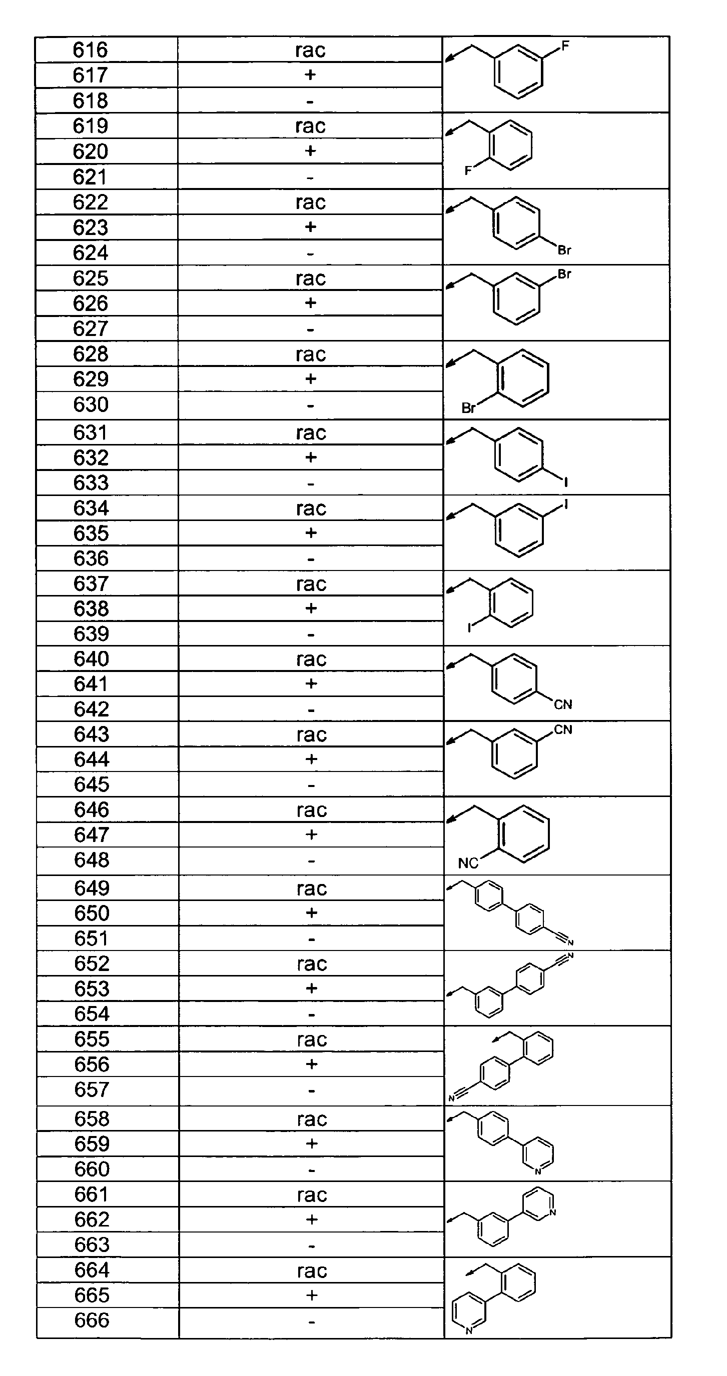

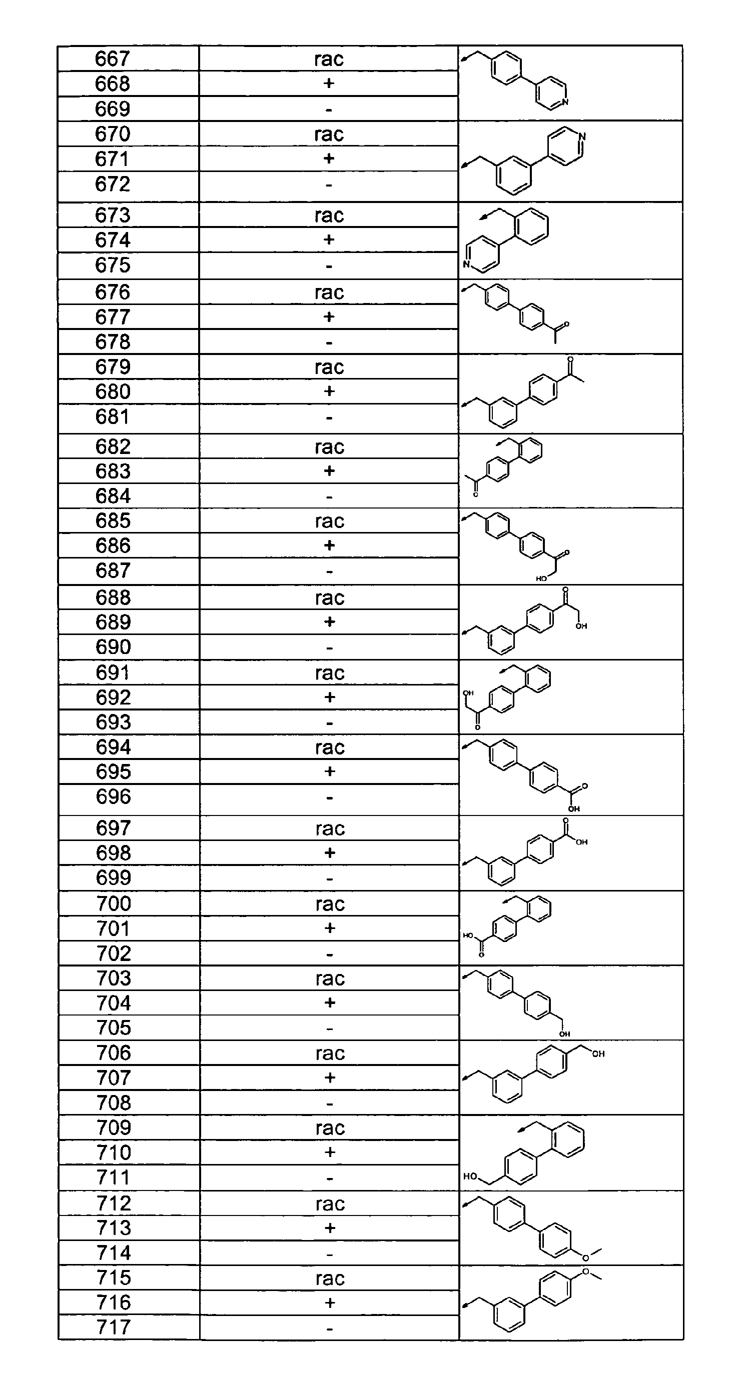

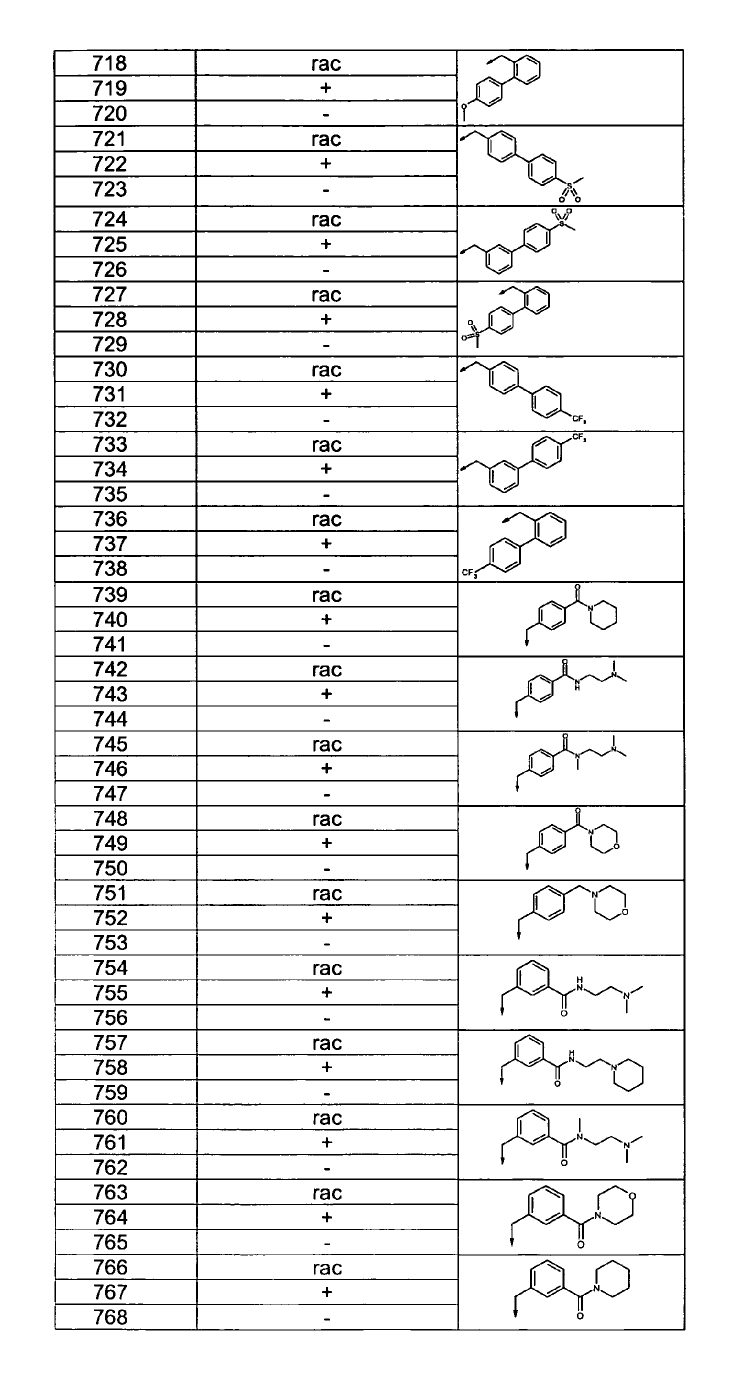

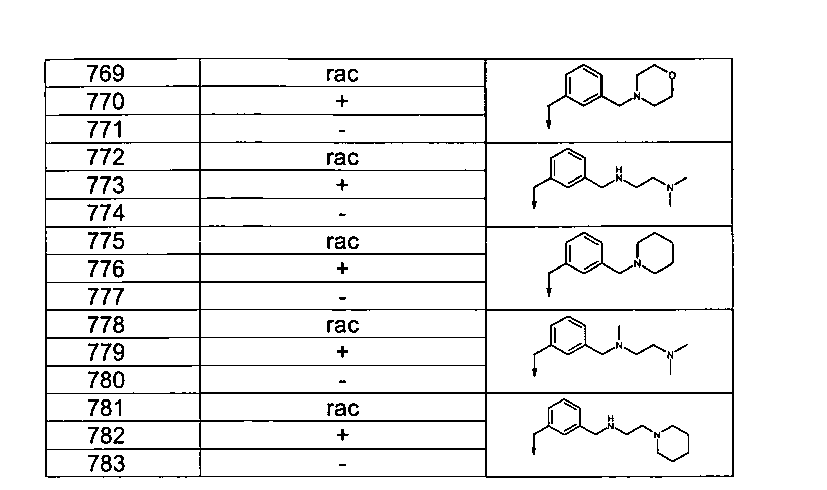

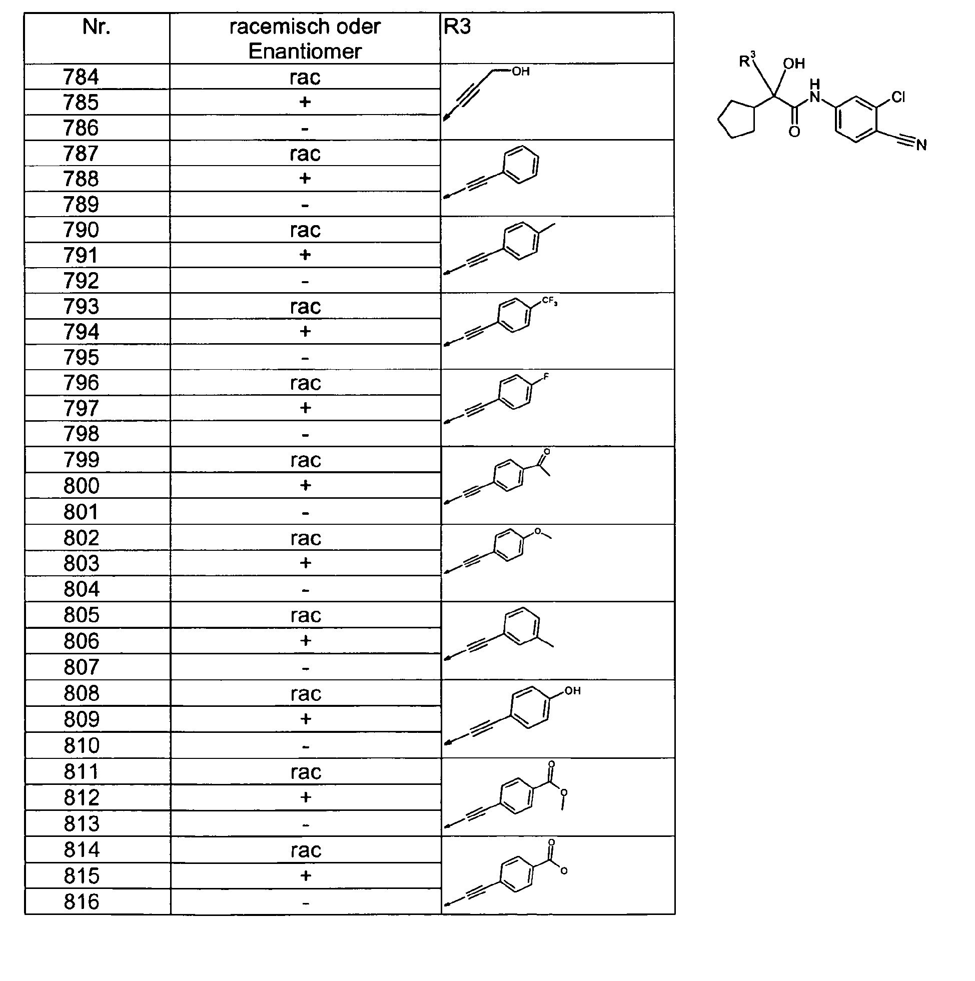

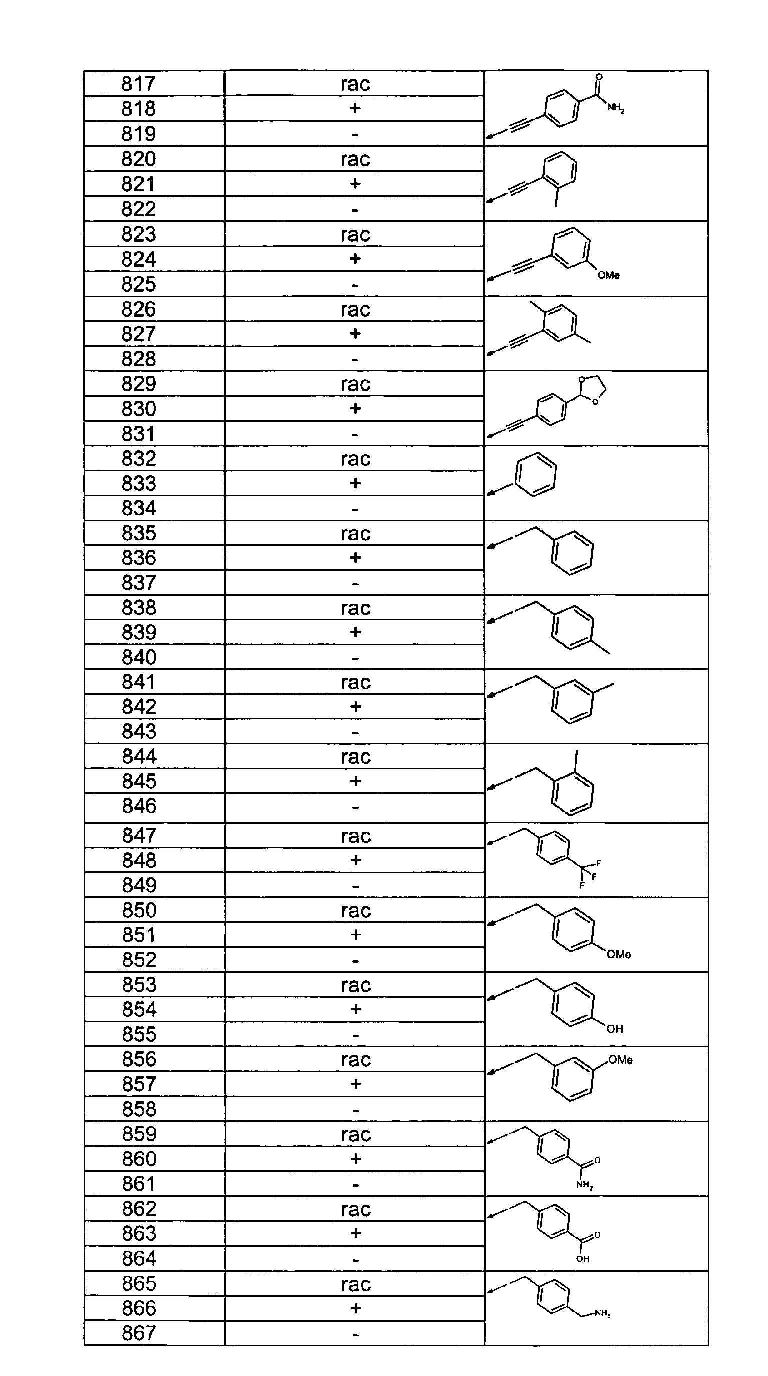

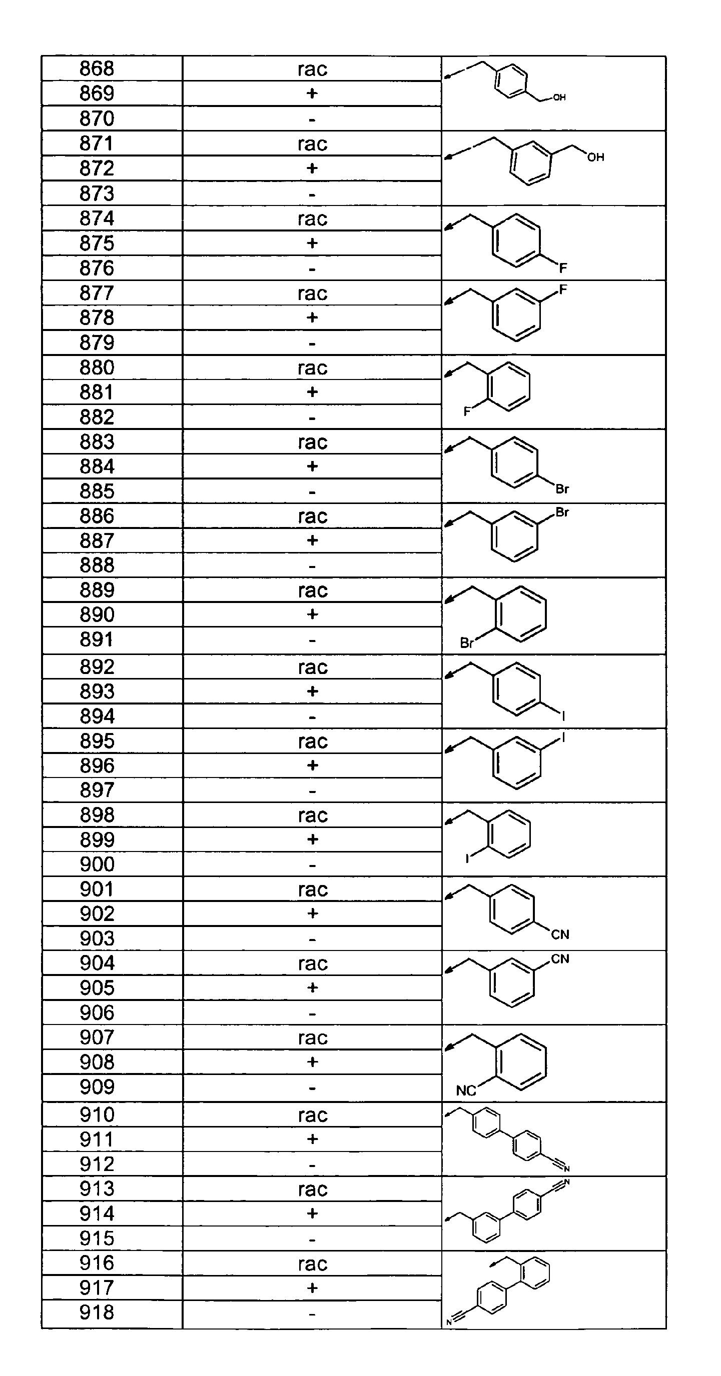

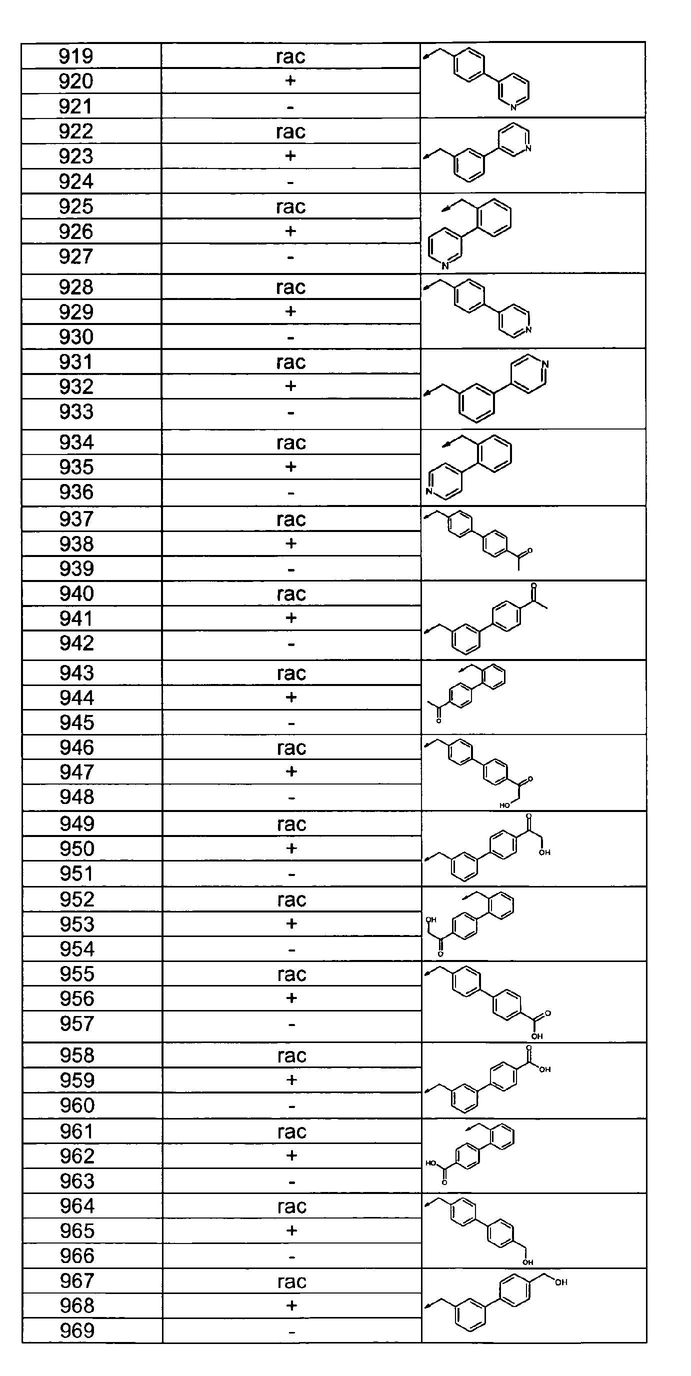

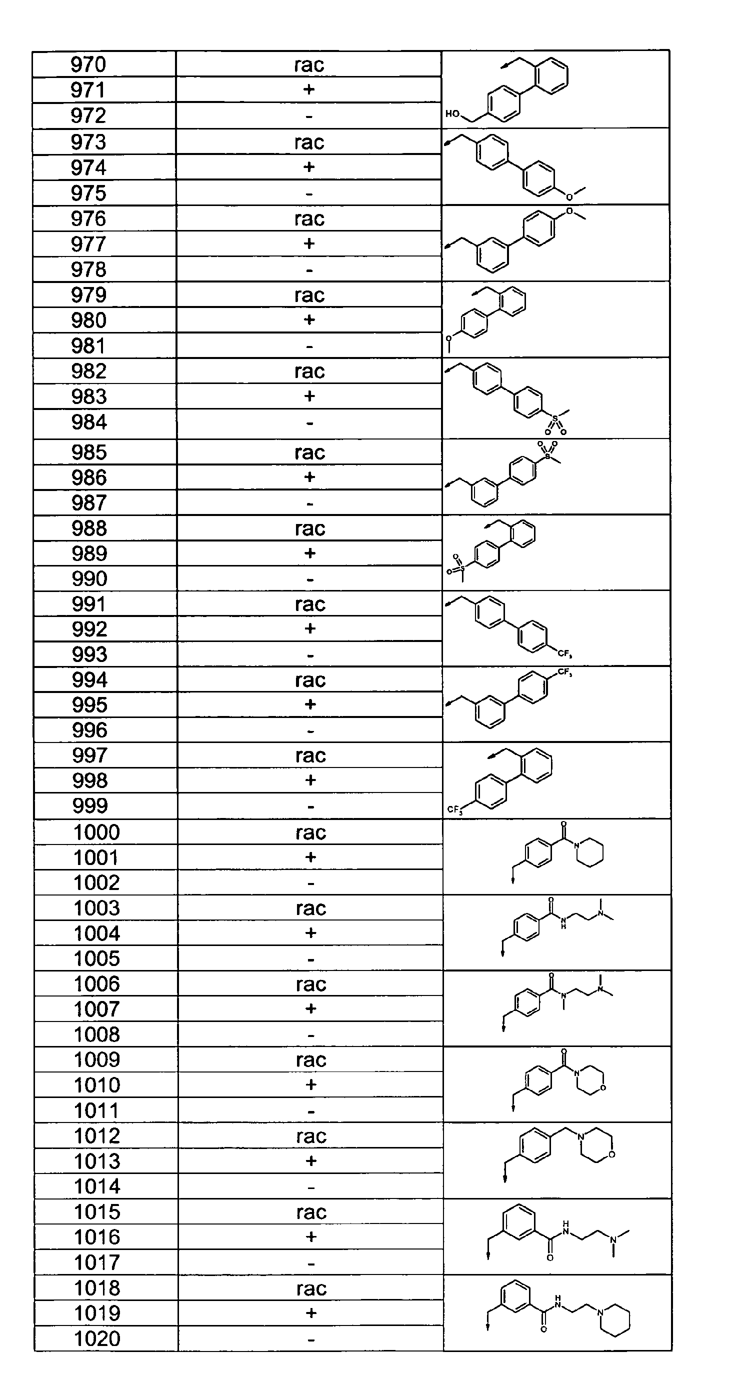

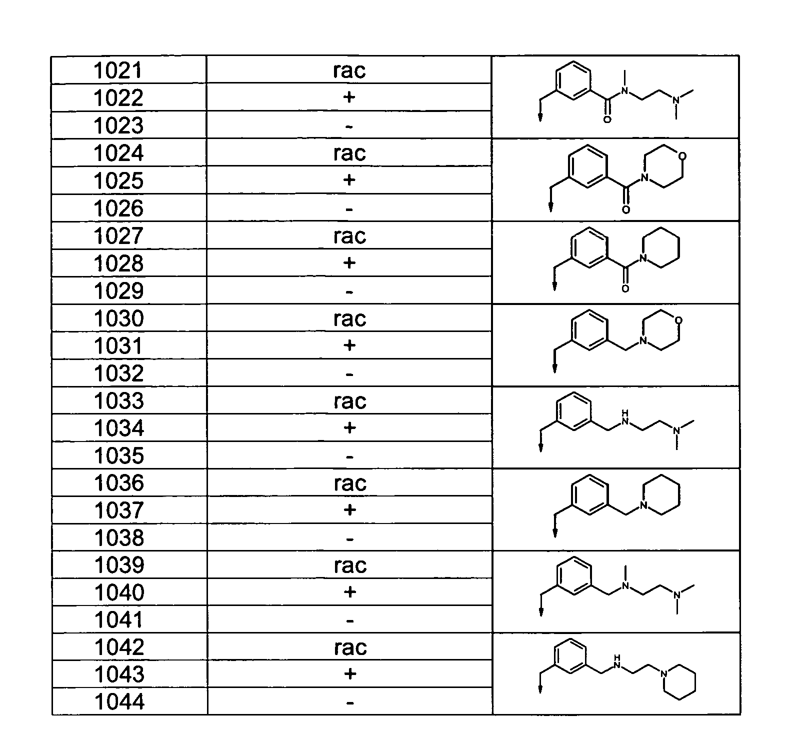

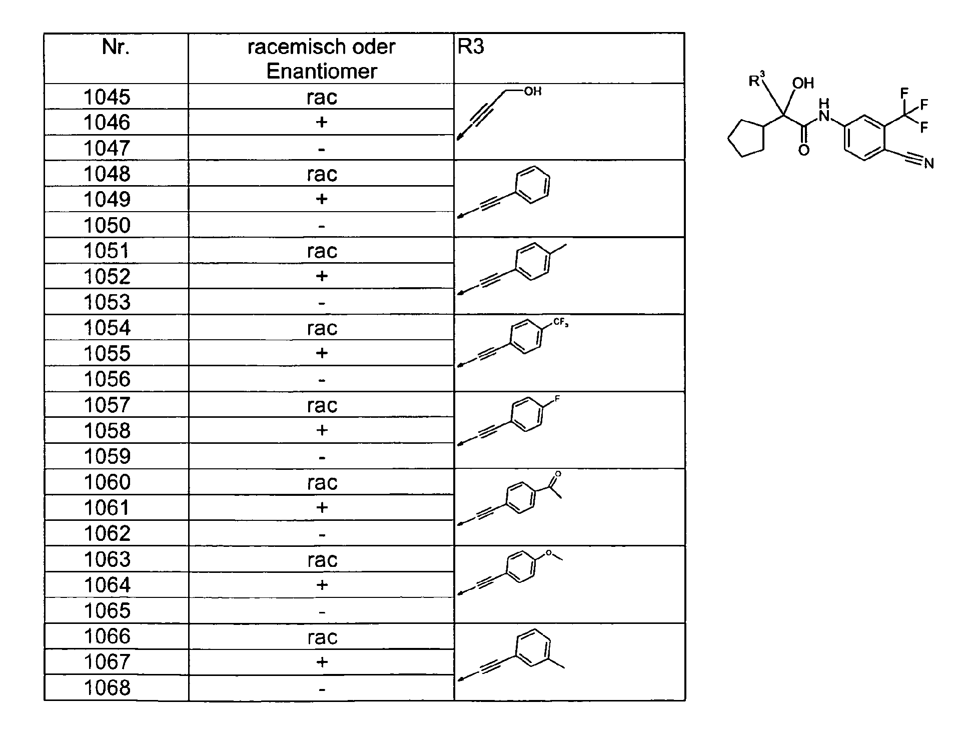

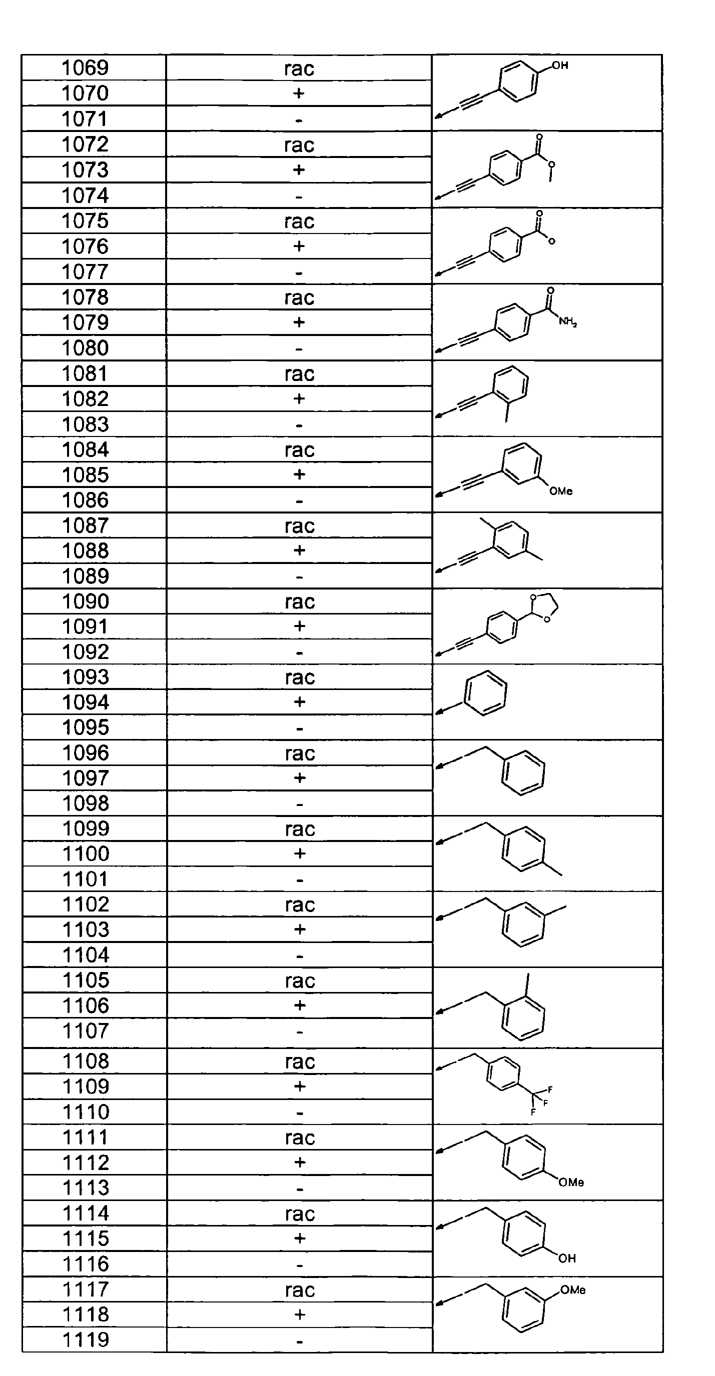

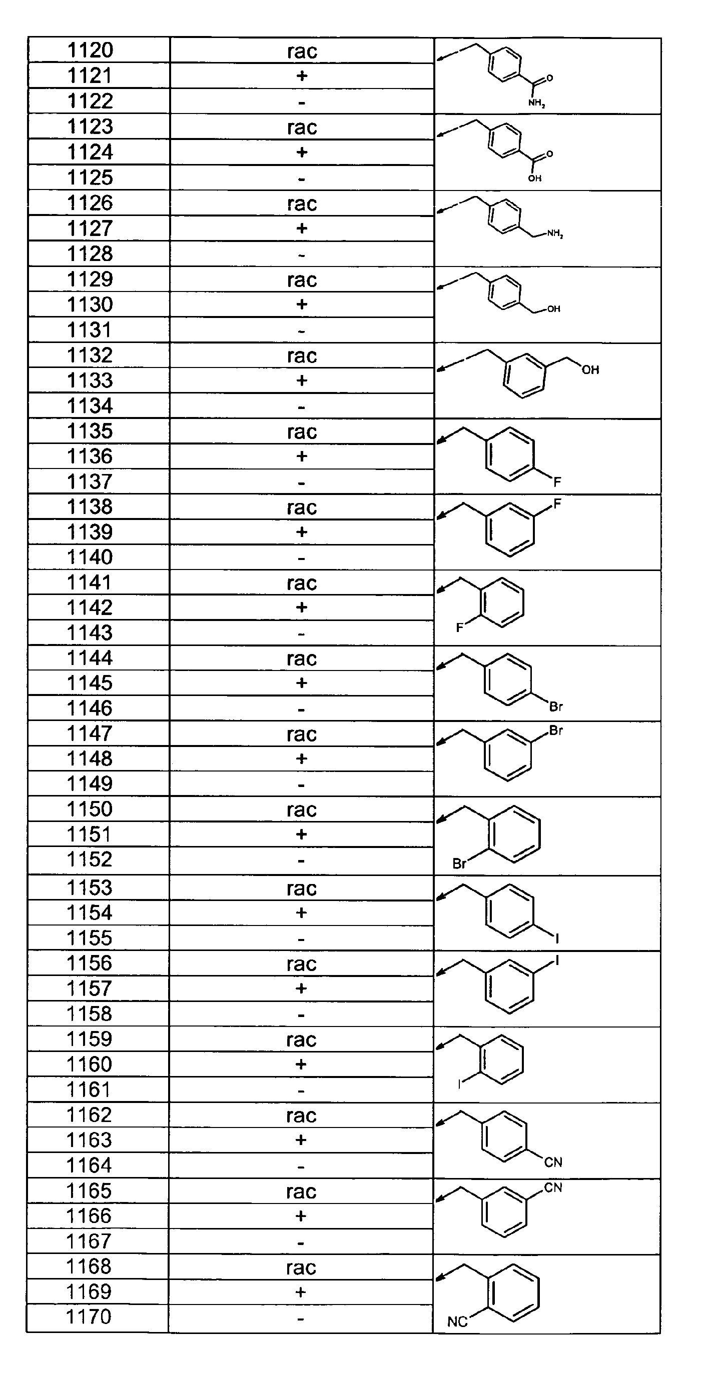

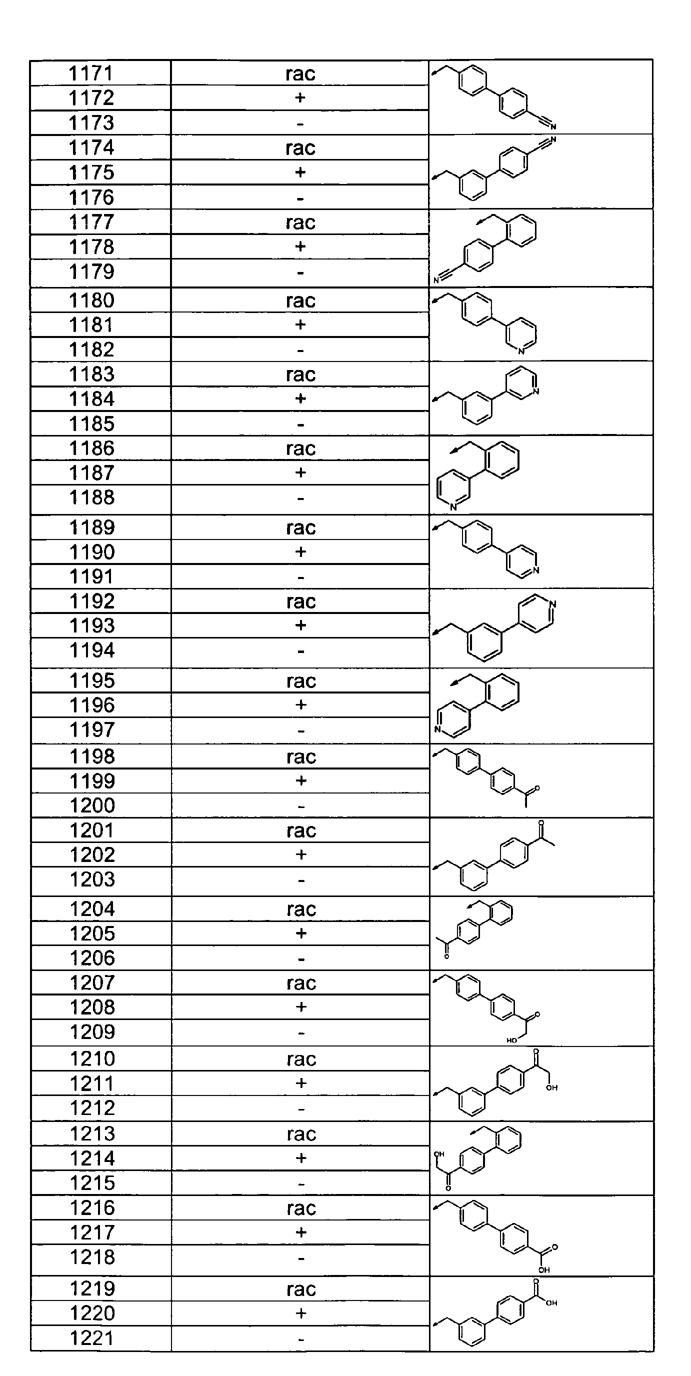

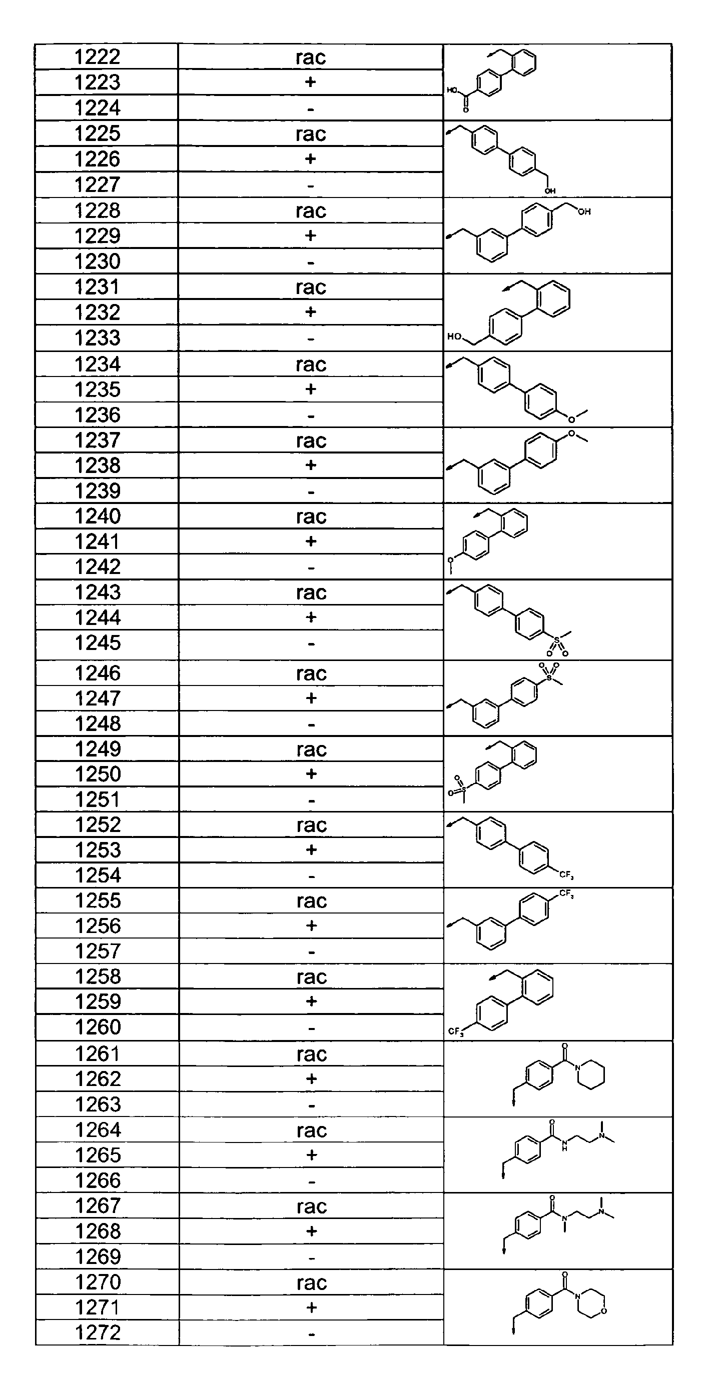

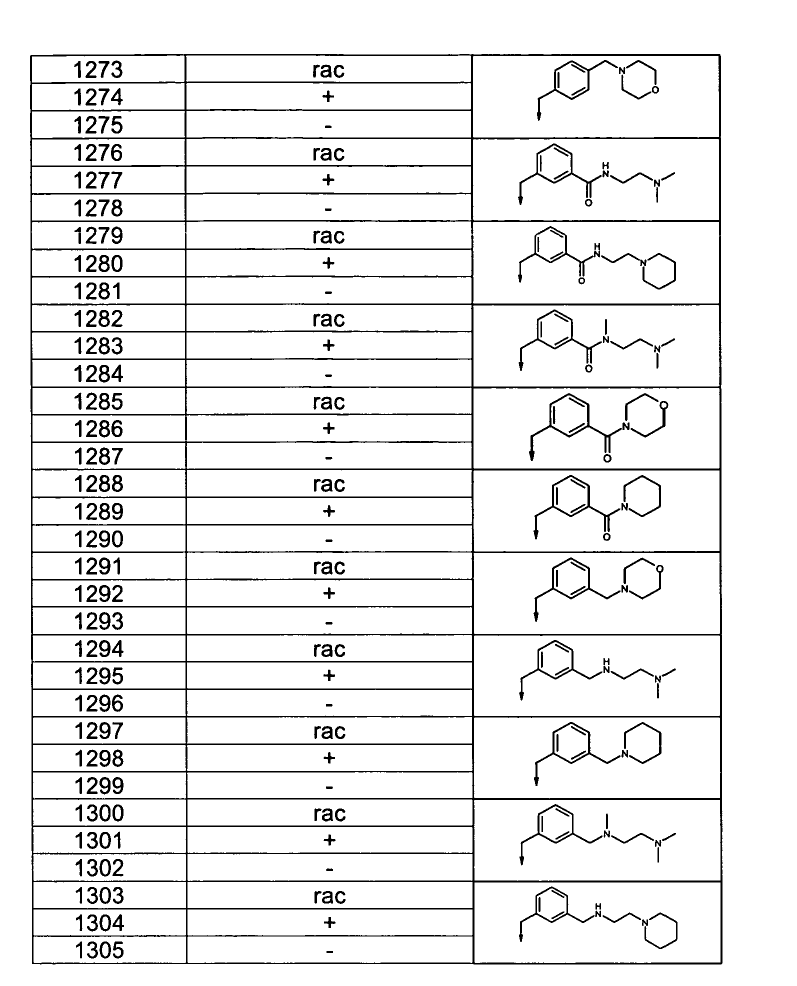

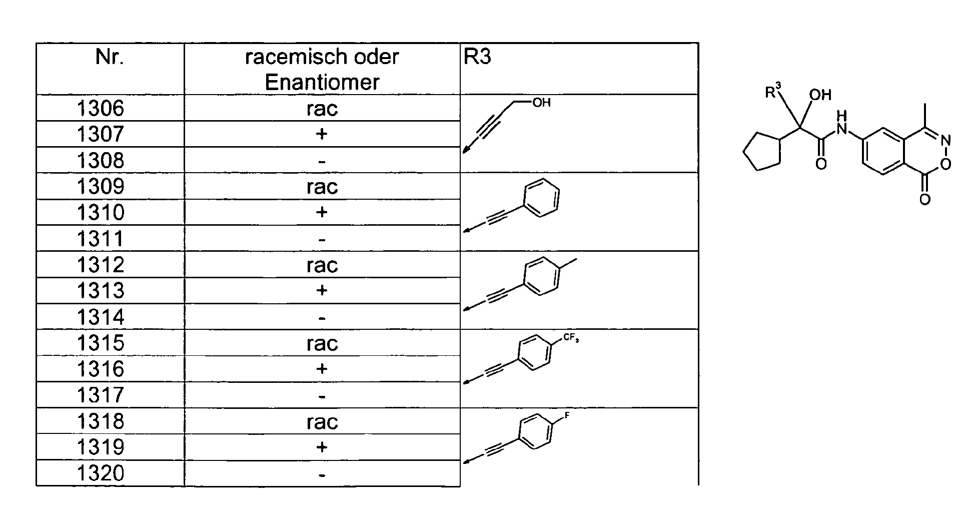

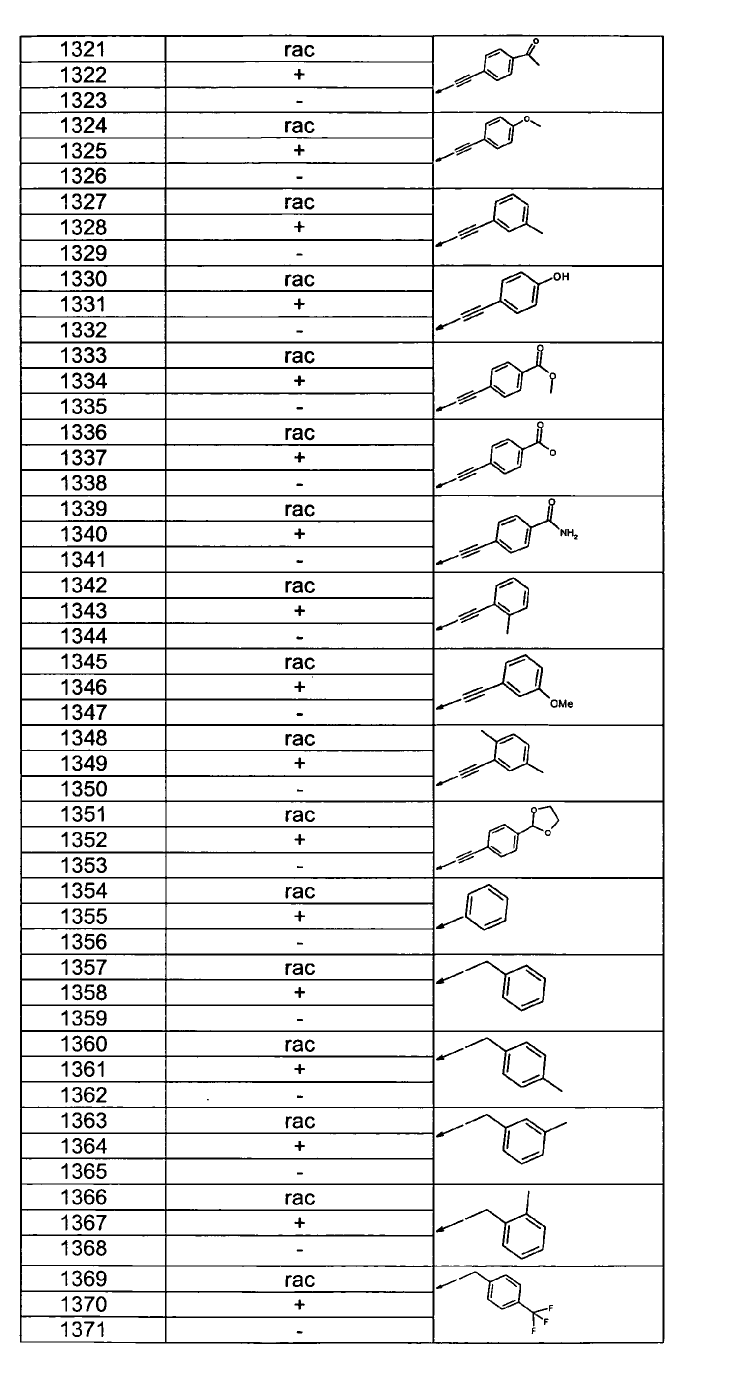

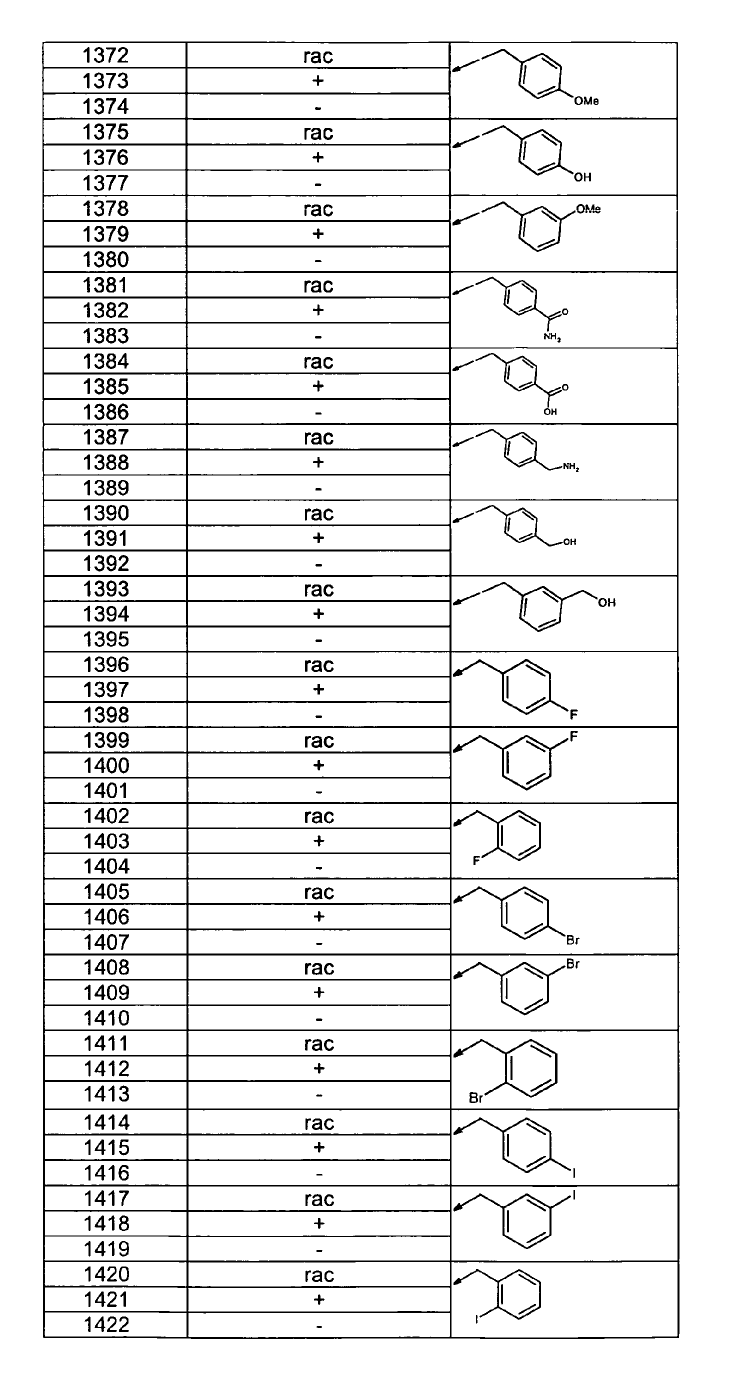

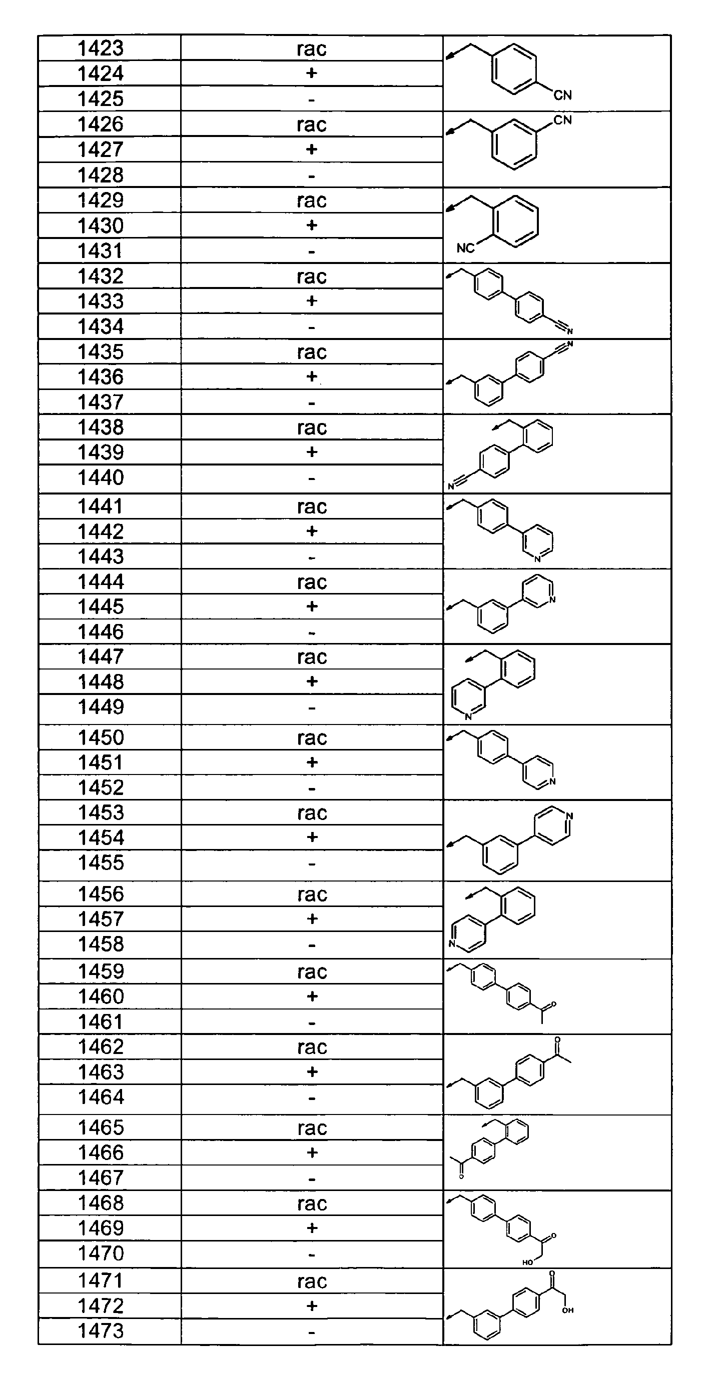

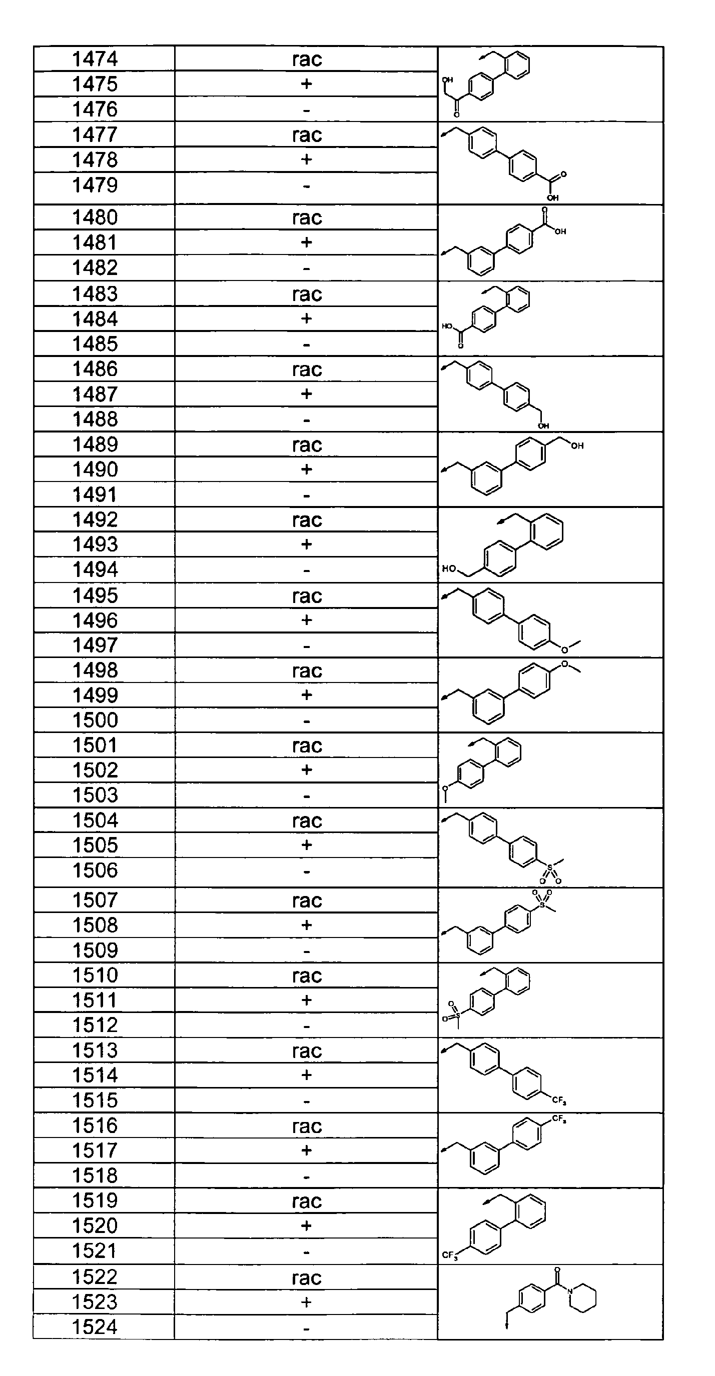

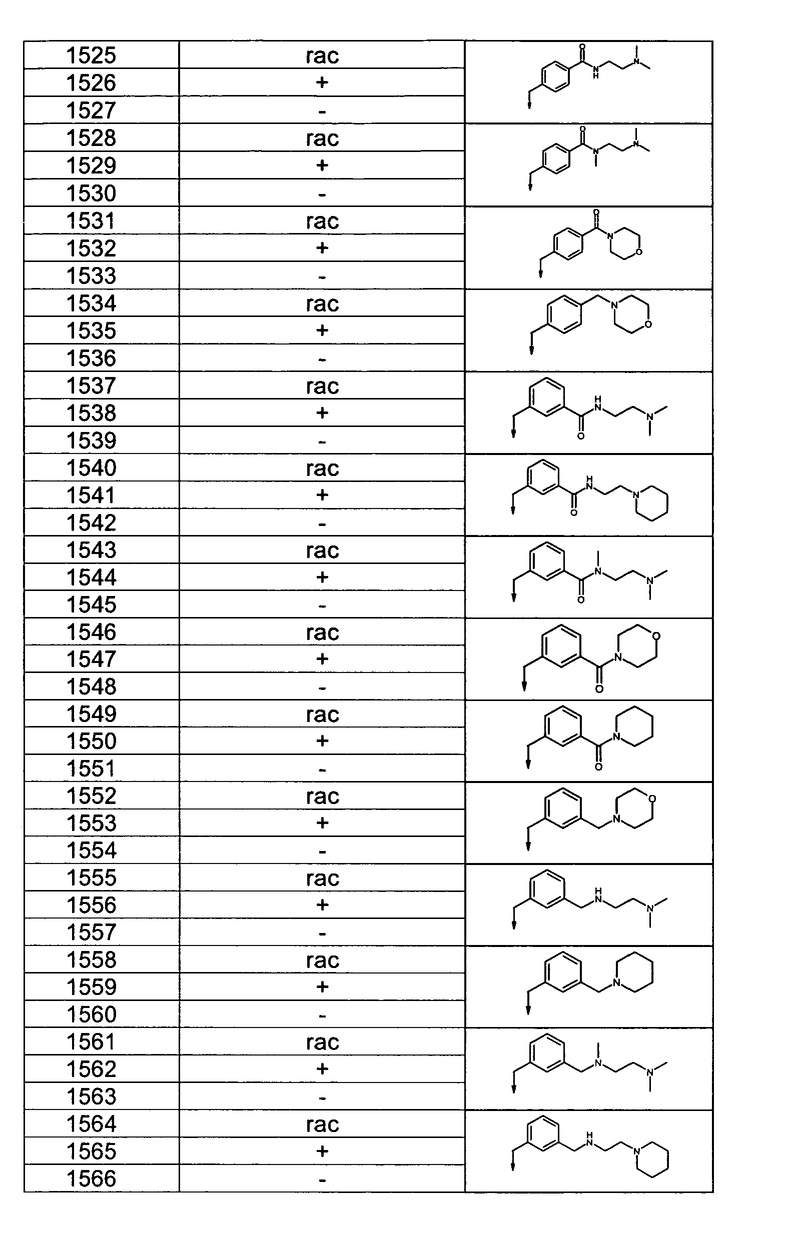

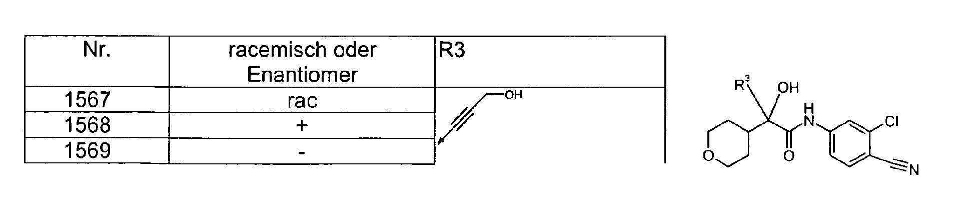

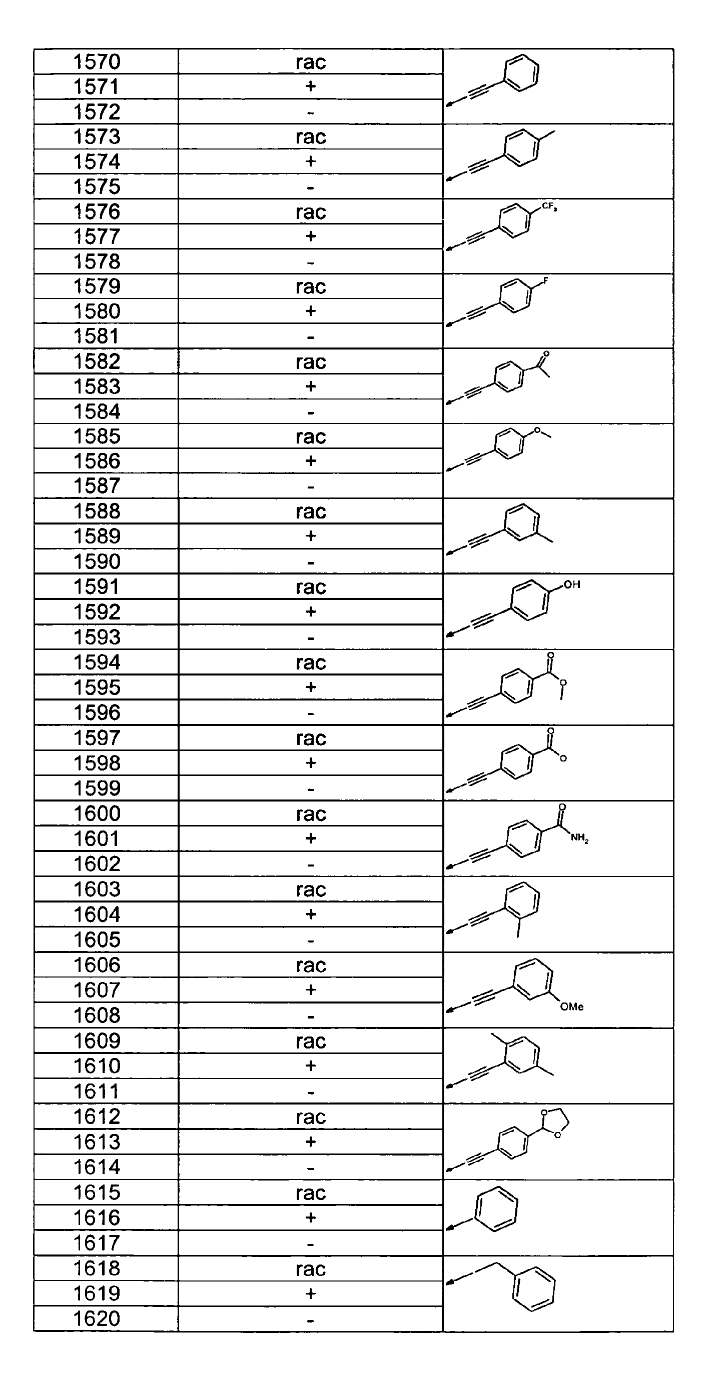

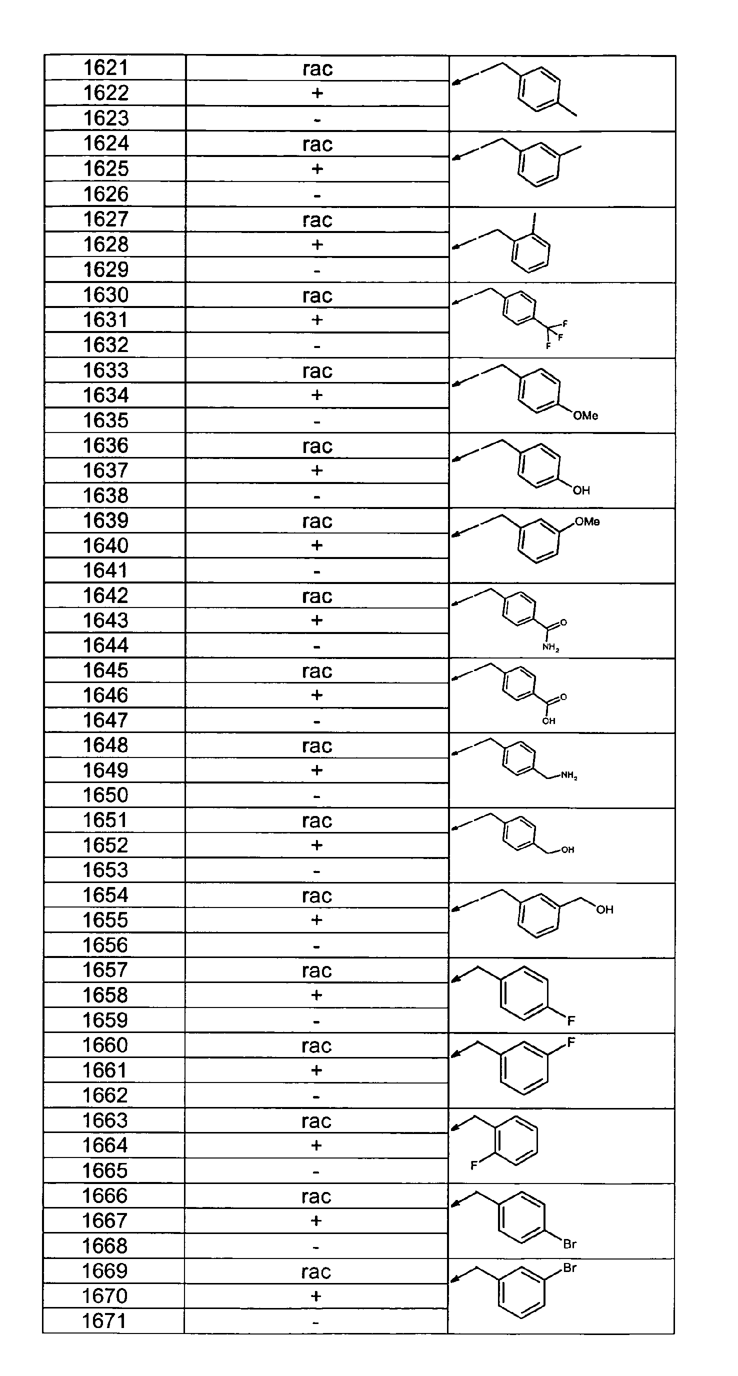

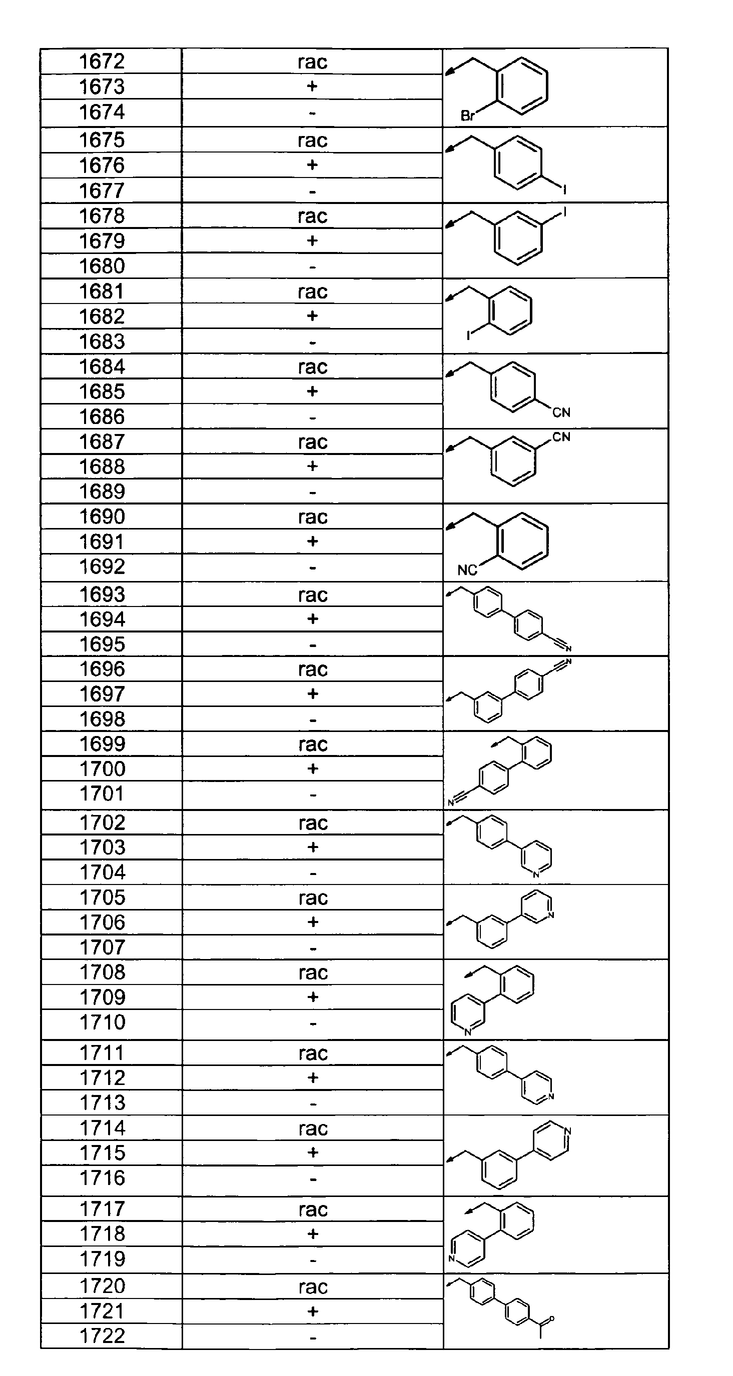

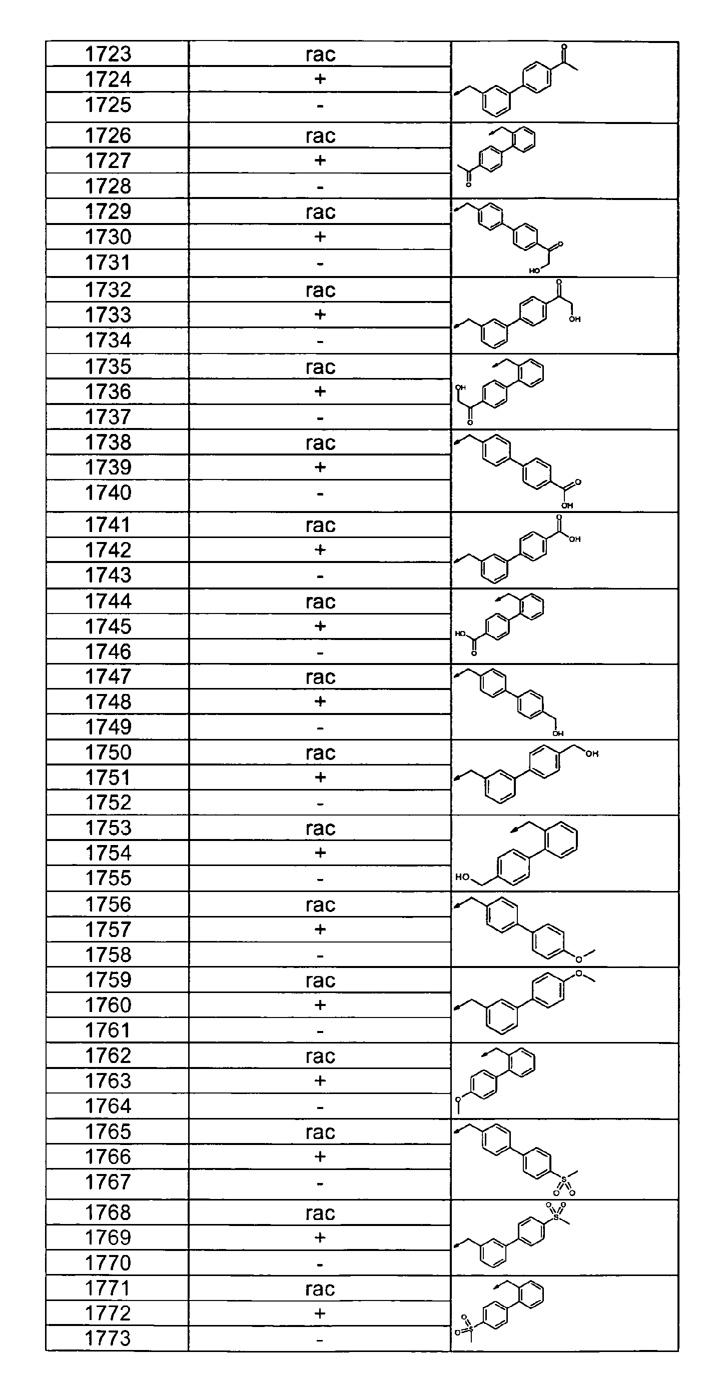

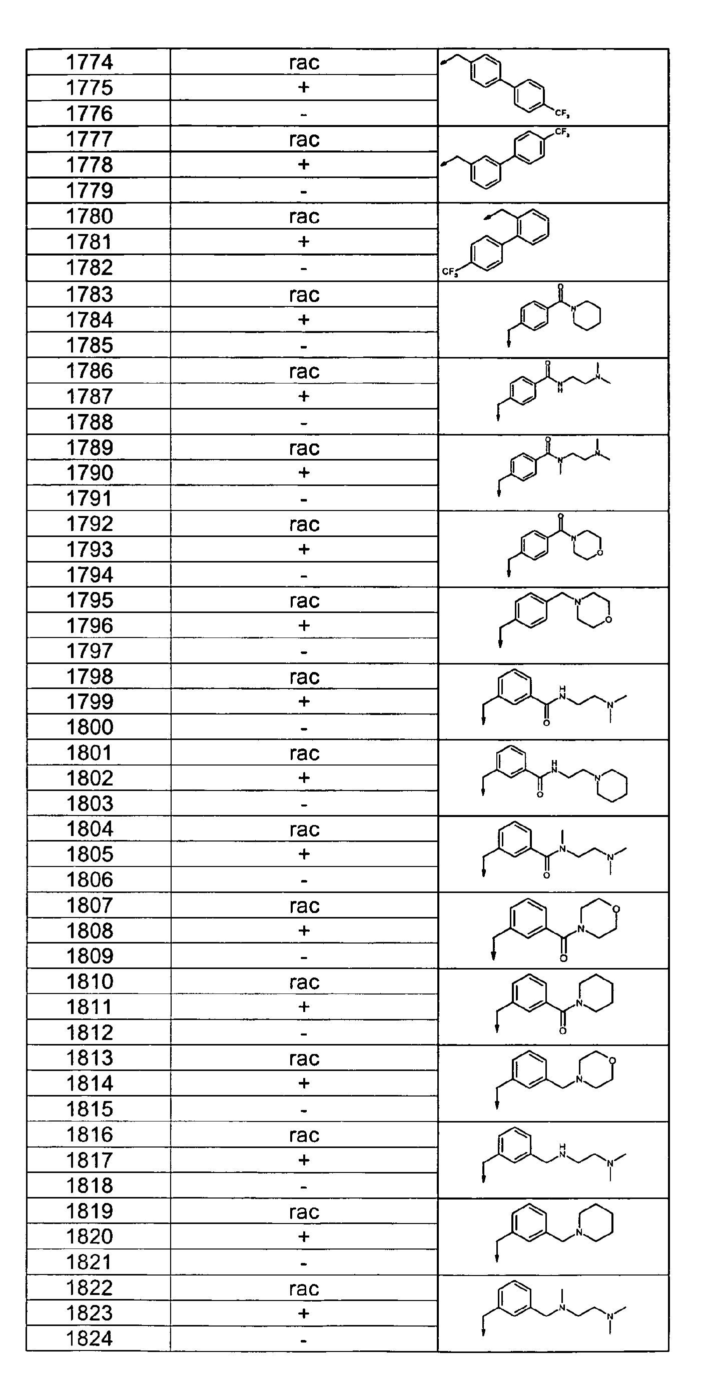

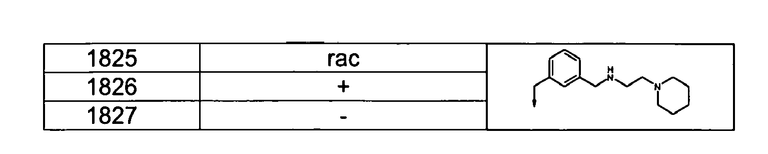

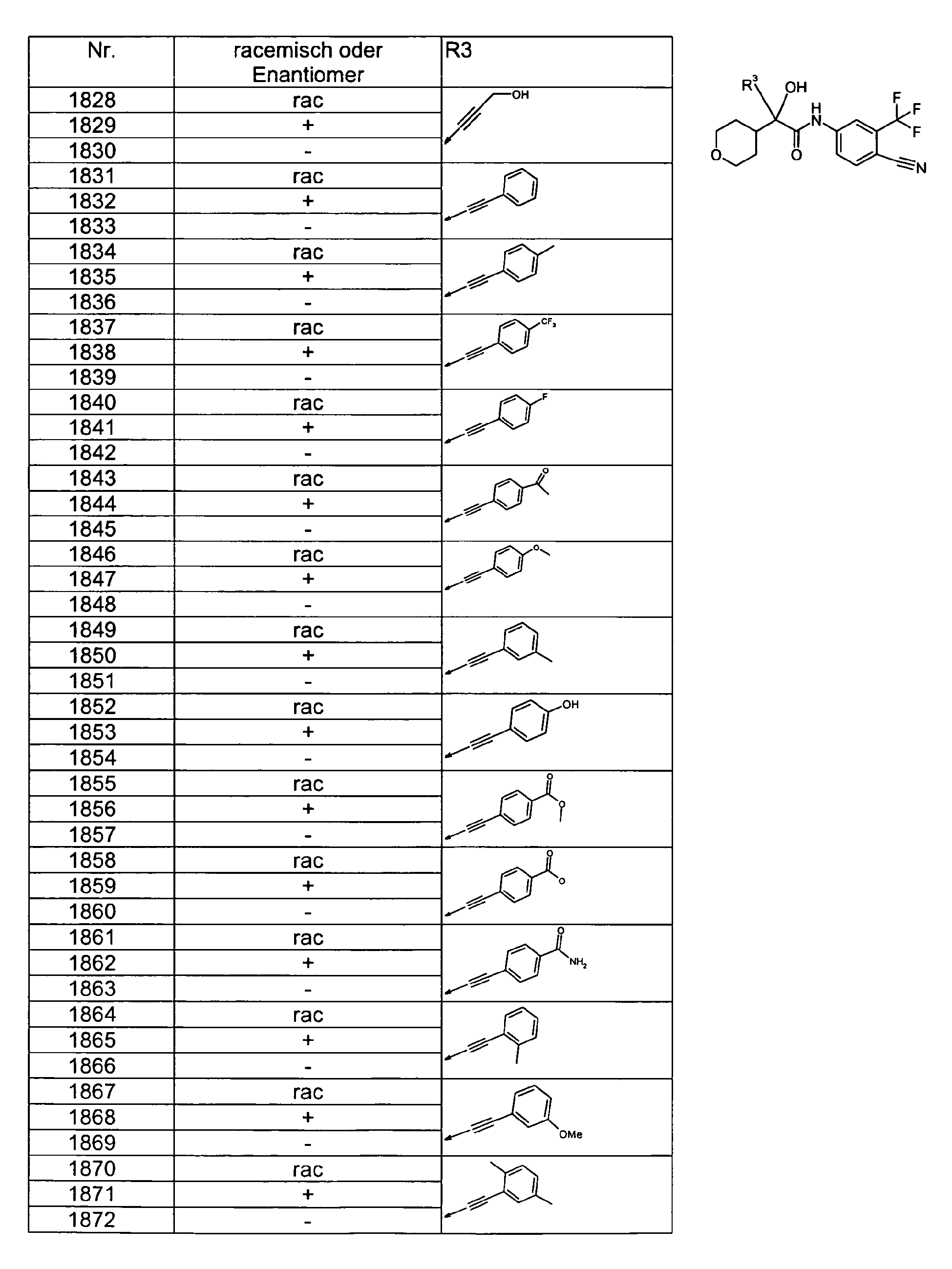

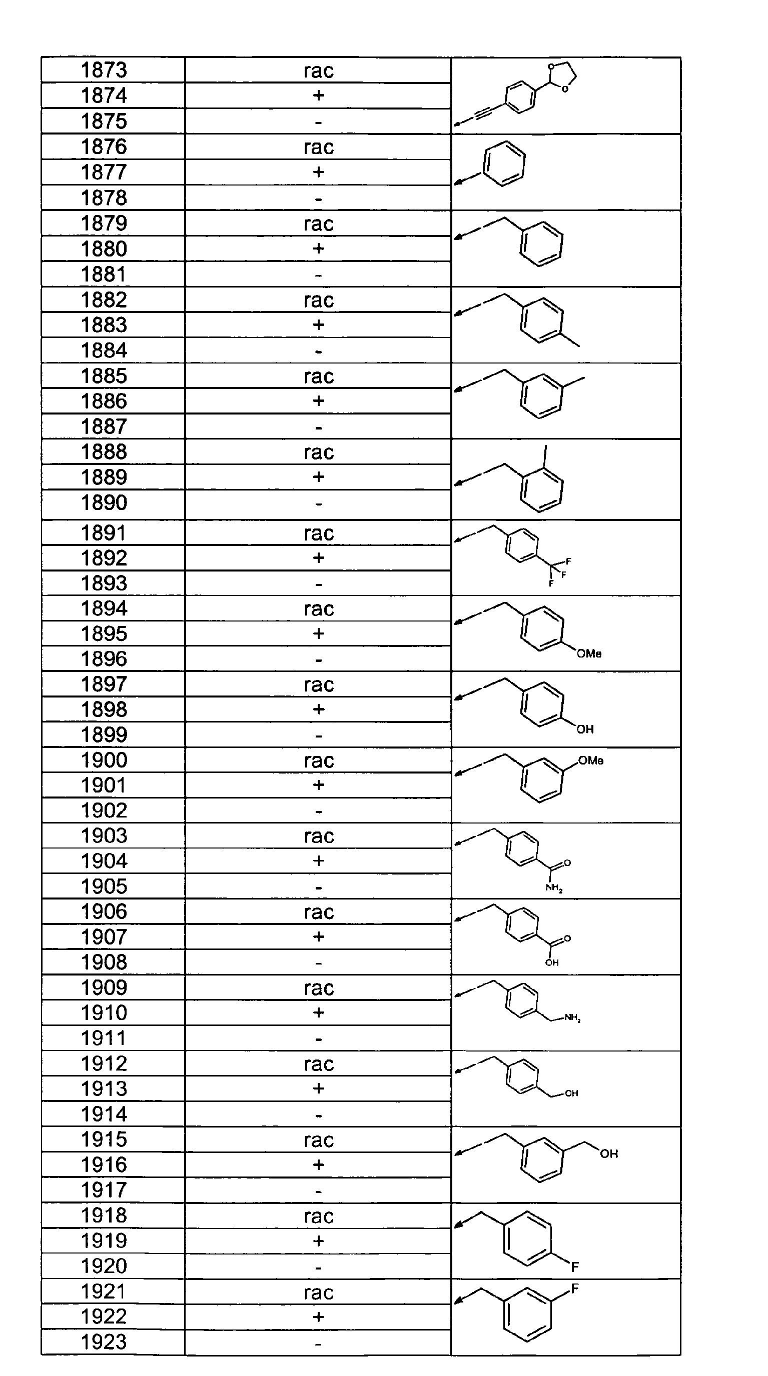

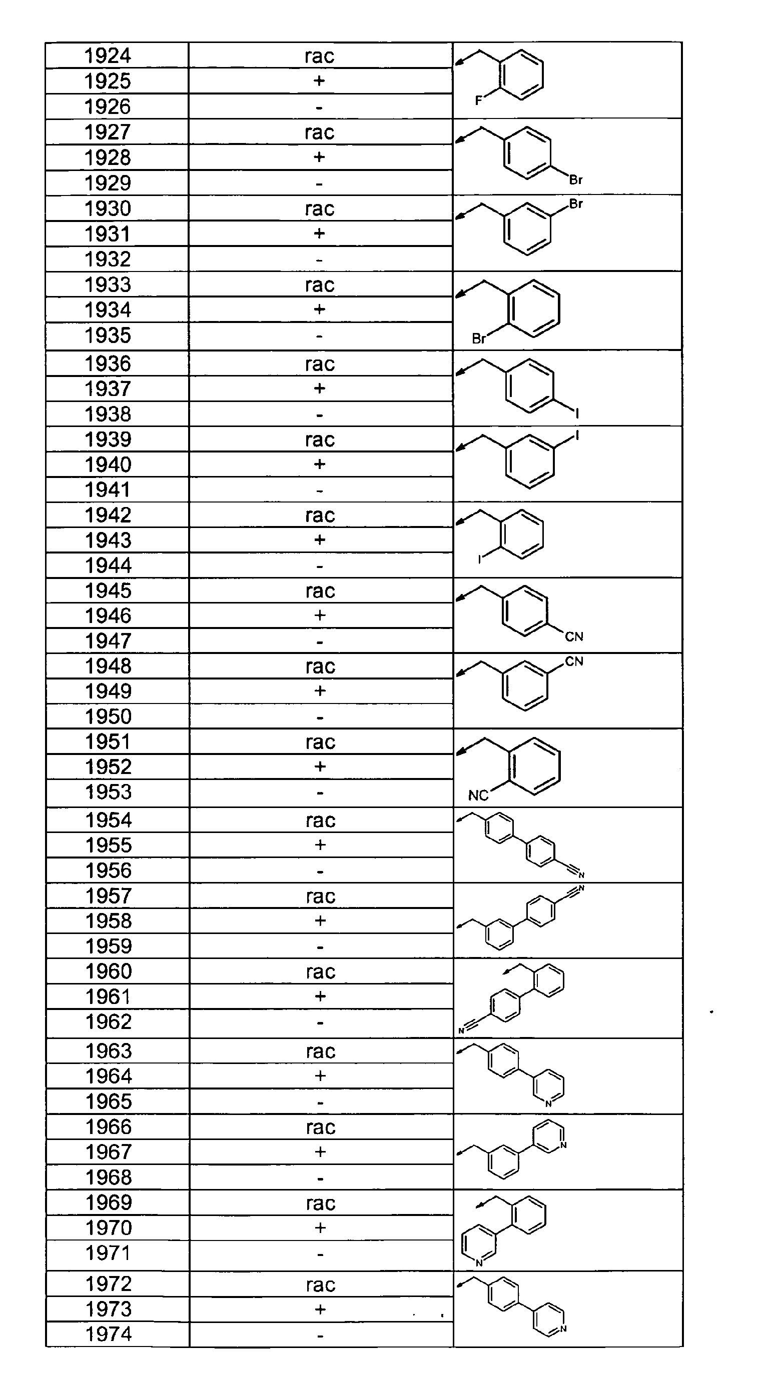

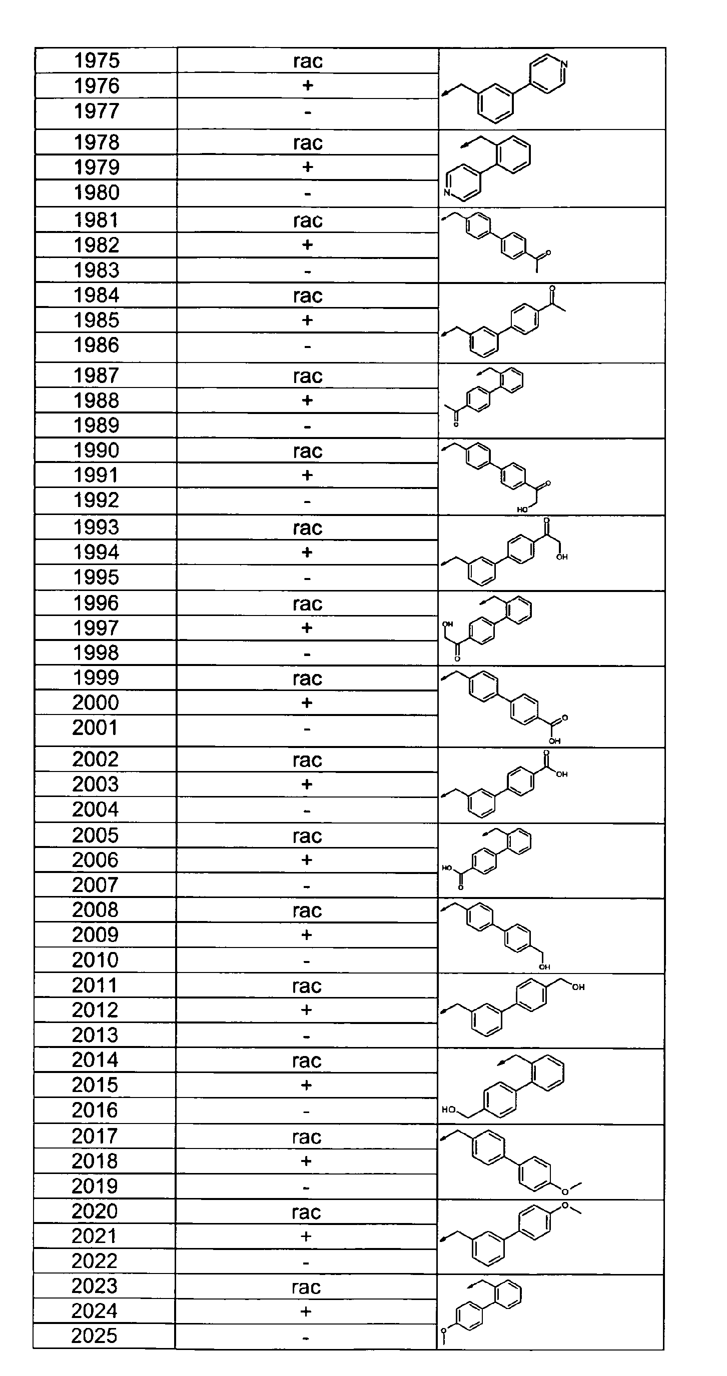

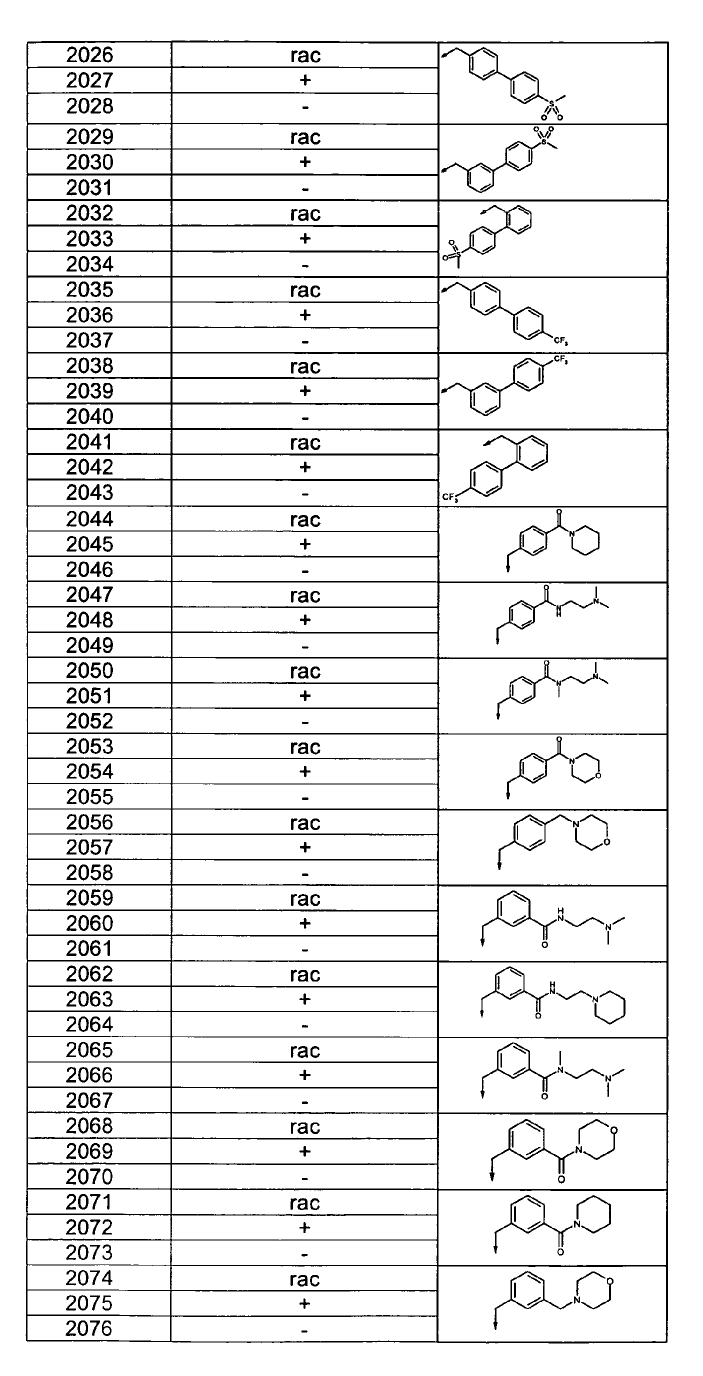

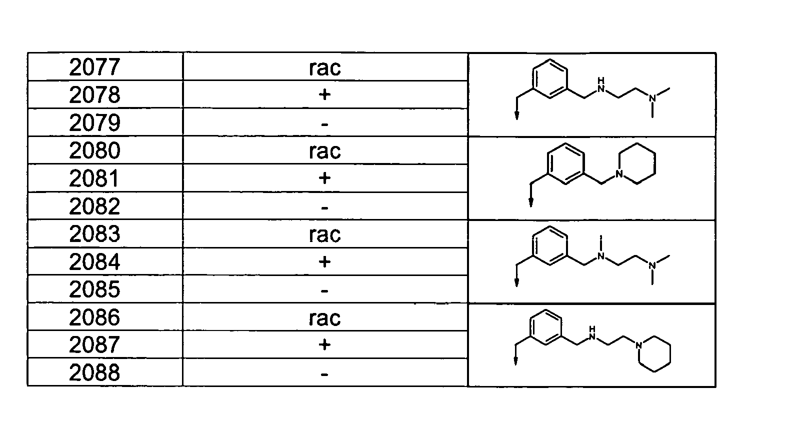

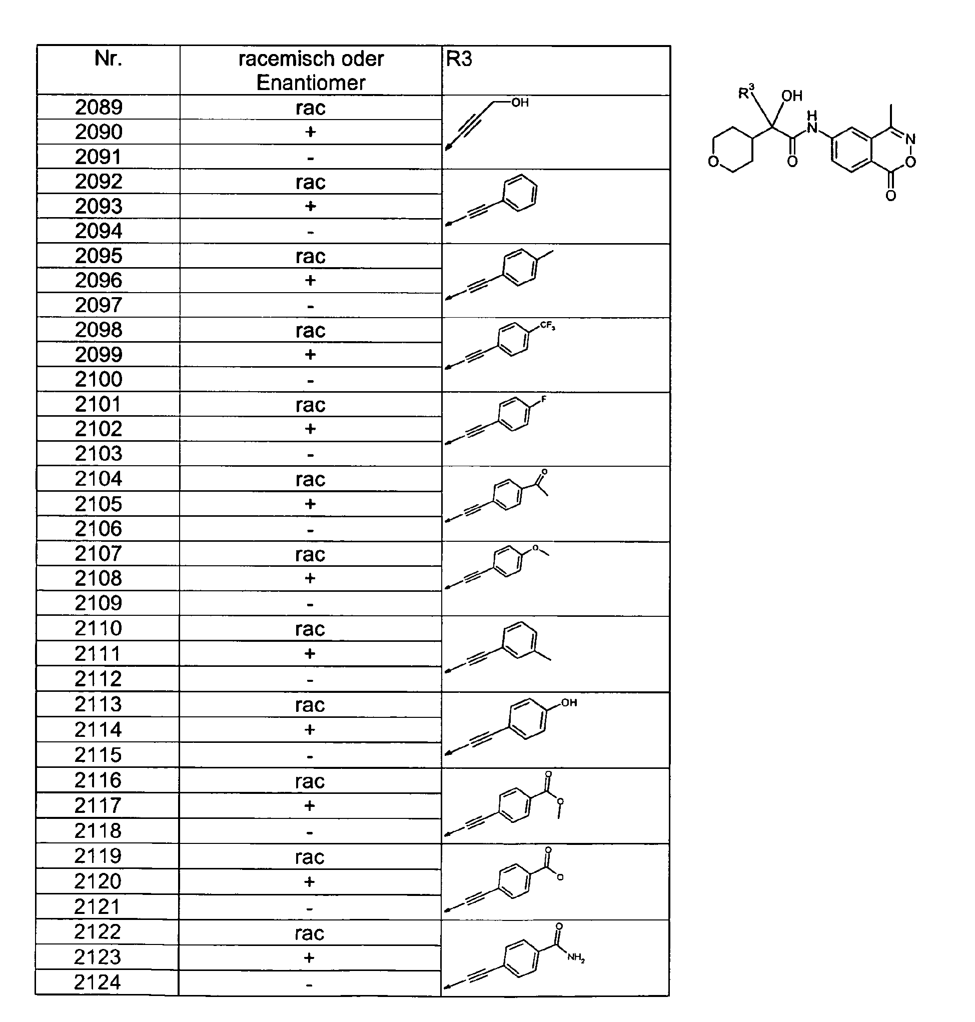

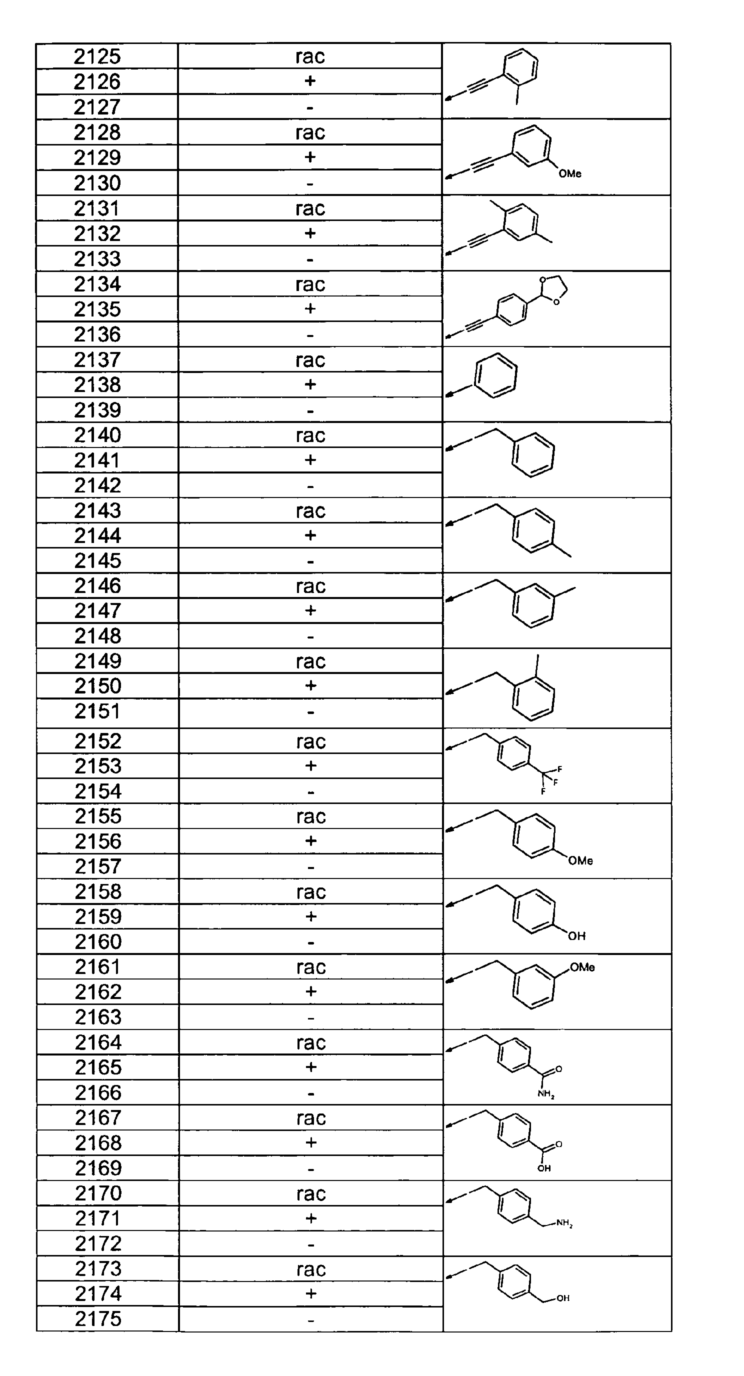

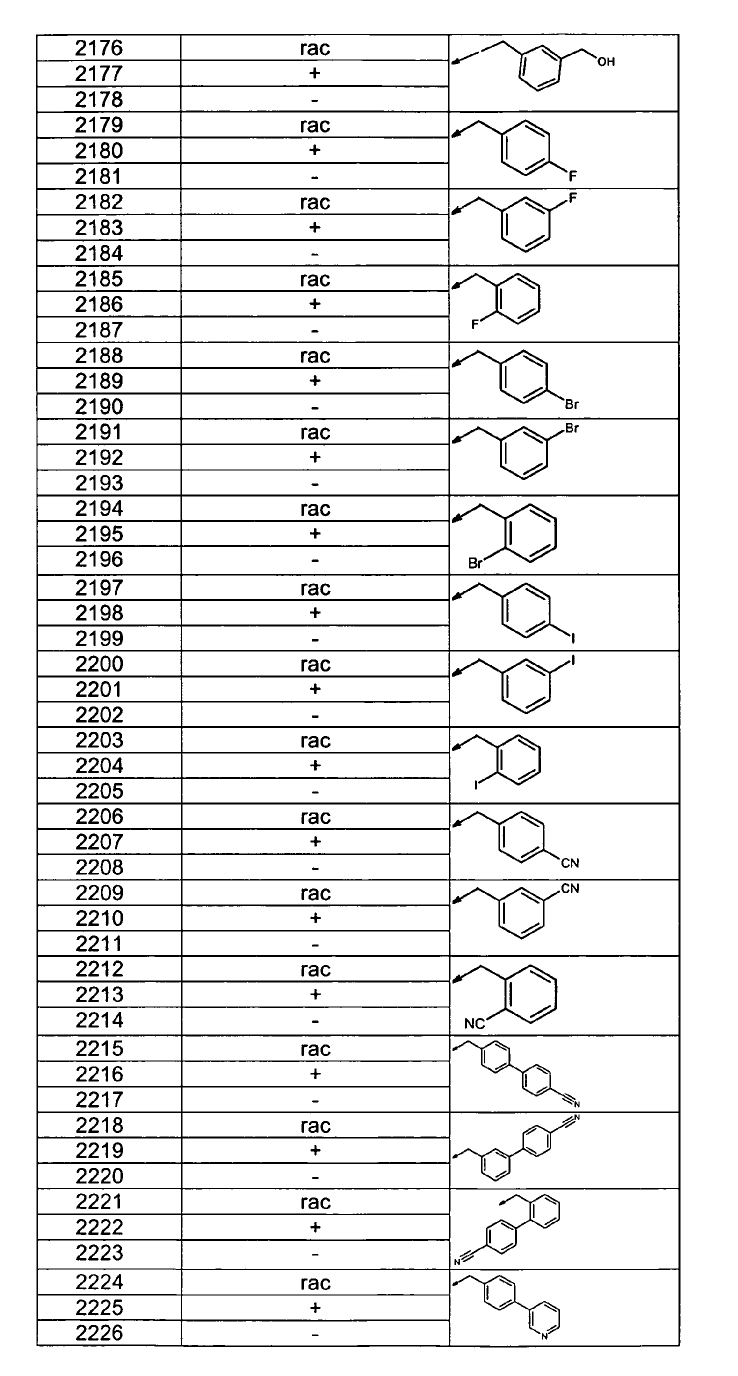

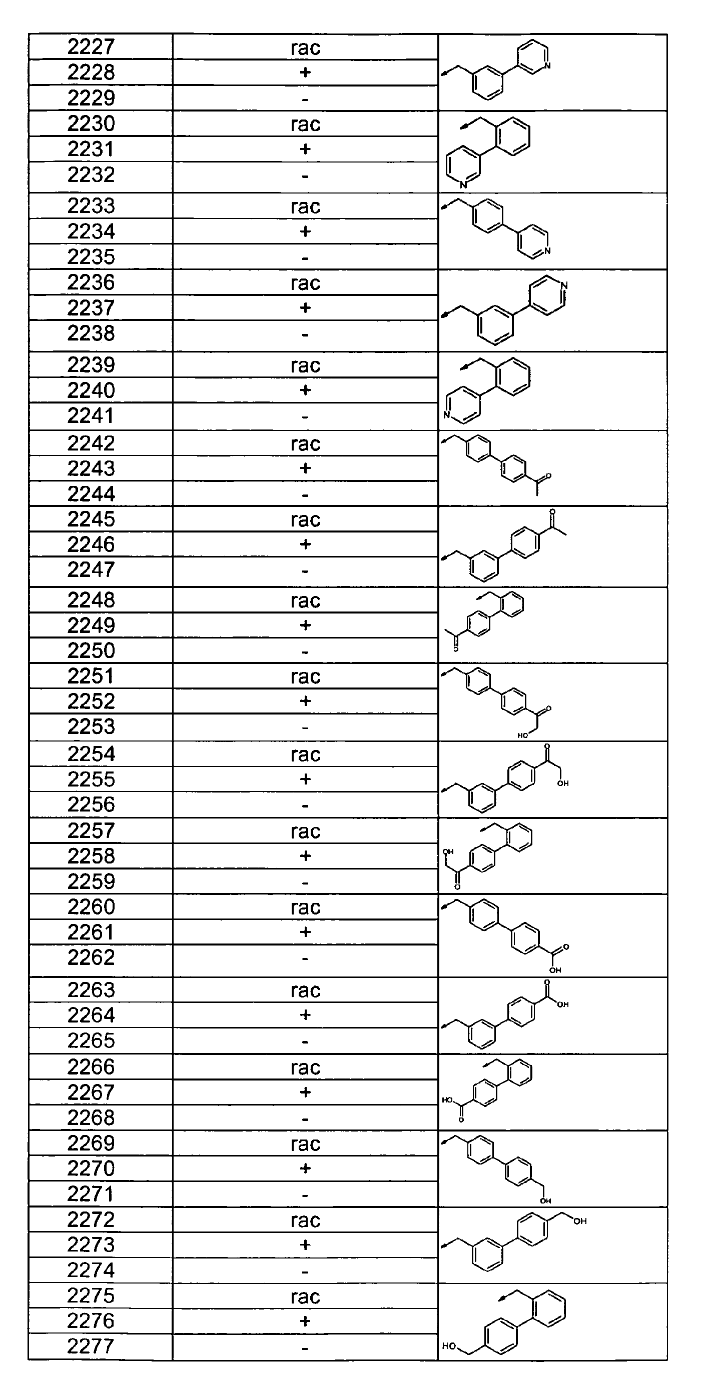

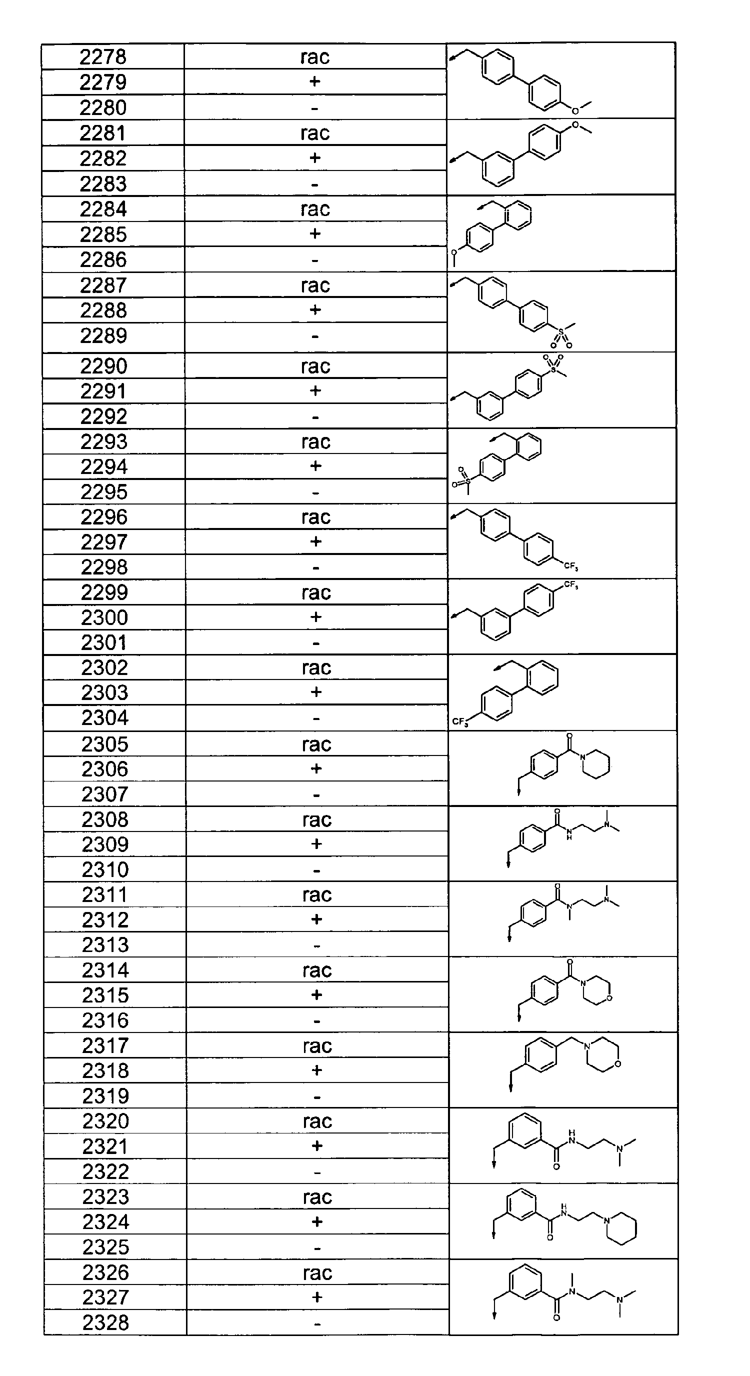

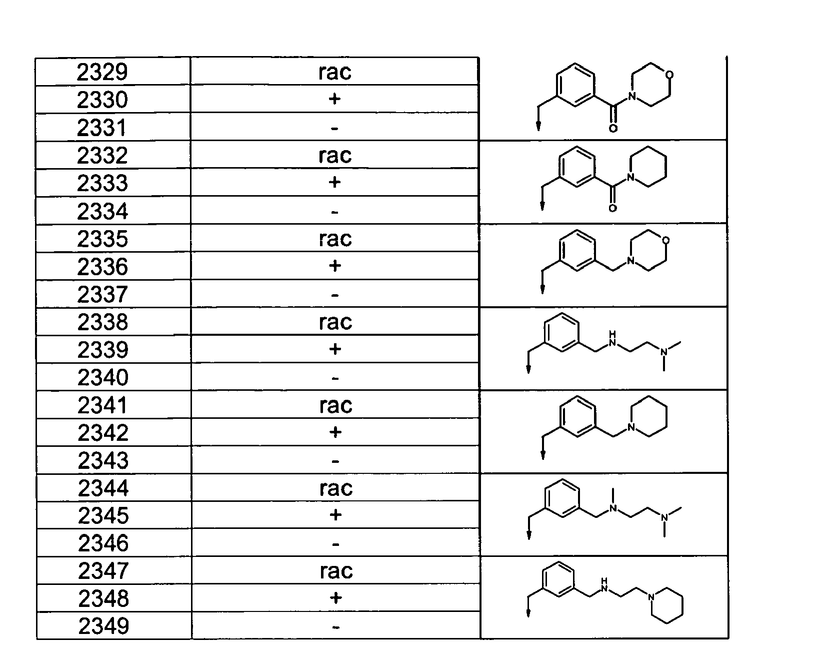

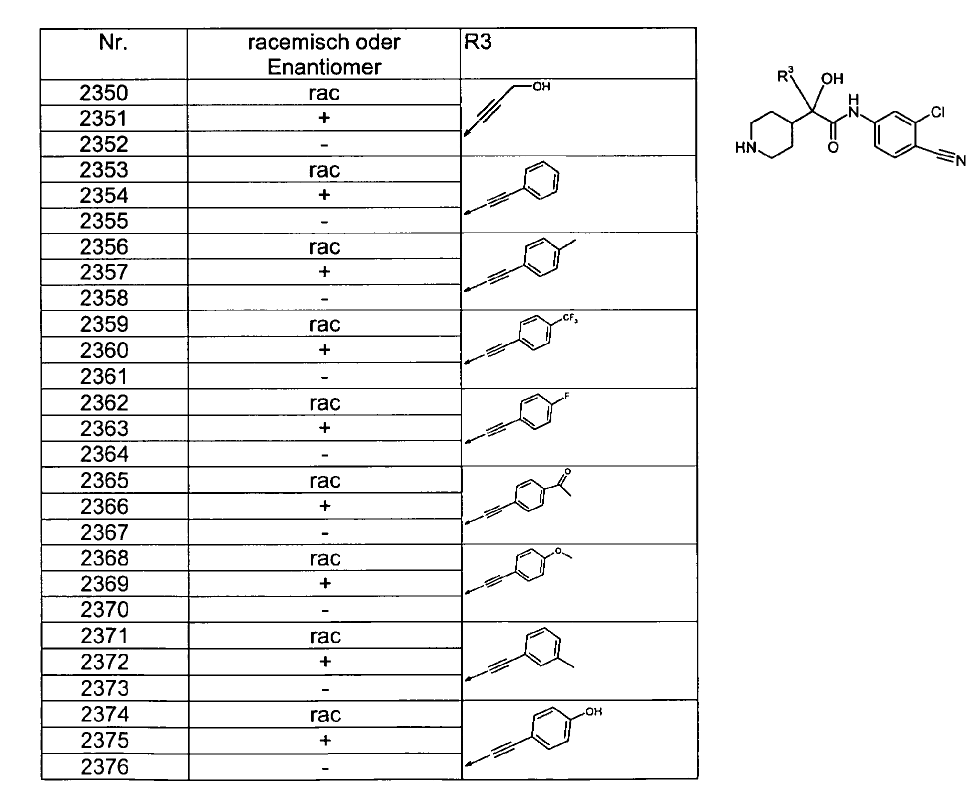

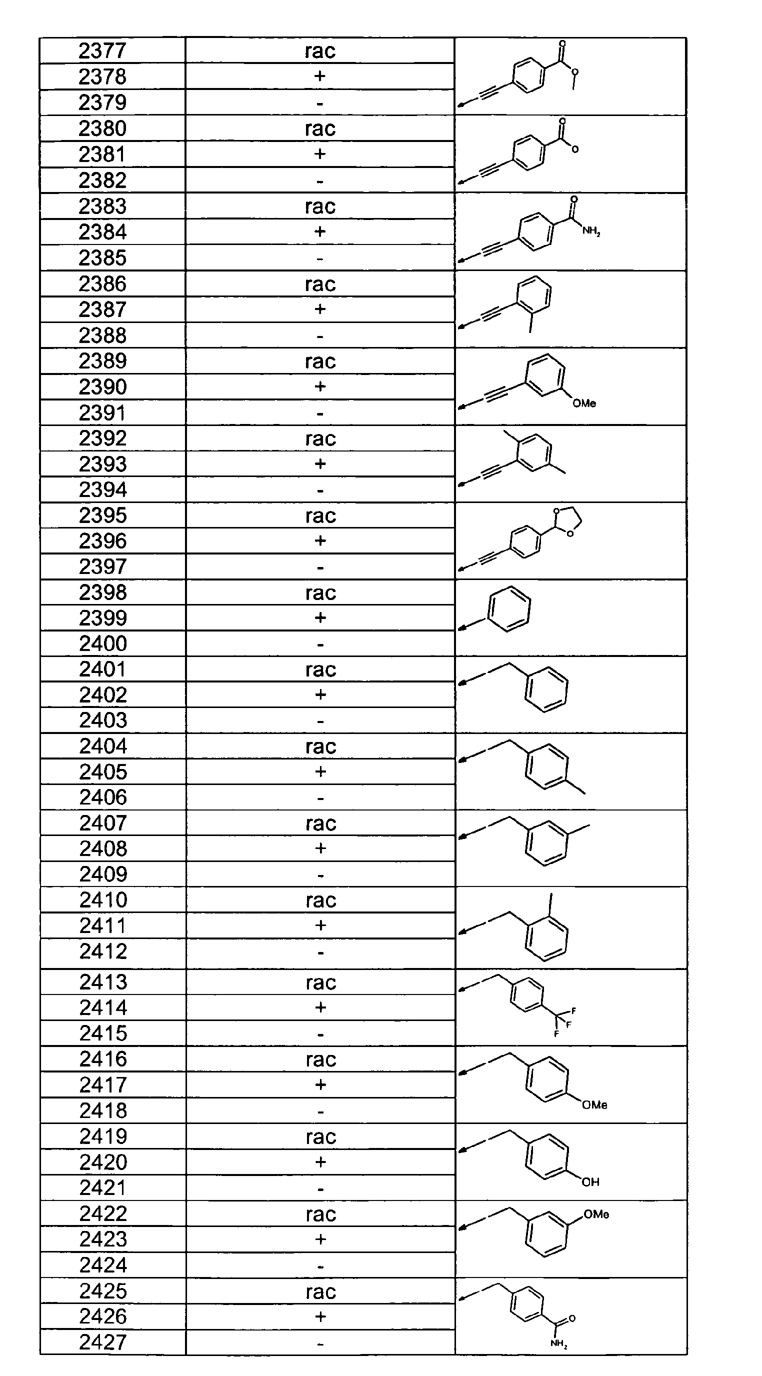

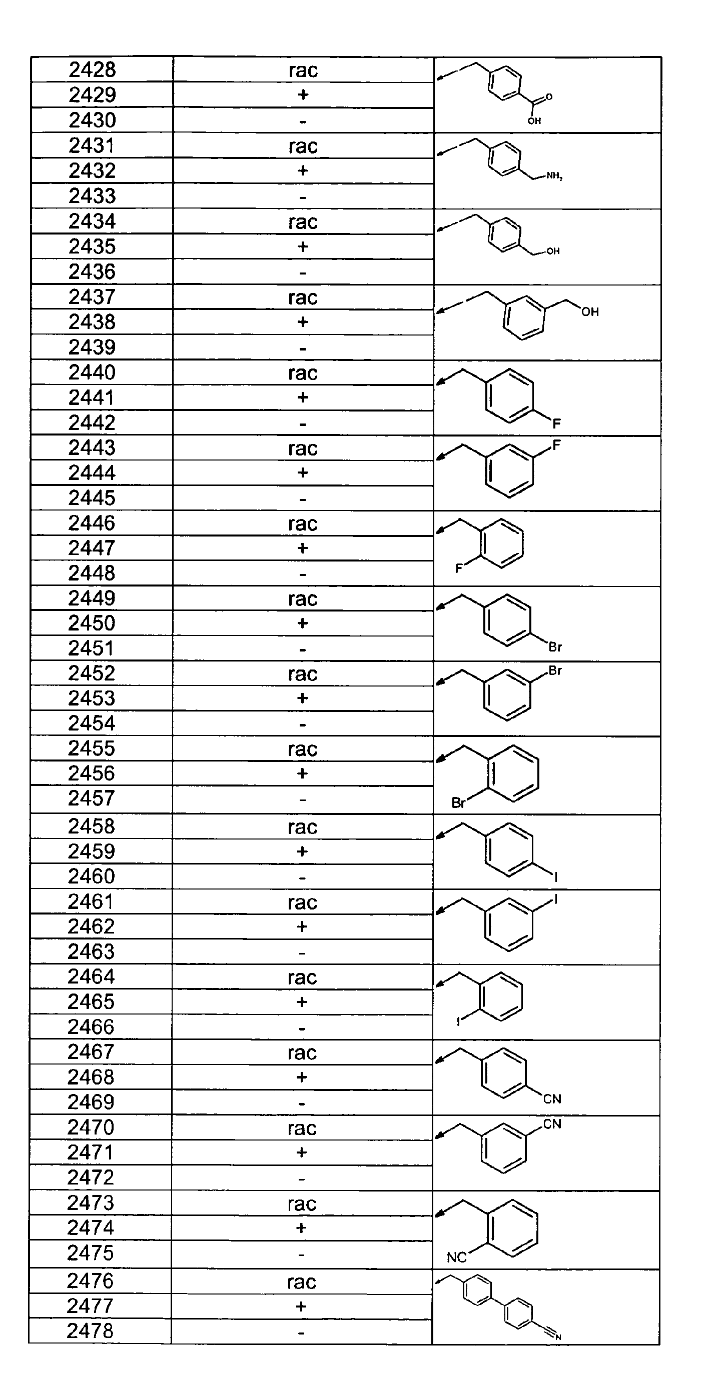

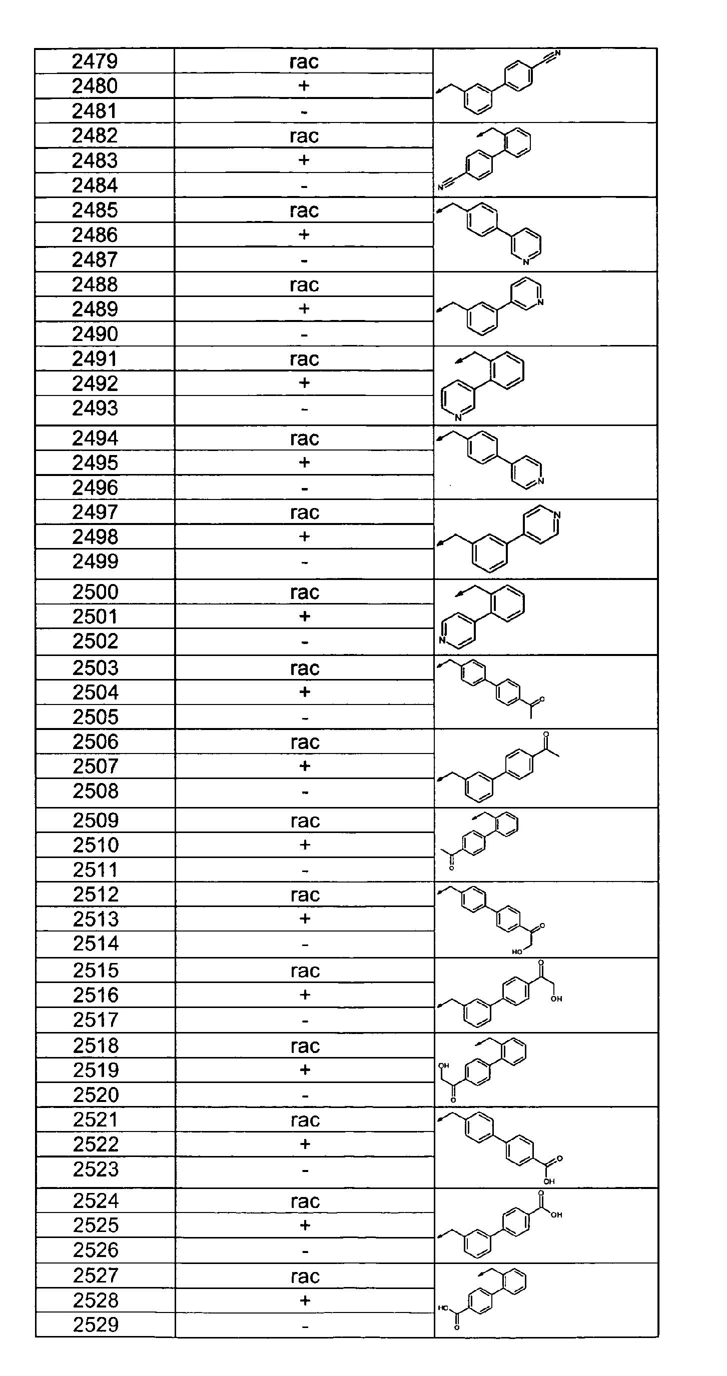

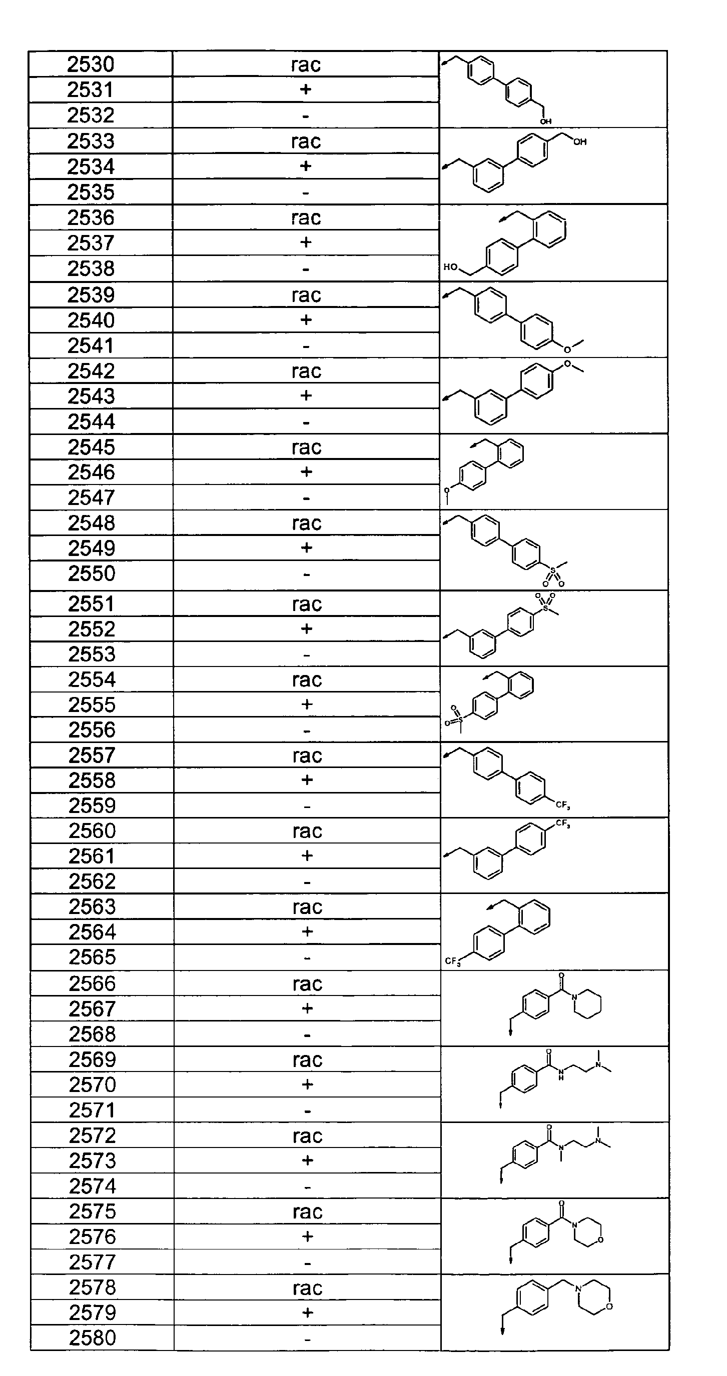

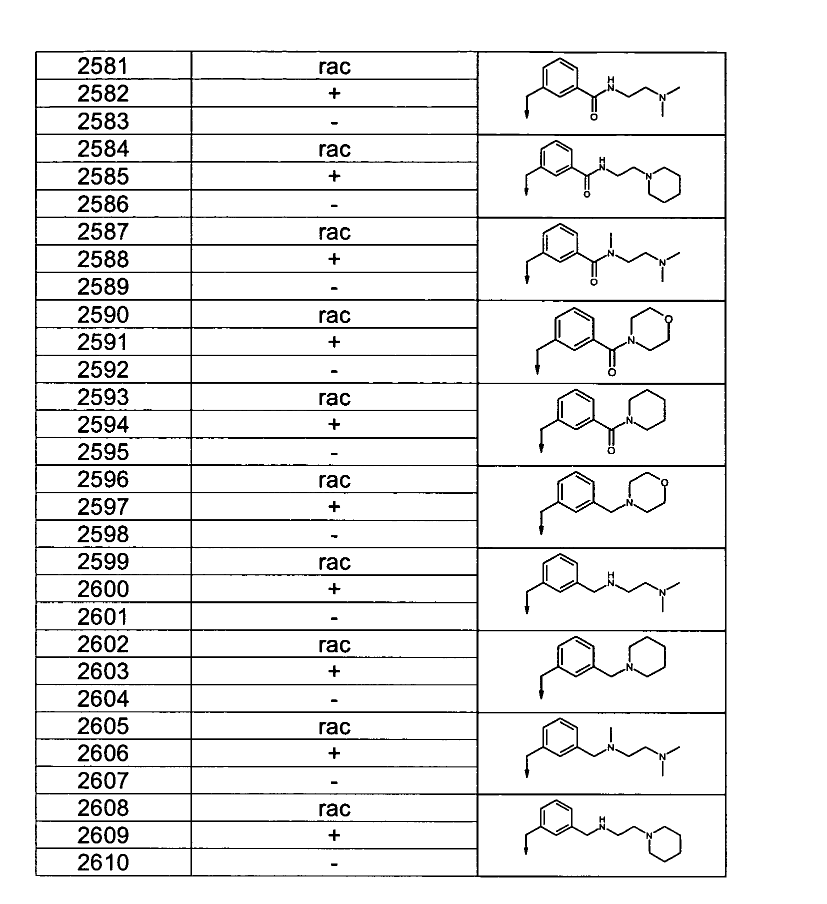

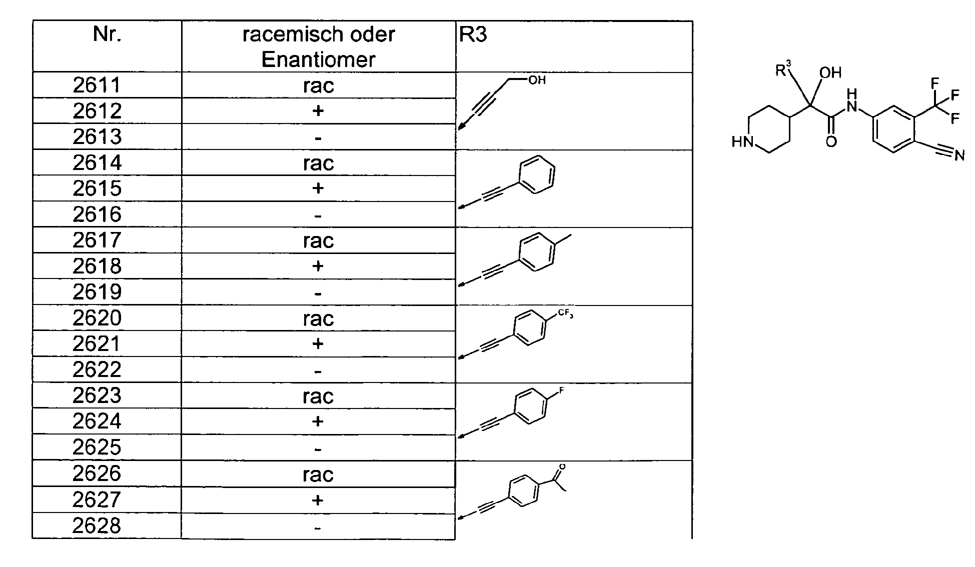

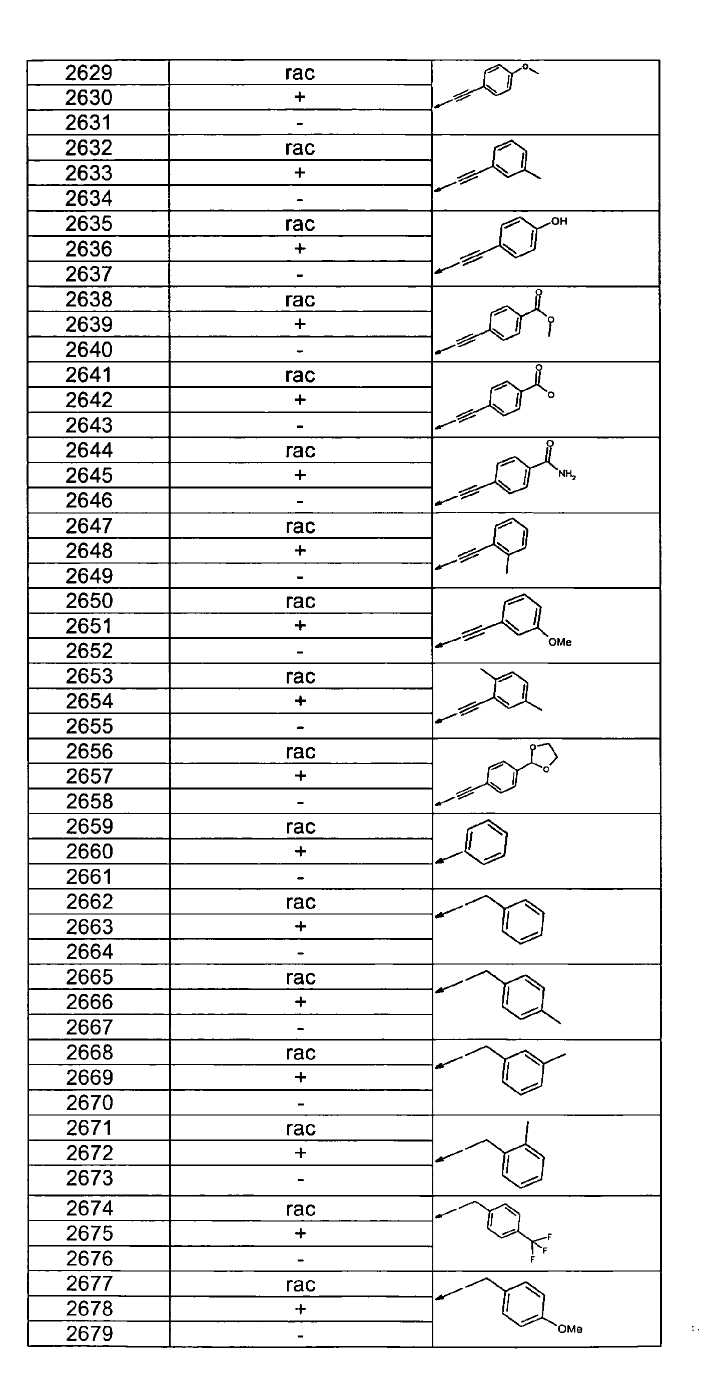

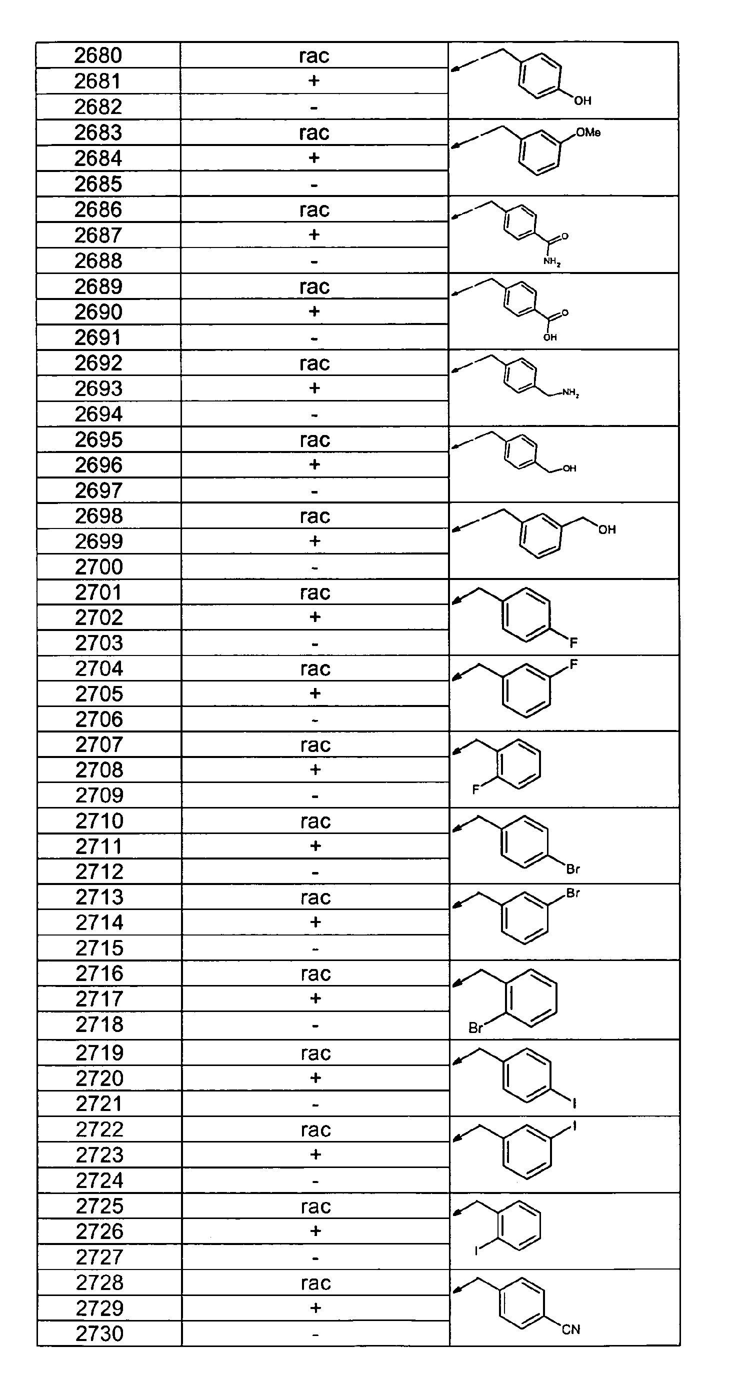

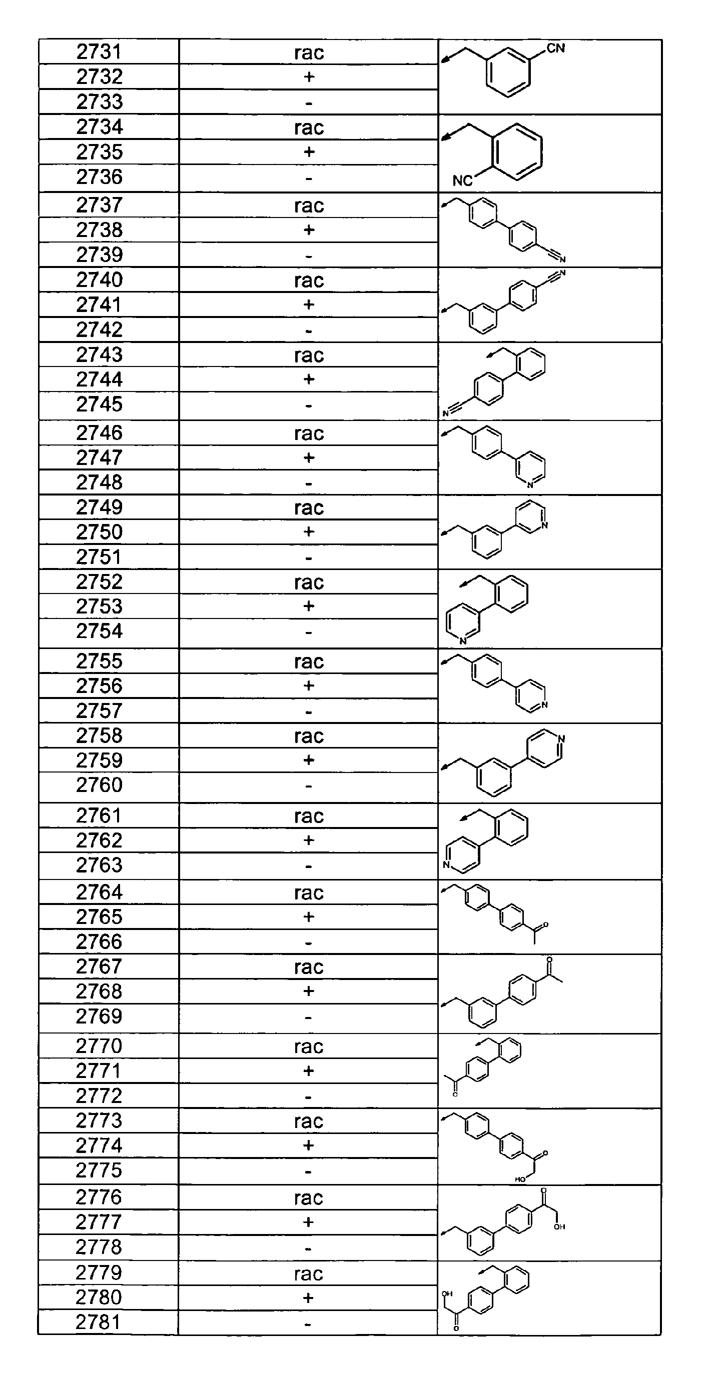

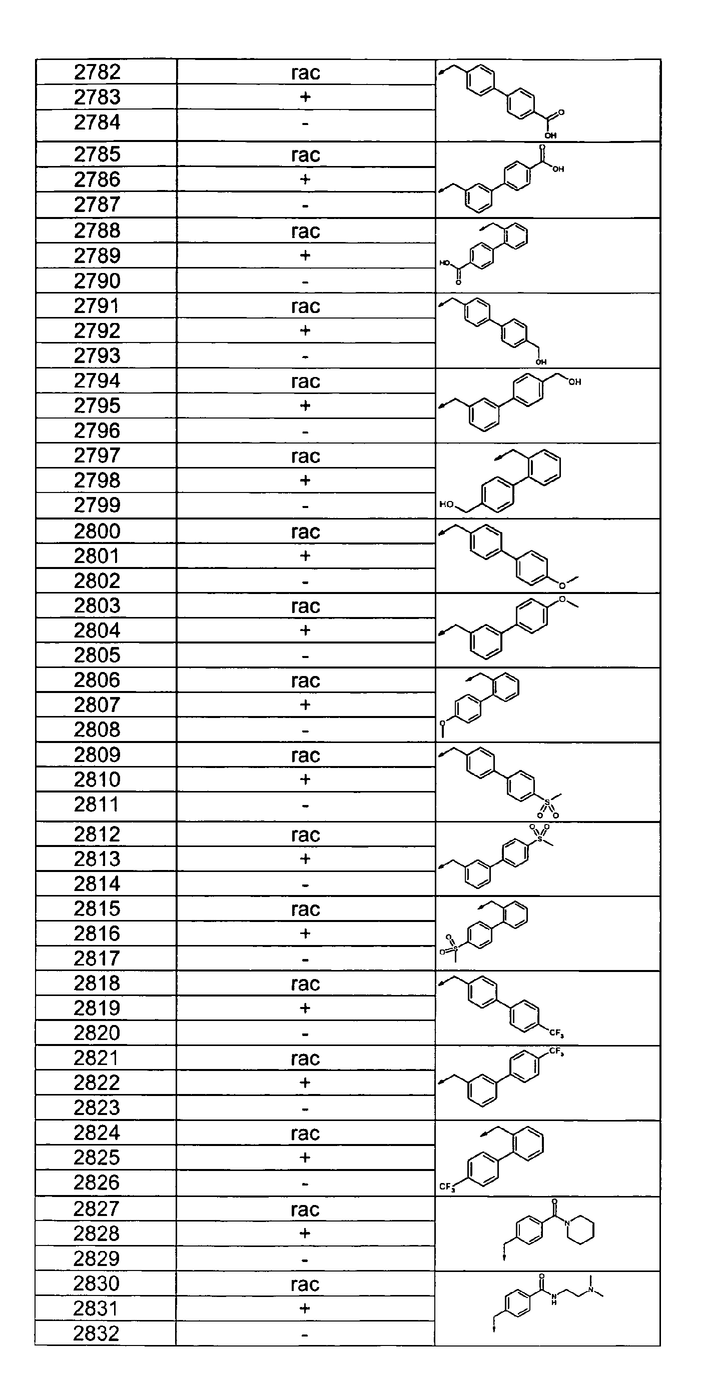

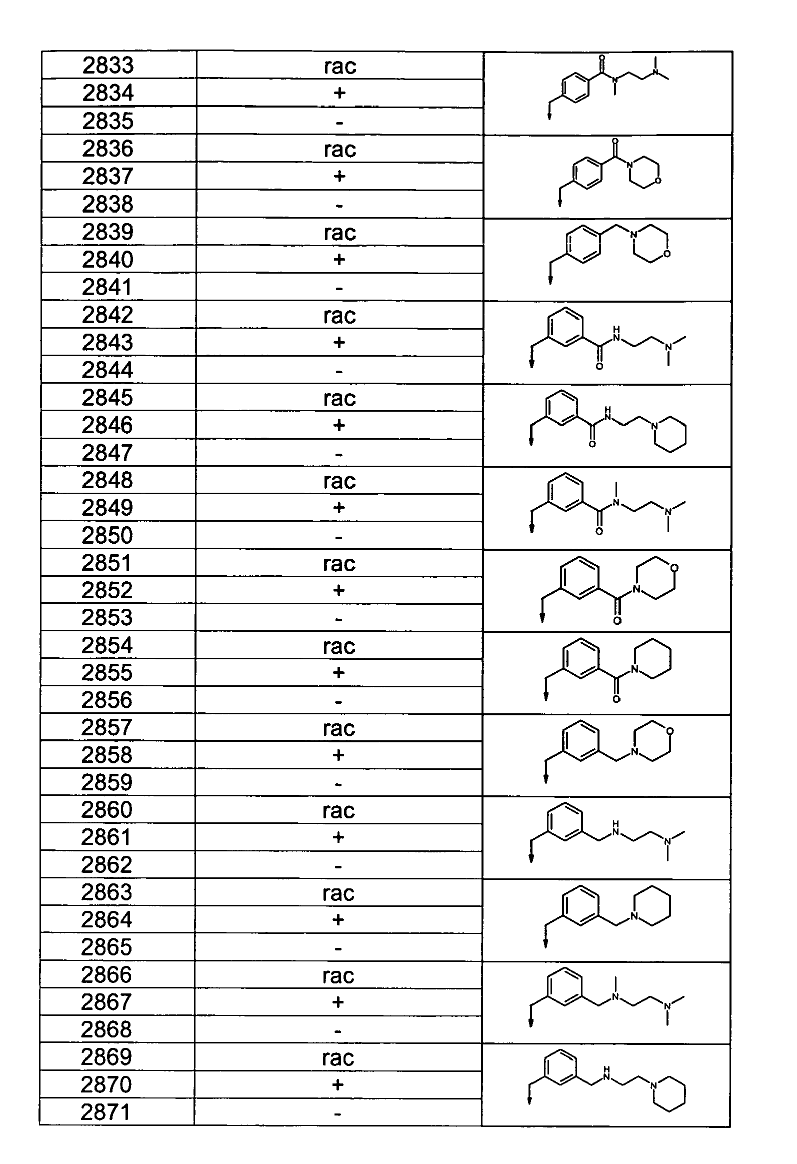

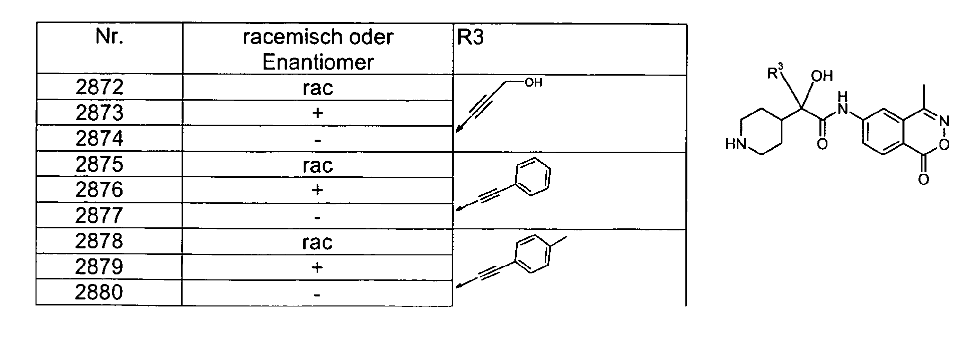

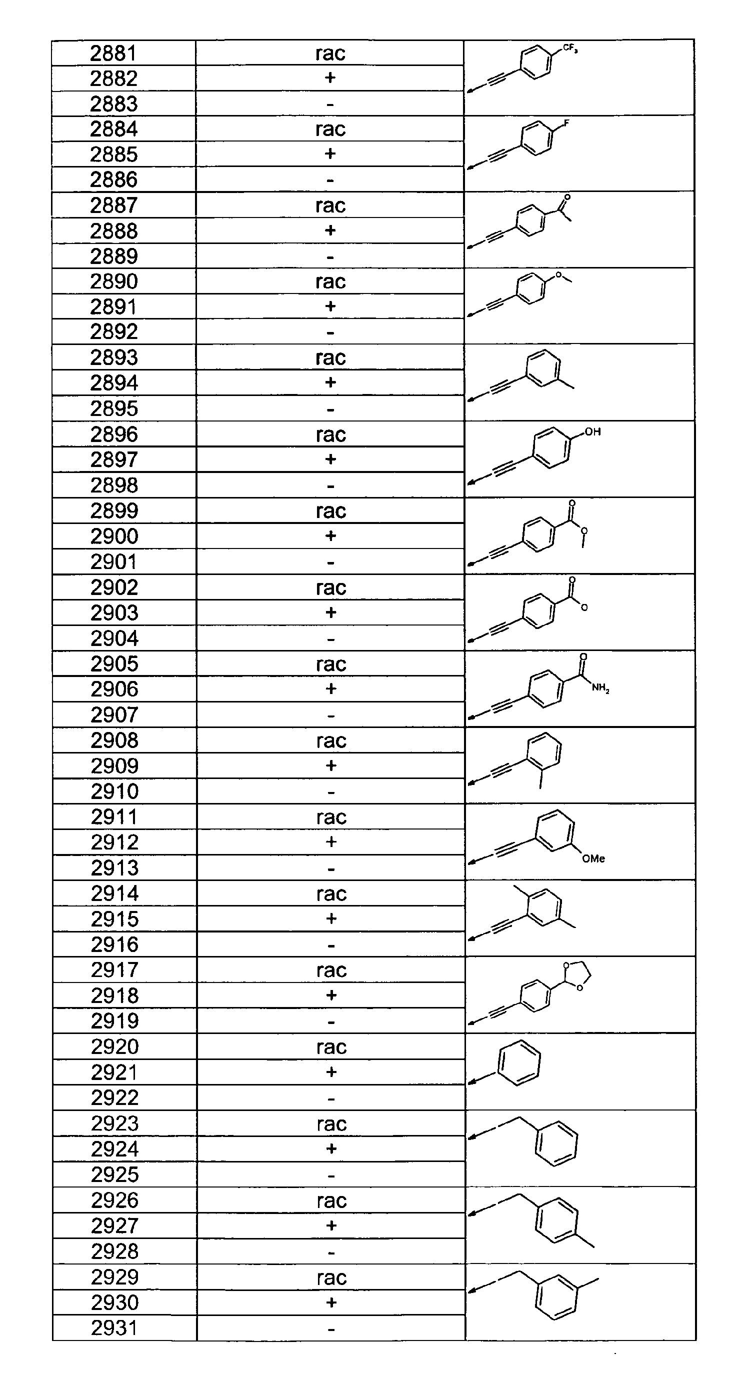

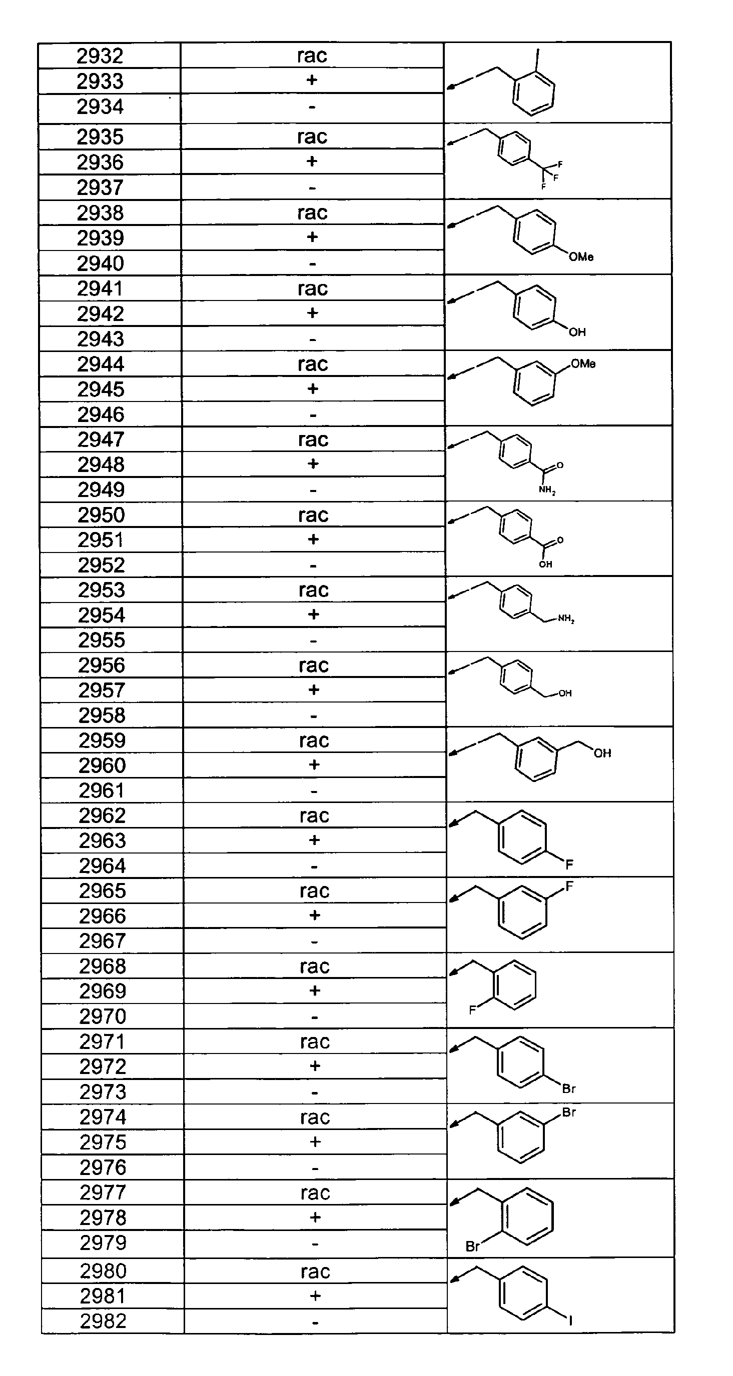

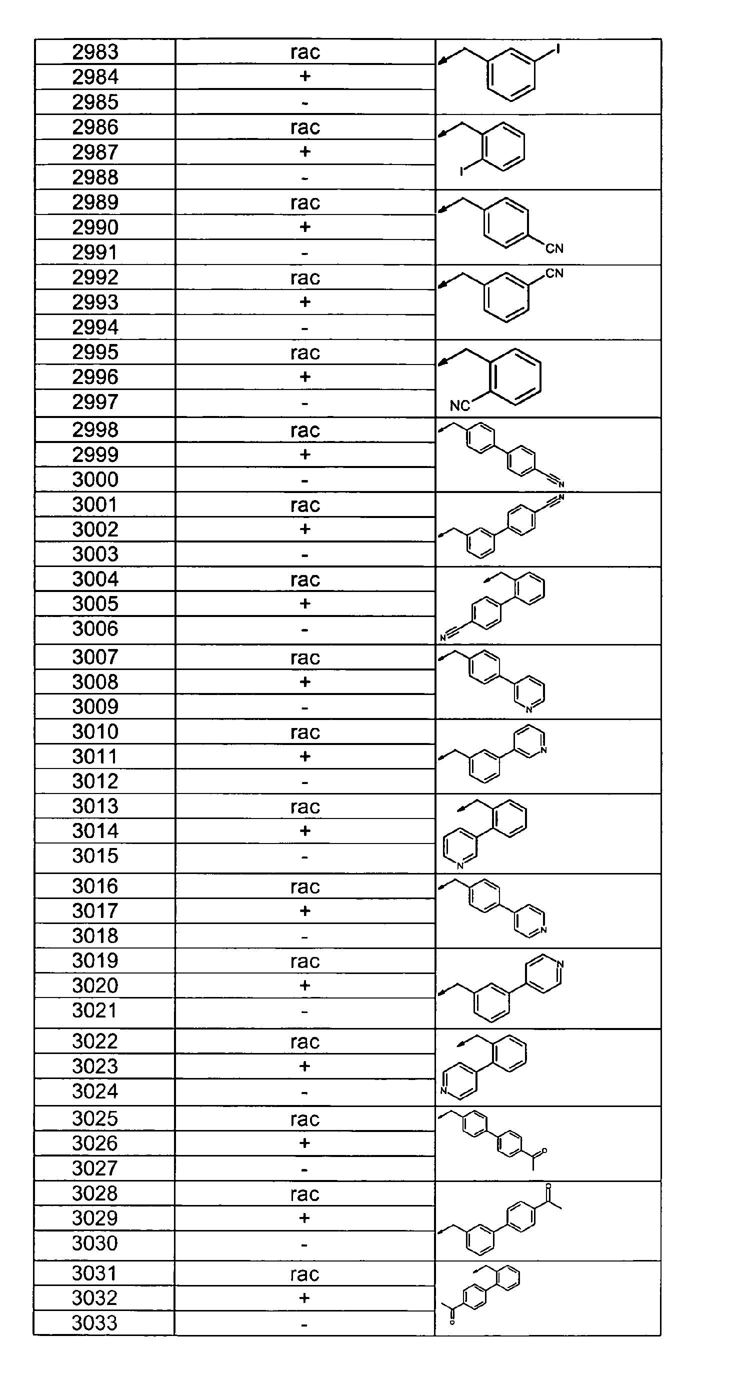

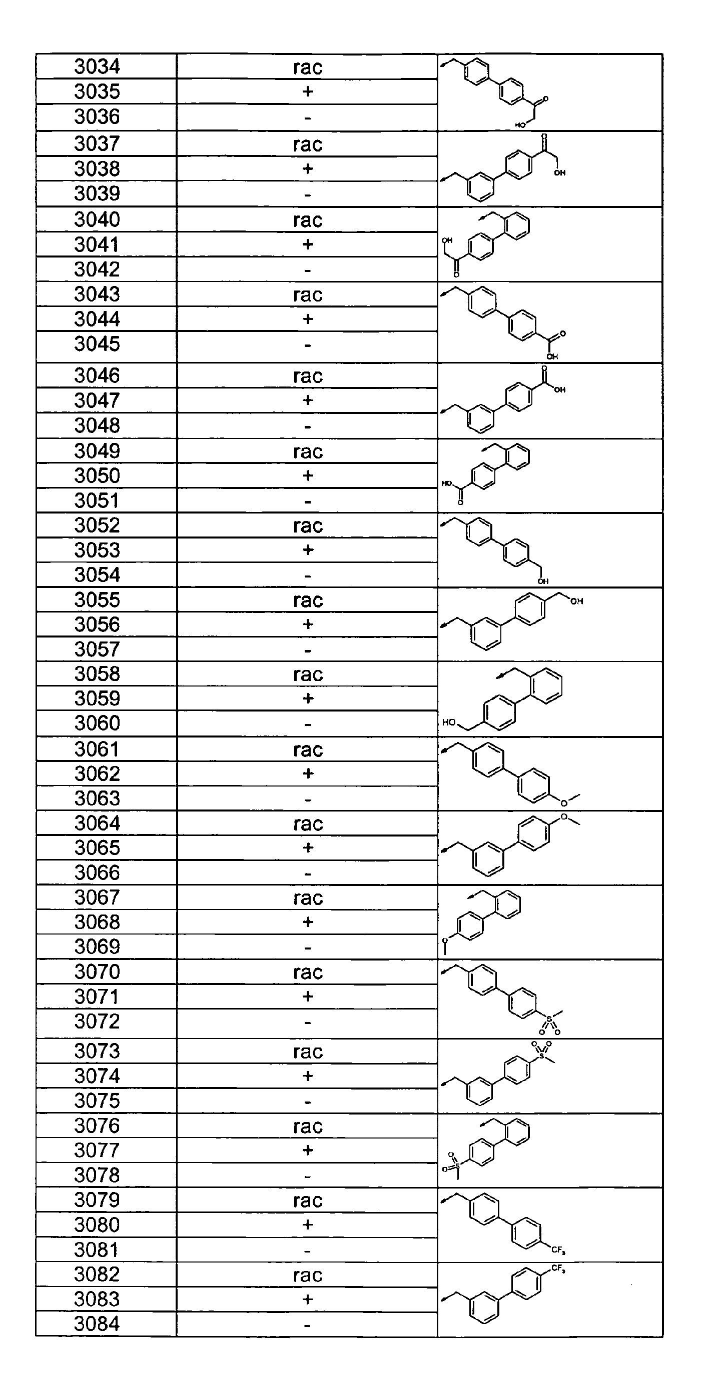

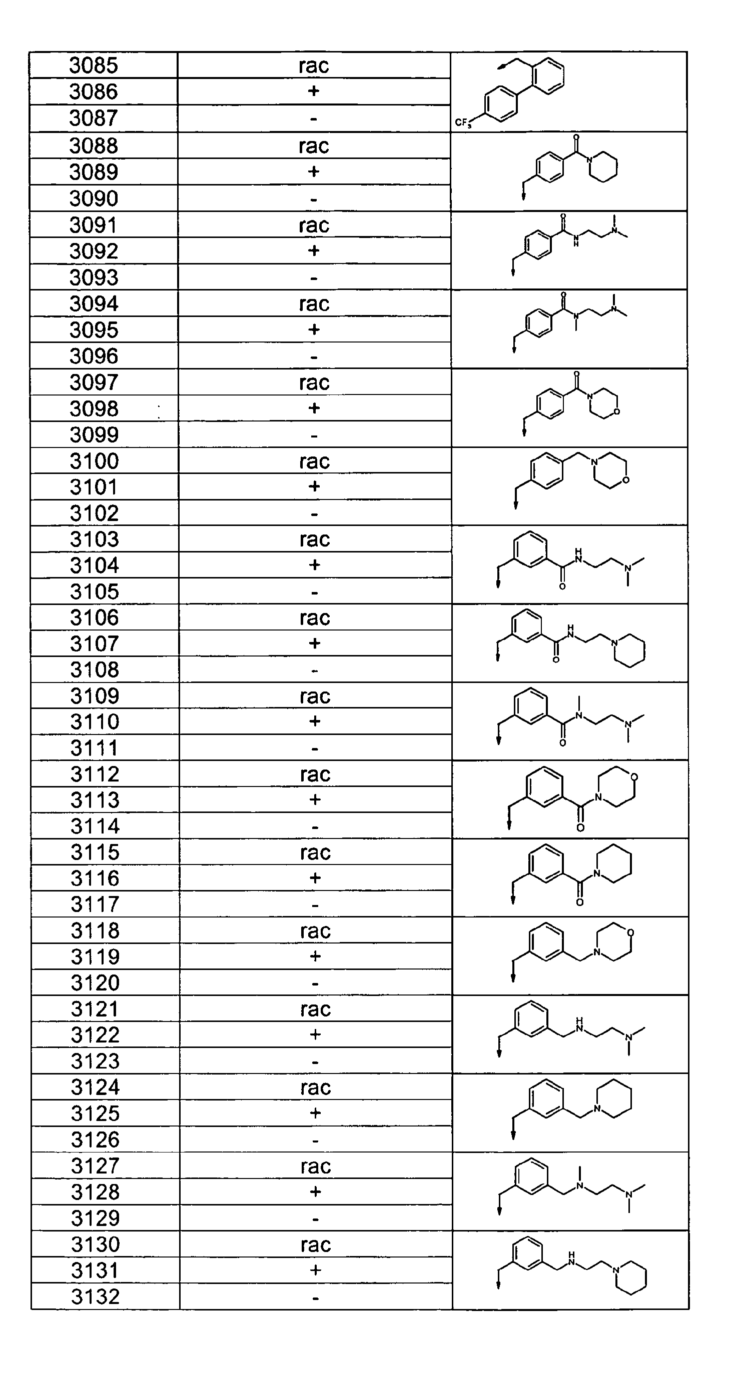

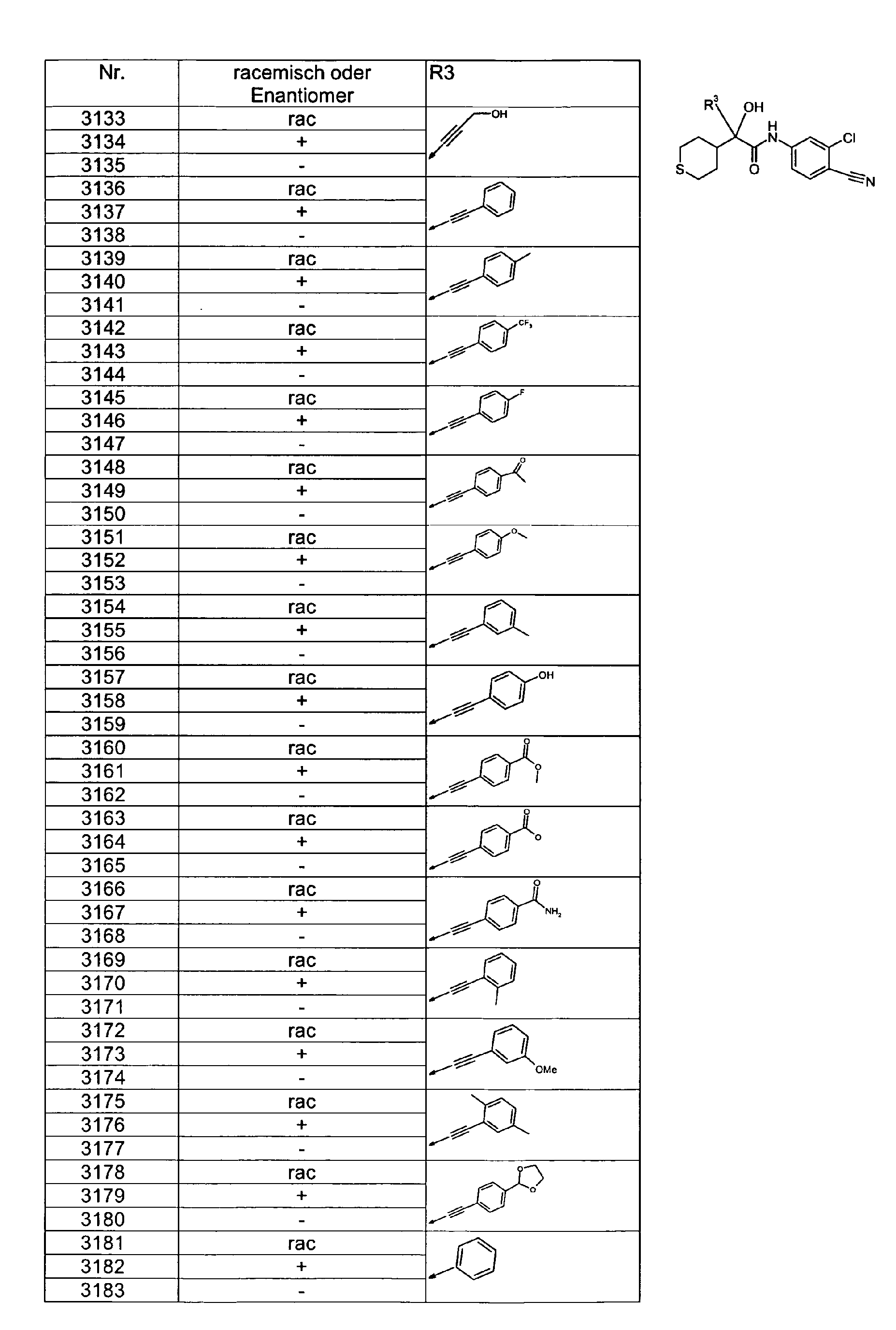

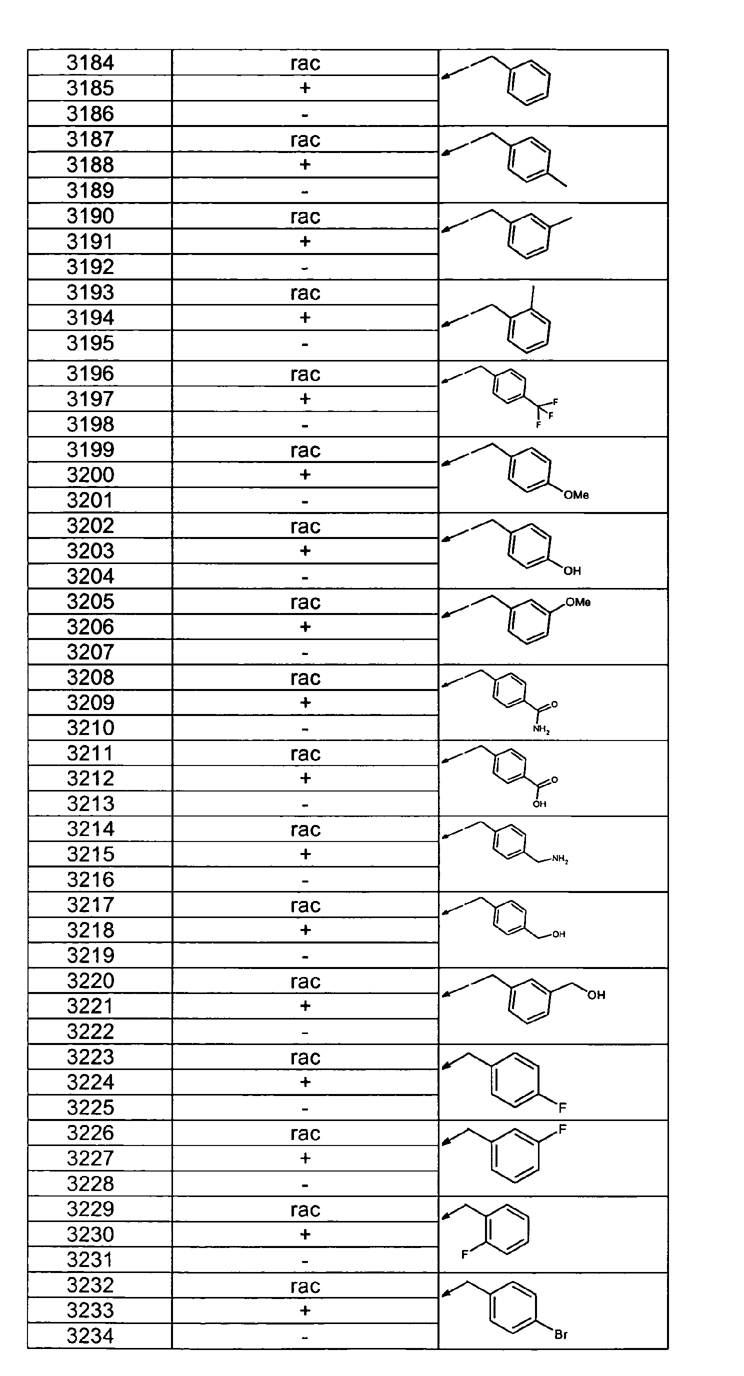

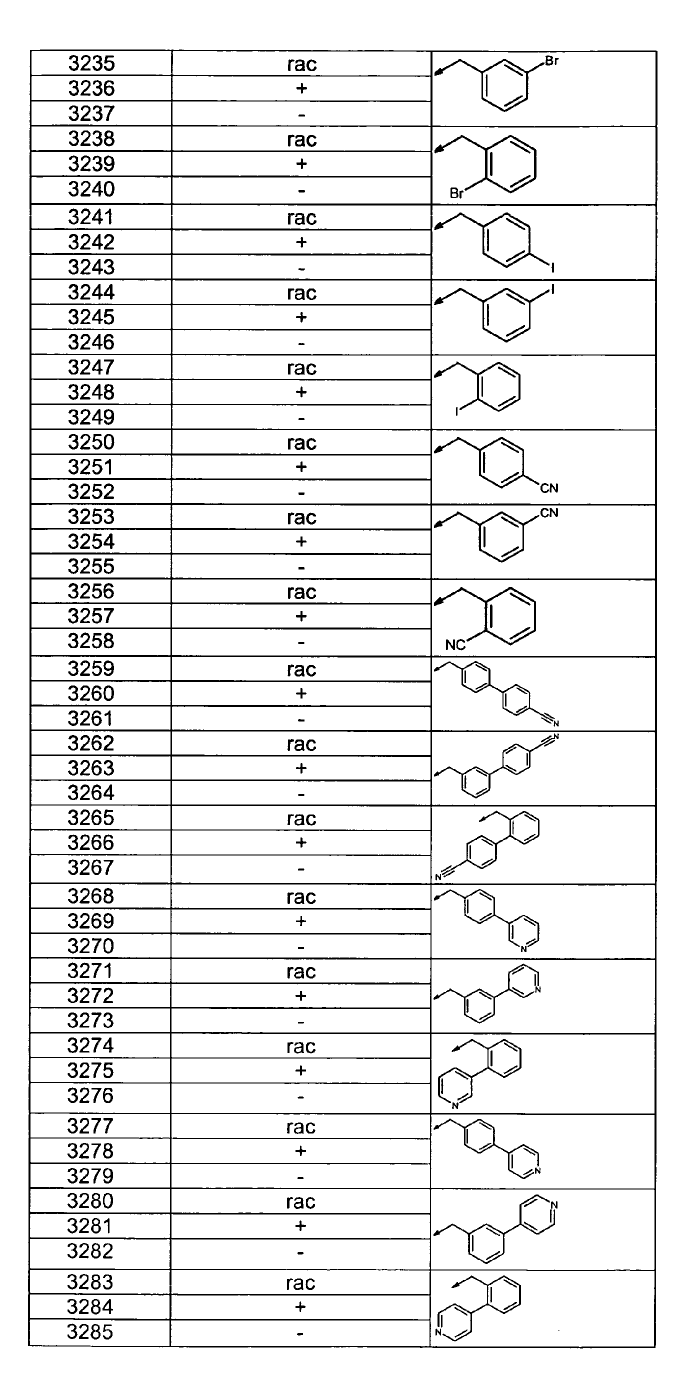

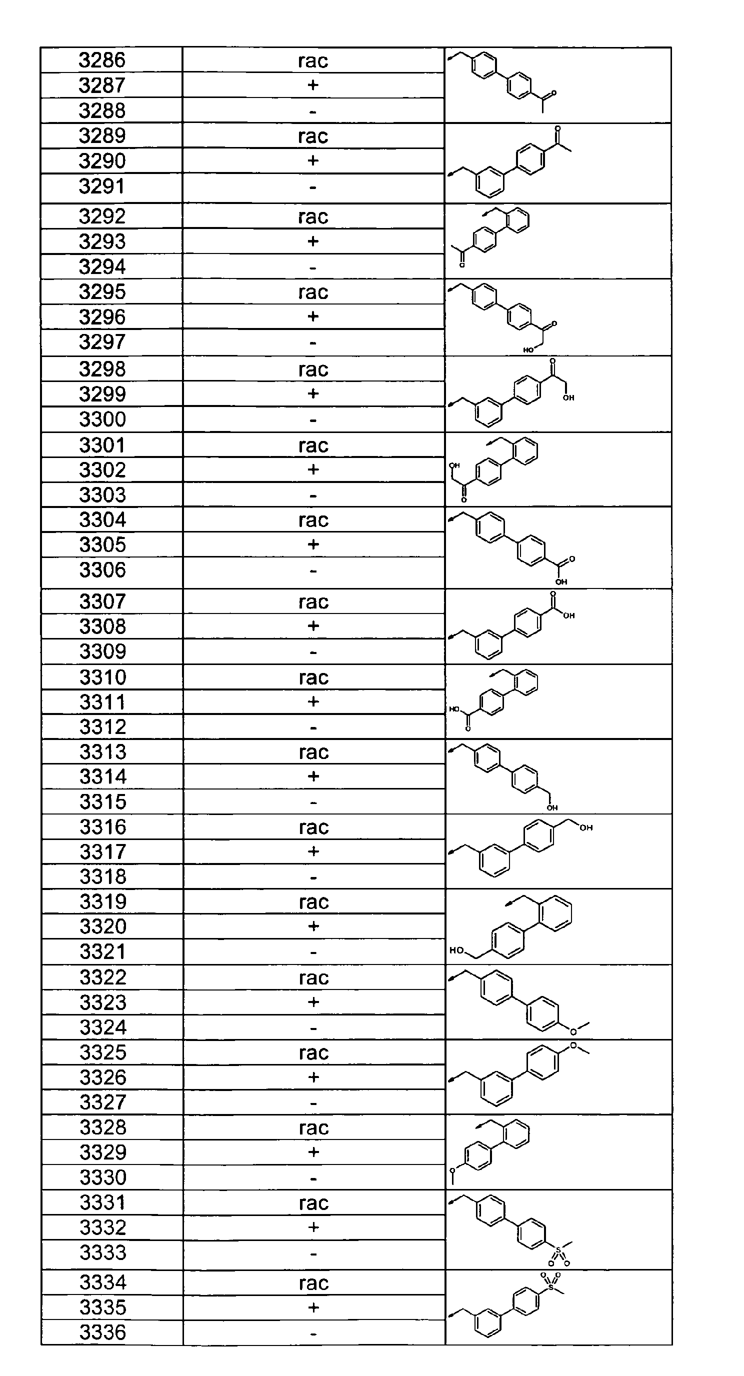

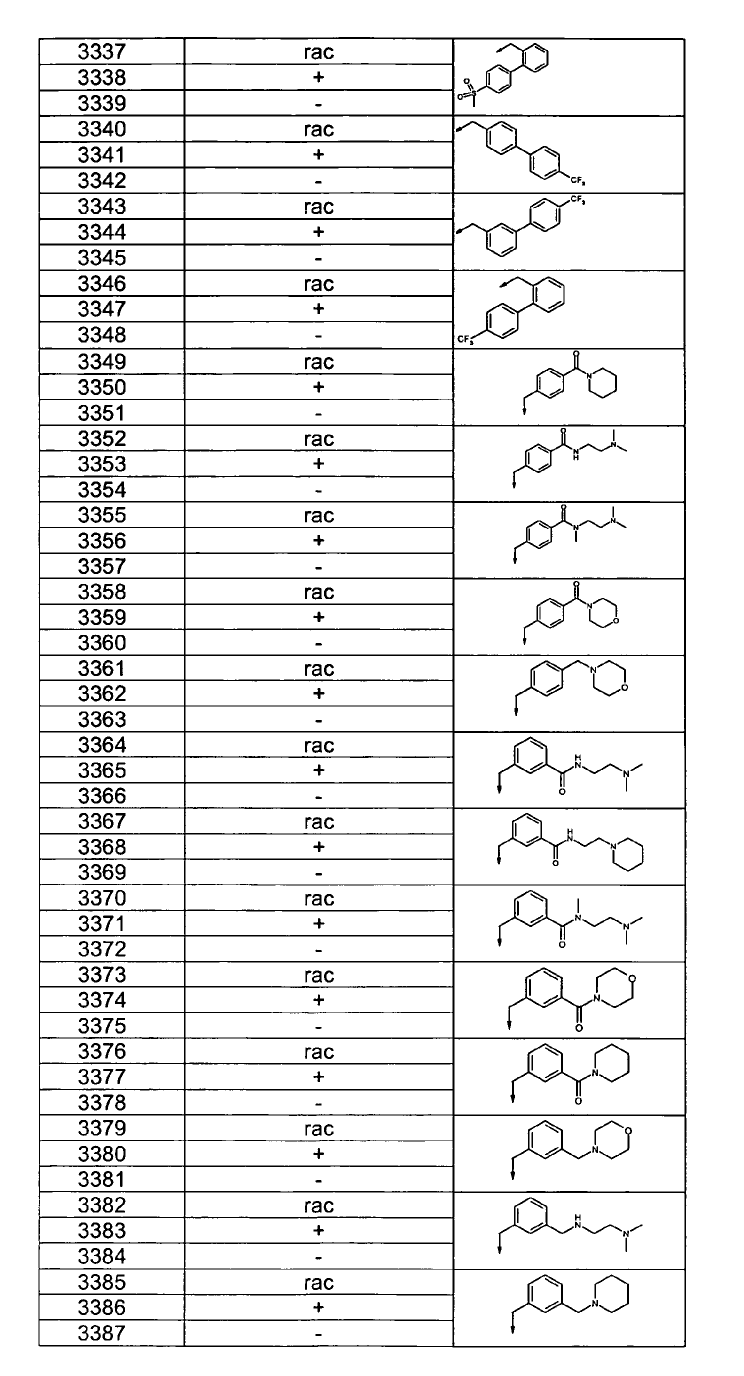

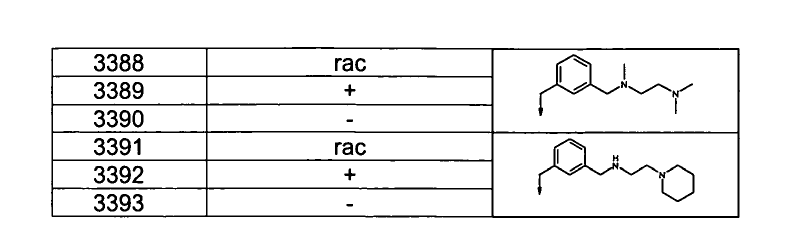

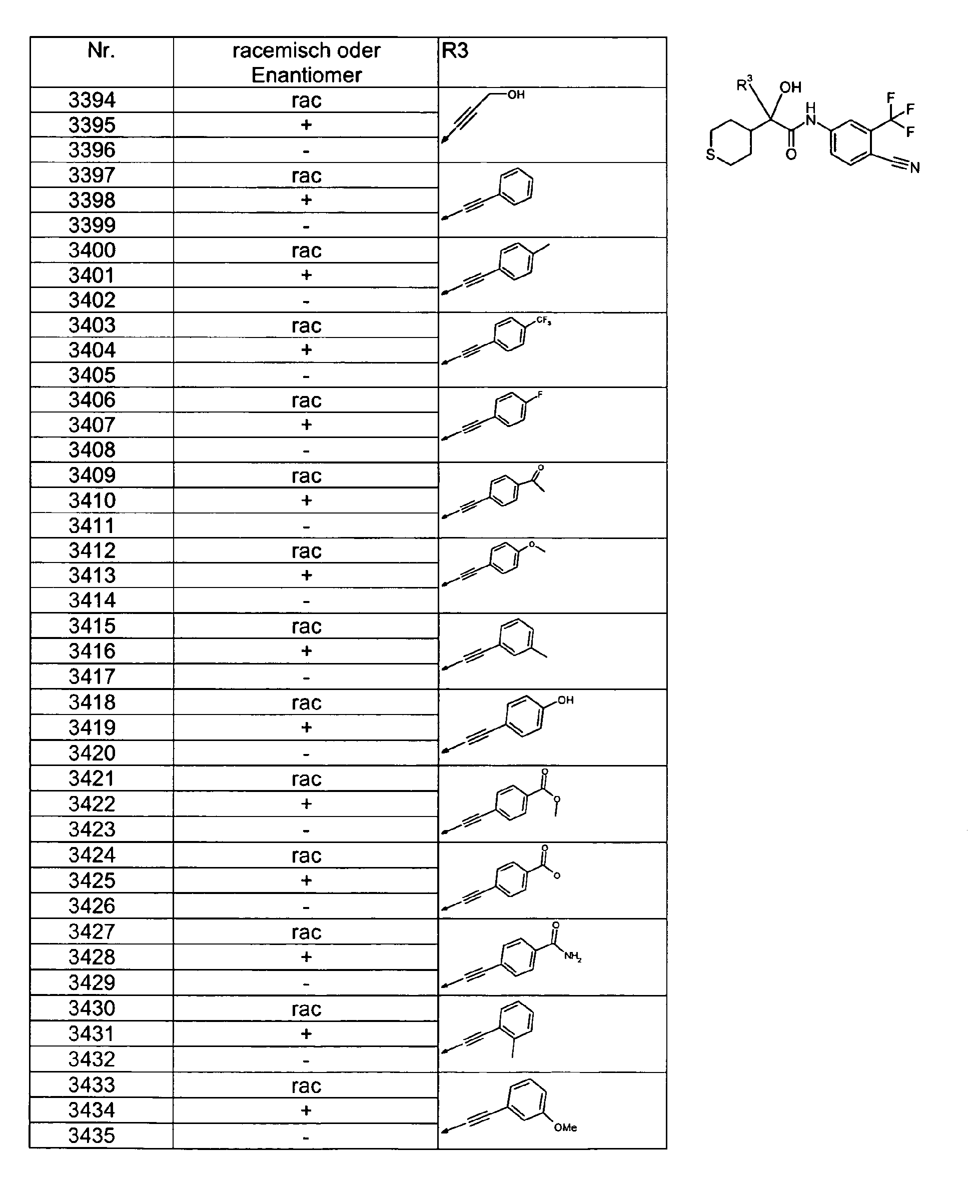

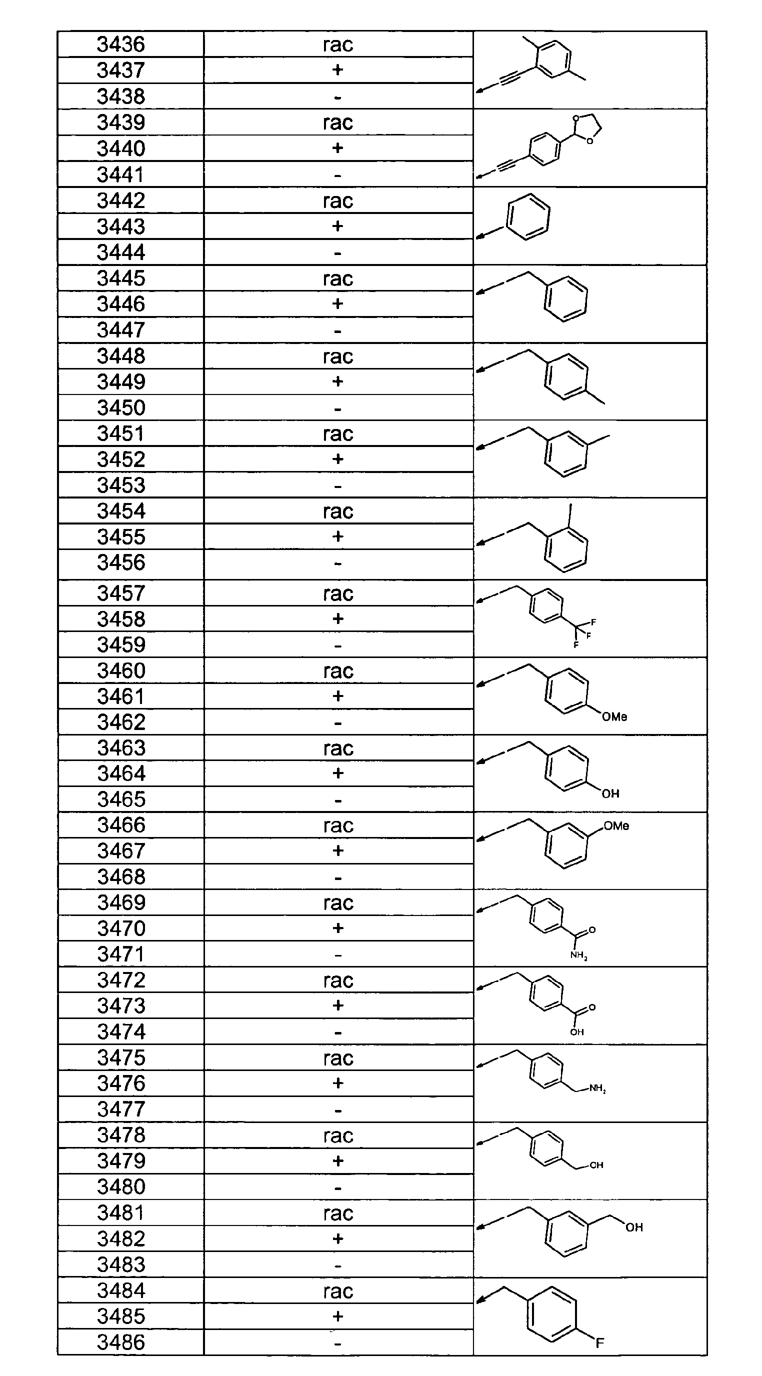

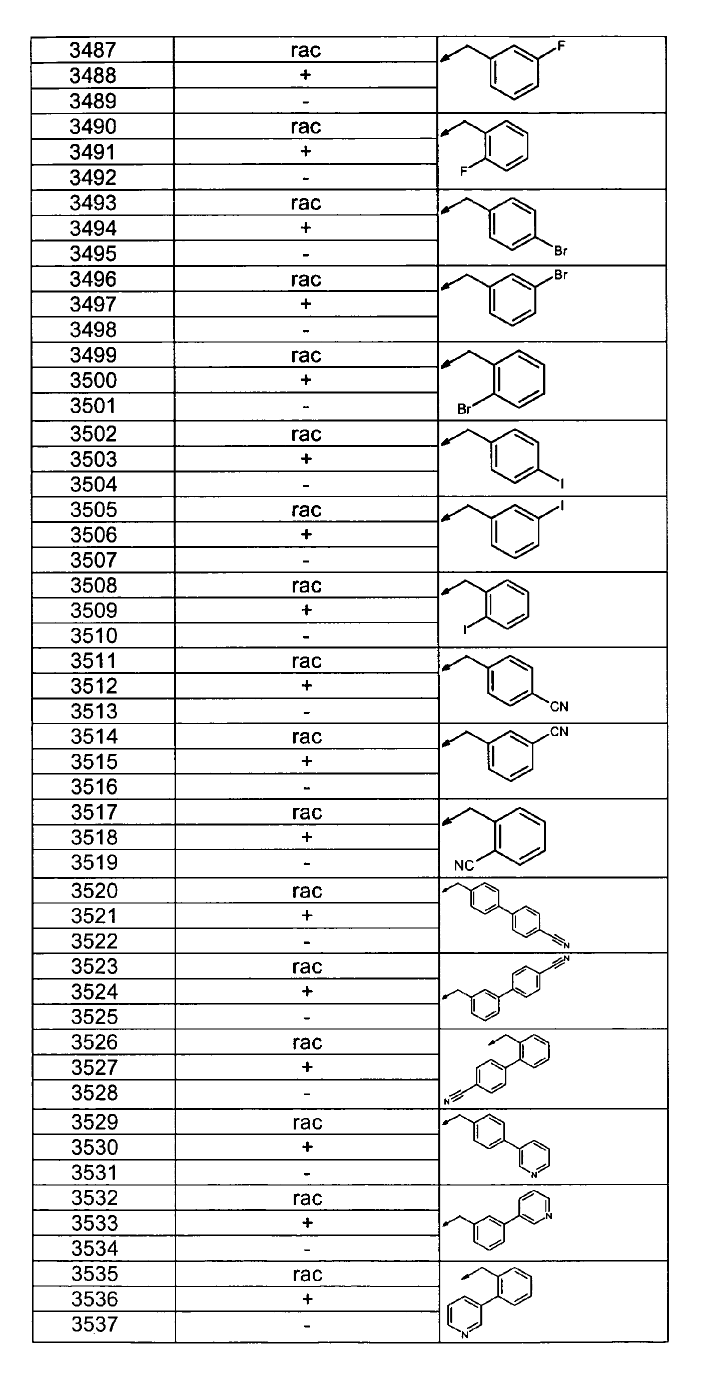

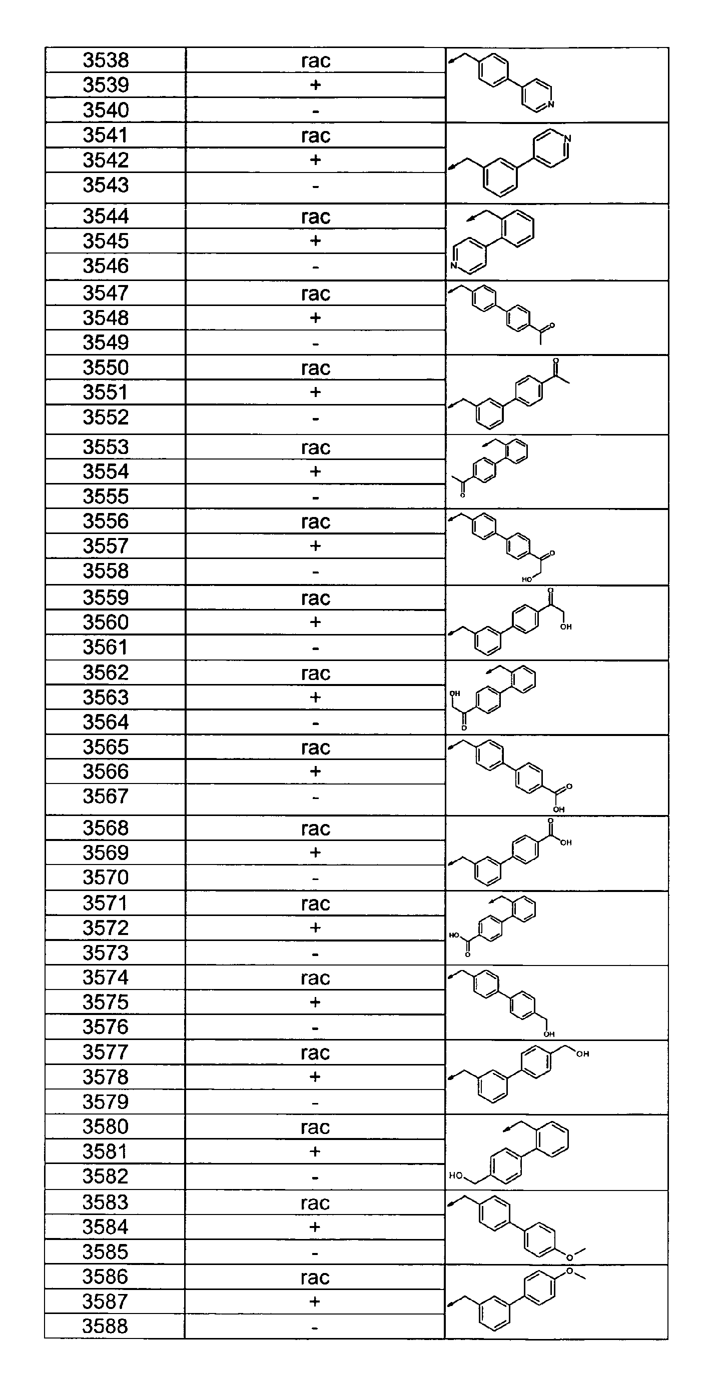

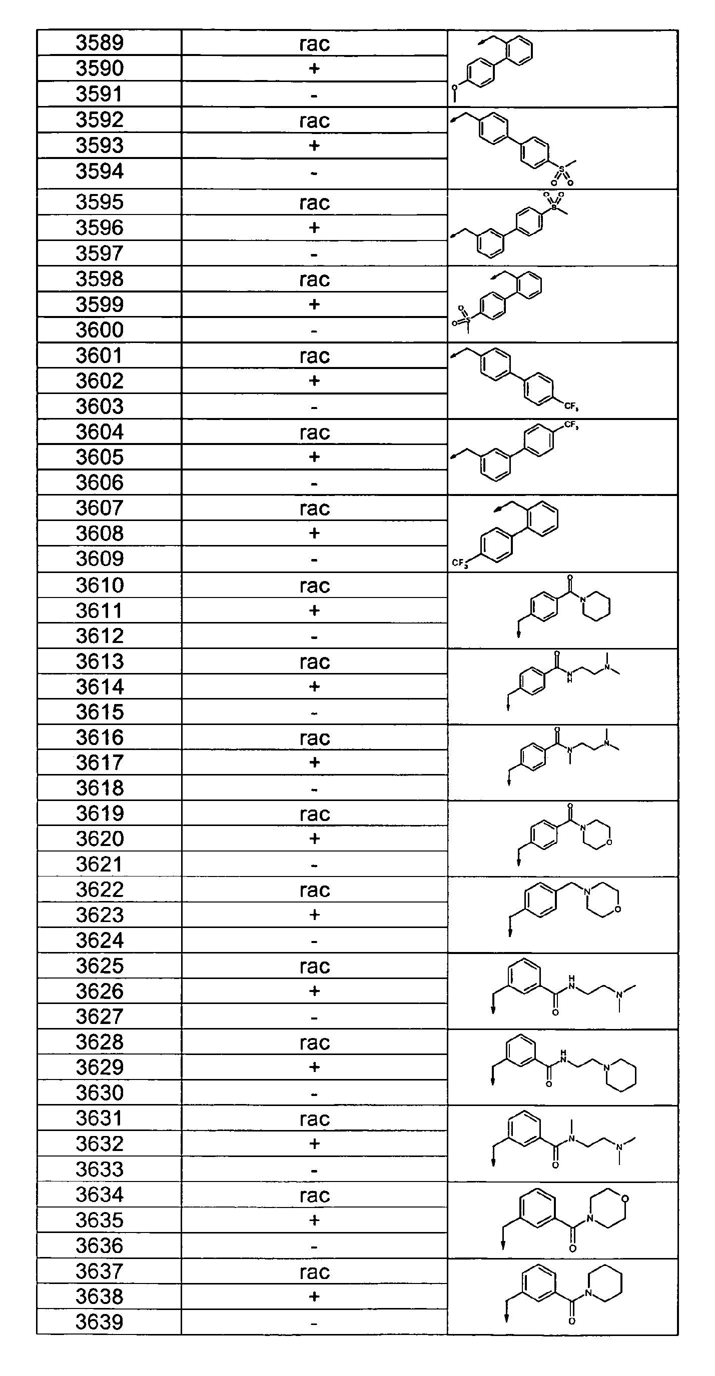

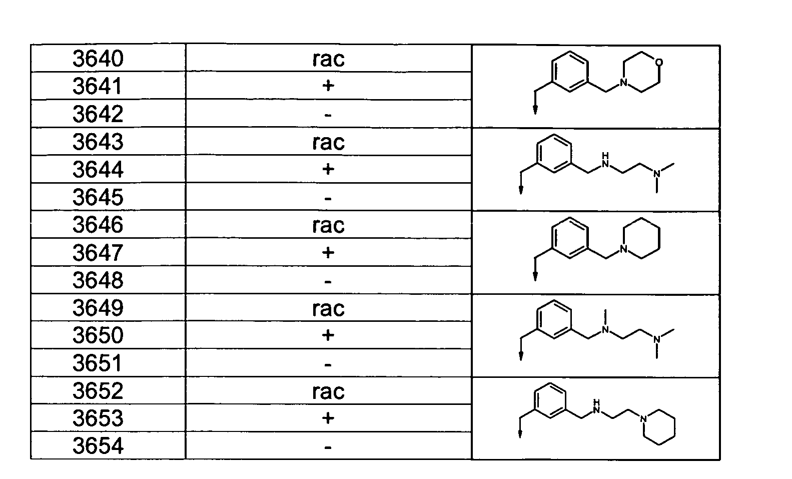

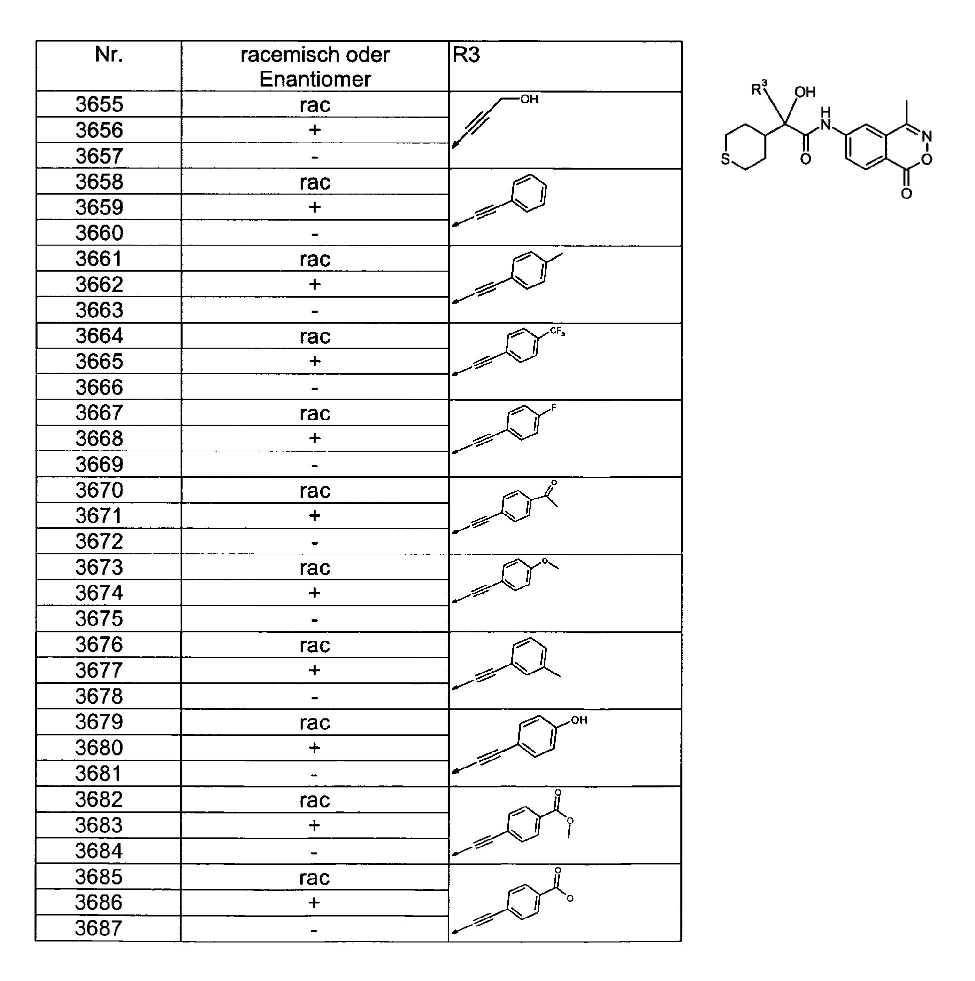

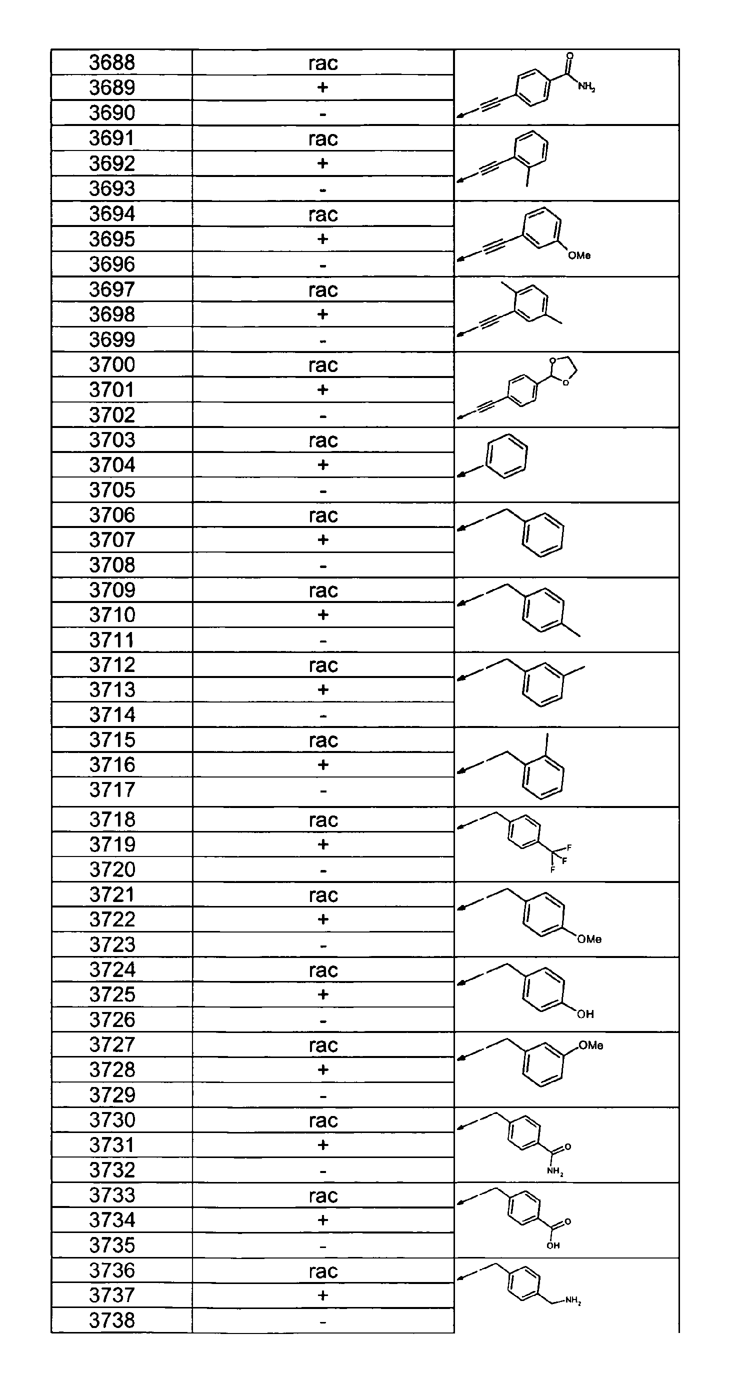

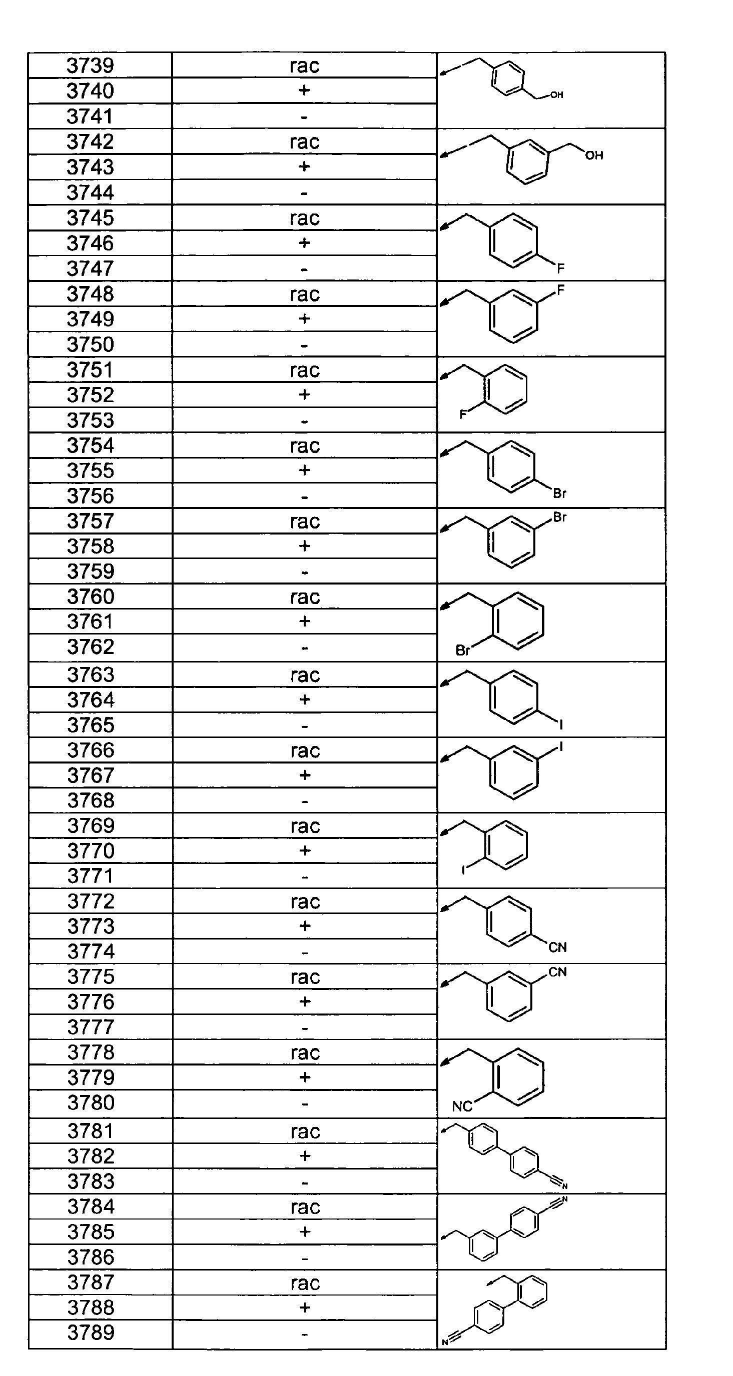

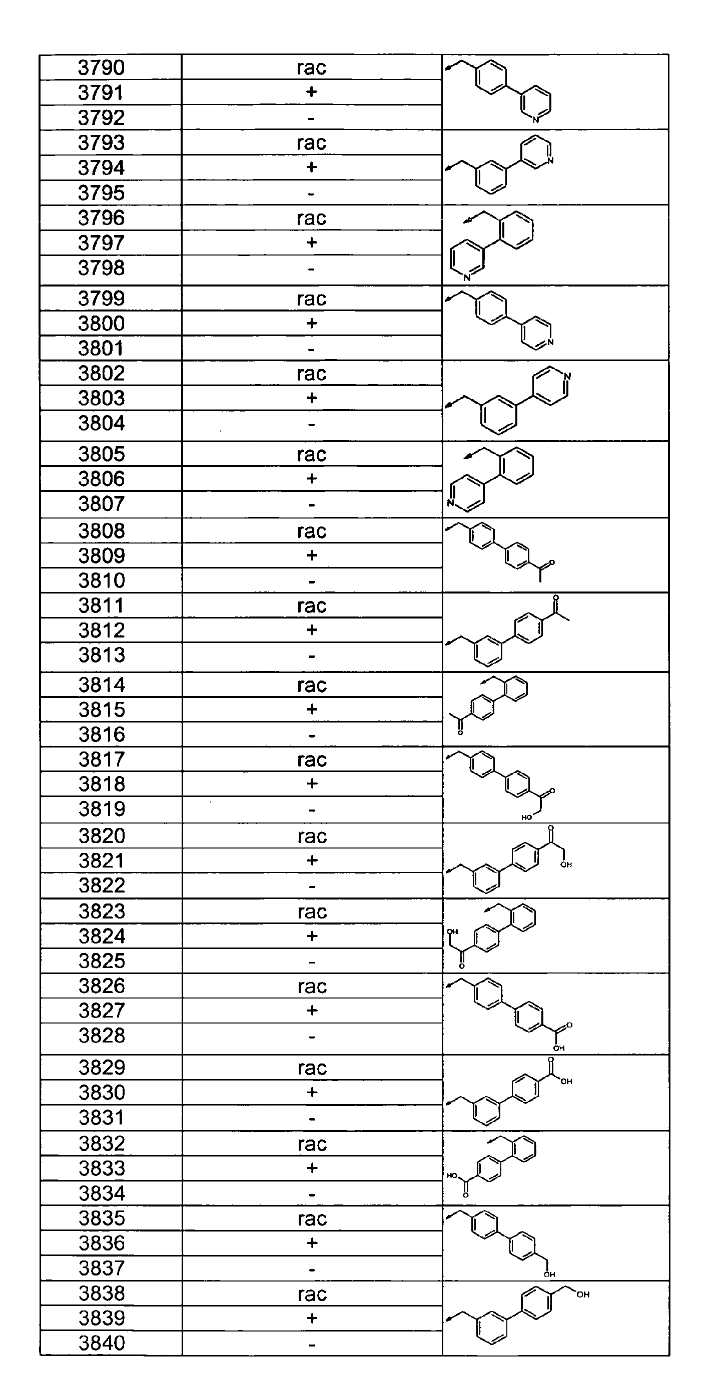

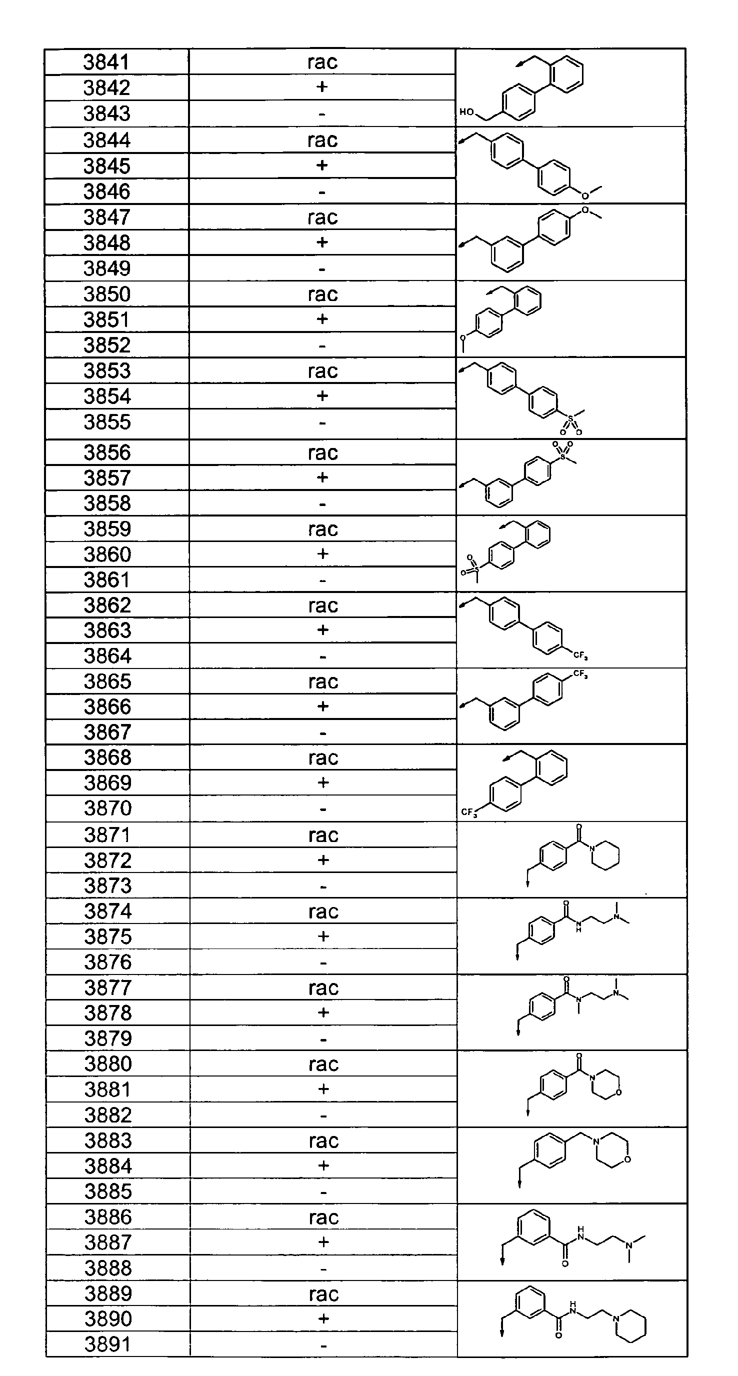

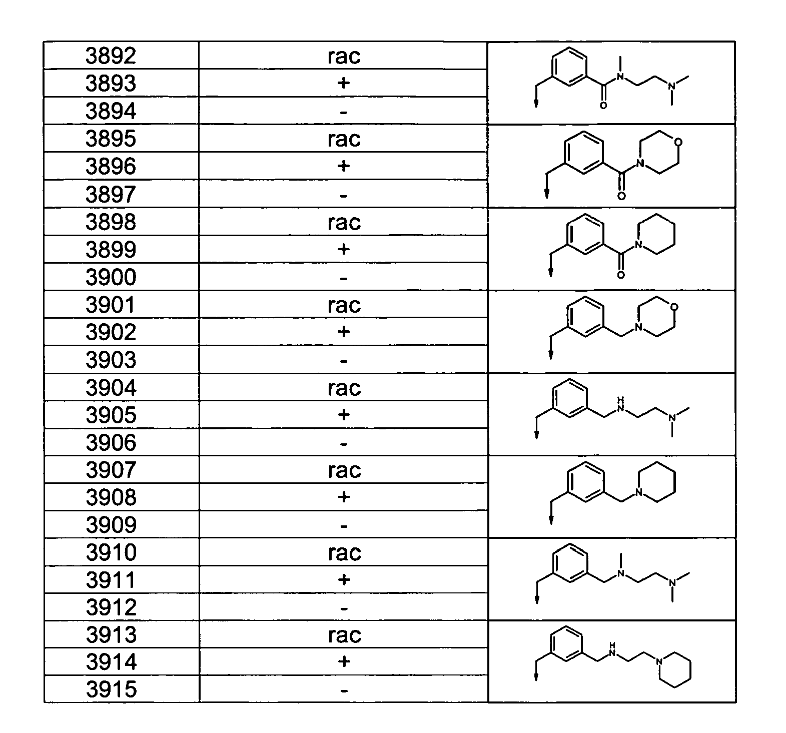

13. Pharmazeutische Zusammensetzung enthaltend mindestens eine Verbindung der allgemeinen Formel I nach einem der Ansprüche 1 bis 12 und gegebenenfalls mindestens einen weiteren Wirkstoff zusammen mit pharmazeutisch verträglichen Hilfs- und/ oder Trägerstoffen.

14. Pharmazeutische Zusammensetzung gemäß Anspruch 13, wobei der weitere Wirkstoff ein SERM (Selektiver-Estrogen-Rezeptor-Modulator), ein Aromatasehemmer, Antiestrogen oder ein Prostaglandin ist.

15. Pharmazeutische Zusammensetzung gemäß Anspruch 14, wobei die weiteren Wirkstoffe Tamoxifen, 5-(4-{5-[(RS)-(4,4,5,5,5-Pentafluorpentyl)sulfinyl]pentyloxy}-phenyl)-6-phenyl-8,9-dihydro-7H-benzocyclohepten-2-ol, ICI 182 780 (7alpha-[9-(4,4,5,5-Pentafluorpentylsulfinyl)nonyl]estra-1,3,5(10)-trien-3,17-beta-diol), 11beta-Fluor-7alpha-[5-(methyl{3-[(4,4,5,5,5-pentafluorpentyl)sulfanyl]propyl}amino)-pentyl]estra-1,3,5(10)-trien-3,17beta-diol, 11beta-Fluor-7alpha-{5-[methyl(7,7,8,8,-9,9,10,10,10-nonafluordecyl)amino]pentyl}estra-1,3,5(10)-trien-3,17beta-diol, 11beta-Fluor-17alpha-methyl-7alpha-{5-[methyl(8,8,9,9,9-pentafluornonyl)amino]pentyl}estra-1,3,5(10)-trien-3,17beta-diol, Clomifen, Raloxifen, Fadrozol, Formestan, Letrozol, Anastrozol oder Atamestan sein können.

16. Verbindungen nach einem der Ansprüche 1 bis 12 zur Herstellung eines Arzneimittels.

17. Verwendung von Verbindungen nach einem der Ansprüche 1 bis 12 zur Herstellung eines Arzneimittels zur Therapie und/ oder Prophylaxe von gynäkologischen Erkrankungen wie Endometriose, Leiomyomen des Uterus, dysfunktionelle Blutungen und Dysmenorrhoe.

18. Verwendung von Verbindungen nach einem der Ansprüche 1 bis 12 zur Herstellung eines Arzneimittels zur Therapie und/ oder Prophylaxe von hormonabhängigen Tumoren.

19. Verwendung von Verbindungen nach einem der Ansprüche 1 bis 12 zur Herstellung eines Arzneimittels zur Therapie und/ oder Prophylaxe von Mammakarzinomen.

20. Verwendung von Verbindungen nach einem der Ansprüche 1 bis 12 zur Herstellung eines Arzneimittels zur Therapie und/ oder Prophylaxe des Endometriumskarzinoms.

21. Verwendung von Verbindungen nach einem der Ansprüche 1 bis 12 zur Herstellung eines Arzneimittels zur Therapie und/ oder Prophylaxe von Ovarkarzinomen.

22. Verwendung von Verbindungen nach einem der Ansprüche 1 bis 12 zur Herstellung eines Arzneimittels zur Therapie und/ oder Prophylaxe von Prostatakarzinomen.

23. Verwendung von Verbindungen nach einem der Ansprüche 1 bis 12 zur Herstellung eines Arzneimittels für die weibliche Hormonersatztherapie.

24. Verwendung von Verbindungen nach einem der Ansprüche 1 bis 12 für die weibliche Fertilitätskontrolle.
